# EUROPEAN PATENT APPLICATION

(11) **EP 3 252 078 A1**
(43) Date of publication of application: **06.12.2017**
(21) Application number: 17174320.6
(22) Date of filing: 02.06.2017
(51) Int. Cl.: C07K 16/28, A61K 39/395, C07K 16/30

(54) **TYPE II ANTI-CD20 ANTIBODY AND ANTI-CD20/CD3 BISPECIFIC ANTIBODY FOR TREATMENT OF CANCER**

(30) Priority: 02.06.2016 EP 16172739; 06.12.2016 WO PCT/EP2016/079800
(71) Applicant: F. Hoffmann-La Roche AG, 4070 Basel (CH)
(72) Inventor: Bacac, Marina, 8952 Schlieren (CH); Klein, Christian, 8952 Schlieren (CH); Umaña, Pablo, 8952 Schlieren (CH); Colombetti, Sara, 8952 Schlieren (CH)
(74) Representative: Winkler, Thomas

(57) **Abstract**

The present invention relates to combination treatment methods of treating a disease comprising a type II anti-CD20 antibody and an anti-CD20/anti-CD3 bispecific antibody. The present invention further relates to methods for reduction of cytokine release associated with the administration of a T-cell activating therapeutic agent.

## Description

### Field of the Invention

The present invention relates to methods of treating a disease, particularly a B-cell proliferative disorder, and methods for reduction of adverse effects in response to the administration of a T-cell activating therapeutic agent. The present invention further relates to combination treatment methods of treating a disease and antibodies for the use in such methods.

### Background

B-cell proliferative disorders describe a heterogeneous group of malignancies that includes both leukemias and lymphomas. Lymphomas develop from lymphatic cells and include two main categories: Hodgkin lymphomas (HL) and the non-Hodgkin lymphomas (NHL). In the United States, lymphomas of B cell origin constitute approximately 80-85% of all non-Hodgkin lymphoma cases, and there is considerable heterogeneity within the B-cell subset, based upon genotypic and phenotypic expression patterns in the B-cell of origin. For example, B cell lymphoma subsets include the slow-growing indolent and incurable diseases, such as Follicular lymphoma (FL) or chronic lymphocytic leukemia (CLL), as well as the more aggressive subtypes, mantle cell lymphoma (MCL) and diffuse large B cell lymphoma (DLBCL).

Despite the availability of various agents for the treatment of B-cell proliferative disorders, there is an ongoing need for development of safe and effective therapies to prolong remission and improve cure rates in patients.

A strategy currently being investigated is the engagement of T cells against malignant B cells. In order to effectively engage T cells against malignant B cells, two recent approaches have been developed. These two approaches are: 1) the administration of T cells engineered ex vivo to recognize tumour cells (also known as chimeric antigen receptor-modified T cell therapy [CAR-T cells]) (Maude et al., N Engl J Med (2014) 371,1507-1517); and, 2) the administration of agents that activate endogenous T cells, such as bispecific antibodies (Oak and Bartlett, Expert Opin Investig Drugs (2015) 24, 715-724).

An example of the first approach is reported in the study by Maude et al., in which 30 adult and pediatric patients were treated with autologous T cells transduced with a CD19-directed chimeric antigen receptor lentiviral vector (CTL019 CAR-T cells). The result was a sustained remission based upon a 6-month event-free survival rate of 67% and an overall survival rate of 78%. However, all patients had cytokine release syndrome (CRS) (associated with tumour burden), with 27% of patients having severe CRS. Central nervous system toxicities of unknown cause were also noted at high frequencies.

In contrast, the second approach, which involves activating endogenous T cells to recognize tumour targets, bypasses this hurdle of scalability, and can also provide competitive efficacy, safety data and potentially long term durations of response. In different CD20⁺ hematologic malignancies, this approach is best exemplified by blinatumomab, a CD 19 CD3 targeting T cell bispecific molecule (Bargou et al., Science (2008) 321, 974-977) that was recently approved for patients with minimal residual disease-positive acute lymphocytic leukemia (ALL). This compound, which is composed of two single chain Fv fragments (the so called BiTE® format), directs the lysis of CD19⁺ cells by cytolytic T cells. The primary constraint of blinatumomab is its short half-life (approximately 2 hours), which necessitates continuous infusion via a pump over 4-8 weeks. Nonetheless, it has potent efficacy in patients with both relapsed/refractory Non-Hodgkin Lymphoma (r/r NHL) and ALL, with step-up dosing (SUD) required to mitigate severe cytokine release syndrome and CNS toxicities (Nagorsen and Baeuerle, Exp Cell Res (2011) 317, 1255-1260).

The CD20 CD3 targeting T cell bispecific molecule, CD20XCD3 bsAB, is another example of a next generation of B cell targeting antibody. CD20XCD3 bsAB is a T cell bispecific (TCB) antibody targeting CD20 expressed on B cells and CD3 epsilon chain (CD3e) present on T cells.

The mechanism of action of CD20XCD3 bsAB comprises simultaneous binding to CD20⁺ B cells and CD3⁺ T cells, leading to T-cell activation and T-cell mediated killing of B cells. In the presence of CD20⁺ B cells, whether circulating or tissue resident, pharmacologically active doses will trigger T-cell activation and associated cytokine release. CD20XCD3 bsAB has shown enhanced potency in nonclinical models over competitive T cell engaging agents and, having an IgG-based format, has a greatly improved half-life over blinatumomab.

Cytokine release is the result of activation of T cells. In a phase 1 study conducted by TeGenero (Suntharalingam et al., N Engl J Med (2006) 355,1018-1028), all 6 healthy volunteers experienced near fatal, severe cytokine release syndrome (CRS) rapidly post-infusion of an inappropriately-dosed, T-cell stimulating super-agonist anti-CD28 monoclonal antibody. More recently, in the above-mentioned study by Maude et al. of CD19-targeting, chimeric antigen receptor T cell (CAR-T cell) treatment of patients with relapsed ALL, all 30 patients had cytokine release, which was categorized as severe in 27% of the patients. CRS is a common but severe complication of CAR-T cell therapy (reviewed in Xu and Tang, Cancer Letters (2014) 343, 172-178).

Severe CRS and CNS toxicity have also been frequently observed with the CD19-CD3 T cell bispecific agent, blinatumomab (Klinger et al., Blood. 2012;119(26):6226-6233). In patients receiving blinatumomab in all clinical trials, neurological toxicities have occurred in approximately 50% of patients, and the types of toxicities observed are well-defined in the package insert.

It is not well understood if or how CNS toxicity is related to earlier cytokine release or T cell activation. Similar to blinatumomab, CNS AEs (ranging from delirium to global encephalopathy) were reported for 43% (13/30) of the patients with r/r ALL treated with CD19-targeting CAR-T cells (Maude et al., N Engl J Med (2014) 371,1507-1517; Ghorashian et al., Br J Haematol (2015) 169, 463-478). Neurologic toxic effects typically occurred after symptoms of CRS had peaked and started to resolve; however no direct, unequivocal association with severe CRS was found. The authors proposed that the mechanism of neurotoxicity could involve direct CAR-T-cell-mediated toxicity or it could be cytokine-mediated. In contrast, an association between severe CRS and neurotoxicity (e.g., encephalopathy) has been suggested in another study of CD19-targeting CAR-T cell therapy (Davila et al., Sci Transl Med (2014) 6, 224ra25) and speculated to be due to general T cell activation, versus direct CAR-T-induced damage.

Cytokine release and/or CNS-related toxicities are particularly pronounced in T cell bispecific antibodies that link CD3⁺ cells to B cells, as compared to other T cell bispecific antibodies that link CD3⁺ cells to tissue-restricted (i.e., non-circulating) target cells.

There is thus a need for methods to reduce or prevent such adverse effects of these promising agents which have the potential to significantly contribute to the treatment of patients with B-cell proliferative disorders such as NHL and CLL.

### Summary of the Invention

The present invention is based on the surprising finding that the cytokine release associated with administration of a T-cell activating therapeutic agent, such as CD20XCD3 bsAB, to a subject can be significantly reduced by pre-treatment of said subject with a Type II anti-CD20 antibody, such as obinutuzumab.

Obinutuzumab is a humanized glyco-engineered type II anti-CD20 mAb that binds with high-affinity to the CD20 antigen, inducing antibody-dependent cellular cytotoxicity (ADCC) and antibody-dependent cellular phagocytosis (ADCP), low complement-dependent cytotoxicity (CDC) activity, and high direct cell death induction.

Without wishing to be bound by theory, the use of GAZYVA® pre-treatment (Gpt) should aid in the rapid depletion of B cells, both in the peripheral blood and in secondary lymphoid organs, such that the risk of highly relevant adverse events (AEs) from strong systemic T cell activation by T-cell activating therapeutic agents (e.g. CRS) is reduced, while supporting exposure levels of T-cell activating therapeutic agents that are high enough from the start of dosing to mediate tumour cell elimination. To date, the safety profile of obinutuzumab (including cytokine release) has been assessed and managed in hundreds of patients in ongoing obinutuzumab clinical trials. Finally, in addition to supporting the safety profile of T-cell activating therapeutic agents such as CD20XCD3 bsAB, Gpt should also help prevent the formation of anti-drug antibodies (ADAs) to these unique molecules.

For patients, Gpt should translate into better drug exposure with an enhanced safety profile.

Gpt should be more effective in accomplishing the above goals compared to other methods used with T cell bispecific agents, such as step up dosing (SUD). A single dose of obinutuzumab should allow relapsed/refractory patients to receive the full therapeutic dose of T-cell activating therapeutic agent such as CD20XCD3 bsAB, once determined, without a time delay from step up dosing. For example, it was recently reported that the blinatumomab dosing regimen for patients with r/r DLBCL in an ongoing Phase 2 trial incorporates a double step up approach (i.e., 9 →28→112 µg/m²/day), thus, requiring 14 days to reach the maximum dose of 112 µg/m²/day (Viardot el at., Hematol Oncol (2015) 33, 242(Abstract 285)).

As shown in the Examples, following pretreatment with obinutuzumab, administration of CD20XCD3 bsAB to cynomolgus monkeys was tolerated up to a level that was ten times higher than that tolerated without Gpt.

Efficient peripheral blood B-cell depletion and anti-tumour activity along with strongly reduced cytokine release in the peripheral blood associated with the first CD20XCD3 bsAB injection was observed upon Gpt.

Accordingly, in a first aspect the present invention provides a method for reducing cytokine release associated with administration of a T-cell activating therapeutic agent in a subject, comprising administration of a Type II anti-CD20 antibody to the subject prior to administration of the therapeutic agent. In one embodiment the period of time between the administration of the Type II anti-CD20 antibody and administration of the therapeutic agent is sufficient for reduction of the number of B-cells in the subject in response to the administration of the Type II anti-CD20 antibody.

In a further aspect, the invention provides a method of treating a disease in a subject, the method comprising a treatment regimen comprising
(i) administration to the subject of a Type II anti-CD20 antibody,
   and consecutively after a period of time
(ii) administration to the subject of a T-cell activating therapeutic agent,
   wherein the period of time between the administration of the Type II anti-CD20 antibody and the administration of the therapeutic agent is sufficient for reduction of the number of B-cells in the subject in response to the administration of the Type II anti-CD20 antibody.

In one embodiment, the treatment regimen effectively reduces cytokine release associated with the administration of the therapeutic agent in the subject as compared to a corresponding treatment regimen without the administration of the Type II anti-CD20 antibody.

In a further aspect, the invention provides a Type II anti-CD20 antibody for use in a method for reducing cytokine release associated with the administration a T-cell activating therapeutic agent in a subject, comprising administration of the Type II anti-CD20 antibody to the subject prior to administration of the therapeutic agent.

In one embodiment, the period of time between the administration of the Type II anti-CD20 antibody and administration of the therapeutic agent is sufficient for reduction of the number of B-cells in the subject in response to the administration of the CD20 antibody.

In a further aspect, the invention provides a Type II anti-CD20 antibody for use in a method of treating a disease in a subject, the method comprising a treatment regimen comprising
(i) administration to the subject of the Type II anti-CD20 antibody,
   and consecutively after a period of time
(ii) administration to the subject of a T-cell activating therapeutic agent,
   wherein the period of time between the administration of the Type II anti-CD20 antibody and the administration of the therapeutic agent is sufficient for reduction of the number of B-cells in the subject in response to the administration of the Type II anti-CD20 antibody.

In one embodiment, the treatment regimen effectively reduces cytokine release associated with the administration of the therapeutic agent in the subject as compared to a corresponding treatment regimen without the administration of the Type II anti-CD20 antibody.

In a further aspect, the invention provides the use of a Type II anti-CD20 antibody in the manufacture of a medicament for the reduction of cytokine release associated with administration of a T-cell activating therapeutic agent in a subject,
wherein the medicament is to be used in a treatment regimen comprising
(i) administration to the subject of the Type II anti-CD20 antibody,
   and consecutively after a period of time
(ii) administration to the subject of a T-cell activating therapeutic agent,
   wherein the period of time between the administration of the Type II anti-CD20 antibody and the administration of the therapeutic agent is sufficient for reduction of the number of B-cells in the subject in response to the administration of the Type II anti-CD20 antibody.

In one embodiment, the treatment regimen effectively reduces cytokine release associated with administration of the T-cell activating therapeutic agent in the subject as compared to a corresponding treatment regimen without the administration of the Type II anti-CD20 antibody.

In still a further aspect, the invention provides a kit for the reduction of cytokine release associated with administration of a T-cell activating therapeutic agent in a subject, comprising a package comprising a Type II anti-CD20 antibody composition and instructions for using the Type II anti-CD20 antibody composition in a treatment regimen comprising
(i) administration to the subject of the Type II anti-CD20 antibody composition,
   and consecutively after a period of time
(ii) administration to the subject of a T-cell activating therapeutic agent,
   wherein the period of time between the administration of the Type II anti-CD20 antibody composition and the administration of the therapeutic agent is sufficient for reduction of the number of B-cells in the subject in response to the administration of the CD20 antibody.

In one embodiment, the treatment regimen effectively reduces cytokine release associated with administration of the T-cell activating therapeutic agent in the subject as compared to a corresponding treatment regimen without the administration of the Type II anti-CD20 antibody composition. In one embodiment, the kit further comprises a therapeutic agent composition.

The invention in a further aspect as provides a T-cell activating therapeutic agent for use in a method of treating a disease in a subject, the method comprising a treatment regimen comprising
(i) administration to the subject of a Type II anti-CD20 antibody,
   and consecutively after a period of time
(ii) administration to the subject of the T-cell activating therapeutic agent,
   wherein the period of time between the administration of the Type II anti-CD20 antibody and the administration of the therapeutic agent is sufficient for reduction of the number of B-cells in the subject in response to the administration of the CD20 antibody.

In one embodiment, the treatment regimen effectively reduces cytokine release associated with administration of the T-cell activating therapeutic agent in the subject as compared to a corresponding treatment regimen without the administration of the Type II anti-CD20 antibody.

The invention in still a further aspect provides the use of a T-cell activating therapeutic agent in the manufacture of a medicament for treatment of a disease in a subject, wherein the treatment comprises a treatment regimen comprising
(i) administration to the subject of a Type II anti-CD20 antibody,
   and consecutively after a period of time
(ii) administration to the subject of the T-cell activating therapeutic agent,
   wherein the period of time between the administration of the Type II anti-CD20 antibody and the administration of the therapeutic agent is sufficient for reduction of the number of B-cells in the subject in response to the administration of the Type II anti-CD20 antibody.

In one embodiment, the treatment regimen effectively reduces cytokine release associated with administration of the T-cell activating therapeutic agent in the subject as compared to a corresponding treatment regimen without the administration of the Type II anti-CD20 antibody.

The invention in a further aspect provides a kit for the treatment of a disease in a subject, comprising a package comprising a T-cell activating therapeutic agent composition and instructions for using the therapeutic agent composition in a treatment regimen comprising
(i) administration to the subject of a Type II anti-CD20 antibody,
   and consecutively after a period of time
(ii) administration to the subject of the T-cell activating therapeutic agent composition,
   wherein the period of time between the administration of the Type II anti-CD20 antibody and the administration of the therapeutic agent composition is sufficient for reduction of the number of B-cells in the subject in response to the administration of the CD20 antibody.

In one embodiment, the treatment regimen effectively reduces cytokine release associated with administration of the T-cell activating therapeutic agent in the subject as compared to a corresponding treatment regimen without the administration of the Type II anti-CD20 antibody composition. In one embodiment, the kit further comprises a Type II anti-CD20 antibody composition.

The methods, uses, Type II anti-CD20 antibodies, therapeutic agents and kits of the invention may incorporate, singly or in combination, any of the features described hereinbelow.

In one embodiment, the Type II anti-CD20 antibody comprises a heavy chain variable region comprising the heavy chain CDR (HCDR) 1 of SEQ ID NO: 4, the HCDR2 of SEQ ID NO: 5, and the HCDR3 of SEQ ID NO: 6; and a light chain variable region comprising the light chain CDR (LCDR) 1 of SEQ ID NO: 7, the LCDR2 of SEQ ID NO: 8 and the LCDR3 of SEQ ID NO: 9.

In a more specific embodiment, the Type II anti-CD20 antibody comprises the heavy chain variable region sequence of SEQ ID NO: 10 and the light chain variable region sequence of SEQ ID NO: 11.

In one embodiment, the Type II anti-CD20 antibody is an IgG antibody, particularly an IgG₁ antibody.

In one embodiment, the Type II anti-CD20 antibody is engineered to have an increased proportion of non-fucosylated oligosaccharides in the Fc region as compared to a non-engineered antibody. In one embodiment, at least about 40% of the N-linked oligosaccharides in the Fc region of the Type II anti-CD20 antibody are non-fucosylated.

In a particular embodiment the Type II anti-CD20 antibody is obinutuzumab.

In one embodiment, the T-cell activating therapeutic agent comprises an antibody, particularly a multispecific (e.g. a bispecific) antibody.

In one embodiment, the antibody specifically binds to an activating T cell antigen.

In one embodiment, the antibody specifically binds to an antigen selected from the group of CD3, CD28, CD137 (also known as 4-1BB), CD40, CD226, OX40, GITR, CD27, HVEM, and CD 127.

In one embodiment, the antibody specifically binds to CD3, particularly CD3c.

In one embodiment, the antibody comprises a heavy chain variable region comprising the heavy chain CDR (HCDR) 1 of SEQ ID NO: 12, the HCDR2 of SEQ ID NO: 13, and the HCDR3 of SEQ ID NO: 14; and a light chain variable region comprising the light chain CDR (LCDR) 1 of SEQ ID NO: 15, the LCDR2 of SEQ ID NO: 16 and the LCDR3 of SEQ ID NO: 17.

In one embodiment, the antibody comprises the heavy chain variable region sequence of SEQ ID NO: 18 and the light chain variable region sequence of SEQ ID NO: 19.

In one embodiment, the antibody specifically binds to a B-cell antigen, particularly a malignant B-cell antigen.

In one embodiment, the antibody specifically binds to an antigen selected from the group consisting of CD20, CD19, CD22, ROR-1, CD37 and CD5, particularly to CD20 or CD19.

In one embodiment, the antibody specifically binds to CD20.

In one embodiment, the antibody comprises a heavy chain variable region comprising the heavy chain CDR (HCDR) 1 of SEQ ID NO: 4, the HCDR2 of SEQ ID NO: 5, and the HCDR3 of SEQ ID NO: 6; and a light chain variable region comprising the light chain CDR (LCDR) 1 of SEQ ID NO: 7, the LCDR2 of SEQ ID NO: 8 and the LCDR3 of SEQ ID NO: 9.

In one embodiment, the antibody comprises the heavy chain variable region sequence of SEQ ID NO: 10 and the light chain variable region sequence of SEQ ID NO: 11.

In one embodiment, the antibody is a multispecific antibody, particularly a bispecific antibody.

In one embodiment, the multispecific antibody specifically binds to (i) an activating T cell antigen and (ii) a B cell antigen.

In one embodiment, the multispecific antibody specifically binds to (i) CD3 and (ii) an antigen selected from CD20 and CD 19.

In one embodiment, the multispecific antibody specifically binds to CD3 and CD20.

In one embodiment, the therapeutic agent comprises a bispecific antibody comprising
(i) an antigen binding moiety that specifically binds to CD3 and comprises a heavy chain variable region comprising the heavy chain CDR (HCDR) 1 of SEQ ID NO: 12, the HCDR2 of SEQ ID NO: 13, and the HCDR3 of SEQ ID NO: 14; and a light chain variable region comprising the light chain CDR (LCDR) 1 of SEQ ID NO: 15, the LCDR2 of SEQ ID NO: 16 and the LCDR3 of SEQ ID NO: 17; and
(ii) an antigen binding moiety that specifically binds to CD20 and comprises a heavy chain variable region comprising the heavy chain CDR (HCDR) 1 of SEQ ID NO: 4, the HCDR2 of SEQ ID NO: 5, and the HCDR3 of SEQ ID NO: 6; and a light chain variable region comprising the light chain CDR (LCDR) 1 of SEQ ID NO: 7, the LCDR2 of SEQ ID NO: 8 and the LCDR3 of SEQ ID NO: 9.

In a particular embodiment, the therapeutic agent comprises CD20XCD3 bsAB.

In one embodiment, the therapeutic agent comprises a chimeric antigen receptor (CAR) or a T cell expressing a CAR, particularly a CAR that specifically binds to a B-cell antigen, more particularly a CAR that specifically binds to an antigen selected from the group of CD20, CD 19, CD22, ROR-1, CD37 and CD5.

In one embodiment, the disease is a B cell proliferative disorder, particularly a CD20-positive B-cell disorder.

In one embodiment, the disease is selected from the group consisting of Non-Hodgkin lymphoma (NHL), acute lymphocytic leukemia (ALL), chronic lymphocytic leukemia (CLL), diffuse large B-cell lymphoma (DLBCL), follicular lymphoma (FL), mantle-cell lymphoma (MCL), marginal zone lymphoma (MZL), Multiple myeloma (MM), and Hodgkin lymphoma (HL).

In a further aspect the present invention provides a Type II anti-CD20 antibody for the use in a method for treating or delaying progression of cancer of an individual. The Type II anti-CD20 antibody is used in combination with an anti-CD20/anti-CD3 bispecific antibody.

The anti-CD20/anti-CD3 bispecific antibody and the Type II anti-CD20 antibody may be administered together in a single composition or administered separately in two or more different compositions.

The anti-CD20/anti-CD3 bispecific antibody and the Type II anti-CD20 antibody may be administered in two or more different composition. The two or more different compositions may be administered at different points in time.

The Type II anti-CD20 antibody may comprise a heavy chain variable region comprising the heavy chain CDR(HCDR) 1 of SEQ ID NO: 4, the HCDR2 of SEQ ID NO: 5, and the HCDR3 of SEQ ID NO:6. The Type II anti-CD20 antibody may further comprise a light chain variable region comprising the light chain CDR (LCDR) 1 of SEQ IDNO: 7, the LCDR2 of SEQ ID NO: 8 and the LCDR3 of SEQ ID NO: 9.

The Type II anti-CD20 antibody may comprise the heavy chain variable region sequence of SEQ ID NO: 10 and the light chain variable region sequence of SEQ ID NO: 11.

The Type II anti-CD20 antibody may be an IgG antibody, particularly an IgG1 antibody. At least about 40% of the N-linked oligosaccharides in the Fc region of the anti-CD20 antibody may be nonfucosylated.

Particularly, the Type II anti-CD20 antibody is Obinutuzumab.

The Type II anti-CD20 antibody may be administered concurrently with, prior to, or subsequently to the anti-CD20/anti-CD3 bispecific antibody.

Furthermore, an anti-PD-L1 antibody, preferably Atezolizumab, may be administered.

The anti-PD-L1 antibody may be administered separately or in combination with at least one of the anti-CD20/anti-CD3 bispecific antibody and the Type II anti-CD20 antibody. Here, "in combination with at least one of" means that the anti-PD-L1 antibody is either administered together with the anti-CD20/anti-CD3 bispecific antibody or together with the Type II anti-CD20 antibody or together with both.

The anti-CD20/anti-CD3 bispecific antibody may comprise a first antigen binding domain that binds to CD3, and a second antigen binding domain that binds to CD20.

The anti-CD20/anti-CD3 bispecific antibody may comprise a first antigen binding domain comprising a heavy chain variable region (VHCD3) and a light chain variable region (VLCD3), and a second antigen binding domain comprising a heavy chain variable region (VHCD20) and a light chain variable region (VLCD20).

The first antigen binding domain of the anti-CD20/anti-CD3 bispecific antibody may comprise a heavy chain variable region (VHCD3) comprising CDR-H1 sequence of SEQ ID NO: 97, CDR-H2 sequence of SEQ ID NO: 98, and CDR-H3 sequence of SEQ ID NO: 99; and/or a light chain variable region (VLCD3) comprising CDR-L1 sequence of SEQ ID NO: 100, CDR-L2 sequence of SEQ ID NO: 101, and CDR-L3 sequence of SEQ ID NO:102.

The first antigen binding domain of the anti-CD20/anti-CD3 bispecific antibody may comprise a heavy chain variable region (VHCD3) comprising the amino acid sequence of SEQ ID NO: 103 and/or a light chain variable region (VLCD3) comprising the amino acid sequence of SEQ ID NO: 104.

The second antigen binding domain of the anti-CD20/anti-CD3 bispecific antibody may comprise a heavy chain variable region (VHCD20) comprising CDR-H1 sequence of SEQ ID NO: 4, CDR-H2 sequence of SEQ ID NO: 5, and CDR-H3 sequence of SEQ ID NO: 6, and/or a light chain variable region (VLCD20) comprising CDR-L1 sequence of SEQ ID NO: 7, CDR-L2 sequence of SEQ ID NO: 8, and CDR-L3 sequence of SEQ ID NO: 9.

The second antigen binding domain of the anti-CD20/anti-CD3 bispecific antibody may comprise a heavy chain variable region (VHCD20) comprising the amino acid sequence of SEQ ID NO: 10 and/or a light chain variable region (VLCD20) comprising the amino acid sequence of SEQ ID NO: 11.

The anti-CD20/anti-CD3 bispecific antibody may comprise a third antigen binding domain that binds to CD20.

The third antigen binding domain of the anti-CD20/anti-CD3 bispecific antibody comprises a heavy chain variable region (VHCD20) comprising CDR-H1 sequence of SEQ ID NO: 4, CDR-H2 sequence of SEQ ID NO: 5, and CDR-H3 sequence of SEQ ID NO: 6; and/or a light chain variable region (VLCD20) comprising CDR-L1 sequence of SEQ ID NO: 7, CDR-L2 sequence of SEQ ID NO: 8, and CDR-L3 sequence of SEQ ID NO: 9.

The third antigen binding domain of the anti-CD20/anti-CD3 bispecific antibody may comprise a heavy chain variable region (VHCD20) comprising the amino acid sequence of SEQ ID NO: 10 and/or a light chain variable region (VLCD20) comprising the amino acid sequence of SEQ ID NO: 11.

The first antigen binding domain of the anti-CD20/anti-CD3 bispecific antibody may be a cross-Fab molecule wherein the variable domains or the constant domains of the Fab heavy and light chain are exchanged, and the second and third, if present, antigen binding domain may be a conventional Fab molecule.

The anti-CD20/anti-CD3 bispecific antibody may comprise an IgG1 Fc domain. The IgG1 Fc domain of the anti-CD20/anti-CD3 bispecific antibody may comprise one or more amino acid substitutions that reduce binding to an Fc receptor and/or effector function. The IgG1 Fc domain of the anti-CD20/anti-CD3 bispecific antibody may comprise the amino acid substitutions L234A, L235A and P329G (numbering according to Kabat EU index).

The anti-CD20/anti-CD3 bispecific antibody may comprise a third antigen binding domain, wherein (i) the second antigen binding domain of the anti-CD20/anti-CD3 bispecific antibody is fused at the C-terminus of the Fab heavy chain to the N-terminus of the Fab heavy chain of the first antigen binding domain, the first antigen binding domain of the anti-CD20/anti-CD3 bispecific antibody is fused at the C-terminus of the Fab heavy chain to the N-terminus of the first subunit of the Fc domain, and the third antigen binding domain of the anti-CD20/anti-CD3 bispecific antibody is fused at the C-terminus of the Fab heavy chain to the N-terminus of the second subunit of the Fc domain. Alternatively, (ii) the first antigen binding domain of the anti-CD20/anti-CD3 bispecific antibody is fused at the C-terminus of the Fab heavy chain to the N-terminus of the Fab heavy chain of the second antigen binding domain, the second antigen binding domain of the anti-CD20/anti-CD3 bispecific antibody is fused at the C-terminus of the Fab heavy chain to the N-terminus of the first subunit of the Fc domain, and the third antigen binding domain of the anti-CD20/anti-CD3 bispecific antibody is fused at the C-terminus of the Fab heavy chain to the N-terminus of the second subunit of the Fc domain.

The combination of the Type II CD20-antibody and the anti-CD20/anti CD3 bispecific antibody may be administered at intervals from about one week to three weeks.

Furthermore, a pretreatment with a Type II anti-CD20 antibody, preferably Obinutuzumab, may be performed prior to the combination treatment. The period of time between the pretreatment and the combination treatment may be sufficient for the reduction of B-cells in the individual in response to the Type II anti-CD20 antibody, preferably Obinutuzumab. The Type II anti-CD20 antibody used in the pretreatment may have one or more features of Type II anti-CD20 antibodies as described above and below.

A further aspect of the present invention relates to a method for treating or delaying progression of a proliferative disease, particularly cancer, in an individual. The method comprises administering a Type II anti-CD20 antibody and an anti-CD20/anti-CD3 bispecific antibody, wherein the Type II anti-CD20 antibody and the anti-CD20/anti-CD3 bispecific antibody are administered in a single composition or in two or more compositions.

A further aspect of the present invention relates to a pharmaceutical composition comprising a Type II anti-CD20 antibody for the use in a combination treatment and an optional pharmaceutically acceptable carrier, and a second medicament comprising an anti-CD20/anti-CD3 bispecific antibody and an optional pharmaceutically acceptable carrier, and optionally a third medicament comprising an anti-PD-L1 antibody and an optional pharmaceutically acceptable carrier, for the use in the combined treatment of a disease, in particular cancer. The elements of the pharmaceutical composition may be sequentially or simultaneously used in the combined treatment.

A further aspect of the present invention relates to kit comprising a first medicament comprising a Type II anti-CD20 antibody and an optional pharmaceutically acceptable carrier, and a second medicament comprising an anti-CD20/anti-CD3 bispecific antibody and an optional pharmaceutically acceptable carrier for the use in the combined treatment of a disease, in particular cancer. Optionally, the kit comprises a third medicament comprising an anti-PD-L1 antibody and an optional pharmaceutically acceptable carrier, for the use in the aforementioned combined treatment of a disease, in particular cancer. The elements of the kit may be sequentially or simultaneously used in the combined treatment

The kit may furthermore comprise instructions for use of the first medicament and the second medicament and optionally the third medicament for treating or delaying the progression of cancer in an individual. Instructions for use may be a package insert.

A further aspect of the present invention relates to the use of a combination of a Type II anti-CD20 antibody and an anti-CD20/anti-CD3 bispecific antibody in the manufacture of a medicament for therapeutic application, preferably for treating or delaying the progression of a proliferative disease, particularly cancer, in an individual.

A further aspect of the present invention relates to the use of a Type II anti-CD20 antibody in the manufacture of a medicament for treating or delaying progression of cancer in an individual, wherein the medicament comprises the Type II anti-CD20 antibody and an optional pharmaceutically acceptable carrier, and wherein the treatment comprises administration of the medicament in combination with a composition comprising an anti-CD20/anti-CD3 bispecific antibody and an optional pharmaceutically acceptable carrier.

A further aspect of the present invention relates to the use of an anti-CD20/anti-CD3 bispecific antibody in the manufacture of a medicament for treating or delaying progression of cancer in an individual, wherein the medicament comprises the anti-CD20/anti-CD3 bispecific antibody and an optional pharmaceutically acceptable carrier, and wherein the treatment comprises administration of the medicament in combination with a composition comprising an anti-CD20 antibody and an optional pharmaceutically acceptable carrier.

A further aspect of the present invention relates to the manufacture of an anti-CD20 antibody for the use in a method for treating or delaying progression of cancer of an individual, wherein the Type II anti-CD20 antibody is used in combination with an anti- CD20/anti-CD3 bispecific antibody.

A further aspect of the present invention relates to the manufacture of an anti-CD20/anti-CD3 bispecific antibody for the use in a method for treating or delaying progression of cancer of an individual, wherein the anti-CD20/anti-CD3 bispecific antibody is used in combination with a Type II anti-CD20 antibody.

A further aspect of the present invention relates to a method for treating or delaying progression of cancer in an individual comprising administering a Type II anti-CD20 antibody and administering of an anti-CD20/anti-CD3 bispecific antibody to the individual. The Type II anti-CD20 antibody and the anti-CD20/anti-CD30 bispecific antibody are administered so that the combination of both represents an effective amount. Conversely, the Type II anti-CD20 antibody, itself, is not administered in an effective amount and the anti-CD20/anti-CD30 bispecific antibody, itself, is not administered in an effective amount. However, the combination of both leads to an effective amount.

In addition, an anti-PD-L1 antibody may be administered to the individual. The combination including the PD-L1 antibody represents an effective amount.

A further aspect of the present invention relates to an anti-CD20/anti-CD3 bispecific antibody for the use in a method for treating or delaying progression of cancer of an individual. The anti-CD20/anti-CD3 bispecific antibody is used in combination with a Type II anti-CD20 antibody.

The anti-CD20/anti-CD3 bispecific antibody and the Type II anti-CD20 antibody may be administered together in a single composition or administered separately in two or more different compositions.

The anti-CD20/anti-CD3 bispecific antibody and the Type II anti-CD20 antibody may be administered in two or more different composition. The two or more different compositions may be administered at different points in time.

The Type II anti-CD20 antibody may comprise a heavy chain variable region comprising the heavy chain CDR(HCDR) 1 of SEQ ID NO: 4, the HCDR2 of SEQ ID NO: 5, and the HCDR3 of SEQ ID NO: 6. The Type II anti-CD20 antibody may further comprise a light chain variable region comprising the light chain CDR (LCDR) 1 of SEQ IDNO: 7, the LCDR2 of SEQ ID NO: 8 and the LCDR3 5 of SEQ ID NO: 9.

The Type II anti-CD20 antibody may comprise the heavy chain variable region sequence of SEQ ID NO: 10 and the light chain variable region sequence of SEQ ID NO: 11.

The Type II anti-CD20 antibody may be an IgG antibody, particularly an IgG1 antibody. At least about 40% of the N-linked oligosaccharides in the Fc region of the anti-CD20 antibody may be nonfucosylated.

Particularly, the Type II anti-CD20 antibody is Obinutuzumab.

The Type II anti-CD20 antibody may be administered concurrently with, prior to, or subsequently to the anti-CD20/anti-CD3 bispecific antibody.

Furthermore, an anti-PD-L1 antibody, preferably Atezolizumab, may be administered.

The anti-PD-L1 antibody may be administered separately or in combination with at least one of the anti-CD20/anti-CD3 bispecific antibody and the Type II anti-CD20 antibody. Here, "in combination with at least one of" means that the anti-PD-L1 antibody is either administered together with the anti-CD20/anti-CD3 bispecific antibody or together with the Type II anti-CD20 antibody or together with both.

The anti-CD20/anti-CD3 bispecific antibody may comprise a first antigen binding domain that binds to CD3, and a second antigen binding domain that binds to CD20.

The anti-CD20/anti-CD3 bispecific antibody may comprise a first antigen binding domain comprising a heavy chain variable region (VHCD3) and a light chain variable region (VLCD3), and a second antigen binding domain comprising a heavy chain variable region (VHCD20) and a light chain variable region (VLCD20).

The first antigen binding domain of the anti-CD20/anti-CD3 bispecific antibody may comprise a heavy chain variable region (VHCD3) comprising CDR-H1 sequence of SEQ ID NO: 97, CDR-H2 sequence of SEQ ID NO: 98, and CDR-H3 sequence of SEQ ID NO: 99; and/or a light chain variable region (VLCD3) comprising CDR-L1 sequence of SEQ ID NO: 100, CDR-L2 sequence of SEQ ID NO: 101, and CDR-L3 sequence of SEQ ID NO: 102. The first antigen binding domain of the anti-CD20/anti-CD3 bispecific antibody may comprise a heavy chain variable region (VHCD3) comprising the amino acid sequence of SEQ ID NO: 103 and/or a light chain variable region (VLCD3) comprising the amino acid sequence of SEQ ID NO: 104.

The second antigen binding domain of the anti-CD20/anti-CD3 bispecific antibody may comprise a heavy chain variable region (VHCD20) comprising CDR-H1 sequence of SEQ ID NO: 4, CDR-H2 sequence of SEQ ID NO: 5, and CDR-H3 sequence of SEQ ID NO: 6, and/or a light chain variable region (VLCD20) comprising CDR-L1 sequence of SEQ ID NO: 7, CDR-L2 sequence of SEQ ID NO: 8, and CDR-L3 sequence of SEQ ID NO: 9.

The second antigen binding domain of the anti-CD20/anti-CD3 bispecific antibody may comprise a heavy chain variable region (VHCD20) comprising the amino acid sequence of SEQ ID NO: 10 and/or a light chain variable region (VLCD20) comprising the amino acid sequence of SEQ ID NO: 11.

The anti-CD20/anti-CD3 bispecific antibody may comprise a third antigen binding domain that binds to CD20.

The third antigen binding domain of the anti-CD20/anti-CD3 bispecific antibody may comprise a heavy chain variable region (VHCD20) comprising CDR-H1 sequence of SEQ ID NO: 4, CDR-H2 sequence of SEQ ID NO: 5, and CDR-H3 sequence of SEQ ID NO: 6; and/or a light chain variable region (VLCD20) comprising CDR-L1 sequence of SEQ ID NO: 7, CDR-L2 sequence of SEQ ID NO: 8, and CDR-L3 sequence of SEQ ID NO: 9.

The third antigen binding domain of the anti-CD20/anti-CD3 bispecific antibody may comprise a heavy chain variable region (VHCD20) comprising the amino acid sequence of SEQ ID NO: 10 and/or a light chain variable region (VLCD20) comprising the amino acid sequence of SEQ ID NO: 11.

The first antigen binding domain of the anti-CD20/anti-CD3 bispecific antibody may be a cross-Fab molecule wherein the variable domains or the constant domains of the Fab heavy and light chain are exchanged, and the second and third, if present, antigen binding domain may be a conventional Fab molecule.

The anti-CD20/anti-CD3 bispecific antibody may comprise an IgG1 Fc domain. The IgG1 Fc domain of the anti-CD20/anti-CD3 bispecific antibody may comprise one or more amino acid substitutions that reduce binding to an Fc receptor and/or effector function. The IgG1 Fc domain of the anti-CD20/anti-CD3 bispecific antibody may comprise the amino acid substitutions L234A, L235A and P329G (numbering according to Kabat EU index).

The anti-CD20/anti-CD3 bispecific antibody may comprise a third antigen binding domain, wherein (i) the second antigen binding domain of the anti-CD20/anti-CD3 bispecific antibody is fused at the C-terminus of the Fab heavy chain to the N-terminus of the Fab heavy chain of the first antigen binding domain, the first antigen binding domain of the anti-CD20/anti-CD3 bispecific antibody is fused at the C-terminus of the Fab heavy chain to the N-terminus of the first subunit of the Fc domain, and the third antigen binding domain of the anti-CD20/anti-CD3 bispecific antibody is fused at the C-terminus of the Fab heavy chain to the N-terminus of the second subunit of the Fc domain. Alternatively, (ii) the first antigen binding domain of the anti-CD20/anti-CD3 bispecific antibody is fused at the C-terminus of the Fab heavy chain to the N-terminus of the Fab heavy chain of the second antigen binding domain, the second antigen binding domain of the anti-CD20/anti-CD3 bispecific antibody is fused at the C-terminus of the Fab heavy chain to the N-terminus of the first subunit of the Fc domain, and the third antigen binding domain of the anti-CD20/anti-CD3 bispecific antibody is fused at the C-terminus of the Fab heavy chain to the N-terminus of the second subunit of the Fc domain.

The combination of the Type II CD20-antibody and the anti-CD20/anti CD3 bispecific antibody may be administered at intervals from about one week to three weeks.

Furthermore, a pretreatment with a Type II anti-CD20 antibody, preferably Obinutuzumab, is performed prior to the combination treatment. The period of time between the pretreatment and the combination treatment may be sufficient for the reduction of B-cells in the individual in response to the Type II anti-CD20 antibody, preferably Obinutuzumab. The Type II anti-CD20 antibody used in the pretreatment may have one or more features of Type II anti-CD20 antibodies as described above and below.

The anti-CD20/anti-CD3 bispecific antibody and the Type II anti-CD20 antibody may be administered together in a single composition or administered separately in two or more different compositions.

The anti-CD20/anti-CD3 bispecific antibody and the Type II anti-CD20 antibody may be administered in two or more different composition, wherein the two or more different compositions are administered at different points in time.

The Type II anti-CD20 antibody may comprise a heavy chain variable region comprising the heavy chain CDR(HCDR) 1 of SEQ ID NO: 4, the HCDR2 of SEQ ID NO: 5, and the HCDR3 of SEQ ID NO: 6; and a light chain variable region comprising the light chain CDR (LCDR) 1 of SEQ IDNO: 7, the LCDR2 of SEQ ID NO: 8 and the LCDR3 of SEQ ID NO: 9.

The Type II anti-CD20 antibody may comprise the heavy chain variable region sequence of SEQ ID NO: 10 and the light chain variable region sequence of SEQ ID NO: 11.

The Type II anti-CD20 antibody may be an IgG antibody, particularly an IgG1 antibody, and wherein at least about 40% of the N-linked oligosaccharides in the Fc region of the anti-CD20 antibody are nonfucosylated. The Type II anti-CD20 antibody may be Obinutuzumab.

The Type II anti-CD20 antibody may be administered concurrently with, prior to, or subsequently to the anti-CD20/anti-CD3 bispecific antibody.

Furthermore, an anti-PD-L1 antibody, preferably Atezolizumab, may be administered.

The anti-PD-L1 antibody may be administered separately or in combination with at least one of the anti-CD20/anti-CD3 bispecific antibody and the Type II anti-CD20 antibody.

The anti-CD20/anti-CD3 bispecific antibody may comprises a first antigen binding domain that binds to CD3, and a second antigen binding domain that binds to CD20.

The anti-CD20/anti-CD3 bispecific antibody may comprise a first antigen binding domain comprising a heavy chain variable region (VHCD3) and a light chain variable region (VLCD3), and a second antigen binding domain comprising a heavy chain variable region (VHCD20) and a light chain variable region (VLCD20).

The first antigen binding domain of the anti-CD20/anti-CD3 bispecific antibody may comprise a heavy chain variable region (VHCD3) comprising CDR-H1 sequence of SEQ ID NO: 97, CDR-H2 sequence of SEQ ID NO: 98, and CDR-H3 sequence of SEQ ID NO: 99; and/or a light chain variable region (VLCD3) comprising CDR-L1 sequence of SEQ ID NO: 100, CDR-L2 sequence of SEQ ID NO: 101, and CDR-L3 sequence of SEQ ID NO: 102.

The first antigen binding domain of the anti-CD20/anti-CD3 bispecific antibody may comprise a heavy chain variable region (VHCD3) comprising the amino acid sequence of SEQ ID NO: 103 and/or a light chain variable region (VLCD3) comprising the amino acid sequence of SEQ ID NO: 104.

The second antigen binding domain of the anti-CD20/anti-CD3 bispecific antibody may comprise a heavy chain variable region (VHCD20) comprising CDR-H1 sequence of SEQ ID NO: 4, CDR-H2 sequence of SEQ ID NO: 5, and CDR-H3 sequence of SEQ ID NO: 6, and/or a light chain variable region (VLCD20) comprising CDR-L1 sequence of SEQ ID NO: 7, CDR-L2 sequence of SEQ ID NO: 8, and CDR-L3 sequence of SEQ ID NO: 9.

The second antigen binding domain of the anti-CD20/anti-CD3 bispecific antibody may comprise a heavy chain variable region (VHCD20) comprising the amino acid sequence of SEQ ID NO: 10 and/or a light chain variable region (VLCD20) comprising the amino acid sequence of SEQ ID NO: 11.

The anti-CD20/anti-CD3 bispecific antibody may comprise a third antigen binding domain that binds to CD20.

The third antigen binding domain of the anti-CD20/anti-CD3 bispecific antibody may comprise a heavy chain variable region (VHCD20) comprising CDR-H1 sequence of SEQ ID NO: 4, CDR-H2 sequence of SEQ ID NO: 5, and CDR-H3 sequence of SEQ ID NO: 6; and/or a light chain variable region (VLCD20) comprising CDR-L1 sequence of SEQ ID NO: 7, CDR-L2 sequence of SEQ ID NO: 8, and CDR-L3 sequence of SEQ ID NO: 9.

The third antigen binding domain of the anti-CD20/anti-CD3 bispecific antibody may comprise a heavy chain variable region (VHCD20) comprising the amino acid sequence of SEQ ID NO: 10 and/or a light chain variable region (VLCD20) comprising the amino acid sequence of SEQ ID NO: 11.

The first antigen binding domain of the anti-CD20/anti-CD3 bispecific antibody may be a cross-Fab molecule wherein the variable domains or the constant domains of the Fab heavy and light chain are exchanged, and the second and third, if present, antigen binding domain may be a conventional Fab molecule.

The anti-CD20/anti-CD3 bispecific antibody may comprise an IgG1 Fc domain. The IgG1 Fc domain of the anti-CD20/anti-CD3 bispecific antibody may comprise one or more amino acid substitutions that reduce binding to an Fc receptor and/or effector function. The IgG1 Fc domain of the anti-CD20/anti-CD3 bispecific antibody may comprise the amino acid substitutions L234A, L235A and P329G (numbering according to Kabat EU index).

The anti-CD20/anti-CD3 bispecific antibody comprises a third antigen binding domain. (i) the second antigen binding domain of the anti-CD20/anti-CD3 bispecific antibody may be fused at the C-terminus of the Fab heavy chain to the N-terminus of the Fab heavy chain of the first antigen binding domain, the first antigen binding domain of the anti-CD20/anti-CD3 bispecific antibody may be fused at the C-terminus of the Fab heavy chain to the N-terminus of the first subunit of the Fc domain, and the third antigen binding domain of the anti-CD20/anti-CD3 bispecific antibody may be fused at the C-terminus of the Fab heavy chain to the N-terminus of the second subunit of the Fc domain. Alternatively, (ii) the first antigen binding domain of the anti-CD20/anti-CD3 bispecific antibody may be fused at the C-terminus of the Fab heavy chain to the N-terminus of the Fab heavy chain of the second antigen binding domain, the second antigen binding domain of the anti-CD20/anti-CD3 bispecific antibody may be fused at the C-terminus of the Fab heavy chain to the N-terminus of the first subunit of the Fc domain, and the third antigen binding domain of the anti-CD20/anti-CD3 bispecific antibody may be fused at the C-terminus of the Fab heavy chain to the N-terminus of the second subunit of the Fc domain.

The combination of the anti-CD20/anti CD3 bispecific antibody and the Type II CD20-antibody may be administered at intervals from about one week to three weeks.

A pretreatment with a Type II anti-CD20 antibody, preferably Obinutuzumab, may be performed prior to the combination treatment. The period of time between the pretreatment and the combination treatment may be sufficient for the reduction of B-cells in the individual in response to the Type II anti-CD20 antibody, preferably Obinutuzumab. The Type II anti-CD20 antibody used in the pretreatment may have one or more features of Type II anti-CD20 antibodies as described above and below.

A further aspect of the present invention relates to a pharmaceutical composition comprising an anti-CD20/anti-CD3 bispecific antibody for the use in a combination treatment and an optional pharmaceutically acceptable carrier, and a second medicament comprising an Type II anti-CD20 antibody and an optional pharmaceutically acceptable carrier, and optionally a third medicament comprising an anti-PD-L1 antibody and an optional pharmaceutically acceptable carrier, for the use in the combined treatment of a disease, in particular cancer. The elements of the pharmaceutical composition may be sequentially or simultaneously used in the combined treatment.

A further aspect of the present invention relates to the invention as described hereinbefore.

### Brief Description of the Drawings

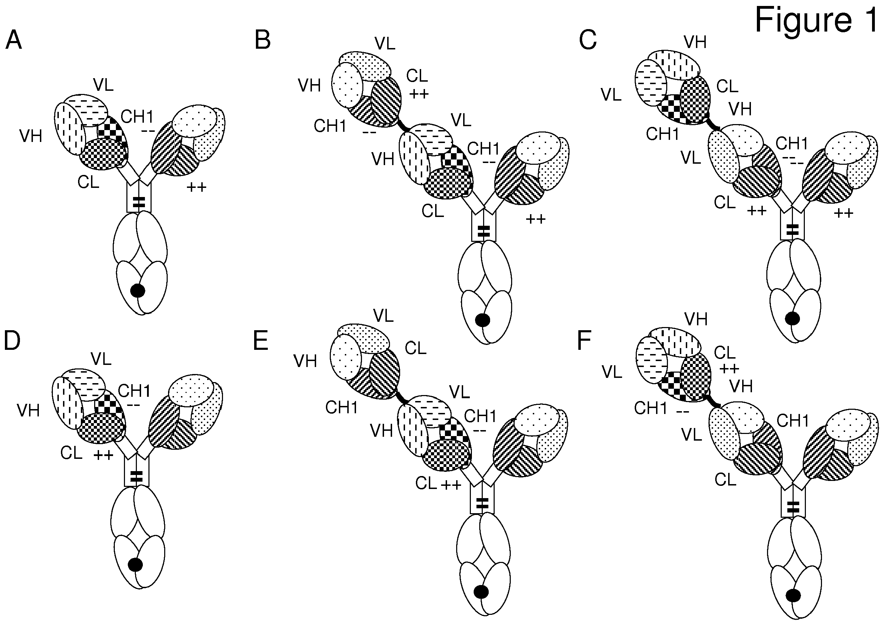

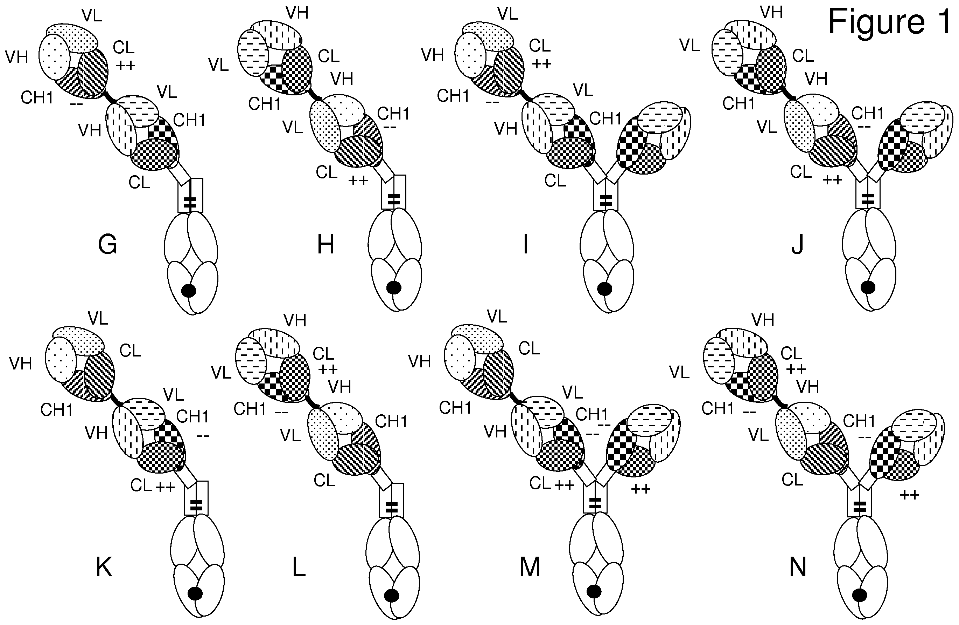

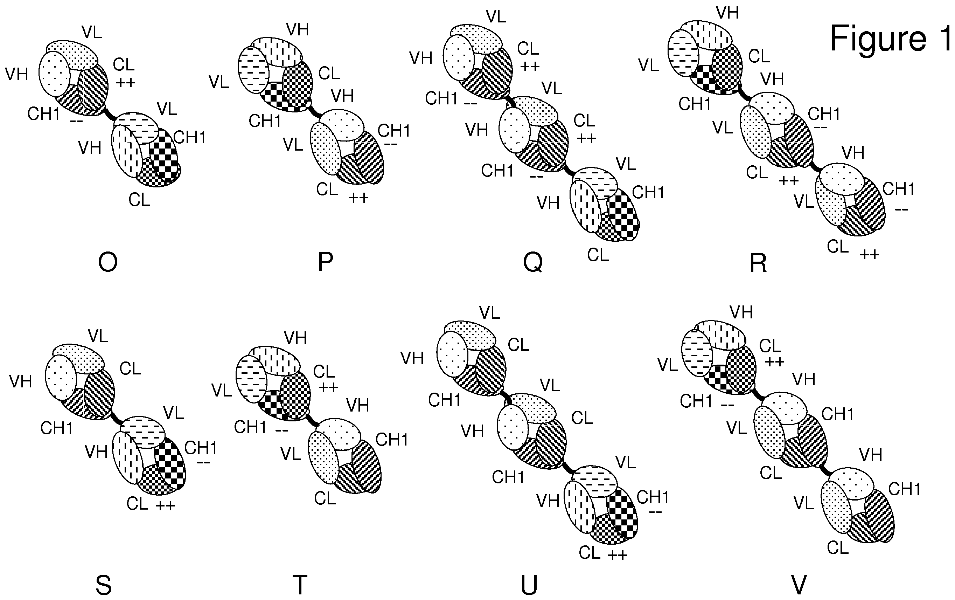

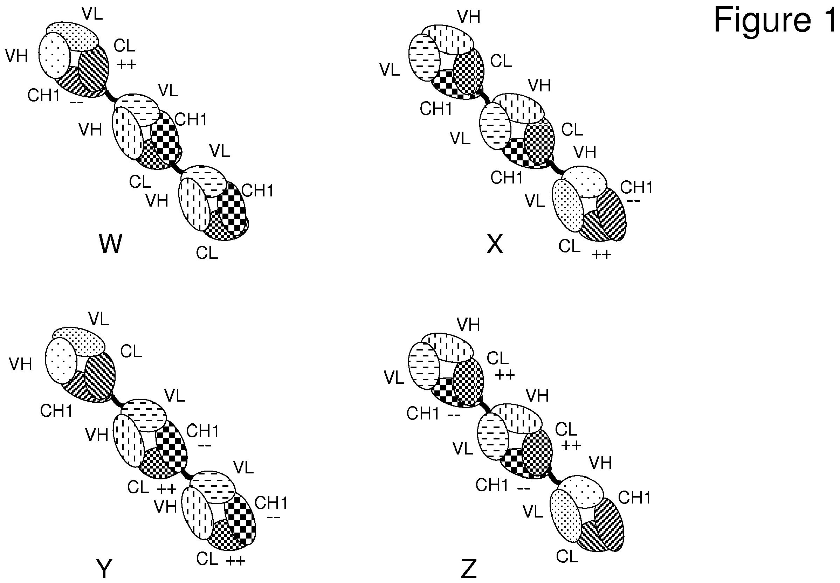

Figure 1. Exemplary configurations of the T cell activating bispecific antigen binding molecules (TCBs) of the invention. (A, D) Illustration of the "1+1 CrossMab" molecule. (B, E) Illustration of the "2+1 IgG Crossfab" molecule with alternative order of Crossfab and Fab components ("inverted"). (C, F) Illustration of the "2+1 IgG Crossfab" molecule. (G, K) Illustration of the "1+1 IgG Crossfab" molecule with alternative order of Crossfab and Fab components ("inverted"). (H, L) Illustration of the "1+1 IgG Crossfab" molecule. (I, M) Illustration of the "2+1 IgG Crossfab" molecule with two CrossFabs. (J, N) Illustration of the "2+1 IgG Crossfab" molecule with two CrossFabs and alternative order of Crossfab and Fab components ("inverted"). (O, S) Illustration of the "Fab-Crossfab" molecule. (P, T) Illustration of the "Crossfab-Fab" molecule. (Q, U) Illustration of the "(Fab)₂-Crossfab" molecule. (R, V) Illustration of the "Crossfab-(Fab)₂" molecule. (W, Y) Illustration of the "Fab-(Crossfab)₂" molecule. (X, Z) Illustration of the "(Crossfab)₂-Fab" molecule. Black dot: optional modification in the Fc domain promoting heterodimerization. ++, --: amino acids of opposite charges optionally introduced in the CH1 and CL domains. Crossfab molecules are depicted as comprising an exchange of VH and VL regions, but may - in embodiments wherein no charge modifications are introduced in CH1 and CL domains - alternatively comprise an exchange of the CH1 and CL domains.

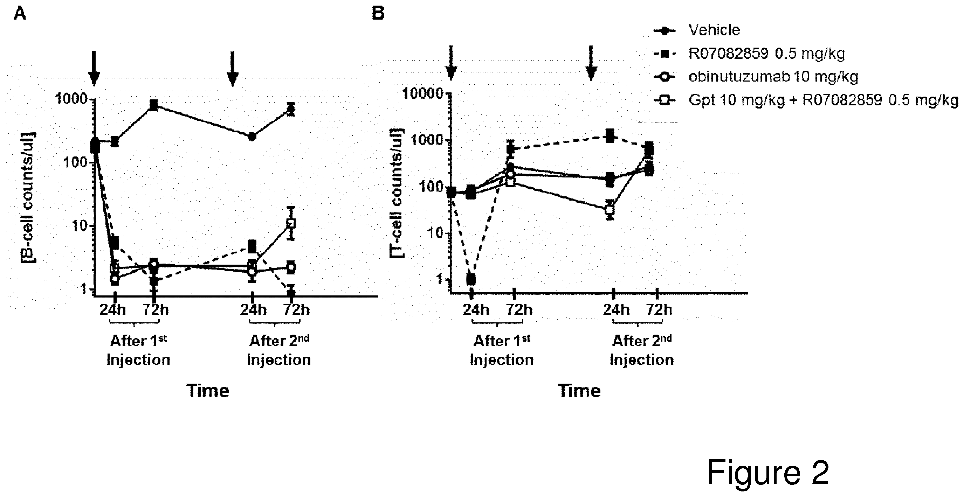

Figure 2. B Cell and T Cell Counts in the Peripheral Blood in the Different Treatment Groups. Flow cytometry analysis of CD19⁺ B cells (A) and CD3⁺ T cells (B) in the peripheral blood of vehicle and CD20XCD3 bsAB-treated fully humanized NOG mice, 24 hours and 72 hours after first and second CD20XCD3 bsAB administration. Black arrows indicate days of CD20XCD3 bsAB administration.

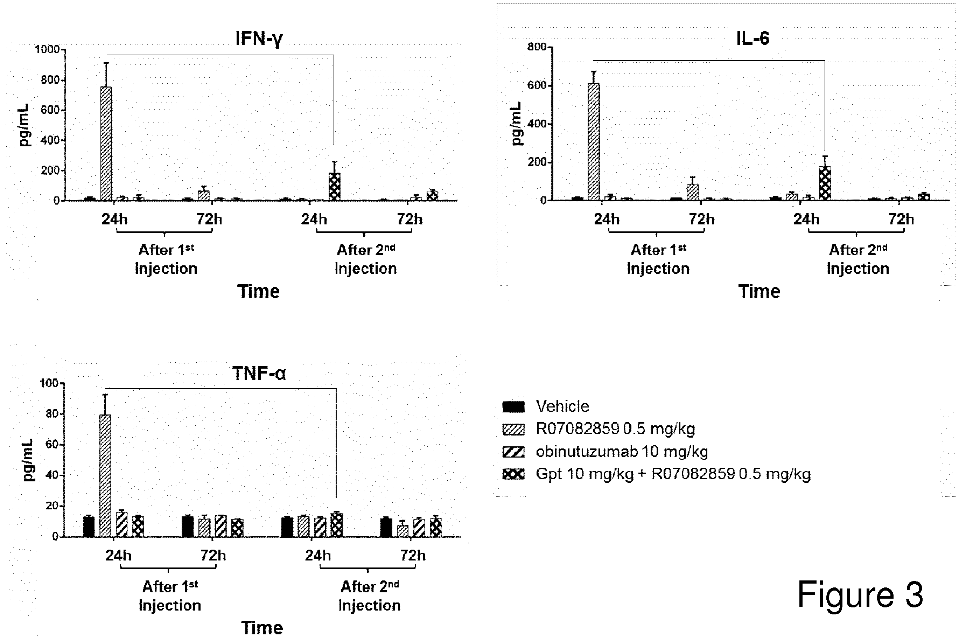

Figure 3. Cytokines Released in Peripheral Blood Among the Different Treatment Groups. Multiplex analysis of cytokines in blood of vehicle and treated mice, 24 hours and 72 hours after the first and second administration of CD20XCD3 bsAB. Histogram bars represent the mean of 5 animals with error bars indicating the standard deviation. Representative graphs for IFNy, TNFα and IL-6 are shown. Compare the cytokine release of the first injection of CD20XCD3 bsAB with and without obinutuzumab pre-treatment (bars to be compared are indicated by connecting lines).

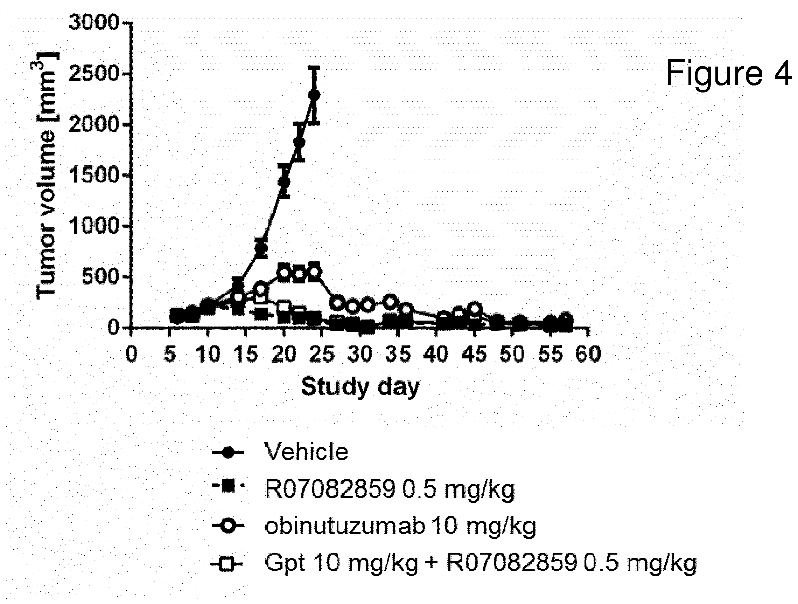

Figure 4. Anti-Tumour Activity of CD20XCD3 bsAB, Obinutuzumab, and
   Gpt + CD20XCD3 bsAB. Anti-tumour activity of CD20XCD3 bsAB and obinutuzumab as monotherapy or Gpt + CD20XCD3 bsAB in fully humanized NOG mice. Black arrow indicates start of therapy. (8<n<10). Tumour model: WSU-DLCL2.

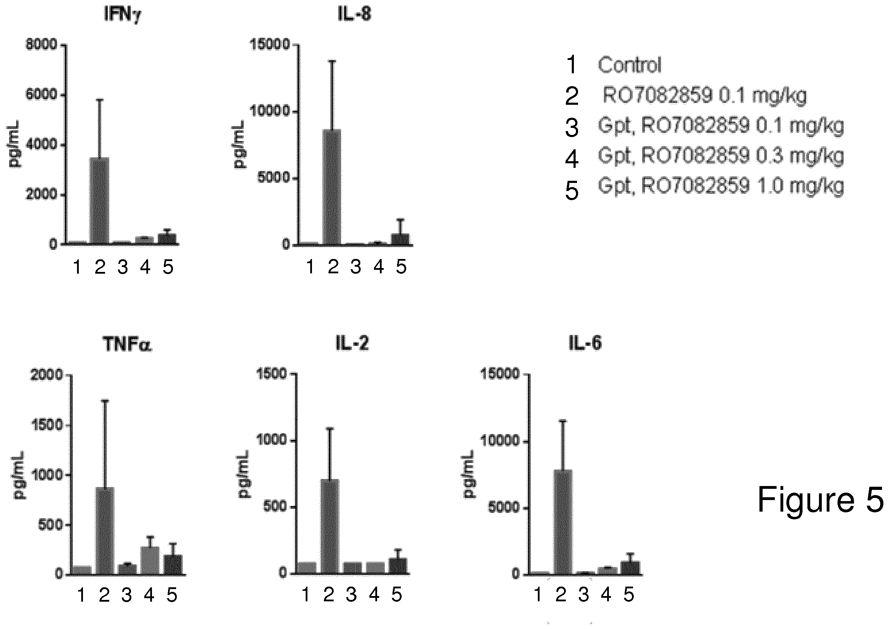

Figure 5. Cytokines Released in Peripheral Blood of Cynomolgus Monkeys following dosing with CD20XCD3 bsAB and Gpt + CD20XCD3 bsAB Treatments.

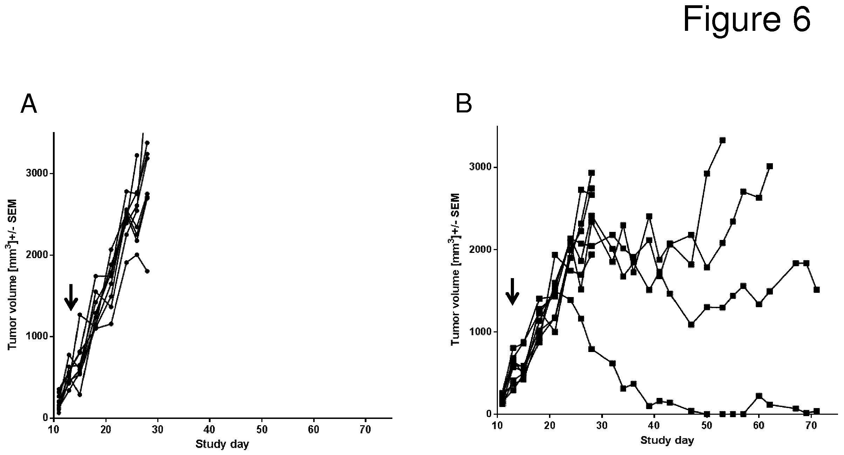

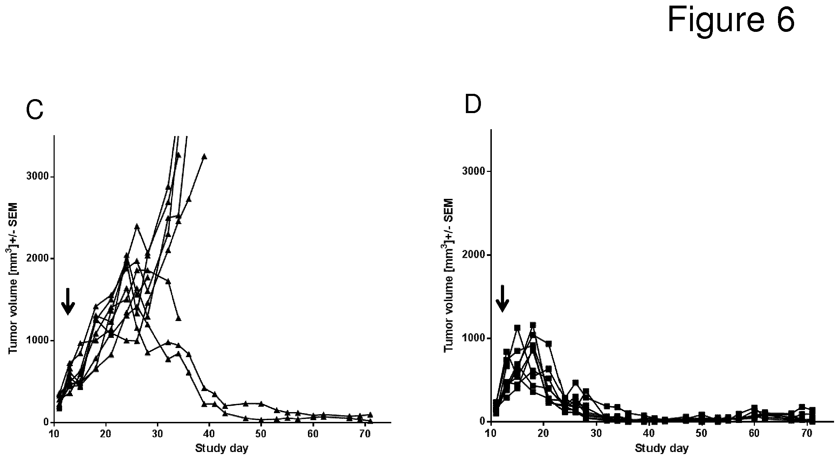

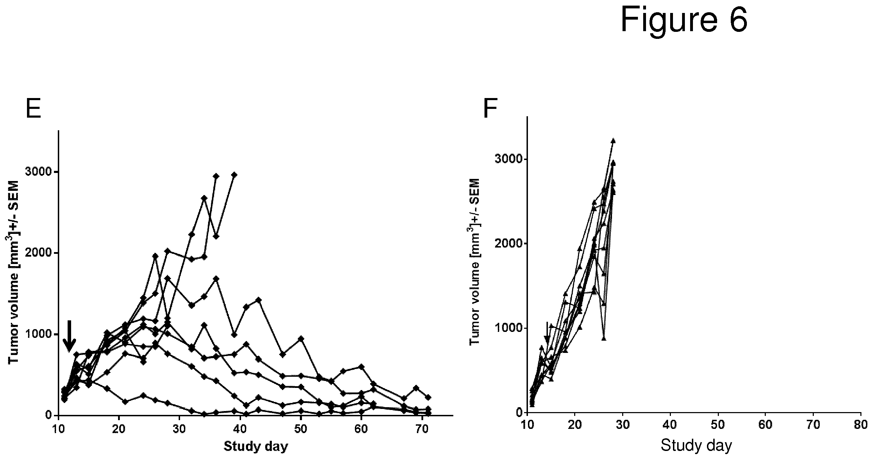

Figure 6. (A-F) Analysis of the anti-tumor activity in combination treatments of an anti-CD20/CD3 bispecific antibody with either Obinutuzumab or Atezolizumab in human hematopoietic stem-cell humanized mice (HSC-NSG mice) bearing aggressive lymphoma mode (WSU-DLCL2 tumor). (A) Efficacy of the vehicle, (B) efficacy of treatment of an anti-CD20-anti-CD3 T-cell bispecific antibody, (C) efficacy of treatment of Obinutuzumab (GAZYVA®), (D) efficacy of the combination treatment of an anti-CD20/CD3 bispecific antibody with Obinutuzumab (GAZYVA®), (E) efficacy of the combination treatment of an anti-CD20/CD3 bispecific antibody with Atezolizumab, (F) Efficacy of treatment of an anti-PD-L1 antibody.

### Detailed Description of the Invention

### Definitions

Terms are used herein as generally used in the art, unless otherwise defined in the following.

CD20 (also known as B-lymphocyte antigen CD20, B-lymphocyte surface antigen B1, Leu-16, Bp35, BM5, and LF5; the human protein is characterized in UniProt database entry P11836) is a hydrophobic transmembrane protein with a molecular weight of approximately 35 kD expressed on pre-B and mature B lymphocytes (Valentine, M.A. et al., J. Biol. Chem. 264 (1989) 11282-11287; Tedder, T.F., et al., Proc. Natl. Acad. Sci. U.S.A. 85 (1988) 208-212; Stamenkovic, I., et al., J. Exp. Med. 167 (1988) 1975-1980; Einfeld, D.A., et al., EMBO J. 7 (1988) 711-717; Tedder, T.F., et al., J. Immunol. 142 (1989) 2560-2568). The corresponding human gene is Membrane-spanning 4-domains, subfamily A, member 1, also known as MS4A1. This gene encodes a member of the membrane-spanning 4A gene family. Members of this nascent protein family are characterized by common structural features and similar intron/exon splice boundaries and display unique expression patterns among hematopoietic cells and nonlymphoid tissues. This gene encodes the B-lymphocyte surface molecule which plays a role in the development and differentiation of B-cells into plasma cells. This family member is localized to 11q12, among a cluster of family members. Alternative splicing of this gene results in two transcript variants which encode the same protein.

The term "CD20" as used herein, refers to any native CD20 from any vertebrate source, including mammals such as primates (e.g. humans) and rodents (e.g., mice and rats), unless otherwise indicated. The term encompasses "full-length," unprocessed CD20 as well as any form of CD20 that results from processing in the cell. The term also encompasses naturally occurring variants of CD20, e.g., splice variants or allelic variants. In one embodiment, CD20 is human CD20. The amino acid sequence of an exemplary human CD20 is shown in SEQ ID NO: 1.

The terms "anti-CD20 antibody" and "an antibody that binds to CD20" refer to an antibody that is capable of binding CD20 with sufficient affinity such that the antibody is useful as a diagnostic and/or therapeutic agent in targeting CD20. In one embodiment, the extent of binding of an anti-CD20 antibody to an unrelated, non-CD20 protein is less than about 10% of the binding of the antibody to CD20 as measured, e.g., by a radioimmunoassay (RIA). In certain embodiments, an antibody that binds to CD20 has a dissociation constant (Kd) of ≤ 1µM, ≤ 100 nM, ≤ 10 nM, ≤ 1 nM, ≤ 0.1 nM, ≤ 0.01 nM, or ≤ 0.001 nM (e.g. 10⁻⁸ M or less, e.g. from 10⁻⁸ M to 10⁻¹³ M, e.g., from 10⁻⁹ M to 10⁻¹³ M). In certain embodiments, an anti-CD20 antibody binds to an epitope of CD20 that is conserved among CD20 from different species.

By "Type II anti-CD20 antibody" is meant an anti-CD20 antibody having binding properties and biological activities of Type II anti-CD20 antibodies as described in Cragg et al., Blood 103 (2004) 2738-2743; Cragg et al., Blood 101 (2003) 1045-1052, Klein et al., mAbs 5 (2013), 22-33, and summarized in Table 1 below.

**Table 1. Properties of type I and type II anti-CD20 antibodies**

| **type I anti-CD20 antibodies** | **type II anti-CD20 antibodies** |
|---|---|
| Bind class I CD20 epitope | Bind class II CD20 epitope |
| Localize CD20 to lipid rafts | Do not localize CD20 to lipid rafts |
| High CDC * | Low CDC * |
| ADCC activity * | ADCC activity * |
| Full binding capacity to B cells | Approx. half binding capacity to B cells |
| Weak homotypic aggregation | Homotypic aggregation |
| Low cell death induction | Strong cell death induction |

| | |
|---|---|
| * if IgG₁ isotype | |

Examples of type II anti-CD20 antibodies include e.g. obinutuzumab (GA101), tositumumab (B1), humanized B-Ly1 antibody IgG1 (a chimeric humanized IgG1 antibody as disclosed in WO 2005/044859), 11B8 IgG1 (as disclosed in WO 2004/035607) and AT80 IgG1.

Examples of type I anti-CD20 antibodies include e.g. rituximab, ofatumumab, veltuzumab, ocaratuzumab, ocrelizumab, PRO131921, ublituximab, HI47 IgG3 (ECACC, hybridoma), 2C6 IgG1 (as disclosed in WO 2005/103081), 2F2 IgG1 (as disclosed in WO 2004/035607 and WO 2005/103081) and 2H7 IgG1 (as disclosed in WO 2004/056312).

The term "humanized B-Ly1 antibody" refers to humanized B-Ly1 antibody as disclosed in WO 2005/044859 and WO 2007/031875, which were obtained from the murine monoclonal anti-CD20 antibody B-Ly1 (variable region of the murine heavy chain (VH): SEQ ID NO: 2; variable region of the murine light chain (VL): SEQ ID NO: 3 (see Poppema, S. and Visser, L., Biotest Bulletin 3 (1987) 131-139) by chimerization with a human constant domain from IgG1 and following humanization (see WO 2005/044859 and WO 2007/031875). These "humanized B-Ly1 antibodies" are disclosed in detail in WO 2005/044859 and WO 2007/031875.

As used herein, the term "release of cytokines" or "cytokine release" is synonymous with "cytokine storm" or "cytokine release syndrome" (abbreviated as "CRS"), and refers to an increase in the levels of cytokines, particularly tumor necrosis factor alpha (TNF-α), interferon gamma (IFN-γ), interleukin-6 (IL-6), interleukin-10 (IL-10), interleukin-2 (IL-2) and/or interleukin-8 (IL-8), in the blood of a subject during or shortly after (e.g. within 1 day of) administration of a therapeutic agent, resulting in adverse symptoms. Cytokine release is a type of infusion-related reaction (IRR), which are common adverse drug reactions to therapeutic agent and timely related to administration of the therapeutic agent. IRRs typically occur during or shortly after an administration of the therapeutic agent, i.e. typically within 24 hours after infusion, predominantly at the first infusion. In some instances, e.g. after the administration of CAR-T cells, CRS can also occur only later, e.g. several days after administration upon expansion of the CAR-T cells. The incidence and severity typically decrease with subsequent infusions. Symptoms may range from symptomatic discomfort to fatal events, and may include fever, chills, dizziness, hypertension, hypotension, dyspnea, restlessness, sweating, flushing, skin rash, tachycardia, tachypnoea, headache, tumour pain, nausea, vomiting and/or organ failure.

The term "amino acid mutation" as used herein is meant to encompass amino acid substitutions, deletions, insertions, and modifications. Any combination of substitution, deletion, insertion, and modification can be made to arrive at the final construct, provided that the final construct possesses the desired characteristics, e.g., reduced binding to an Fc receptor. Amino acid sequence deletions and insertions include amino- and/or carboxy-terminal deletions and insertions of amino acids. Particular amino acid mutations are amino acid substitutions. For the purpose of altering e.g. the binding characteristics of an Fc region, non-conservative amino acid substitutions, i.e. replacing one amino acid with another amino acid having different structural and/or chemical properties, are particularly preferred. Amino acid substitutions include replacement by non-naturally occurring amino acids or by naturally occurring amino acid derivatives of the twenty standard amino acids (e.g. 4-hydroxyproline, 3-methylhistidine, ornithine, homoserine, 5-hydroxylysine). Amino acid mutations can be generated using genetic or chemical methods well known in the art. Genetic methods may include site-directed mutagenesis, PCR, gene synthesis and the like. It is contemplated that methods of altering the side chain group of an amino acid by methods other than genetic engineering, such as chemical modification, may also be useful. Various designations may be used herein to indicate the same amino acid mutation. For example, a substitution from proline at position 329 of the Fc region to glycine can be indicated as 329G, G329, G₃₂₉, P329G, or Pro329Gly.

"Affinity" refers to the strength of the sum total of non-covalent interactions between a single binding site of a molecule (e.g., a receptor) and its binding partner (e.g., a ligand). Unless indicated otherwise, as used herein, "binding affinity" refers to intrinsic binding affinity which reflects a 1:1 interaction between members of a binding pair (e.g., receptor and a ligand). The affinity of a molecule X for its partner Y can generally be represented by the dissociation constant (K_{D}), which is the ratio of dissociation and association rate constants (k_{off} and kₒₙ, respectively). Thus, equivalent affinities may comprise different rate constants, as long as the ratio of the rate constants remains the same. Affinity can be measured by well established methods known in the art. A particular method for measuring affinity is Surface Plasmon Resonance (SPR).

"Reduction" (and grammatical variations thereof such as "reduce" or "reducing"), for example reduction of the number of B cells or cytokine release, refers to a decrease in the respective quantity, as measured by appropriate methods known in the art. For clarity the term includes also reduction to zero (or below the detection limit of the analytical method), i.e. complete abolishment or elimination. Conversely, "increased" refers to an increase in the respective quantity.

As used herein, the term "antigen binding moiety" refers to a polypeptide molecule that specifically binds to an antigenic determinant. In one embodiment, an antigen binding moiety is able to direct the entity to which it is attached (e.g. a cytokine or a second antigen binding moiety) to a target site, for example to a specific type of tumor cell or tumor stroma bearing the antigenic determinant. Antigen binding moieties include antibodies and fragments thereof as further defined herein. Preferred antigen binding moieties include an antigen binding domain of an antibody, comprising an antibody heavy chain variable region and an antibody light chain variable region. In certain embodiments, the antigen binding moieties may include antibody constant regions as further defined herein and known in the art. Useful heavy chain constant regions include any of the five isotypes: α, δ, ε, γ, or µ. Useful light chain constant regions include any of the two isotypes: κ and λ.

By "specifically binds" is meant that the binding is selective for the antigen and can be discriminated from unwanted or non-specific interactions. The ability of an antigen binding moiety to bind to a specific antigenic determinant can be measured either through an enzyme-linked immunosorbent assay (ELISA) or other techniques familiar to one of skill in the art, e.g. surface plasmon resonance technique (analyzed on a BIAcore instrument) (Liljeblad et al., Glyco J 17, 323-329 (2000)), and traditional binding assays (Heeley, Endocr Res 28, 217-229 (2002)). In one embodiment, the extent of binding of an antigen binding moiety to an unrelated protein is less than about 10% of the binding of the antigen binding moiety to the antigen as measured, e.g., by SPR. In certain embodiments, an antigen binding moiety that binds to the antigen, or an antigen binding molecule comprising that antigen binding moiety, has a dissociation constant (K_{D}) of ≤ 1 µM, ≤ 100 nM, ≤ 10 nM, ≤ 1 nM, ≤ 0.1 nM, ≤ 0.01 nM, or ≤ 0.001 nM (e.g. 10⁻⁸ M or less, e.g. from 10⁻⁸ M to 10⁻¹³ M, e.g., from 10⁻⁹ M to 10⁻¹³ M).

"Reduced binding", for example reduced binding to an Fc receptor, refers to a decrease in affinity for the respective interaction, as measured for example by SPR. For clarity the term includes also reduction of the affinity to zero (or below the detection limit of the analytic method), i.e. complete abolishment of the interaction. Conversely, "increased binding" refers to an increase in binding affinity for the respective interaction.

As used herein, the term "antigen binding molecule" refers in its broadest sense to a molecule that specifically binds an antigenic determinant. Examples of antigen binding molecules are immunoglobulins and derivatives, e.g. fragments, thereof.

As used herein, the term "antigenic determinant" is synonymous with "antigen" and "epitope," and refers to a site (e.g. a contiguous stretch of amino acids or a conformational configuration made up of different regions of non-contiguous amino acids) on a polypeptide macromolecule to which an antigen binding moiety binds, forming an antigen binding moiety-antigen complex. Useful antigenic determinants can be found, for example, on the surfaces of tumor cells, on the surfaces of virus-infected cells, on the surfaces of other diseased cells, free in blood serum, and/or in the extracellular matrix (ECM). The proteins referred to as antigens herein (e.g. CD3) can be any native form the proteins from any vertebrate source, including mammals such as primates (e.g. humans) and rodents (e.g. mice and rats), unless otherwise indicated. In a particular embodiment the antigen is a human protein. Where reference is made to a specific protein herein, the term encompasses the "full-length", unprocessed protein as well as any form of the protein that results from processing in the cell. The term also encompasses naturally occurring variants of the protein, e.g. splice variants or allelic variants. An exemplary human protein useful as antigen is CD3, particularly the epsilon subunit of CD3 (see UniProt no. P07766 (version 130), NCBI RefSeq no. NP_000724.1, SEQ ID NO: 105 for the human sequence; or UniProt no. Q95LI5 (version 49), NCBI GenBank no. BAB71849.1, SEQ ID NO: 106 for the cynomolgus [Macaca fascicularis] sequence). In certain embodiments the T cell activating bispecific antigen binding molecule of the invention binds to an epitope of CD3 or a target cell antigen that is conserved among the CD3 or target cell antigen from different species.

As used herein, term "polypeptide" refers to a molecule composed of monomers (amino acids) linearly linked by amide bonds (also known as peptide bonds). The term "polypeptide" refers to any chain of two or more amino acids, and does not refer to a specific length of the product. Thus, peptides, dipeptides, tripeptides, oligopeptides, "protein," "amino acid chain," or any other term used to refer to a chain of two or more amino acids, are included within the definition of "polypeptide," and the term "polypeptide" may be used instead of, or interchangeably with any of these terms. The term "polypeptide" is also intended to refer to the products of post-expression modifications of the polypeptide, including without limitation glycosylation, acetylation, phosphorylation, amidation, derivatization by known protecting/blocking groups, proteolytic cleavage, or modification by non-naturally occurring amino acids. A polypeptide may be derived from a natural biological source or produced by recombinant technology, but is not necessarily translated from a designated nucleic acid sequence. It may be generated in any manner, including by chemical synthesis. A polypeptide of the invention may be of a size of about 3 or more, 5 or more, 10 or more, 20 or more, 25 or more, 50 or more, 75 or more, 100 or more, 200 or more, 500 or more, 1,000 or more, or 2,000 or more amino acids. Polypeptides may have a defined three-dimensional structure, although they do not necessarily have such structure. Polypeptides with a defined three-dimensional structure are referred to as folded, and polypeptides which do not possess a defined three-dimensional structure, but rather can adopt a large number of different conformations, and are referred to as unfolded.

By an "isolated" polypeptide or a variant, or derivative thereof is intended a polypeptide that is not in its natural milieu. No particular level of purification is required. For example, an isolated polypeptide can be removed from its native or natural environment. Recombinantly produced polypeptides and proteins expressed in host cells are considered isolated for the purpose of the invention, as are native or recombinant polypeptides which have been separated, fractionated, or partially or substantially purified by any suitable technique.

"Percent (%) amino acid sequence identity" with respect to a reference polypeptide sequence is defined as the percentage of amino acid residues in a candidate sequence that are identical with the amino acid residues in the reference polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for aligning sequences, including any algorithms needed to achieve maximal alignment over the full length of the sequences being compared. For purposes herein, however, % amino acid sequence identity values are generated using the sequence comparison computer program ALIGN-2. The ALIGN-2 sequence comparison computer program was authored by Genentech, Inc., and the source code has been filed with user documentation in the U.S. Copyright Office, Washington D.C., 20559, where it is registered under U.S. Copyright Registration No. TXU510087. The ALIGN-2 program is publicly available from Genentech, Inc., South San Francisco, California, or may be compiled from the source code. The ALIGN-2 program should be compiled for use on a UNIX operating system, including digital UNIX V4.0D. All sequence comparison parameters are set by the ALIGN-2 program and do not vary. In situations where ALIGN-2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:
100 times the fraction X/Y
where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. Unless specifically stated otherwise, all % amino acid sequence identity values used herein are obtained as described in the immediately preceding paragraph using the ALIGN-2 computer program.

The term "antibody" herein is used in the broadest sense and encompasses various antibody structures, including but not limited to monoclonal antibodies, polyclonal antibodies, multispecific antibodies (e.g. bispecific antibodies), and antibody fragments so long as they exhibit the desired antigen binding activity.

The terms "full length antibody," "intact antibody," and "whole antibody" are used herein interchangeably to refer to an antibody having a structure substantially similar to a native antibody structure or having heavy chains that contain an Fc region as defined herein.

An "antibody fragment" refers to a molecule other than an intact antibody that comprises a portion of an intact antibody that binds the antigen to which the intact antibody binds. Examples of antibody fragments include but are not limited to Fv, Fab, Fab', Fab'-SH, F(ab')₂, diabodies, linear antibodies, single-chain antibody molecules (e.g. scFv), and multispecific antibodies formed from antibody fragments. The term "antibody fragment" as used herein also encompasses single-domain antibodies.

The term "immunoglobulin molecule" refers to a protein having the structure of a naturally occurring antibody. For example, immunoglobulins of the IgG class are heterotetrameric glycoproteins of about 150,000 daltons, composed of two light chains and two heavy chains that are disulfide-bonded. From N- to C-terminus, each heavy chain has a variable region (VH), also called a variable heavy domain or a heavy chain variable domain, followed by three constant domains (CH1, CH2, and CH3), also called a heavy chain constant region. Similarly, from N- to C-terminus, each light chain has a variable region (VL), also called a variable light domain or a light chain variable domain, followed by a constant light (CL) domain, also called a light chain constant region. The heavy chain of an immunoglobulin may be assigned to one of five classes, called α (IgA), δ (IgD), ε (IgE), γ (IgG), or µ (IgM), some of which may be further divided into subclasses, e.g. γ₁ (IgG₁), γ₂ (IgG₂), γ₃ (IgG₃), γ₄ (IgG₄), α₁ (IgA₁) and α₂ (IgA₂). The light chain of an immunoglobulin may be assigned to one of two types, called kappa (κ) and lambda (λ), based on the amino acid sequence of its constant domain. An immunoglobulin essentially consists of two Fab molecules and an Fc domain, linked via the immunoglobulin hinge region.

The term "antigen binding domain" refers to the part of an antibody that comprises the area which specifically binds to and is complementary to part or all of an antigen. An antigen binding domain may be provided by, for example, one or more antibody variable domains (also called antibody variable regions). Preferably, an antigen binding domain comprises an antibody light chain variable region (VL) and an antibody heavy chain variable region (VH).

The term "variable region" or "variable domain" refers to the domain of an antibody heavy or light chain that is involved in binding the antibody to antigen. The variable domains of the heavy chain and light chain (VH and VL, respectively) of a native antibody generally have similar structures, with each domain comprising four conserved framework regions (FRs) and three hypervariable regions (HVRs). See, e.g., Kindt et al., Kuby Immunology, 6th ed., W.H. Freeman and Co., page 91 (2007). A single VH or VL domain may be sufficient to confer antigen binding specificity.

A "human antibody" is one which possesses an amino acid sequence which corresponds to that of an antibody produced by a human or a human cell or derived from a non-human source that utilizes human antibody repertoires or other human antibody-encoding sequences. This definition of a human antibody specifically excludes a humanized antibody comprising non-human antigen-binding residues.

A "humanized" antibody refers to a chimeric antibody comprising amino acid residues from non-human HVRs and amino acid residues from human FRs. In certain embodiments, a humanized antibody will comprise substantially all of at least one, and typically two, variable domains, in which all or substantially all of the HVRs (e.g., CDRs) correspond to those of a non-human antibody, and all or substantially all of the FRs correspond to those of a human antibody. A humanized antibody optionally may comprise at least a portion of an antibody constant region derived from a human antibody. A "humanized form" of an antibody, e.g., a non-human antibody, refers to an antibody that has undergone humanization.

The term "hypervariable region" or "HVR" as used herein refers to each of the regions of an antibody variable domain which are hypervariable in sequence ("complementarity determining regions" or "CDRs") and/or form structurally defined loops ("hypervariable loops") and/or contain the antigen-contacting residues ("antigen contacts"). Generally, antibodies comprise six HVRs: three in the VH (H1, H2, H3), and three in the VL (L1, L2, L3). Exemplary HVRs herein include:
(a) hypervariable loops occurring at amino acid residues 26-32 (L1), 50-52 (L2), 91-96 (L3), 26-32 (H1), 53-55 (H2), and 96-101 (H3) (Chothia and Lesk, J. Mol. Biol. 196:901-917 (1987));
(b) CDRs occurring at amino acid residues 24-34 (L1), 50-56 (L2), 89-97 (L3), 31-35b (H1), 50-65 (H2), and 95-102 (H3) (Kabat et al., Sequences of Proteins of Immunological Interest, 5th Ed. Public Health Service, National Institutes of Health, Bethesda, MD (1991));
(c) antigen contacts occurring at amino acid residues 27c-36 (L1), 46-55 (L2), 89-96 (L3), 30-35b (H1), 47-58 (H2), and 93-101 (H3) (MacCallum et al. J. Mol. Biol. 262: 732-745 (1996)); and
(d) combinations of (a), (b), and/or (c), including HVR amino acid residues 46-56 (L2), 47-56 (L2), 48-56 (L2), 49-56 (L2), 26-35 (H1), 26-35b (H1), 49-65 (H2), 93-102 (H3), and 94-102 (H3).

Unless otherwise indicated, HVR residues and other residues in the variable domain (e.g., FR residues) are numbered herein according to Kabat et al., *supra.*

"Framework" or "FR" refers to variable domain residues other than hypervariable region (HVR) residues. The FR of a variable domain generally consists of four FR domains: FR1, FR2, FR3, and FR4. Accordingly, the HVR and FR sequences generally appear in the following sequence in VH (or VL): FR1-H1(L1)-FR2-H2(L2)-FR3-H3(L3)-FR4.

The "class" of an antibody refers to the type of constant domain or constant region possessed by its heavy chain. There are five major classes of antibodies: IgA, IgD, IgE, IgG, and IgM, and several of these may be further divided into subclasses (isotypes), e.g., IgG1, IgG₂, IgG₃, IgG₄, IgA₁, and IgA₂. The heavy chain constant domains that correspond to the different classes of immunoglobulins are called α, δ, ε, γ, and µ, respectively.

The term "Fc domain" or "Fc region" herein is used to define a C-terminal region of an immunoglobulin heavy chain that contains at least a portion of the constant region. The term includes native sequence Fc regions and variant Fc regions. Although the boundaries of the Fc region of an IgG heavy chain might vary slightly, the human IgG heavy chain Fc region is usually defined to extend from Cys226, or from Pro230, to the carboxyl-terminus of the heavy chain. However, antibodies produced by host cells may undergo post-translational cleavage of one or more, particularly one or two, amino acids from the C-terminus of the heavy chain. Therefore an antibody produced by a host cell by expression of a specific nucleic acid molecule encoding a full-length heavy chain may include the full-length heavy chain, or it may include a cleaved variant of the full-length heavy chain (also referred to herein as a "cleaved variant heavy chain"). This may be the case where the final two C-terminal amino acids of the heavy chain are glycine (G446) and lysine (K447, numbering according to Kabat EU index). Therefore, the C-terminal lysine (Lys447), or the C-terminal glycine (Gly446) and lysine (K447), of the Fc region may or may not be present. Unless otherwise specified herein, numbering of amino acid residues in the Fc region or constant region is according to the EU numbering system, also called the EU index, as described in Kabat et al., Sequences of Proteins of Immunological Interest, 5th Ed. Public Health Service, National Institutes of Health, Bethesda, MD, 1991 (see also above). A "subunit" of an Fc domain as used herein refers to one of the two polypeptides forming the dimeric Fc domain, i.e. a polypeptide comprising C-terminal constant regions of an immunoglobulin heavy chain, capable of stable self-association. For example, a subunit of an IgG Fc domain comprises an IgG CH2 and an IgG CH3 constant domain.

A "modification promoting the association of the first and the second subunit of the Fc domain" is a manipulation of the peptide backbone or the post-translational modifications of an Fc domain subunit that reduces or prevents the association of a polypeptide comprising the Fc domain subunit with an identical polypeptide to form a homodimer. A modification promoting association as used herein particularly includes separate modifications made to each of the two Fc domain subunits desired to associate (i.e. the first and the second subunit of the Fc domain), wherein the modifications are complementary to each other so as to promote association of the two Fc domain subunits. For example, a modification promoting association may alter the structure or charge of one or both of the Fc domain subunits so as to make their association sterically or electrostatically favorable, respectively. Thus, (hetero)dimerization occurs between a polypeptide comprising the first Fc domain subunit and a polypeptide comprising the second Fc domain subunit, which might be non-identical in the sense that further components fused to each of the subunits (e.g. antigen binding moieties) are not the same. In some embodiments the modification promoting association comprises an amino acid mutation in the Fc domain, specifically an amino acid substitution. In a particular embodiment, the modification promoting association comprises a separate amino acid mutation, specifically an amino acid substitution, in each of the two subunits of the Fc domain.

An "activating Fc receptor" is an Fc receptor that following engagement by an Fc region of an antibody elicits signaling events that stimulate the receptor-bearing cell to perform effector functions. Activating Fc receptors include FcγRIIIa (CD16a), FcγRI (CD64), FcγRIIa (CD32), and FcαRI (CD89).

The term "effector functions" when used in reference to antibodies refer to those biological activities attributable to the Fc region of an antibody, which vary with the antibody isotype. Examples of antibody effector functions include: C1q binding and complement dependent cytotoxicity (CDC), Fc receptor binding, antibody-dependent cell-mediated cytotoxicity (ADCC), antibody-dependent cellular phagocytosis (ADCP), cytokine secretion, immune complex-mediated antigen uptake by antigen presenting cells, down regulation of cell surface receptors (e.g. B cell receptor), and B cell activation.

As used herein, the term "effector cells" refers to a population of lymphocytes that display effector moiety receptors, e.g. cytokine receptors, and/or Fc receptors on their surface through which they bind an effector moiety, e.g. a cytokine, and/or an Fc region of an antibody and contribute to the destruction of target cells, e.g. tumor cells. Effector cells may for example mediate cytotoxic or phagocytic effects. Effector cells include, but are not limited to, effector T cells such as CD8⁺cytotoxic T cells, CD4⁺ helper T cells, γδ T cells, NK cells, lymphokine-activated killer (LAK) cells and macrophages/monocytes.

As used herein, the terms "engineer, engineered, engineering," are considered to include any manipulation of the peptide backbone or the post-translational modifications of a naturally occurring or recombinant polypeptide or fragment thereof. Engineering includes modifications of the amino acid sequence, of the glycosylation pattern, or of the side chain group of individual amino acids, as well as combinations of these approaches. "Engineering", particularly with the prefix "glyco-", as well as the term "glycosylation engineering" includes metabolic engineering of the glycosylation machinery of a cell, including genetic manipulations of the oligosaccharide synthesis pathways to achieve altered glycosylation of glycoproteins expressed in cells. Furthermore, glycosylation engineering includes the effects of mutations and cell environment on glycosylation. In one embodiment, the glycosylation engineering is an alteration in glycosyltransferase activity. In a particular embodiment, the engineering results in altered glucosaminyltransferase activity and/or fucosyltransferase activity. Glycosylation engineering can be used to obtain a "host cell having increased GnTIII activity" (e.g. a host cell that has been manipulated to express increased levels of one or more polypeptides having β(1,4)-N-acetylglucosaminyltransferase III (GnTIII) activity), a "host cell having increased ManII activity" (e.g. a host cell that has been manipulated to express increased levels of one or more polypeptides having α-mannosidase II (ManII) activity), or a "host cell having decreased α(1,6) fucosyltransferase activity" (e.g. a host cell that has been manipulated to express decreased levels of α(1,6) fucosyltransferase).

The terms "host cell," "host cell line," and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell, but may contain mutations. Mutant progeny that have the same function or biological activity as screened or selected for in the originally transformed cell are included herein. A host cell is any type of cellular system that can be used to generate proteins used for the present invention. In one embodiment, the host cell is engineered to allow the production of an antibody with modified oligosaccharides. In certain embodiments, the host cells have been manipulated to express increased levels of one or more polypeptides having β(1,4)-N-acetylglucosaminyltransferase III (GnTIII) activity. In certain embodiments the host cells have been further manipulated to express increased levels of one or more polypeptides having α-mannosidase II (ManII) activity. Host cells include cultured cells, e.g. mammalian cultured cells, such as CHO cells, BHK cells, NS0 cells, SP2/0 cells, YO myeloma cells, P3X63 mouse myeloma cells, PER cells, PER.C6 cells or hybridoma cells, yeast cells, insect cells, and plant cells, to name only a few, but also cells comprised within a transgenic animal, transgenic plant or cultured plant or animal tissue.

As used herein, the term "polypeptide having GnTIII activity" refers to polypeptides that are able to catalyze the addition of a N-acetylglucosamine (GlcNAc) residue in β-1,4 linkage to the β-linked mannoside of the trimannosyl core of N-linked oligosaccharides. This includes fusion polypeptides exhibiting enzymatic activity similar to, but not necessarily identical to, an activity of β(1,4)-N-acetylglucosaminyltransferase III, also known as β-1,4-mannosyl-glycoprotein 4-beta-N-acetylglucosaminyl-transferase (EC 2.4.1.144), according to the Nomenclature Committee of the International Union of Biochemistry and Molecular Biology (NC-IUBMB), as measured in a particular biological assay, with or without dose dependency. In the case where dose dependency does exist, it need not be identical to that of GnTIII, but rather substantially similar to the dose-dependency in a given activity as compared to the GnTIII (i.e. the candidate polypeptide will exhibit greater activity or not more than about 25-fold less and, preferably, not more than about ten-fold less activity, and most preferably, not more than about three-fold less activity relative to the GnTIII). In certain embodiments the polypeptide having GnTIII activity is a fusion polypeptide comprising the catalytic domain of GnTIII and the Golgi localization domain of a heterologous Golgi resident polypeptide. Particularly, the Golgi localization domain is the localization domain of mannosidase II or GnTI, most particularly the localization domain of mannosidase II. Alternatively, the Golgi localization domain is selected from the group consisting of: the localization domain of mannosidase I, the localization domain of GnTII, and the localization domain of α1,6 core fucosyltransferase. Methods for generating such fusion polypeptides and using them to produce antibodies with increased effector functions are disclosed in WO2004/065540, U.S. Provisional Pat. Appl. No. 60/495,142 and U.S. Pat. Appl. Publ. No. 2004/0241817, the entire contents of which are expressly incorporated herein by reference.

As used herein, the term "Golgi localization domain" refers to the amino acid sequence of a Golgi resident polypeptide which is responsible for anchoring the polypeptide to a location within the Golgi complex. Generally, localization domains comprise amino terminal "tails" of an enzyme.

As used herein, the term "polypeptide having ManII activity" refers to polypeptides that are able to catalyze the hydrolysis of the terminal 1,3- and 1,6-linked α-D-mannose residues in the branched GlcNAcMan₅GlcNAc₂ mannose intermediate of N-linked oligosaccharides. This includes polypeptides exhibiting enzymatic activity similar to, but not necessarily identical to, an activity of Golgi α-mannosidase II, also known as mannosyl oligosaccharide 1,3-1,6-α-mannosidase II (EC 3.2.1.114), according to the Nomenclature Committee of the International Union of Biochemistry and Molecular Biology (NC-IUBMB).

Antibody-dependent cell-mediated cytotoxicity (ADCC) is an immune mechanism leading to the lysis of antibody-coated target cells by immune effector cells. The target cells are cells to which antibodies or fragments thereof comprising an Fc region specifically bind, generally via the protein part that is N-terminal to the Fc region. As used herein, the term "increased/reduced ADCC" is defined as either an increase/reduction in the number of target cells that are lysed in a given time, at a given concentration of antibody in the medium surrounding the target cells, by the mechanism of ADCC defined above, and/or a reduction/increase in the concentration of antibody, in the medium surrounding the target cells, required to achieve the lysis of a given number of target cells in a given time, by the mechanism of ADCC. The increase/reduction in ADCC is relative to the ADCC mediated by the same antibody produced by the same type of host cells, using the same standard production, purification, formulation and storage methods (which are known to those skilled in the art), but that has not been engineered. For example the increase in ADCC mediated by an antibody produced by host cells engineered to have an altered pattern of glycosylation (e.g. to express the glycosyltransferase, GnTIII, or other glycosyltransferases) by the methods described herein, is relative to the ADCC mediated by the same antibody produced by the same type of non-engineered host cells.

By "antibody having increased/reduced antibody dependent cell-mediated cytotoxicity (ADCC)" is meant an antibody having increased/reducedADCC as determined by any suitable method known to those of ordinary skill in the art. One accepted *in vitro* ADCC assay is as follows:
1) the assay uses target cells that are known to express the target antigen recognized by the antigen-binding region of the antibody;
2) the assay uses human peripheral blood mononuclear cells (PBMCs), isolated from blood of a randomly chosen healthy donor, as effector cells;
3) the assay is carried out according to following protocol:
   i) the PBMCs are isolated using standard density centrifugation procedures and are suspended at 5 x 10⁶ cells/ml in RPMI cell culture medium;
   ii) the target cells are grown by standard tissue culture methods, harvested from the exponential growth phase with a viability higher than 90%, washed in RPMI cell culture medium, labeled with 100 micro-Curies of ⁵¹Cr, washed twice with cell culture medium, and resuspended in cell culture medium at a density of 10⁵ cells/ml;
   iii) 100 microliters of the final target cell suspension above are transferred to each well of a 96-well microtiter plate;
   iv) the antibody is serially-diluted from 4000 ng/ml to 0.04 ng/ml in cell culture medium and 50 microliters of the resulting antibody solutions are added to the target cells in the 96-well microtiter plate, testing in triplicate various antibody concentrations covering the whole concentration range above;
   v) for the maximum release (MR) controls, 3 additional wells in the plate containing the labeled target cells, receive 50 microliters of a 2% (V/V) aqueous solution of non-ionic detergent (Nonidet, Sigma, St. Louis), instead of the antibody solution (point iv above);
   vi) for the spontaneous release (SR) controls, 3 additional wells in the plate containing the labeled target cells, receive 50 microliters of RPMI cell culture medium instead of the antibody solution (point iv above);
   vii) the 96-well microtiter plate is then centrifuged at 50 x g for 1 minute and incubated for 1 hour at 4°C;
   viii) 50 microliters of the PBMC suspension (point i above) are added to each well to yield an effector:target cell ratio of 25:1 and the plates are placed in an incubator under 5% CO₂ atmosphere at 37°C for 4 hours;
   ix) the cell-free supernatant from each well is harvested and the experimentally released radioactivity (ER) is quantified using a gamma counter;
   x) the percentage of specific lysis is calculated for each antibody concentration according to the formula (ER-MR)/(MR-SR) x 100, where ER is the average radioactivity quantified (see point ix above) for that antibody concentration, MR is the average radioactivity quantified (see point ix above) for the MR controls (see point v above), and SR is the average radioactivity quantified (see point ix above) for the SR controls (see point vi above);
4) "increased/reduced ADCC" is defined as either an increase/reduction in the maximum percentage of specific lysis observed within the antibody concentration range tested above, and/or a reduction/increase in the concentration of antibody required to achieve one half of the maximum percentage of specific lysis observed within the antibody concentration range tested above. The increase/reduction in ADCC is relative to the ADCC, measured with the above assay, mediated by the same antibody, produced by the same type of host cells, using the same standard production, purification, formulation and storage methods, which are known to those skilled in the art, but that has not been engineered.

The term "monoclonal antibody" as used herein refers to an antibody obtained from a population of substantially homogeneous antibodies, i.e., the individual antibodies comprising the population are identical and/or bind the same epitope, except for possible variant antibodies, e.g., containing naturally occurring mutations or arising during production of a monoclonal antibody preparation, such variants generally being present in minor amounts. In contrast to polyclonal antibody preparations, which typically include different antibodies directed against different determinants (epitopes), each monoclonal antibody of a monoclonal antibody preparation is directed against a single determinant on an antigen. Thus, the modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by a variety of techniques, including but not limited to the hybridoma method, recombinant DNA methods, phage-display methods, and methods utilizing transgenic animals containing all or part of the human immunoglobulin loci, such methods and other exemplary methods for making monoclonal antibodies being described herein.

As used herein, the terms "first", "second", "third" etc. with respect to antigen binding moieties etc., are used for convenience of distinguishing when there is more than one of each type of moiety. Use of these terms is not intended to confer a specific order or orientation unless explicitly so stated.

The terms "multispecific" and "bispecific" mean that the antigen binding molecule is able to specifically bind to at least two distinct antigenic determinants. Typically, a bispecific antigen binding molecule comprises two antigen binding sites, each of which is specific for a different antigenic determinant. In certain embodiments a bispecific antigen binding molecule is capable of simultaneously binding two antigenic determinants, particularly two antigenic determinants expressed on two distinct cells.

The term "valent" as used herein denotes the presence of a specified number of antigen binding sites in an antigen binding molecule. As such, the term "monovalent binding to an antigen" denotes the presence of one (and not more than one) antigen binding site specific for the antigen in the antigen binding molecule.

An "antigen binding site" refers to the site, i.e. one or more amino acid residues, of an antigen binding molecule which provides interaction with the antigen. For example, the antigen binding site of an antibody comprises amino acid residues from the complementarity determining regions (CDRs). A native immunoglobulin molecule typically has two antigen binding sites, a Fab molecule typically has a single antigen binding site.

A "T cell activating therapeutic agent" as used herein refers to a therapeutic agent capable of inducing T cell activation in a subject, particularly a therapeutic agent designed for inducing T-cell activation in a subject. Examples of T cell activating therapeutic agents include bispecific antibodies that specifically bind an activating T cell antigen, such as CD3, and a target cell antigen, such as CD20 or CD19. Further examples include chimeric antigen receptors (CARs) which comprise a T cell activating domain and an antigen binding moiety that specifically binds to a target cell antigen, such as CD20 or CD 19.

An "activating T cell antigen" as used herein refers to an antigenic determinant expressed by a T lymphocyte, particularly a cytotoxic T lymphocyte, which is capable of inducing or enhancing T cell activation upon interaction with an antigen binding molecule. Specifically, interaction of an antigen binding molecule with an activating T cell antigen may induce T cell activation by triggering the signaling cascade of the T cell receptor complex. An exemplary activating T cell antigen is CD3. In a particular embodiment the activating T cell antigen is CD3, particularly the epsilon subunit of CD3 (see UniProt no. P07766 (version 130), NCBI RefSeq no. NP_000724.1, SEQ ID NO: 105 for the human sequence; or UniProt no. Q95LI5 (version 49), NCBI GenBank no. BAB71849.1, SEQ ID NO: 106 for the cynomolgus [Macaca fascicularis] sequence).

"T cell activation" as used herein refers to one or more cellular response of a T lymphocyte, particularly a cytotoxic T lymphocyte, selected from: proliferation, differentiation, cytokine secretion, cytotoxic effector molecule release, cytotoxic activity, and expression of activation markers. The T cell activating therapeutic agents used in the present invention are capable of inducing T cell activation. Suitable assays to measure T cell activation are known in the art described herein.

A "target cell antigen" as used herein refers to an antigenic determinant presented on the surface of a target cell, for example a cell in a tumor such as a cancer cell or a cell of the tumor stroma. In a particular embodiment, the target cell antigen is CD20, particularly human CD20 (see UniProt no. P11836).

A "B-cell antigen" as used herein refers to an antigenic determinant presented on the surface of a B lymphocyte, particularly a malignant B lymphocyte (in that case the antigen also being referred to as "malignant B-cell antigen").

A "T-cell antigen" as used herein refers to an antigenic determinant presented on the surface of a T lymphocyte, particularly a cytotoxic T lymphocyte.

A "Fab molecule" refers to a protein consisting of the VH and CH1 domain of the heavy chain (the "Fab heavy chain") and the VL and CL domain of the light chain (the "Fab light chain") of an immunoglobulin.

By "chimeric antigen receptor" or "CAR" is meant a genetically engineered receptor protein comprising an antigen binding moiety, e.g. a single-chain variable fragment (scFv) of a targeting antibody, a transmembrane domain, an intracellular T-cell activating signaling domain (e.g. the CD3 zeta chain of the T-cell receptor) and optionally one or more intracellular co-stimulatory domains (e.g. of CD28, CD27, CD137 (4-1BB), Ox40). CARs mediate antigen recognition, T cell activation, and - in the case of second-generation CARs - costimulation to augment T cell functionality and persistence. For a review see e.g. Jackson et al., Nat Rev Clin Oncol (2016) 13, 370-383.

By "B cell proliferative disorder" is meant a disease wherein the number of B cells in a patient is increased as compared to the number of B cells in a healthy subject, and particularly wherein the increase in the number of B cells is the cause or hallmark of the disease. A "CD20-positive B cell proliferative disorder" is a B cell proliferative disorder wherein B-cells, particularly malignant B-cells (in addition to normal B-cells), express CD20.

Exemplary B cell proliferation disorders include Non-Hodgkin lymphoma (NHL), acute lymphocytic leukemia (ALL), chronic lymphocytic leukemia (CLL), diffuse large B-cell lymphoma (DLBCL), follicular lymphoma (FL), mantle-cell lymphoma (MCL), marginal zone lymphoma (MZL), as well as some types of Multiple myeloma (MM) and Hodgkin lymphoma (HL).

By "fused" is meant that the components (e.g. a Fab molecule and an Fc domain subunit) are linked by peptide bonds, either directly or via one or more peptide linkers.

An "effective amount" of an agent refers to the amount that is necessary to result in a physiological change in the cell or tissue to which it is administered.

A "therapeutically effective amount" of an agent, e.g. a pharmaceutical composition, refers to an amount effective, at dosages and for periods of time necessary, to achieve the desired therapeutic or prophylactic result. A therapeutically effective amount of an agent for example eliminates, decreases, delays, minimizes or prevents adverse effects of a disease.

By "therapeutic agent" is meant an active ingredient, e.g. of a pharmaceutical composition, that is administered to a subject in an attempt to alter the natural course of a disease in the subject being treated, and can be performed either for prophylaxis or during the course of clinical pathology. An "immunotherapeutic agent" refers to a therapeutic agent that is administered to a subject in an attempt to restore or enhance the subject's immune response, e.g. to a tumor.

An "individual" or "subject" is a mammal. Mammals include, but are not limited to, domesticated animals (e.g. cows, sheep, cats, dogs, and horses), primates (e.g. humans and non-human primates such as monkeys), rabbits, and rodents (e.g. mice and rats). Preferably, the individual or subject is a human.

The term "pharmaceutical composition" refers to a preparation which is in such form as to permit the biological activity of an active ingredient contained therein to be effective, and which contains no additional components which are unacceptably toxic to a subject to which the composition would be administered.

A "pharmaceutically acceptable carrier" refers to an ingredient in a pharmaceutical composition, other than an active ingredient, which is nontoxic to a subject. A pharmaceutically acceptable carrier includes, but is not limited to, a buffer, excipient, stabilizer, or preservative.

As used herein, "treatment" (and grammatical variations thereof such as "treat" or "treating") refers to clinical intervention in an attempt to alter the natural course of a disease in the individual being treated, and can be performed either for prophylaxis or during the course of clinical pathology. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In some embodiments, methods of the invention are used to delay development of a disease or to slow the progression of a disease.

The term "package insert" or "instructions for use" is used to refer to instructions customarily included in commercial packages of therapeutic products, that contain information about the indications, usage, dosage, administration, combination therapy, contraindications and/or warnings concerning the use of such therapeutic products.

The term "combination treatment" noted herein encompasses combined administration (where two or more therapeutic agents are included in the same or separate formulations), and separate administration, in which case, administration of an antibody as reported herein can occur prior to, simultaneously, and/or following, administration of the additional therapeutic agent or agents, preferably an antibody or antibodies.

"CD3" refers to any native CD3 from any vertebrate source, including mammals such as primates (e.g. humans), non-human primates (e.g. cynomolgus monkeys) and rodents (e.g. mice and rats), unless otherwise indicated. The term encompasses "full-length," unprocessed CD3 as well as any form of CD3 that results from processing in the cell. The term also encompasses naturally occurring variants of CD3, e.g., splice variants or allelic variants. In one embodiment, CD3 is human CD3, particularly the epsilon subunit of human CD3 (CD3ε). The amino acid sequence of human CD3ε is shown in UniProt (www.uniprot.org) accession no. P07766 (version 144), or NCBI (www.ncbi.nlm.nih.gov/) RefSeq NP_000724.1. See also SEQ ID NO: 91. The amino acid sequence of cynomolgus [Macaca fascicularis] CD3ε is shown in NCBI GenBank no. BAB71849.1. See also SEQ ID NO: 92.

"CD19" refers to B-lymphocyte antigen CD19, also known as B-lymphocyte surface antigen B4 or T-cell surface antigen Leu-12 and includes any native CD19 from any vertebrate source, including mammals such as primates (e.g. humans) and rodents (e.g., mice and rats), unless otherwise indicated. The term encompasses "full-length," unprocessed CD19 as well as any form of CD 19 that results from processing in the cell. The term also encompasses naturally occurring variants of CD19, e.g., splice variants or allelic variants. In one embodiment, CD 19 is human CD 19. The amino acid sequence of an exemplary human CD 19 is shown in UniProt (www.uniprot.org) accession no. P15391 (version 174), or NCBI (www.ncbi.nlm.nih.gov/) RefSeq NP_001770.5, and SEQ ID NO: 93.

By a "crossover" Fab molecule (also termed "Crossfab") is meant a Fab molecule wherein the variable domains or the constant domains of the Fab heavy and light chain are exchanged (i.e. replaced by each other), i.e. the crossover Fab molecule comprises a peptide chain composed of the light chain variable domain VL and the heavy chain constant domain 1 CH1 (VL-CH1, in N- to C-terminal direction), and a peptide chain composed of the heavy chain variable domain VH and the light chain constant domain CL (VH-CL, in N- to C-terminal direction). For clarity, in a crossover Fab molecule wherein the variable domains of the Fab light chain and the Fab heavy chain are exchanged, the peptide chain comprising the heavy chain constant domain 1 CH1 is referred to herein as the "heavy chain" of the (crossover) Fab molecule. Conversely, in a crossover Fab molecule wherein the constant domains of the Fab light chain and the Fab heavy chain are exchanged, the peptide chain comprising the heavy chain variable domain VH is referred to herein as the "heavy chain" of the (crossover) Fab molecule.

In contrast thereto, by a "conventional" Fab molecule is meant a Fab molecule in its natural format, i.e. comprising a heavy chain composed of the heavy chain variable and constant domains (VH-CH1, in N- to C-terminal direction), and a light chain composed of the light chain variable and constant domains (VL-CL, in N- to C-terminal direction).

The term "polynucleotide" refers to an isolated nucleic acid molecule or construct, e.g. messenger RNA (mRNA), virally-derived RNA, or plasmid DNA (pDNA). A polynucleotide may comprise a conventional phosphodiester bond or a non-conventional bond (e.g. an amide bond, such as found in peptide nucleic acids (PNA). The term "nucleic acid molecule" refers to any one or more nucleic acid segments, e.g. DNA or RNA fragments, present in a polynucleotide.

By "isolated" nucleic acid molecule or polynucleotide is intended a nucleic acid molecule, DNA or RNA, which has been removed from its native environment. For example, a recombinant polynucleotide encoding a polypeptide contained in a vector is considered isolated for the purposes of the present invention. Further examples of an isolated polynucleotide include recombinant polynucleotides maintained in heterologous host cells or purified (partially or substantially) polynucleotides in solution. An isolated polynucleotide includes a polynucleotide molecule contained in cells that ordinarily contain the polynucleotide molecule, but the polynucleotide molecule is present extrachromosomally or at a chromosomal location that is different from its natural chromosomal location. Isolated RNA molecules include in vivo or in vitro RNA transcripts of the present invention, as well as positive and negative strand forms, and double-stranded forms. Isolated polynucleotides or nucleic acids according to the present invention further include such molecules produced synthetically. In addition, a polynucleotide or a nucleic acid may be or may include a regulatory element such as a promoter, ribosome binding site, or a transcription terminator.

By a nucleic acid or polynucleotide having a nucleotide sequence at least, for example, 95% "identical" to a reference nucleotide sequence of the present invention, it is intended that the nucleotide sequence of the polynucleotide is identical to the reference sequence except that the polynucleotide sequence may include up to five point mutations per each 100 nucleotides of the reference nucleotide sequence. In other words, to obtain a polynucleotide having a nucleotide sequence at least 95% identical to a reference nucleotide sequence, up to 5% of the nucleotides in the reference sequence may be deleted or substituted with another nucleotide, or a number of nucleotides up to 5% of the total nucleotides in the reference sequence may be inserted into the reference sequence. These alterations of the reference sequence may occur at the 5' or 3' terminal positions of the reference nucleotide sequence or anywhere between those terminal positions, interspersed either individually among residues in the reference sequence or in one or more contiguous groups within the reference sequence. As a practical matter, whether any particular polynucleotide sequence is at least 80%, 85%, 90%, 95%, 96%, 97%, 98% or 99% identical to a nucleotide sequence of the present invention can be determined conventionally using known computer programs, such as the ones discussed above for polypeptides (e.g. ALIGN-2).

The term "expression cassette" refers to a polynucleotide generated recombinantly or synthetically, with a series of specified nucleic acid elements that permit transcription of a particular nucleic acid in a target cell. The recombinant expression cassette can be incorporated into a plasmid, chromosome, mitochondrial DNA, plastid DNA, virus, or nucleic acid fragment. Typically, the recombinant expression cassette portion of an expression vector includes, among other sequences, a nucleic acid sequence to be transcribed and a promoter. In certain embodiments, the expression cassette of the invention comprises polynucleotide sequences that encode bispecific antigen binding molecules of the invention or fragments thereof.

The term "vector" or "expression vector" is synonymous with "expression construct" and refers to a DNA molecule that is used to introduce and direct the expression of a specific gene to which it is operably associated in a target cell. The term includes the vector as a self-replicating nucleic acid structure as well as the vector incorporated into the genome of a host cell into which it has been introduced. The expression vector of the present invention comprises an expression cassette. Expression vectors allow transcription of large amounts of stable mRNA. Once the expression vector is inside the target cell, the ribonucleic acid molecule or protein that is encoded by the gene is produced by the cellular transcription and/or translation machinery. In one embodiment, the expression vector of the invention comprises an expression cassette that comprises polynucleotide sequences that encode bispecific antigen binding molecules of the invention or fragments thereof.

### Type II anti-CD20 antibodies

The CD20 molecule (also called human B-lymphocyte-restricted differentiation antigen or Bp35) is a hydrophobic transmembrane protein expressed on the surface of malignant and non-malignant pre-B and mature B lymphocytes that has been described extensively (Valentine, M.A., et al., J. Biol. Chem. 264 (1989) 11282-11287; and Einfeld, D.A., et al., EMBO J. 7 (1988) 711-717; Tedder, T.F., et al., Proc. Natl. Acad. Sci. U.S.A. 85 (1988) 208-212; Stamenkovic, I., et al., J. Exp. Med. 167 (1988) 1975-1980; Tedder, T.F., et al., J. Immunol. 142 (1989) 2560-2568).

CD20 is highly expressed by over 90% of B cell non-Hodgkin's lymphomas (NHL) (Anderson, K.C., et al., Blood 63 (1984) 1424-1433) but is not found on hematopoietic stem cells, pro-B cells, normal plasma cells, or other normal tissues (Tedder, T.F., et al., J, Immunol. 135 (1985) 973- 979).

There exist two different types of anti-CD20 antibodies differing significantly in their mode of CD20 binding and biological activities (Cragg, M.S., et al., Blood 103 (2004) 2738-2743; and Cragg, M.S., et al., Blood 101 (2003) 1045-1052). Type I anti-CD20 antibodies primarily utilize complement to kill target cells, while Type II antibodies primarily operate through direct induction of cell death.

Type I and Type II anti-CD20 antibodies and their characteristics are reviewed e.g. in Klein et al., mAbs 5 (2013), 22-33. Type II anti-CD20 antibodies do not localize CD20 to lipid rafts, show low CDC activity, show only about half the binding capacity to B cells as compared to Type I anti-CD20 antibodies, and induce homotypic aggregation and direct cell death. In constrast thereto, Type I antibodies localize CD20 to lipid rafts, show high CDC activity, full binding capacity to B cells, and only weak induction of homotypic aggregation and direct cell death.

Obinutuzumab and tositumumab (CAS number 192391-48) are examples of Type II anti-CD20 antibodies, while rituximab, ofatumumab, veltuzumab, ocaratuzumab, ocrelizumab, PRO131921 and ublituximab are examples of Type I anti-CD20 antibodies.

According to the invention, the anti-CD20 antibody is a Type II anti-CD20 antibody. In one embodiment according to the present invention, the Type II anti-CD20 antibody is capable of reducing the number of B cells in a subject. In one embodiment, the Type II anti-CD20 antibody is an IgG antibody, particularly an IgG1 antibody. In one embodiment, the Type II anti-CD20 antibody is a full-length antibody. In one embodiment, the Type II anti-CD20 antibody comprises an Fc region, particularly an IgG Fc region or, more particularly, an IgG1 Fc region. In one embodiment the Type II anti-CD20 antibody is a humanized B-Ly1 antibody. Particularly, the Type II anti-CD20 antibody is a humanized, IgG-class Type II anti-CD20 antibody, having the binding specificity of the murine B-Ly1 antibody (Poppema and Visser, Biotest Bulletin 3, 131-139 (1987); SEQ ID NOs 2 and 3).

In one embodiment, the Type II anti-CD20 antibody comprises a heavy chain variable region comprising the heavy chain CDR (HCDR) 1 of SEQ ID NO: 4, the HCDR2 of SEQ ID NO: 5, and the HCDR3 of SEQ ID NO: 6; and a light chain variable region comprising the light chain CDR (LCDR) 1 of SEQ ID NO: 7, the LCDR2 of SEQ ID NO: 8 and the LCDR3 of SEQ ID NO: 9. Particularly, the heavy chain variable region framework regions (FRs) FR1, FR2, and FR3 of said Type II anti-CD20 antibody are human FR sequences encoded by the VH1_10 human germ-line sequence, the heavy chain variable region FR4 of said Type II anti-CD20 antibody is a human FR sequence encoded by the JH4 human germ-line sequence, the light chain variable region FRs FR1, FR2, and FR3 of said Type II anti-CD20 antibody are human FR sequences encoded by the VK_2_40 human germ-line sequence, and the light chain variable region FR4 of said Type II anti-CD20 antibody is a human FR sequence encoded by the JK4 human germ-line sequence. In one embodiment, the Type II anti-CD20 antibody comprises the heavy chain variable region sequence of SEQ ID NO: 10 and the light chain variable region sequence of SEQ ID NO: 11.

In a particular embodiment, the Type II anti-CD20 antibody is obinutuzumab (recommended INN, WHO Drug Information, Vol. 26, No. 4, 2012, p. 453). As used herein, obinutuzumab is synonymous for GA101. The tradename is GAZYVA® or GAZYVARO®. This replaces all previous versions (e.g. Vol. 25, No. 1, 2011, p.75-76), and is formerly known as afutuzumab (recommended INN, WHO Drug Information, Vol. 23, No. 2, 2009, p. 176; Vol. 22, No. 2, 2008, p. 124). In one embodiment, the Type II anti-CD20 antibody is tositumomab.

The Type II anti-CD20 antibody useful in the present invention may be engineered to have increased effector function, as compared to a corresponding non-engineered antibody. In one embodiment the antibody engineered to have increased effector function has at least 2-fold, at least 10-fold or even at least 100-fold increased effector function, compared to a corresponding non-engineered antibody. The increased effector function can include, but is not limited to, one or more of the following: increased Fc receptor binding, increased C1q binding and complement dependent cytotoxicity (CDC), increased antibody-dependent cell-mediated cytotoxicity (ADCC), increased antibody-dependent cellular phagocytosis (ADCP), increased cytokine secretion, increased immune complex-mediated antigen uptake by antigen-presenting cells, increased binding to NK cells, increased binding to macrophages, increased binding to monocytes, increased binding to polymorphonuclear cells, increased direct signaling inducing apoptosis, increased crosslinking of target-bound antibodies, increased dendritic cell maturation, or increased T cell priming.

In one embodiment the increased effector function one or more selected from the group of increased Fc receptor binding, increased CDC, increased ADCC, increased ADCP, and increased cytokine secretion. In one embodiment the increased effector function is increased binding to an activating Fc receptor. In one such embodiment the binding affinity to the activating Fc receptor is increased at least 2-fold, particularly at least 10-fold, compared to the binding affinity of a corresponding non-engineered antibody. In a specific embodiment the activating Fc receptor is selected from the group of FcγRIIIa, FcγRI, and FcγRIIa. In one embodiment the activating Fc receptor is FcγRIIIa, particularly human FcγRIIIa. In another embodiment the increased effector function is increased ADCC. In one such embodiment the ADCC is increased at least 10-fold, particularly at least 100-fold, compared to the ADCC mediated by a corresponding non-engineered antibody. In yet another embodiment the increased effector function is increased binding to an activating Fc receptor and increased ADCC.

Increased effector function can be measured by methods known in the art. A suitable assay for measuring ADCC is described herein. Other examples of *in vitro* assays to assess ADCC activity of a molecule of interest are described in U.S. Patent No. 5,500,362; Hellstrom et al. Proc Natl Acad Sci USA 83, 7059-7063 (1986) and Hellstrom et al., Proc Natl Acad Sci USA 82, 1499-1502 (1985); U.S. Patent No. 5,821,337; Bruggemann et al., J Exp Med 166, 1351-1361 (1987). Alternatively, non-radioactive assays methods may be employed (see, for example, ACTI™ non-radioactive cytotoxicity assay for flow cytometry (CellTechnology, Inc. Mountain View, CA); and CytoTox 96^{®} non-radioactive cytotoxicity assay (Promega, Madison, WI)). Useful effector cells for such assays include peripheral blood mononuclear cells (PBMC) and Natural Killer (NK) cells. Alternatively, or additionally, ADCC activity of the molecule of interest may be assessed in vivo, e.g. in a animal model such as that disclosed in Clynes et al., Proc Natl Acad Sci USA 95, 652-656 (1998). Binding to Fc receptors can be easily determined e.g. by ELISA, or by Surface Plasmon Resonance (SPR) using standard instrumentation such as a BIAcore instrument (GE Healthcare), and Fc receptors such as may be obtained by recombinant expression. According to a particular embodiment, binding affinity to an activating Fc receptor is measured by surface plasmon resonance using a BIACORE® T100 machine (GE Healthcare) at 25°C. Alternatively, binding affinity of antibodies for Fc receptors may be evaluated using cell lines known to express particular Fc receptors, such as NK cells expressing FcγIIIa receptor. C1q binding assays may also be carried out to determine whether the antibody is able to bind C1q and hence has CDC activity. See e.g., C1q and C3c binding ELISA in WO 2006/029879 and WO 2005/100402. To assess complement activation, a CDC assay may be performed (see, for example, Gazzano-Santoro et al., J Immunol Methods 202, 163 (1996); Cragg et al., Blood 101, 1045-1052 (2003); and Cragg and Glennie, Blood 103, 2738-2743 (2004)).

Increased effector function may result e.g. from glycoengineering of the Fc region or the introduction of amino acid mutations in the Fc region of the antibody. In one embodiment the anti-CD20 antibody is engineered by introduction of one or more amino acid mutations in the Fc region. In a specific embodiment the amino acid mutations are amino acid substitutions. In an even more specific embodiment the amino acid substitutions are at positions 298, 333, and/or 334 of the Fc region (EU numbering of residues). Further suitable amino acid mutations are described e.g. in Shields et al., J Biol Chem 9(2), 6591-6604 (2001); U.S. Patent No. 6,737,056; WO 2004/063351 and WO 2004/099249. Mutant Fc regions can be prepared by amino acid deletion, substitution, insertion or modification using genetic or chemical methods well known in the art. Genetic methods may include site-specific mutagenesis of the encoding DNA sequence, PCR, gene synthesis, and the like. The correct nucleotide changes can be verified for example by sequencing.

In another embodiment the Type II anti-CD20 antibody is engineered by modification of the glycosylation in the Fc region. In a specific embodiment the Type II anti-CD20 antibody is engineered to have an increased proportion of non-fucosylated oligosaccharides in the Fc region as compared to a non-engineered antibody. An increased proportion of non-fucosylated oligosaccharides in the Fc region of an antibody results in the antibody having increased effector function, in particular increased ADCC.

In a more specific embodiment, at least about 20%, about 25%, about 30%, about 35%, about 40%, about 45%, about 50%, about 55%, about 60%, about 65%, about 70%, about 75%, about 80%, about 85%, about 90%, about 95%, or about 100%, preferably at least about 40%, of the N-linked oligosaccharides in the Fc region of the Type II anti-CD20 antibody are non-fucosylated. In one embodiment, between about 40% and about 80% of the N-linked oligosaccharides in the Fc region of the Type II anti-CD20 antibody are non-fucosylated. In one embodiment, between about 40% and about 60% of the N-linked oligosaccharides in the Fc region of the Type II anti-CD20 antibody are non-fucosylated. The non-fucosylated oligosaccharides may be of the hybrid or complex type.

In another specific embodiment the Type II anti-CD20 antibody is engineered to have an increased proportion of bisected oligosaccharides in the Fc region as compared to a non-engineered antibody. In a more specific embodiment, at least about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100%, preferably at least about 40%, of the N-linked oligosaccharides in the Fc region of the Type II anti-CD20 antibody are bisected. In one embodiment, between about 40% and about 80% of the N-linked oligosaccharides in the Fc region of the anti-CD20 antibody are bisected. In one embodiment, between about 40% and about 60% of the N-linked oligosaccharides in the Fc region of the anti-CD20 antibody are bisected. The bisected oligosaccharides may be of the hybrid or complex type.

In yet another specific embodiment the Type II anti-CD20 antibody is engineered to have an increased proportion of bisected, non-fucosylated oligosaccharides in the Fc region, as compared to a non-engineered antibody. In a more specific embodiment, at least about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, or about 100%, preferably at least about 15%, more preferably at least about 25%, of the N-linked oligosaccharides in the Fc region of the Type II anti-CD20 antibody are bisected, non-fucosylated. The bisected, non-fucosylated oligosaccharides may be of the hybrid or complex type.

The oligosaccharide structures in the antibody Fc region can be analysed by methods well known in the art, e.g. by MALDI TOF mass spectrometry as described in Umana et al., Nat Biotechnol 17, 176-180 (1999) or Ferrara et al., Biotechn Bioeng 93, 851-861 (2006). The percentage of non-fucosylated oligosaccharides is the amount of oligosaccharides lacking fucose residues, relative to all oligosaccharides attached to Asn 297 (e. g. complex, hybrid and high mannose structures) and identified in an N-glycosidase F treated sample by MALDI TOF MS. Asn 297 refers to the asparagine residue located at about position 297 in the Fc region (EU numbering of Fc region residues); however, Asn297 may also be located about ± 3 amino acids upstream or downstream of position 297, i.e., between positions 294 and 300, due to minor sequence variations in antibodies. The percentage of bisected, or bisected non-fucosylated, oligosaccharides is determined analogously.

In one embodiment the Type II anti-CD20 antibody is engineered to have modified glycosylation in the Fc region, as compared to a non-engineered antibody, by producing the antibody in a host cell having altered activity of one or more glycosyltransferase. Glycosyltransferases include β(1,4)-N-acetylglucosaminyltransferase III (GnTIII), β(1,4)-galactosyltransferase (GalT), β(1,2)-N-acetylglucosaminyltransferase I (GnTI), β(1,2)-N-acetylglucosaminyltransferase II (GnTII) and α(1,6)-fucosyltransferase. In a specific embodiment the Type II anti-CD20 antibody is engineered to have an increased proportion of non-fucosylated oligosaccharides in the Fc region, as compared to a non-engineered antibody, by producing the antibody in a host cell having increased β(1,4)-N-acetylglucosaminyltransferase III (GnTIII) activity. In an even more specific embodiment the host cell additionally has increased α-mannosidase II (ManII) activity. The glycoengineering methodology that can be used for engineering antibodies useful for the present invention has been described in greater detail in Umana et al., Nat Biotechnol 17, 176-180 (1999); Ferrara et al., Biotechn Bioeng 93, 851-861 (2006); WO 99/54342 (U.S. Pat. No. 6,602,684; EP 1071700); WO 2004/065540 (U.S. Pat. Appl. Publ. No. 2004/0241817; EP 1587921), WO 03/011878 (U.S. Pat. Appl. Publ. No. 2003/0175884), the entire content of each of which is incorporated herein by reference in its entirety. Antibodies glycoengineered using this methodology are referred to as GlycoMabs herein.

Generally, any type of cultured cell line, including the cell lines discussed herein, can be used to generate cell lines for the production of anti-TNC A2 antibodies with altered glycosylation pattern. Particular cell lines include CHO cells, BHK cells, NS0 cells, SP2/0 cells, YO myeloma cells, P3X63 mouse myeloma cells, PER cells, PER.C6 cells or hybridoma cells, and other mammalian cells. In certain embodiments, the host cells have been manipulated to express increased levels of one or more polypeptides having β(1,4)-N-acetylglucosaminyltransferase III (GnTIII) activity. In certain embodiments the host cells have been further manipulated to express increased levels of one or more polypeptides having α-mannosidase II (ManII) activity. In a specific embodiment, the polypeptide having GnTIII activity is a fusion polypeptide comprising the catalytic domain of GnTIII and the Golgi localization domain of a heterologous Golgi resident polypeptide. Particularly, said Golgi localization domain is the Golgi localization domain of mannosidase II. Methods for generating such fusion polypeptides and using them to produce antibodies with increased effector functions are disclosed in Ferrara et al., Biotechn Bioeng 93, 851-861 (2006) and WO2004/065540, the entire contents of which are expressly incorporated herein by reference.

The host cells which contain the coding sequence of an antibody useful for the invention and/or the coding sequence of polypeptides having glycosyltransferase activity, and which express the biologically active gene products may be identified e.g. by DNA-DNA or DNA-RNA hybridization; the presence or absence of "marker" gene functions; assessing the level of transcription as measured by the expression of the respective mRNA transcripts in the host cell; or detection of the gene product as measured by immunoassay or by its biological activity - methods which are well known in the art. GnTIII or Man II activity can be detected e.g. by employing a lectin which binds to biosynthetis products of GnTIII or ManII, respectively. An example for such a lectin is the E₄-PHA lectin which binds preferentially to oligosaccharides containing bisecting GlcNAc. Biosynthesis products (i.e. specific oligosaccharide structures) of polypeptides having GnTIII or ManII activity can also be detected by mass spectrometric analysis of oligosaccharides released from glycoproteins produced by cells expressing said polypeptides. Alternatively, a functional assay which measures the increased effector function, e.g. increased Fc receptor binding, mediated by antibodies produced by the cells engineered with the polypeptide having GnTIII or ManII activity may be used.

In another embodiment the Type II anti-CD20 antibody is engineered to have an increased proportion of non-fucosylated oligosaccharides in the Fc region, as compared to a non-engineered antibody, by producing the antibody in a host cell having decreased α(1,6)-fucosyltransferase activity. A host cell having decreased α(1,6)-fucosyltransferase activity may be a cell in which the α(1,6)-fucosyltransferase gene has been disrupted or otherwise deactivated, e.g. knocked out (see Yamane-Ohnuki et al., Biotech Bioeng 87, 614 (2004); Kanda et al., Biotechnol Bioeng, 94(4), 680-688 (2006); Niwa et al., J Immunol Methods 306, 151-160 (2006)).

Other examples of cell lines capable of producing defucosylated antibodies include Lec13 CHO cells deficient in protein fucosylation (Ripka et al., Arch Biochem Biophys 249, 533-545 (1986); US Pat. Appl. No. US 2003/0157108; and WO 2004/056312, especially at Example 11). The antibodies useful in the present invention can alternatively be glycoengineered to have reduced fucose residues in the Fc region according to the techniques disclosed in EP 1 176 195 A1, WO 03/084570, WO 03/085119 and U.S. Pat. Appl. Pub. Nos. 2003/0115614, 2004/093621, 2004/110282, 2004/110704, 2004/132140, US Pat. No. 6,946,292 (Kyowa), e.g. by reducing or abolishing the activity of a GDP-fucose transporter protein in the host cells used for antibody production.

Glycoengineered antibodies useful in the invention may also be produced in expression systems that produce modified glycoproteins, such as those taught in WO 03/056914 (GlycoFi, Inc.) or in WO 2004/057002 and WO 2004/024927 (Greenovation).

### T-cell activating therapeutic agents

The present invention is useful in connection with various therapeutic agents, particularly with therapeutic agents that are activating T-cells in the subject, i.e. have the ability of inducing T-cell activation in the subject. Such therapeutic agents include, for example, antibodies directed to T-cell antigens (particularly activating T-cell antigens), or T-cells modified with chimeric antigen receptors (CAR) or recombinant T-cell receptors (TCR). The invention is particularly useful in connection with B-cell targeted T-cell activating therapeutic agents.

In one embodiment, the therapeutic agent induces cytokine release in a subject when administered to the subject in a treatment regimen without the administration of a Type II anti-CD20 antibody.

In one embodiment, the therapeutic agent is a biologic agent. In one embodiment, the therapeutic agent comprises a polypeptide, particularly a recombinant polypeptide. In one embodiment, the therapeutic agent comprises a polypeptide that does not naturally occur in the subject. In one embodiment, the therapeutic agent to be systemically administered. In one embodiment, the therapeutic agent is to be administered by infusion, particulary intravenous infusion.

In one embodiment, the therapeutic agent comprises an antigen binding polypeptide. In one embodiment, the therapeutic agent comprises a polypeptide selected from the group of an antibody, an antibody fragment, an antigen receptor or an antigen-binding fragment thereof, and a receptor ligand or a receptor-binding fragment thereof. In one embodiment, the therapeutic agent comprises an antibody. In one embodiment, the antibody is a monoclonal antibody. In one embodiment, the antibody is a polyclonal antibody. In one embodiment the antibody is a human antibody. In one embodiment, the antibody is humanized antibody. In one embodiment the antibody is a chimeric antibody. In one embodiment the antibody is full-length antibody. In one embodiment the antibody is an IgG-class antibody, particularly an IgG1 subclass antibody. In one embodiment, the antibody is a recombinant antibody.

In certain embodiments, the therapeutic agent comprises an antibody fragment. Antibody fragments include, but are not limited to, Fab, Fab', Fab'-SH, F(ab')₂, Fv, and scFv fragments, and other fragments described below. For a review of certain antibody fragments, see Hudson et al. Nat. Med. 9:129-134 (2003). For a review of scFv fragments, see, e.g., Plückthun, in The Pharmacology of Monoclonal Antibodies, vol. 113, Rosenburg and Moore eds., (Springer-Verlag, New York), pp. 269-315 (1994); see also WO 93/16185; and U.S. Patent Nos. 5,571,894 and 5,587,458. For discussion of Fab and F(ab')₂ fragments comprising salvage receptor binding epitope residues and having increased in vivo half-life, see U.S. Patent No. 5,869,046. In one embodiment, the antibody fragment is a Fab fragment or a scFv fragment.

Diabodies are antibody fragments with two antigen-binding sites that may be bivalent or bispecific. See, for example, EP 404,097; WO 1993/01161; Hudson et al., Nat. Med. 9:129-134 (2003); and Hollinger et al., Proc. Natl. Acad. Sci. USA 90: 6444-6448 (1993). Triabodies and tetrabodies are also described in Hudson et al., Nat. Med. 9:129-134 (2003).

Single-domain antibodies are antibody fragments comprising all or a portion of the heavy chain variable domain or all or a portion of the light chain variable domain of an antibody. In certain embodiments, a single-domain antibody is a human single-domain antibody (Domantis, Inc., Waltham, MA; *see,* e.g., U.S. Patent No. 6,248,516 B1).

Antibody fragments can be made by various techniques, including but not limited to proteolytic digestion of an intact antibody as well as production by recombinant host cells (e.g. *E. coli* or phage), as described herein.

In certain embodiments, the therapeutic agent comprises a chimeric antibody. Certain chimeric antibodies are described, e.g., in U.S. Patent No. 4,816,567; and Morrison et al., Proc. Natl. Acad. Sci. USA, 81:6851-6855 (1984)). In one example, a chimeric antibody comprises a non-human variable region (e.g., a variable region derived from a mouse, rat, hamster, rabbit, or non-human primate, such as a monkey) and a human constant region. In a further example, a chimeric antibody is a "class switched" antibody in which the class or subclass has been changed from that of the parent antibody. Chimeric antibodies include antigen-binding fragments thereof.

In certain embodiments, the therapeutic agent comprises a humanized antibody. Typically, a non-human antibody is humanized to reduce immunogenicity to humans, while retaining the specificity and affinity of the parental non-human antibody. Generally, a humanized antibody comprises one or more variable domains in which HVRs, e.g., CDRs, (or portions thereof) are derived from a non-human antibody, and FRs (or portions thereof) are derived from human antibody sequences. A humanized antibody optionally will also comprise at least a portion of a human constant region. In some embodiments, some FR residues in a humanized antibody are substituted with corresponding residues from a non-human antibody (e.g., the antibody from which the HVR residues are derived), e.g., to restore or improve antibody specificity or affinity.

Humanized antibodies and methods of making them are reviewed, e.g., in Almagro and Fransson, Front. Biosci. 13:1619-1633 (2008), and are further described, e.g., in Riechmann et al., Nature 332:323-329 (1988); Queen et al., Proc. Nat'l Acad. Sci. USA 86:10029-10033 (1989); US Patent Nos. 5, 821,337, 7,527,791, 6,982,321, and 7,087,409; Kashmiri et al., Methods 36:25-34 (2005) (describing specificity determining region (SDR) grafting); Padlan, Mol. Immunol. 28:489-498 (1991) (describing "resurfacing"); Dall'Acqua et al., Methods 36:43-60 (2005) (describing "FR shuffling"); and Osbourn et al., Methods 36:61-68 (2005) and Klimka et al., Br. J. Cancer, 83:252-260 (2000) (describing the "guided selection" approach to FR shuffling).

Human framework regions that may be used for humanization include but are not limited to: framework regions selected using the "best-fit" method (see, e.g., Sims et al. J. Immunol. 151:2296 (1993)); framework regions derived from the consensus sequence of human antibodies of a particular subgroup of light or heavy chain variable regions (see, e.g., Carter et al. Proc. Natl. Acad. Sci. USA, 89:4285 (1992); and Presta et al. J. Immunol., 151:2623 (1993)); human mature (somatically mutated) framework regions or human germline framework regions (see, e.g., Almagro and Fransson, Front. Biosci. 13:1619-1633 (2008)); and framework regions derived from screening FR libraries (see, e.g., Baca et al., J. Biol. Chem. 272:10678-10684 (1997) and Rosok et al., J. Biol. Chem. 271:22611-22618 (1996)).

In certain embodiments, the therapeutic agent comprises a human antibody. Human antibodies can be produced using various techniques known in the art. Human antibodies are described generally in van Dijk and van de Winkel, Curr. Opin. Pharmacol. 5: 368-74 (2001) and Lonberg, Curr. Opin. Immunol. 20:450-459 (2008).

Human antibodies may be prepared by administering an immunogen to a transgenic animal that has been modified to produce intact human antibodies or intact antibodies with human variable regions in response to antigenic challenge. Such animals typically contain all or a portion of the human immunoglobulin loci, which replace the endogenous immunoglobulin loci, or which are present extrachromosomally or integrated randomly into the animal's chromosomes. In such transgenic mice, the endogenous immunoglobulin loci have generally been inactivated. For review of methods for obtaining human antibodies from transgenic animals, see Lonberg, Nat. Biotech. 23:1117-1125 (2005). See also, e.g., U.S. Patent Nos. 6,075,181 and 6,150,584 describing XENOMOUSE™ technology; U.S. Patent No. 5,770,429 describing HuMAB® technology; U.S. Patent No. 7,041,870 describing K-M MOUSE® technology, and U.S. Patent Application Publication No. US 2007/0061900, describing VELOCIMOUSE® technology). Human variable regions from intact antibodies generated by such animals may be further modified, e.g., by combining with a different human constant region.

Human antibodies can also be made by hybridoma-based methods. Human myeloma and mouse-human heteromyeloma cell lines for the production of human monoclonal antibodies have been described. (See, e.g., Kozbor J. Immunol., 133: 3001 (1984); Brodeur et al., Monoclonal Antibody Production Techniques and Applications, pp. 51-63 (Marcel Dekker, Inc., New York, 1987); and Boerner et al., J. Immunol., 147: 86 (1991).) Human antibodies generated via human B-cell hybridoma technology are also described in Li et al., Proc. Natl. Acad. Sci. USA, 103:3557-3562 (2006). Additional methods include those described, for example, in U.S. Patent No. 7,189,826 (describing production of monoclonal human IgM antibodies from hybridoma cell lines) and Ni, Xiandai Mianyixue, 26(4):265-268 (2006) (describing human-human hybridomas). Human hybridoma technology (Trioma technology) is also described in Vollmers and Brandlein, Histology and Histopathology, 20(3):927-937 (2005) and Vollmers and Brandlein, Methods and Findings in Experimental and Clinical Pharmacology, 27(3):185-91 (2005).

Human antibodies may also be generated by isolating Fv clone variable domain sequences selected from human-derived phage display libraries. Such variable domain sequences may then be combined with a desired human constant domain. Techniques for selecting human antibodies from antibody libraries are described below.

Antibodies comprised in the therapeutic agent may be isolated by screening combinatorial libraries for antibodies with the desired activity or activities. For example, a variety of methods are known in the art for generating phage display libraries and screening such libraries for antibodies possessing the desired binding characteristics. Such methods are reviewed, e.g., in Hoogenboom et al. in Methods in Molecular Biology 178:1-37 (O'Brien et al., ed., Human Press, Totowa, NJ, 2001) and further described, e.g., in the McCafferty et al., Nature 348:552-554; Clackson et al., Nature 352: 624-628 (1991); Marks et al., J. Mol. Biol. 222: 581-597 (1992); Marks and Bradbury, in Methods in Molecular Biology 248:161-175 (Lo, ed., Human Press, Totowa, NJ, 2003); Sidhu et al., J. Mol. Biol. 338(2): 299-310 (2004); Lee et al., J. Mol. Biol. 340(5): 1073-1093 (2004); Fellouse, Proc. Natl. Acad. Sci. USA 101(34): 12467-12472 (2004); and Lee et al., J. Immunol. Methods 284(1-2): 119-132(2004).

In certain phage display methods, repertoires of VH and VL genes are separately cloned by polymerase chain reaction (PCR) and recombined randomly in phage libraries, which can then be screened for antigen-binding phage as described in Winter et al., Ann. Rev. Immunol., 12: 433-455 (1994). Phage typically display antibody fragments, either as single-chain Fv (scFv) fragments or as Fab fragments. Libraries from immunized sources provide high-affinity antibodies to the immunogen without the requirement of constructing hybridomas. Alternatively, the naive repertoire can be cloned (e.g., from human) to provide a single source of antibodies to a wide range of non-self and also self-antigens without any immunization as described by Griffiths et al., EMBO J, 12: 725-734 (1993). Finally, naive libraries can also be made synthetically by cloning unrearranged V-gene segments from stem cells, and using PCR primers containing random sequence to encode the highly variable CDR3 regions and to accomplish rearrangement *in vitro,* as described by Hoogenboom and Winter, J. Mol. Biol., 227: 381-388 (1992). Patent publications describing human antibody phage libraries include, for example: US Patent No. 5,750,373, and US Patent Publication Nos. 2005/0079574, 2005/0119455, 2005/0266000, 2007/0117126, 2007/0160598, 2007/0237764, 2007/0292936, and 2009/0002360.

Antibodies or antibody fragments isolated from human antibody libraries are considered human antibodies or human antibody fragments herein.

In certain embodiments, the therapeutic agent comprises a multispecific antibody, e.g. a bispecific antibody. Multispecific antibodies are monoclonal antibodies that have binding specificities for at least two different sites. In certain embodiments, the binding specificities are for different antigens. In certain embodiments, the binding specificities are for different epitopes on the same antigen. Bispecific antibodies may also be used to localize cytotoxic agents to cells which express an antigen. Bispecific antibodies can be prepared as full length antibodies or antibody fragments.

Techniques for making multispecific antibodies include, but are not limited to, recombinant co-expression of two immunoglobulin heavy chain-light chain pairs having different specificities (see Milstein and Cuello, Nature 305: 537 (1983)), WO 93/08829, and Traunecker et al., EMBO J. 10: 3655 (1991)), and "knob-in-hole" engineering (see, e.g., U.S. Patent No. 5,731,168). Multi-specific antibodies may also be made by engineering electrostatic steering effects for making antibody Fc-heterodimeric molecules (WO 2009/089004A1); cross-linking two or more antibodies or fragments (see, e.g., US Patent No. 4,676,980, and Brennan et al., Science, 229: 81 (1985)); using leucine zippers to produce bi-specific antibodies (see, e.g., Kostelny et al., J. Immunol., 148(5):1547-1553 (1992)); using "diabody" technology for making bispecific antibody fragments (see, e.g., Hollinger et al., Proc. Natl. Acad. Sci. USA, 90:6444-6448 (1993)); and using single-chain Fv (sFv) dimers (see,e.g. Gruber et al., J. Immunol., 152:5368 (1994)); and preparing trispecific antibodies as described, e.g., in Tutt et al. J. Immunol. 147: 60 (1991).

Engineered antibodies with three or more functional antigen binding sites, including "Octopus antibodies," are also included herein (see, e.g. US 2006/0025576A1).

The antibody or fragment herein also includes a "Dual Acting FAb" or "DAF" comprising an antigen binding site that binds to two different antigens (see, US 2008/0069820, for example).

"Crossmab" antibodies are also included herein (see e.g. WO2009080251, WO2009080252, WO2009080253, WO2009080254).

Another technique for making bispecific antibody fragments is the "bispecific T cell engager" or BiTE® approach (see, e.g., WO2004/106381, WO2005/061547, WO2007/042261, and WO2008/119567). This approach utilizes two antibody variable domains arranged on a single polypeptide. For example, a single polypeptide chain includes two single chain Fv (scFv) fragments, each having a variable heavy chain (VH) and a variable light chain (VL) domain separated by a polypeptide linker of a length sufficient to allow intramolecular association between the two domains. This single polypeptide further includes a polypeptide spacer sequence between the two scFv fragments. Each scFv recognizes a different epitope, and these epitopes may be specific for different cell types, such that cells of two different cell types are brought into close proximity or tethered when each scFv is engaged with its cognate epitope. One particular embodiment of this approach includes a scFv recognizing a cell-surface antigen expressed by an immune cell, e.g., a CD3 polypeptide on a T cell, linked to another scFv that recognizes a cell-surface antigen expressed by a target cell, such as a malignant or tumor cell.

As it is a single polypeptide, the bispecific T cell engager may be expressed using any prokaryotic or eukaryotic cell expression system known in the art, e.g., a CHO cell line.

However, specific purification techniques (see, e.g., EP1691833) may be necessary to separate monomeric bispecific T cell engagers from other multimeric species, which may have biological activities other than the intended activity of the monomer. In one exemplary purification scheme, a solution containing secreted polypeptides is first subjected to a metal affinity chromatography, and polypeptides are eluted with a gradient of imidazole concentrations. This eluate is further purified using anion exchange chromatography, and polypeptides are eluted using with a gradient of sodium chloride concentrations. Finally, this eluate is subjected to size exclusion chromatography to separate monomers from multimeric species.

Antibodies with more than two valencies are contemplated. For example, trispecific antibodies can be prepared. Tuft et al. J. Immunol. 147: 60 (1991).

In certain embodiments, an antibody comprised in the therapeutic agent may be further modified to contain additional nonproteinaceous moieties that are known in the art and readily available. The moieties suitable for derivatization of the antibody include but are not limited to water soluble polymers. Non-limiting examples of water soluble polymers include, but are not limited to, polyethylene glycol (PEG), copolymers of ethylene glycol/propylene glycol, carboxymethylcellulose, dextran, polyvinyl alcohol, polyvinyl pyrrolidone, poly-1, 3-dioxolane, poly-1,3,6-trioxane, ethylene/maleic anhydride copolymer, polyaminoacids (either homopolymers or random copolymers), and dextran or poly(n-vinyl pyrrolidone)polyethylene glycol, propropylene glycol homopolymers, prolypropylene oxide/ethylene oxide copolymers, polyoxyethylated polyols (e.g., glycerol), polyvinyl alcohol, and mixtures thereof. Polyethylene glycol propionaldehyde may have advantages in manufacturing due to its stability in water. The polymer may be of any molecular weight, and may be branched or unbranched. The number of polymers attached to the antibody may vary, and if more than one polymer are attached, they can be the same or different molecules. In general, the number and/or type of polymers used for derivatization can be determined based on considerations including, but not limited to, the particular properties or functions of the antibody to be improved, whether the antibody derivative will be used in a therapy under defined conditions, etc.

The therapeutic agent may also comprise an antibody conjugated to one or more cytotoxic agents, such as chemotherapeutic agents or drugs, growth inhibitory agents, toxins (e.g., protein toxins, enzymatically active toxins of bacterial, fungal, plant, or animal origin, or fragments thereof), or radioactive isotopes.

In one embodiment, the therapeutic agent comprises an antibody-drug conjugate (ADC) in which an antibody is conjugated to one or more drugs, including but not limited to a maytansinoid (see U.S. Patent Nos. 5,208,020, 5,416,064 and European Patent EP 0 425 235 B1); an auristatin such as monomethylauristatin drug moieties DE and DF (MMAE and MMAF) (see U.S. Patent Nos. 5,635,483 and 5,780,588, and 7,498,298); a dolastatin; a calicheamicin or derivative thereof (see U.S. Patent Nos. 5,712,374, 5,714,586, 5,739,116, 5,767,285, 5,770,701, 5,770,710, 5,773,001, and 5,877,296; Hinman et al., Cancer Res. 53:3336-3342 (1993); and Lode et al., Cancer Res. 58:2925-2928 (1998)); an anthracycline such as daunomycin or doxorubicin (see Kratz et al., Current Med. Chem. 13:477-523 (2006); Jeffrey et al., Bioorganic & Med. Chem. Letters 16:358-362 (2006); Torgov et al., Bioconj. Chem. 16:717-721 (2005); Nagy et al., Proc. Natl. Acad. Sci. USA 97:829-834 (2000); Dubowchik et al., Bioorg. & Med. Chem. Letters 12:1529-1532 (2002); King et al., J. Med. Chem. 45:4336-4343 (2002); and U.S. Patent No. 6,630,579); methotrexate; vindesine; a taxane such as docetaxel, paclitaxel, larotaxel, tesetaxel, and ortataxel; a trichothecene; and CC1065.

In another embodiment, the therapeutic agent comprises an antibody as described herein conjugated to an enzymatically active toxin or fragment thereof, including but not limited to diphtheria A chain, nonbinding active fragments of diphtheria toxin, exotoxin A chain (from Pseudomonas aeruginosa), ricin A chain, abrin A chain, modeccin A chain, alpha-sarcin, Aleurites fordii proteins, dianthin proteins, Phytolaca americana proteins (PAPI, PAPII, and PAP-S), momordica charantia inhibitor, curcin, crotin, saponaria officinalis inhibitor, gelonin, mitogellin, restrictocin, phenomycin, enomycin, and the tricothecenes.

In another embodiment, the therapeutic agent comprises an antibody as described herein conjugated to a radioactive atom to form a radioconjugate. A variety of radioactive isotopes are available for the production of radioconjugates. Examples include At²¹¹, I¹³¹, I¹²⁵, Y⁹⁰, Re¹⁸⁶, Re¹⁸⁸, Sm¹⁵³, Bi²¹², P³², Pb²¹² and radioactive isotopes of Lu. When the radioconjugate is used for detection, it may comprise a radioactive atom for scintigraphic studies, for example Tc^{99m} or I¹²³, or a spin label for nuclear magnetic resonance (NMR) imaging (also known as magnetic resonance imaging, mri), such as iodine-123 again, iodine-131, indium-111, fluorine-19, carbon-13, nitrogen-15, oxygen-17, gadolinium, manganese or iron.

Conjugates of an antibody and cytotoxic agent may be made using a variety of bifunctional protein coupling agents such as N-succinimidyl-3-(2-pyridyldithio) propionate (SPDP), succinimidyl-4-(N-maleimidomethyl) cyclohexane-1-carboxylate (SMCC), iminothiolane (IT), bifunctional derivatives of imidoesters (such as dimethyl adipimidate HCl), active esters (such as disuccinimidyl suberate), aldehydes (such as glutaraldehyde), bis-azido compounds (such as bis (p-azidobenzoyl) hexanediamine), bis-diazonium derivatives (such as bis-(p-diazoniumbenzoyl)-ethylenediamine), diisocyanates (such as toluene 2,6-diisocyanate), and bis-active fluorine compounds (such as 1,5-difluoro-2,4-dinitrobenzene). For example, a ricin immunotoxin can be prepared as described in Vitetta et al., Science 238:1098 (1987). Carbon-14-labeled 1-isothiocyanatobenzyl-3-methyldiethylene triaminepentaacetic acid (MX-DTPA) is an exemplary chelating agent for conjugation of radionucleotide to the antibody. See WO94/11026. The linker may be a "cleavable linker" facilitating release of a cytotoxic drug in the cell. For example, an acid-labile linker, peptidase-sensitive linker, photolabile linker, dimethyl linker or disulfide-containing linker (Chari et al., Cancer Res. 52:127-131 (1992); U.S. Patent No. 5,208,020) may be used.

In one embodiment, the therapeutic agent comprises an antibody indicated for the treatment of cancer. In one embodiment the therapeutic agent is indicated for the treatment of cancer. In one embodiment, cancer is a B-cell proliferative disorder. In one embodiment, the cancer is a CD20-positive B-cell proliferative disorder. In one embodiment, the cancer is selected from the group consisting of Non-Hodgkin lymphoma (NHL), acute lymphocytic leukemia (ALL), chronic lymphocytic leukemia (CLL), diffuse large B-cell lymphoma (DLBCL), follicular lymphoma (FL), mantle-cell lymphoma (MCL), marginal zone lymphoma (MZL), Multiple myeloma (MM), and Hodgkin lymphoma (HL). In one embodiment, the therapeutic agent is an immunotherapeutic agent.

In some embodiments, the therapeutic agent comprises an antibody that specifically binds to an activating T cell antigen. In one embodiment, the therapeutic antibody may comprise an antibody that specifically binds to an antigen selected from the group of CD3, CD28, CD 137 (also known as 4-1BB), CD40, CD226, OX40, GITR, CD27, HVEM, and CD127.

In one embodiment, the therapeutic agent comprises an antibody that specifically binds to CD3, particularly CD3ε.

In one embodiment, the therapeutic agent comprises an antibody that is or can compete for binding with antibody H2C (PCT publication no. WO2008/119567), antibody V9 (Rodrigues et al., Int J Cancer Suppl 7, 45-50 (1992) and US patent no. 6,054,297), antibody FN18 (Nooij et al., Eur J Immunol 19, 981-984 (1986)), antibody SP34 (Pessano et al., EMBO J 4, 337-340 (1985)), antibody OKT3 (Kung et al., Science 206, 347-349 (1979)), antibody WT31 (Spits et al., J Immunol 135, 1922 (1985)), antibody UCHT1 (Burns et al., J Immunol 129, 1451-1457 (1982)), antibody 7D6 (Coulie et al., Eur J Immunol 21, 1703-1709 (1991)) or antibody Leu-4. In some embodiments, the therapeutic agent may also comprise an antibody that specifically binds to CD3 as described in WO 2005/040220, WO 2005/118635, WO 2007/042261, WO 2008/119567, WO 2008/119565, WO 2012/162067, WO 2013/158856, WO 2013/188693, WO 2013/186613, WO 2014/110601, WO 2014/145806, WO 2014/191113, WO 2014/047231, WO 2015/095392, WO 2015/181098, WO 2015/001085, WO 2015/104346, WO 2015/172800, WO 2016/020444, or WO 2016/014974.

In one embodiment, the therapeutic agent may comprise an antibody that specifically binds to a B-cell antigen, particularly a malignant B-cell antigen. In one embodiment, the therapeutic agent may comprise an antibody that specifically binds to an antigen selected from the group consisting of CD20, CD19, CD22, ROR-1, CD37 and CD5, particularly to CD20 or CD19.

In some embodiments, the therapeutic agent may comprise an antibody selected from rituximab, ocrelizumab, ofatumumab, ocaratuzumab, veltuzumab, and ublituximab.

In some embodiments, the therapeutic agent may comprise a multispecific antibody, particularly a bispecific antibody. In some embodiments, the therapeutic agent may comprise a bispecific antibody capable of binding to a T cell and a target cell, e.g. a tumor cell. In some embodiments, the target cell is a B-cell, particularly a malignant B-cell. In some embodiments, the therapeutic agent may comprise a bispecific antibody that specifically binds to (i) an activating T cell antigen and (ii) a B cell antigen. In some embodiments, the therapeutic agent may comprise a bispecific antibody that specifically binds to CD3 on a T cell and to a target cell antigen. In some embodiments, the target cell antigen is a B-cell antigen, particularly a malignant B-cell antigen. In some embodiments, the therapeutic agent may comprise a bispecific T cell engager (BiTE®).

In some embodiments, the therapeutic agent may comprise a bispecific antibody directed against CD3 and CD20. In one embodiment, the bispecific antibody is XmAb^{®}13676. In one embodiment, the bispecific antibody is REGN1979. In one embodiment, the bispecific antibody is FBTA05 (Lymphomun).

In some embodiments, the therapeutic agent may comprise a bispecific antibody directed against CD3 and CD19. In one embodiment, the bispecific antibody is blinatumomab (BLINCYTO®). In one embodiment, the bispecific antibody is AFM11. In one embodiment, the bispecific antibody is MGD011 (JNJ-64052781).

In some embodiments, the therapeutic agent may comprise a bispecific antibody directed against CD3 and CD38. In one embodiment, the bispecific antibody is XmAb^{®} 13551, XmAb^{®} 15426, or XmAb^{®} 14702.

In some embodiments, the therapeutic agent may comprise a bispecific antibody directed against CD3 and BCMA. In one embodiment, the bispecific antibody is BI836909.

In some embodiments, the therapeutic agent may comprise a bispecific antibody directed against CD3 and CD33. In one embodiment, the bispecific antibody is AMG330.

In some embodiments, the therapeutic agent may comprise a bispecific antibody directed against CD3 and CD123. In one embodiment, the bispecific antibody is MGD006. In one embodiment, the bispecific antibody is XmAb^{®} 14045. In one embodiment, the bispecific antibody is JNJ-63709178.

In some embodiments, the therapeutic agent may comprise a recombinant receptor or a fragment thereof. In some embodiments, the receptor is a T cell receptor (TCR). In some embodiments, the therapeutic agent may comprise a chimeric antigen receptor (CAR).

In some embodiments, the therapeutic agent may comprise a T cell (e.g., a cytotoxic T cell or CTL) expressing a chimeric antigen receptor (CAR). In some embodiments, the therapeutic agent may comprise a T cell expressing a recombinant T cell receptor (TCR).

In one embodiment, the therapeutic agent may comprise a CAR that specifically binds to a B-cell antigen, particularly a malignant B-cell antigen. In one embodiment, the therapeutic agent may comprise a CAR that specifically binds to an antigen selected from the group consisting of CD20, CD19, CD22, ROR-1, CD37 and CD5, particularly to CD20 or CD19.

In some embodiments, the therapeutic agent may comprise a CAR directed to CD19, or a T cell expressing a CAR directed to CD19. In some embodiments, the therapeutic agent may comprise KTE-C19, CTL019, JCAR-014, JCAR-015, JCAR-017, BPX-401, UCART19,

In some embodiments, the therapeutic agent may comprise a CAR directed to CD22, or a T cell expressing a CAR directed to CD22. In some embodiments, the therapeutic agent may comprise JCAR-018 or UCART22.

In some embodiments, the therapeutic agent may comprise an agonist directed against an T cell activating co-stimulatory molecule. In some embodiments, a T cell activating co-stimulatory molecule may include CD40, CD226, CD28, OX40, GITR, CD137, CD27, HVEM, or CD 127. In some embodiments, the agonist directed against a T cell activating co-stimulatory molecule is an agonist antibody that binds to CD40, CD226, CD28, OX40, GITR, CD137, CD27, HVEM, or CD127. In some embodiments, the therapeutic agent may comprise an antibody targeting GITR. In some embodiments, the antibody targeting GITR is TRX518.

In some embodiments, the therapeutic agent may comprise an agonist directed against CD 137 (also known as TNFRSF9, 4-1BB, or ILA), for example, an activating antibody. In some embodiments, the therapeutic agent may comprise urelumab (also known as BMS-663513). In some embodiments, the therapeutic agent may comprise ligand of CD137 (also known as TNFRSF9, 4-1BB, or ILA), such as 4-1BBL. In some embodiments, the therapeutic agent may comprise an agonist directed against CD40, for example, an activating antibody. In some embodiments, the therapeutic agent may comprise CP-870893. In some embodiments, the therapeutic agent may comprise an agonist directed against OX40 (also known as CD 134), for example, an activating antibody. In some embodiments, the therapeutic agent may comprise an anti-OX40 antibody (e.g., AgonOX). In some embodiments, the therapeutic agent may comprise a ligand of OX40, such as OX40L. In some embodiments, the therapeutic agent may comprise an agonist directed against CD27, for example, an activating antibody. In some embodiments, the therapeutic agent may comprise CDX-1127.

In some embodiments, the therapeutic agent may comprise a generic, biosimilar or non-comparable biologic version of an agent, e.g. an antibody, named herein.

In one embodiment, the therapeutic agent does not comprise obinutuzumab.

In some embodiments, the therapeutic agent comprises an antibody that specifically binds to CD3, particularly CD3 epsilon. In one embodiment, the antibody that specifically binds to CD3 comprises a heavy chain variable region comprising the heavy chain CDR (HCDR) 1 of SEQ ID NO: 12, the HCDR2 of SEQ ID NO: 13, and the HCDR3 of SEQ ID NO: 14; and a light chain variable region comprising the light chain CDR (LCDR) 1 of SEQ ID NO: 15, the LCDR2 of SEQ ID NO: 16 and the LCDR3 of SEQ ID NO: 17. In a further embodiment, the antibody that specifically binds CD3 comprises a heavy chain variable region sequence that is at least 80%, 85%, 90%, 95%, 96%, 97%, 98%, or 99% identical to of SEQ ID NO: 18 and a light chain variable region sequence that is at least 80%, 85%, 90%, 95%, 96%, 97%, 98%, or 99% identical to the sequence of SEQ ID NO: 19. In a further embodiment, the antibody that specifically binds CD3 comprises the heavy chain variable region sequence of SEQ ID NO: 18 and the light chain variable region sequence of SEQ ID NO: 19.

In one embodiment, the antibody that specifically binds to CD3 is a full-length antibody. In one embodiment, the antibody that specifically binds to CD3 is an antibody of the human IgG class, particularly an antibody of the human IgG₁ class. In one embodiment, the antibody that specifically binds to CD3 is an antibody fragment, particularly a Fab molecule or a scFv molecule, more particularly a Fab molecule. In a particular embodiment, the antibody that specifically binds to CD3 is a crossover Fab molecule wherein the variable domains or the constant domains of the Fab heavy and light chain are exchanged (i.e. replaced by each other). In one embodiment, the antibody that specifically binds to CD3 is a humanized antibody.

In one embodiment, the therapeutic agent comprises a multispecific antibody, particularly a bispecific antibody. In one embodiment, the multispecific antibody specifically binds to (i) an activating T cell antigen and (ii) a B cell antigen. Particular bispecific antibodies are described in PCT publication no. WO 2016/020309 and European patent application nos. EP15188093 and EP16169160 (each incorporated herein by reference in its entirety).

In one embodiment, the bispecific antibody specifically binds to CD3 and CD20. In one embodiment, the bispecific antibody comprises an antigen binding moiety that specifically binds to CD20, and an antigen binding moiety that specifically binds to CD3. In one embodiment, the bispecific antibody comprises a first antigen binding moiety that specifically binds to CD3, and a second and a third antigen binding moiety that specifically bind to CD20. In one embodiment, the first antigen binding moiety is a crossover Fab molecule, and the second and the first antigen binding moiety are each a conventional Fab molecule. In one embodiment, the bispecific antibody further comprises an Fc domain. The bispecific antibody may comprise modifications in the Fc region and/or the antigen binding moieties as described herein.

In one embodiment, the therapeutic agent comprises a bispecific antibody comprising
(i) an antigen binding moiety that specifically binds to CD3 and comprises a heavy chain variable region comprising the heavy chain CDR (HCDR) 1 of SEQ ID NO: 12, the HCDR2 of SEQ ID NO: 13, and the HCDR3 of SEQ ID NO: 14; and a light chain variable region comprising the light chain CDR (LCDR) 1 of SEQ ID NO: 15, the LCDR2 of SEQ ID NO: 16 and the LCDR3 of SEQ ID NO: 17; and
(ii) an antigen binding moiety that specifically binds to CD20 and comprises a heavy chain variable region comprising the heavy chain CDR (HCDR) 1 of SEQ ID NO: 4, the HCDR2 of SEQ ID NO: 5, and the HCDR3 of SEQ ID NO: 6; and a light chain variable region comprising the light chain CDR (LCDR) 1 of SEQ ID NO: 7, the LCDR2 of SEQ ID NO: 8 and the LCDR3 of SEQ ID NO: 9.

In one embodiment, the therapeutic agent comprises a bispecific antibody comprising
(i) an antigen binding moiety that specifically binds to CD3 and comprises a heavy chain variable region of SEQ ID NO: 18; and a light chain variable region of SEQ ID NO: 19; and
(ii) an antigen binding moiety that specifically binds to CD20 and comprises a heavy chain variable region of SEQ ID NO: 10; and a light chain variable region of SEQ ID NO: 11.

In a particular embodiment, the therapeutic agent comprises a bispecific antibody comprising
a) a first Fab molecule which specifically binds to a first antigen;
b) a second Fab molecule which specifically binds to a second antigen, and wherein the variable domains VL and VH of the Fab light chain and the Fab heavy chain are replaced by each other;
c) a third Fab molecule which specifically binds to the first antigen; and
d) an Fc domain composed of a first and a second subunit capable of stable association;
wherein
(i) the first antigen is CD20 and the second antigen is CD3, particularly CD3 epsilon;
(ii) the first Fab molecule under a) and the third Fab molecule under c) each comprise the heavy chain complementarity determining region (CDR) 1 of SEQ ID NO: 4, the heavy chain CDR 2 of SEQ ID NO: 5, the heavy chain CDR 3 of SEQ ID NO: 6, the light chain CDR 1 of SEQ ID NO: 7, the light chain CDR 2 of SEQ ID NO: 8 and the light chain CDR 3 of SEQ ID NO: 9, and the second Fab molecule under b) comprises the heavy chain CDR 1 of SEQ ID NO: 12, the heavy chain CDR 2 of SEQ ID NO: 13, the heavy chain CDR 3 of SEQ ID NO: 14, the light chain CDR 1 of SEQ ID NO: 15, the light chain CDR 2 of SEQ ID NO: 16 and the light chain CDR 3 of SEQ ID NO: 17;
(iii) in the constant domain CL of the first Fab molecule under a) and the third Fab molecule under c) the amino acid at position 124 is substituted by lysine (K) (numbering according to Kabat) and the amino acid at position 123 is substituted by lysine (K) or arginine (R), particularly by arginine (R) (numbering according to Kabat), and wherein in the constant domain CH1 of the first Fab molecule under a) and the third Fab molecule under c) the amino acid at position 147 is substituted by glutamic acid (E) (numbering according to Kabat EU index) and the amino acid at position 213 is substituted by glutamic acid (E) (numbering according to Kabat EU index); and
(iv) the first Fab molecule under a) is fused at the C-terminus of the Fab heavy chain to the N-terminus of the Fab heavy chain of the second Fab molecule under b), and the second Fab molecule under b) and the third Fab molecule under c) are each fused at the C-terminus of the Fab heavy chain to the N-terminus of one of the subunits of the Fc domain under d).

In one embodiment, the first Fab molecule under a) and the third Fab molecule under c) each comprise a heavy chain variable region that is at least 95%, 96%, 97%, 98%, or 99% identical to the sequence of SEQ ID NO: 10, and a light chain variable region that is at least 95%, 96%, 97%, 98%, or 99% identical to the sequence of SEQ ID NO: 11.

In one embodiment, the first Fab molecule under a) and the third Fab molecule under c) each comprise the heavy chain variable region sequence of SEQ ID NO: 10, and the light chain variable region sequence of SEQ ID NO: 11.

In one embodiment, the second Fab molecule under b) comprises a heavy chain variable region that is at least 95%, 96%, 97%, 98%, or 99% identical to the sequence of SEQ ID NO: 18, and a light chain variable region that is at least 95%, 96%, 97%, 98%, or 99% identical to the sequence of SEQ ID NO: 19.

In still a further embodiment, the second Fab molecule under b) comprises the heavy chain variable region sequence of SEQ ID NO: 18, and the light chain variable region sequence of SEQ ID NO: 19.

In a particular embodiment, the bispecific antibody comprises a polypeptide that is at least 95%, 96%, 97%, 98%, or 99% identical to the sequence of SEQ ID NO: 20, a polypeptide that is at least 95%, 96%, 97%, 98%, or 99% identical to the sequence of SEQ ID NO: 21, a polypeptide that is at least 95%, 96%, 97%, 98%, or 99% identical to the sequence of SEQ ID NO: 22, and a polypeptide that is at least 95%, 96%, 97%, 98%, or 99% identical to the sequence of SEQ ID NO: 23. In a further particular embodiment, the bispecific antibody comprises a polypeptide sequence of SEQ ID NO: 20, a polypeptide sequence of SEQ ID NO: 21, a polypeptide sequence of SEQ ID NO: 22 and a polypeptide sequence of SEQ ID NO: 23. (CD20XCD3 bsAB)

In one embodiment, the bispecific antibody comprises an antigen binding moiety that specifically binds to CD 19, and an antigen binding moiety that specifically binds to CD3. In one embodiment, the bispecific antibody comprises a first antigen binding moiety that specifically binds to CD3, and a second and a third antigen binding moiety that specifically bind to CD19. In one embodiment, the first antigen binding moiety is a crossover Fab molecule, and the second and the first antigen binding moiety are each a conventional Fab molecule. In one embodiment, the bispecific antibody further comprises an Fc domain. The bispecific antibody may comprise modifications in the Fc region and/or the antigen binding moieties as described herein.

In one embodiment, the therapeutic agent comprises a bispecific antibody comprising
(i) an antigen binding moiety that specifically binds to CD3 and comprises a heavy chain variable region comprising the heavy chain CDR (HCDR) 1 of SEQ ID NO: 12, the HCDR2 of SEQ ID NO: 13, and the HCDR3 of SEQ ID NO: 14; and a light chain variable region comprising the light chain CDR (LCDR) 1 of SEQ ID NO: 15, the LCDR2 of SEQ ID NO: 16 and the LCDR3 of SEQ ID NO: 17; and
(ii) an antigen binding moiety that specifically binds to CD 19 and comprises a heavy chain variable region comprising the heavy chain CDR (HCDR) 1 of SEQ ID NO: 24, the HCDR2 of SEQ ID NO: 25, and the HCDR3 of SEQ ID NO: 26; and a light chain variable region comprising the light chain CDR (LCDR) 1 of SEQ ID NO: 27, the LCDR2 of SEQ ID NO: 28 and the LCDR3 of SEQ ID NO: 29.

In one embodiment, the therapeutic agent comprises a bispecific antibody comprising
(i) an antigen binding moiety that specifically binds to CD3 and comprises a heavy chain variable region of SEQ ID NO: 18; and a light chain variable region of SEQ ID NO: 19; and
(ii) an antigen binding moiety that specifically binds to CD 19 and comprises a heavy chain variable region of SEQ ID NO: 30; and a light chain variable region of SEQ ID NO: 31.

In a particular embodiment, the therapeutic agent comprises a bispecific antibody comprising
a) a first Fab molecule which specifically binds to a first antigen;
b) a second Fab molecule which specifically binds to a second antigen, and wherein the variable domains VL and VH of the Fab light chain and the Fab heavy chain are replaced by each other;
c) a third Fab molecule which specifically binds to the first antigen; and
d) an Fc domain composed of a first and a second subunit capable of stable association;
wherein
(i) the first antigen is CD19 and the second antigen is CD3, particularly CD3 epsilon;
(ii) the first Fab molecule under a) and the third Fab molecule under c) each comprise the heavy chain complementarity determining region (CDR) 1 of SEQ ID NO: 24, the heavy chain CDR 2 of SEQ ID NO: 25, the heavy chain CDR 3 of SEQ ID NO: 26, the light chain CDR 1 of SEQ ID NO: 27, the light chain CDR 2 of SEQ ID NO: 28 and the light chain CDR 3 of SEQ ID NO: 29, and the second Fab molecule under b) comprises the heavy chain CDR 1 of SEQ ID NO: 12, the heavy chain CDR 2 of SEQ ID NO: 13, the heavy chain CDR 3 of SEQ ID NO: 14, the light chain CDR 1 of SEQ ID NO: 15, the light chain CDR 2 of SEQ ID NO: 16 and the light chain CDR 3 of SEQ ID NO: 17;
(iii) in the constant domain CL of the first Fab molecule under a) and the third Fab molecule under c) the amino acid at position 124 is substituted by lysine (K) (numbering according to Kabat) and the amino acid at position 123 is substituted by lysine (K) or arginine (R), particularly by arginine (R) (numbering according to Kabat), and wherein in the constant domain CH1 of the first Fab molecule under a) and the third Fab molecule under c) the amino acid at position 147 is substituted by glutamic acid (E) (numbering according to Kabat EU index) and the amino acid at position 213 is substituted by glutamic acid (E) (numbering according to Kabat EU index); and
(iv) the first Fab molecule under a) is fused at the C-terminus of the Fab heavy chain to the N-terminus of the Fab heavy chain of the second Fab molecule under b), and the second Fab molecule under b) and the third Fab molecule under c) are each fused at the C-terminus of the Fab heavy chain to the N-terminus of one of the subunits of the Fc domain under d).

In one embodiment, the first Fab molecule under a) and the third Fab molecule under c) each comprise a heavy chain variable region that is at least 95%, 96%, 97%, 98%, or 99% identical to the sequence of SEQ ID NO: 30, and a light chain variable region that is at least 95%, 96%, 97%, 98%, or 99% identical to the sequence of SEQ ID NO: 31.

In one embodiment, the first Fab molecule under a) and the third Fab molecule under c) each comprise the heavy chain variable region sequence of SEQ ID NO: 30, and the light chain variable region sequence of SEQ ID NO: 31.

In one embodiment, the second Fab molecule under b) comprises a heavy chain variable region that is at least 95%, 96%, 97%, 98%, or 99% identical to the sequence of SEQ ID NO: 18, and a light chain variable region that is at least 95%, 96%, 97%, 98%, or 99% identical to the sequence of SEQ ID NO: 19.

In still a further embodiment, the second Fab molecule under b) comprises the heavy chain variable region sequence of SEQ ID NO: 18, and the light chain variable region sequence of SEQ ID NO: 19.

In a particular embodiment, the bispecific antibody comprises a polypeptide that is at least 95%, 96%, 97%, 98%, or 99% identical to the sequence of SEQ ID NO: 23, a polypeptide that is at least 95%, 96%, 97%, 98%, or 99% identical to the sequence of SEQ ID NO: 32, a polypeptide that is at least 95%, 96%, 97%, 98%, or 99% identical to the sequence of SEQ ID NO: 33, and a polypeptide that is at least 95%, 96%, 97%, 98%, or 99% identical to the sequence of SEQ ID NO: 34. In a further particular embodiment, the bispecific antibody comprises a polypeptide sequence of SEQ ID NO: 23, a polypeptide sequence of SEQ ID NO: 32, a polypeptide sequence of SEQ ID NO: 33 and a polypeptide sequence of SEQ ID NO: 34.

### Antibody formats

The components of an antibody comprised in the therapeutic agent, particularly a multispecific antibody, can be fused to each other in a variety of configurations. Exemplary configurations are depicted in Figure 1.

In particular embodiments, the antigen binding moieties comprised in the antibody are Fab molecules. In such embodiments, the first, second, third etc. antigen binding moiety may be referred to herein as first, second, third etc. Fab molecule, respectively. Furthermore, in particular embodiments, the antibody comprises an Fc domain composed of a first and a second subunit capable of stable association.

In some embodiments, the second Fab molecule is fused at the C-terminus of the Fab heavy chain to the N-terminus of the first or the second subunit of the Fc domain.

In one such embodiment, the first Fab molecule is fused at the C-terminus of the Fab heavy chain to the N-terminus of the Fab heavy chain of the second Fab molecule. In a specific such embodiment, the antibody essentially consists of the first and the second Fab molecule, the Fc domain composed of a first and a second subunit, and optionally one or more peptide linkers, wherein the first Fab molecule is fused at the C-terminus of the Fab heavy chain to the N-terminus of the Fab heavy chain of the second Fab molecule, and the second Fab molecule is fused at the C-terminus of the Fab heavy chain to the N-terminus of the first or the second subunit of the Fc domain. Such a configuration is schematically depicted in Figures 1G and 1K. Optionally, the Fab light chain of the first Fab molecule and the Fab light chain of the second Fab molecule may additionally be fused to each other.

In another such embodiment, the first Fab molecule is fused at the C-terminus of the Fab heavy chain to the N-terminus of the first or second subunit of the Fc domain. In a specific such embodiment, the antibody essentially consists of the first and the second Fab molecule, the Fc domain composed of a first and a second subunit, and optionally one or more peptide linkers, wherein the first and the second Fab molecule are each fused at the C-terminus of the Fab heavy chain to the N-terminus of one of the subunits of the Fc domain. Such a configuration is schematically depicted in Figures 1A and 1D. The first and the second Fab molecule may be fused to the Fc domain directly or through a peptide linker. In a particular embodiment the first and the second Fab molecule are each fused to the Fc domain through an immunoglobulin hinge region. In a specific embodiment, the immunoglobulin hinge region is a human IgG₁ hinge region, particularly where the Fc domain is an IgG₁ Fc domain.

In other embodiments, the first Fab molecule is fused at the C-terminus of the Fab heavy chain to the N-terminus of the first or second subunit of the Fc domain.

In one such embodiment, the second Fab molecule is fused at the C-terminus of the Fab heavy chain to the N-terminus of the Fab heavy chain of the first Fab molecule. In a specific such embodiment, the antibody essentially consists of the first and the second Fab molecule, the Fc domain composed of a first and a second subunit, and optionally one or more peptide linkers, wherein the second Fab molecule is fused at the C-terminus of the Fab heavy chain to the N-terminus of the Fab heavy chain of the first Fab molecule, and the first Fab molecule is fused at the C-terminus of the Fab heavy chain to the N-terminus of the first or the second subunit of the Fc domain. Such a configuration is schematically depicted in Figures 1H and 1L. Optionally, the Fab light chain of the first Fab molecule and the Fab light chain of the second Fab molecule may additionally be fused to each other.

The Fab molecules may be fused to the Fc domain or to each other directly or through a peptide linker, comprising one or more amino acids, typically about 2-20 amino acids. Peptide linkers are known in the art and are described herein. Suitable, non-immunogenic peptide linkers include, for example, (G₄S)ₙ, (SG₄)ₙ, (G₄S)ₙ or G₄(SG₄)ₙ peptide linkers. "n" is generally an integer from 1 to 10, typically from 2 to 4. In one embodiment said peptide linker has a length of at least 5 amino acids, in one embodiment a length of 5 to 100, in a further embodiment of 10 to 50 amino acids. In one embodiment said peptide linker is (GxS)ₙ or (GxS)ₙGₘ with G=glycine, S=serine, and (x=3, n= 3, 4, 5 or 6, and m=0, 1, 2 or 3) or (x=4, n=2, 3, 4 or 5 and m= 0, 1, 2 or 3), in one embodiment x=4 and n=2 or 3, in a further embodiment x=4 and n=2. In one embodiment said peptide linker is (G₄S)₂. A particularly suitable peptide linker for fusing the Fab light chains of the first and the second Fab molecule to each other is (G₄S)₂. An exemplary peptide linker suitable for connecting the Fab heavy chains of the first and the second Fab fragments comprises the sequence (D)-(G₄S)₂ (SEQ ID NOs 95 and 96). Another suitable such linker comprises the sequence (G₄S)₄. Additionally, linkers may comprise (a portion of) an immunoglobulin hinge region. Particularly where a Fab molecule is fused to the N-terminus of an Fc domain subunit, it may be fused via an immunoglobulin hinge region or a portion thereof, with or without an additional peptide linker.

An antibody with a single antigen binding moiety (such as a Fab molecule) capable of specific binding to a target cell antigen (for example as shown in Figure 1A, D, G, H, K, L) is useful, particularly in cases where internalization of the target cell antigen is to be expected following binding of a high affinity antigen binding moiety. In such cases, the presence of more than one antigen binding moiety specific for the target cell antigen may enhance internalization of the target cell antigen, thereby reducing its availablity.

In many other cases, however, it will be advantageous to have an antibody comprising two or more antigen binding moieties (such as Fab moelcules) specific for a target cell antigen (see examples shown in Figure 1B, 1C, 1E, 1F, 1I, 1J. 1M or 1N), for example to optimize targeting to the target site or to allow crosslinking of target cell antigens.

Accordingly, in particular embodiments, the antibody further comprises a third Fab molecule which specifically binds to the first antigen. The first antigen preferably is the target cell antigen. In one embodiment, the third Fab molecule is a conventional Fab molecule. In one embodiment, the third Fab molecule is identical to the first Fab molecule (i.e. the first and the third Fab molecule comprise the same heavy and light chain amino acid sequences and have the same arrangement of domains (i.e. conventional or crossover)). In a particular embodiment, the second Fab molecule specifically binds to an activating T cell antigen, particularly CD3, and the first and third Fab molecule specifically bind to a target cell antigen.

In alternative embodiments, the antibody further comprises a third Fab molecule which specifically binds to the second antigen. In these embodiments, the second antigen preferably is the target cell antigen. In one such embodiment, the third Fab molecule is a crossover Fab molecule (a Fab molecule wherein the variable domains VH and VL or the constant domains CL and CH1 of the Fab heavy and light chains are exchanged / replaced by each other). In one such embodiment, the third Fab molecule is identical to the second Fab molecule (i.e. the second and the third Fab molecule comprise the same heavy and light chain amino acid sequences and have the same arrangement of domains (i.e. conventional or crossover)). In one such embodiment, the first Fab molecule specifically binds to an activating T cell antigen, particularly CD3, and the second and third Fab molecule specifically bind to a target cell antigen.

In one embodiment, the third Fab molecule is fused at the C-terminus of the Fab heavy chain to the N-terminus of the first or second subunit of the Fc domain.

In a particular embodiment, the second and the third Fab molecule are each fused at the C-terminus of the Fab heavy chain to the N-terminus of one of the subunits of the Fc domain, and the first Fab molecule is fused at the C-terminus of the Fab heavy chain to the N-terminus of the Fab heavy chain of the second Fab molecule. In a specific such embodiment, the antibody essentially consists of the first, the second and the third Fab molecule, the Fc domain composed of a first and a second subunit, and optionally one or more peptide linkers, wherein the first Fab molecule is fused at the C-terminus of the Fab heavy chain to the N-terminus of the Fab heavy chain of the second Fab molecule, and the second Fab molecule is fused at the C-terminus of the Fab heavy chain to the N-terminus of the first subunit of the Fc domain, and wherein the third Fab molecule is fused at the C-terminus of the Fab heavy chain to the N-terminus of the second subunit of the Fc domain. Such a configuration is schematically depicted in Figure 1B and 1E (particular embodiments, wherein the third Fab molecule is a conventional Fab molecule and preferably identical to the first Fab molecule), and Figure 1I and 1M (alternative embodiments, wherein the third Fab molecule is a crossover Fab molecule and preferably identical to the second Fab molecule). The second and the third Fab molecule may be fused to the Fc domain directly or through a peptide linker. In a particular embodiment the second and the third Fab molecule are each fused to the Fc domain through an immunoglobulin hinge region. In a specific embodiment, the immunoglobulin hinge region is a human IgG₁ hinge region, particularly where the Fc domain is an IgG₁ Fc domain. Optionally, the Fab light chain of the first Fab molecule and the Fab light chain of the second Fab molecule may additionally be fused to each other.

In another embodiment, the first and the third Fab molecule are each fused at the C-terminus of the Fab heavy chain to the N-terminus of one of the subunits of the Fc domain, and the second Fab molecule is fused at the C-terminus of the Fab heavy chain to the N-terminus of the Fab heavy chain of the first Fab molecule. In a specific such embodiment, the antibody essentially consists of the first, the second and the third Fab molecule, the Fc domain composed of a first and a second subunit, and optionally one or more peptide linkers, wherein the second Fab molecule is fused at the C-terminus of the Fab heavy chain to the N-terminus of the Fab heavy chain of the first Fab molecule, and the first Fab molecule is fused at the C-terminus of the Fab heavy chain to the N-terminus of the first subunit of the Fc domain, and wherein the third Fab molecule is fused at the C-terminus of the Fab heavy chain to the N-terminus of the second subunit of the Fc domain. Such a configuration is schematically depicted in Figure 1C and 1F (particular embodiments, wherein the third Fab molecule is a conventional Fab molecule and preferably identical to the first Fab molecule) and in Figure 1J and 1N (alternative embodiments, wherein the third Fab molecule is a crossover Fab molecule and preferably identical to the second Fab molecule). The first and the third Fab molecule may be fused to the Fc domain directly or through a peptide linker. In a particular embodiment the first and the third Fab molecule are each fused to the Fc domain through an immunoglobulin hinge region. In a specific embodiment, the immunoglobulin hinge region is a human IgG₁ hinge region, particularly where the Fc domain is an IgG₁ Fc domain. Optionally, the Fab light chain of the first Fab molecule and the Fab light chain of the second Fab molecule may additionally be fused to each other.

In configurations of the antibody wherein a Fab molecule is fused at the C-terminus of the Fab heavy chain to the N-terminus of each of the subunits of the Fc domain through an immunoglobulin hinge regions, the two Fab molecules, the hinge regions and the Fc domain essentially form an immunoglobulin molecule. In a particular embodiment the immunoglobulin molecule is an IgG class immunoglobulin. In an even more particular embodiment the immunoglobulin is an IgG₁ subclass immunoglobulin. In another embodiment the immunoglobulin is an IgG₄ subclass immunoglobulin. In a further particular embodiment the immunoglobulin is a human immunoglobulin. In other embodiments the immunoglobulin is a chimeric immunoglobulin or a humanized immunoglobulin.

In some of the antibodies, the Fab light chain of the first Fab molecule and the Fab light chain of the second Fab molecule are fused to each other, optionally via a peptide lnker. Depending on the configuration of the first and the second Fab molecule, the Fab light chain of the first Fab molecule may be fused at its C-terminus to the N-terminus of the Fab light chain of the second Fab molecule, or the Fab light chain of the second Fab molecule may be fused at its C-terminus to the N-terminus of the Fab light chain of the first Fab molecule. Fusion of the Fab light chains of the first and the second Fab molecule further reduces mispairing of unmatched Fab heavy and light chains, and also reduces the number of plasmids needed for expression of some of the antibodies.

In certain embodiments the antibody comprises a polypeptide wherein the Fab light chain variable region of the second Fab molecule shares a carboxy-terminal peptide bond with the Fab heavy chain constant region of the second Fab molecule (i.e. the second Fab molecule comprises a crossover Fab heavy chain, wherein the heavy chain variable region is replaced by a light chain variable region), which in turn shares a carboxy-terminal peptide bond with an Fc domain subunit (VL₍₂₎-CH1₍₂₎-CH2-CH3(-CH4)), and a polypeptide wherein the Fab heavy chain of the first Fab molecule shares a carboxy-terminal peptide bond with an Fc domain subunit (VH₍₁₎-CH1₍₁₎-CH2-CH3(-CH4)). In some embodiments the antibody further comprises a polypeptide wherein the Fab heavy chain variable region of the second Fab molecule shares a carboxy-terminal peptide bond with the Fab light chain constant region of the second Fab molecule (VH₍₂₎-CL₍₂₎) and the Fab light chain polypeptide of the first Fab molecule (VL₍₁₎-CL₍₁₎). In certain embodiments the polypeptides are covalently linked, e.g., by a disulfide bond.

In certain embodiments the antibody comprises a polypeptide wherein the Fab heavy chain variable region of the second Fab molecule shares a carboxy-terminal peptide bond with the Fab light chain constant region of the second Fab molecule (i.e. the second Fab molecule comprises a crossover Fab heavy chain, wherein the heavy chain constant region is replaced by a light chain constant region), which in turn shares a carboxy-terminal peptide bond with an Fc domain subunit (VH₍₂₎-CL₍₂₎-CH2-CH3(-CH4)), and a polypeptide wherein the Fab heavy chain of the first Fab molecule shares a carboxy-terminal peptide bond with an Fc domain subunit (VH₍₁₎-CH1₍₁₎-CH2-CH3(-CH4)). In some embodiments the antibody further comprises a polypeptide wherein the Fab light chain variable region of the second Fab molecule shares a carboxy-terminal peptide bond with the Fab heavy chain constant region of the second Fab molecule (VL₍₂₎-CH1₍₂₎) and the Fab light chain polypeptide of the first Fab molecule (VL₍₁₎-CL₍₁₎). In certain embodiments the polypeptides are covalently linked, e.g., by a disulfide bond.

In some embodiments, the antibody comprises a polypeptide wherein the Fab light chain variable region of the second Fab molecule shares a carboxy-terminal peptide bond with the Fab heavy chain constant region of the second Fab molecule (i.e. the second Fab molecule comprises a crossover Fab heavy chain, wherein the heavy chain variable region is replaced by a light chain variable region), which in turn shares a carboxy-terminal peptide bond with the Fab heavy chain of the first Fab molecule, which in turn shares a carboxy-terminal peptide bond with an Fc domain subunit (VL₍₂₎-CH1₍₂₎-VH₍₁₎-CH1₍₁₎-CH2-CH3(-CH4)). In other embodiments, the antibody comprises a polypeptide wherein the Fab heavy chain of the first Fab molecule shares a carboxy-terminal peptide bond with the Fab light chain variable region of the second Fab molecule which in turn shares a carboxy-terminal peptide bond with the Fab heavy chain constant region of the second Fab molecule (i.e. the second Fab molecule comprises a crossover Fab heavy chain, wherein the heavy chain variable region is replaced by a light chain variable region), which in turn shares a carboxy-terminal peptide bond with an Fc domain subunit (VH₍₁₎-CH1₍₁₎-VL₍₂₎-CH1₍₂₎-CH2-CH3(-CH4)).

In some of these embodiments the antibody further comprises a crossover Fab light chain polypeptide of the second Fab molecule, wherein the Fab heavy chain variable region of the second Fab molecule shares a carboxy-terminal peptide bond with the Fab light chain constant region of the second Fab molecule (VH₍₂₎-CL₍₂₎), and the Fab light chain polypeptide of the first Fab molecule (VL₍₁₎-CL₍₁₎). In others of these embodiments the antibody further comprises a polypeptide wherein the Fab heavy chain variable region of the second Fab molecule shares a carboxy-terminal peptide bond with the Fab light chain constant region of the second Fab molecule which in turn shares a carboxy-terminal peptide bond with the Fab light chain polypeptide of the first Fab molecule (VH₍₂₎-CL₍₂₎- VL₍₁₎-CL₍₁₎), or a polypeptide wherein the Fab light chain polypeptide of the first Fab molecule shares a carboxy-terminal peptide bond with the Fab heavy chain variable region of the second Fab molecule which in turn shares a carboxy-terminal peptide bond with the Fab light chain constant region of the second Fab molecule (VL₍₁₎-CL₍₁₎-VH₍₂₎-CL₍₂₎), as appropriate.

The antibody according to these embodiments may further comprise (i) an Fc domain subunit polypeptide (CH2-CH3(-CH4)), or (ii) a polypeptide wherein the Fab heavy chain of a third Fab molecule shares a carboxy-terminal peptide bond with an Fc domain subunit (VH₍₃₎-CH1₍₃₎-CH2-CH3(-CH4)) and the Fab light chain polypeptide of a third Fab molecule (VL₍₃₎-CL₍₃₎). In certain embodiments the polypeptides are covalently linked, e.g., by a disulfide bond.

In some embodiments, the antibody comprises a polypeptide wherein the Fab heavy chain variable region of the second Fab molecule shares a carboxy-terminal peptide bond with the Fab light chain constant region of the second Fab molecule (i.e. the second Fab molecule comprises a crossover Fab heavy chain, wherein the heavy chain constant region is replaced by a light chain constant region), which in turn shares a carboxy-terminal peptide bond with the Fab heavy chain of the first Fab molecule, which in turn shares a carboxy-terminal peptide bond with an Fc domain subunit (VH₍₂₎-CL₍₂₎-VH₍₁₎-CH1₍₁₎-CH2-CH3(-CH4)). In other embodiments, the antibody comprises a polypeptide wherein the Fab heavy chain of the first Fab molecule shares a carboxy-terminal peptide bond with the Fab heavy chain variable region of the second Fab molecule which in turn shares a carboxy-terminal peptide bond with the Fab light chain constant region of the second Fab molecule (i.e. the second Fab molecule comprises a crossover Fab heavy chain, wherein the heavy chain constant region is replaced by a light chain constant region), which in turn shares a carboxy-terminal peptide bond with an Fc domain subunit (VH₍₁₎-CH1₍₁₎-VH₍₂₎-CL₍₂₎-CH2-CH3(-CH4)).

In some of these embodiments the antibody further comprises a crossover Fab light chain polypeptide of the second Fab molecule, wherein the Fab light chain variable region of the second Fab molecule shares a carboxy-terminal peptide bond with the Fab heavy chain constant region of the second Fab molecule (VL₍₂₎-CH1₍₂₎), and the Fab light chain polypeptide of the first Fab molecule (VL₍₁₎-CL₍₁₎). In others of these embodiments the antibody further comprises a polypeptide wherein the Fab light chain variable region of the second Fab molecule shares a carboxy-terminal peptide bond with the Fab heavy chain constant region of the second Fab molecule which in turn shares a carboxy-terminal peptide bond with the Fab light chain polypeptide of the first Fab molecule (VL₍₂₎-CH1₍₂₎-VL₍₁₎-CL₍₁₎), or a polypeptide wherein the Fab light chain polypeptide of the first Fab molecule shares a carboxy-terminal peptide bond with the Fab heavy chain variable region of the second Fab molecule which in turn shares a carboxy-terminal peptide bond with the Fab light chain constant region of the second Fab molecule (VL₍₁₎-CL₍₁₎-VH₍₂₎-CL₍₂₎), as appropriate.

The antibody according to these embodiments may further comprise (i) an Fc domain subunit polypeptide (CH2-CH3(-CH4)), or (ii) a polypeptide wherein the Fab heavy chain of a third Fab molecule shares a carboxy-terminal peptide bond with an Fc domain subunit (VH₍₃₎-CH1₍₃₎-CH2-CH3(-CH4)) and the Fab light chain polypeptide of a third Fab molecule (VL₍₃₎-CL₍₃₎). In certain embodiments the polypeptides are covalently linked, e.g., by a disulfide bond.

In some embodiments, the first Fab molecule is fused at the C-terminus of the Fab heavy chain to the N-terminus of the Fab heavy chain of the second Fab molecule. In certain such embodiments, the antibody does not comprise an Fc domain. In certain embodiments, the antibody essentially consists of the first and the second Fab molecule, and optionally one or more peptide linkers, wherein the first Fab molecule is fused at the C-terminus of the Fab heavy chain to the N-terminus of the Fab heavy chain of the second Fab molecule. Such a configuration is schematically depicted in Figures 1O and 1S.

In other embodiments, the second Fab molecule is fused at the C-terminus of the Fab heavy chain to the N-terminus of the Fab heavy chain of the first Fab molecule. In certain such embodiments, the antibody does not comprise an Fc domain. In certain embodiments, the antibody essentially consists of the first and the second Fab molecule, and optionally one or more peptide linkers, wherein the second Fab molecule is fused at the C-terminus of the Fab heavy chain to the N-terminus of the Fab heavy chain of the first Fab molecule. Such a configuration is schematically depicted in Figures 1P and 1T.

In some embodiments, the first Fab molecule is fused at the C-terminus of the Fab heavy chain to the N-terminus of the Fab heavy chain of the second Fab molecule, and the antibody further comprises a third Fab molecule, wherein said third Fab molecule is fused at the C-terminus of the Fab heavy chain to the N-terminus of the Fab heavy chain of the first Fab molecule. In particular such embodiments, said third Fab molecule is a conventional Fab molecule. In other such embodiments, said third Fab molecule is a crossover Fab molecule as described herein, i.e. a Fab molecule wherein the variable domains VH and VL or the constant domains CL and CH1 of the Fab heavy and light chains are exchanged / replaced by each other. In certain such embodiments, the antibody essentially consists of the first, the second and the third Fab molecule, and optionally one or more peptide linkers, wherein the first Fab molecule is fused at the C-terminus of the Fab heavy chain to the N-terminus of the Fab heavy chain of the second Fab molecule, and the third Fab molecule is fused at the C-terminus of the Fab heavy chain to the N-terminus of the Fab heavy chain of the first Fab molecule. Such a configuration is schematically depicted in Figure 1Q and 1U (particular embodiments, wherein the third Fab molecule is a conventional Fab molecule and preferably identical to the first Fab molecule).

In some embodiments, the first Fab molecule is fused at the C-terminus of the Fab heavy chain to the N-terminus of the Fab heavy chain of the second Fab molecule, and the antibody further comprises a third Fab molecule, wherein said third Fab molecule is fused at the N-terminus of the Fab heavy chain to the C-terminus of the Fab heavy chain of the second Fab molecule. In particular such embodiments, said third Fab molecule is a crossover Fab molecule as described herein, i.e. a Fab molecule wherein the variable domains VH and VL or the constant domains CH1 and CL of the Fab heavy and light chains are exchanged / replaced by each other. In other such embodiments, said third Fab molecule is a conventional Fab molecule. In certain such embodiments, the antibody essentially consists of the first, the second and the third Fab molecule, and optionally one or more peptide linkers, wherein the first Fab molecule is fused at the C-terminus of the Fab heavy chain to the N-terminus of the Fab heavy chain of the second Fab molecule, and the third Fab molecule is fused at the N-terminus of the Fab heavy chain to the C-terminus of the Fab heavy chain of the second Fab molecule. Such a configuration is schematically depicted in Figure 1W and 1Y (particular embodiments, wherein the third Fab molecule is a crossover Fab molecule and preferably identical to the second Fab molecule).

In some embodiments, the second Fab molecule is fused at the C-terminus of the Fab heavy chain to the N-terminus of the Fab heavy chain of the first Fab molecule, and the antibody further comprises a third Fab molecule, wherein said third Fab molecule is fused at the N-terminus of the Fab heavy chain to the C-terminus of the Fab heavy chain of the first Fab molecule. In particular such embodiments, said third Fab molecule is a conventional Fab molecule. In other such embodiments, said third Fab molecule is a crossover Fab molecule as described herein, i.e. a Fab molecule wherein the variable domains VH and VL or the constant domains CH1 and CL of the Fab heavy and light chains are exchanged / replaced by each other. In certain such embodiments, the antibody essentially consists of the first, the second and the third Fab molecule, and optionally one or more peptide linkers, wherein the second Fab molecule is fused at the C-terminus of the Fab heavy chain to the N-terminus of the Fab heavy chain of the first Fab molecule, and the third Fab molecule is fused at the N-terminus of the Fab heavy chain to the C-terminus of the Fab heavy chain of the first Fab molecule. Such a configuration is schematically depicted in Figure 1R and 1V (particular embodiments, wherein the third Fab molecule is a conventional Fab molecule and preferably identical to the first Fab molecule).

In some embodiments, the second Fab molecule is fused at the C-terminus of the Fab heavy chain to the N-terminus of the Fab heavy chain of the first Fab molecule, and the antibody further comprises a third Fab molecule, wherein said third Fab molecule is fused at the C-terminus of the Fab heavy chain to the N-terminus of the Fab heavy chain of the second Fab molecule. In particular such embodiments, said third Fab molecule is a crossover Fab molecule as described herein, i.e. a Fab molecule wherein the variable domains VH and VL or the constant domains CH1 and CL of the Fab heavy and light chains are exchanged / replaced by each other. In other such embodiments, said third Fab molecule is a conventional Fab molecule. In certain such embodiments, the antibody essentially consists of the first, the second and the third Fab molecule, and optionally one or more peptide linkers, wherein the second Fab molecule is fused at the C-terminus of the Fab heavy chain to the N-terminus of the Fab heavy chain of the first Fab molecule, and the third Fab molecule is fused at the C-terminus of the Fab heavy chain to the N-terminus of the Fab heavy chain of the second Fab molecule. Such a configuration is schematically depicted in Figure 1X and 1Z (particular embodiments, wherein the third Fab molecule is a crossover Fab molecule and preferably identical to the first Fab molecule).

In certain embodiments the antibody comprises a polypeptide wherein the Fab heavy chain of the first Fab molecule shares a carboxy-terminal peptide bond with the Fab light chain variable region of the second Fab molecule, which in turn shares a carboxy-terminal peptide bond with the Fab heavy chain constant region of the second Fab molecule (i.e. the second Fab molecule comprises a crossover Fab heavy chain, wherein the heavy chain variable region is replaced by a light chain variable region) (VH₍₁₎-CH1₍₁₎-VL₍₂₎-CH1₍₂₎). In some embodiments the antibody further comprises a polypeptide wherein the Fab heavy chain variable region of the second Fab molecule shares a carboxy-terminal peptide bond with the Fab light chain constant region of the second Fab molecule (VH₍₂₎-CL₍₂₎) and the Fab light chain polypeptide of the first Fab molecule (VL₍₁₎-CL₍₁₎).

In certain embodiments the antibody comprises a polypeptide wherein the Fab light chain variable region of the second Fab molecule shares a carboxy-terminal peptide bond with the Fab heavy chain constant region of the second Fab molecule (i.e. the second Fab molecule comprises a crossover Fab heavy chain, wherein the heavy chain variable region is replaced by a light chain variable region), which in turn shares a carboxy-terminal peptide bond with the Fab heavy chain of the first Fab molecule (VL₍₂₎-CH1₍₂₎-VH₍₁₎-CH1₍₁₎). In some embodiments the antibody further comprises a polypeptide wherein the Fab heavy chain variable region of the second Fab molecule shares a carboxy-terminal peptide bond with the Fab light chain constant region of the second Fab molecule (VH₍₂₎-CL₍₂₎) and the Fab light chain polypeptide of the first Fab molecule (VL₍₁₎-CL₍₁₎).

In certain embodiments the antibody comprises a polypeptide wherein the Fab heavy chain variable region of the second Fab molecule shares a carboxy-terminal peptide bond with the Fab light chain constant region of the second Fab molecule (i.e. the second Fab molecule comprises a crossover Fab heavy chain, wherein the heavy chain constant region is replaced by a light chain constant region), which in turn shares a carboxy-terminal peptide bond with the Fab heavy chain of the first Fab molecule (VH₍₂₎-CL₍₂₎-VH₍₁₎-CH1₍₁₎). In some embodiments the antibody further comprises a polypeptide wherein the Fab light chain variable region of the second Fab molecule shares a carboxy-terminal peptide bond with the Fab heavy chain constant region of the second Fab molecule (VL₍₂₎-CH1₍₂₎) and the Fab light chain polypeptide of the first Fab molecule (VL₍₁₎-CL₍₁₎).

In certain embodiments the antibody comprises a polypeptide wherein the Fab heavy chain of a third Fab molecule shares a carboxy-terminal peptide bond with the Fab heavy chain of the first Fab molecule, which in turn shares a carboxy-terminal peptide bond with the Fab light chain variable region of the second Fab molecule, which in turn shares a carboxy-terminal peptide bond with the Fab heavy chain constant region of the second Fab molecule (i.e. the second Fab molecule comprises a crossover Fab heavy chain, wherein the heavy chain variable region is replaced by a light chain variable region) (VH₍₃₎-CH1₍₃₎-VH₍₁₎-CH1₍₁₎-VL₍₂₎-CH1₍₂₎). In some embodiments the antibody further comprises a polypeptide wherein the Fab heavy chain variable region of the second Fab molecule shares a carboxy-terminal peptide bond with the Fab light chain constant region of the second Fab molecule (VH₍₂₎-CL₍₂₎) and the Fab light chain polypeptide of the first Fab molecule (VL₍₁₎-CL₍₁₎). In some embodiments the antibody further comprises the Fab light chain polypeptide of a third Fab molecule (VL₍₃₎-CL₍₃₎).

In certain embodiments the antibody comprises a polypeptide wherein the Fab heavy chain of a third Fab molecule shares a carboxy-terminal peptide bond with the Fab heavy chain of the first Fab molecule, which in turn shares a carboxy-terminal peptide bond with the Fab heavy chain variable region of the second Fab molecule, which in turn shares a carboxy-terminal peptide bond with the Fab light chain constant region of the second Fab molecule (i.e. the second Fab molecule comprises a crossover Fab heavy chain, wherein the heavy chain constant region is replaced by a light chain constant region) (VH₍₃₎-CH1₍₃₎-VH₍₁₎-CH1₍₁₎-VH₍₂₎-CL₍₂₎). In some embodiments the antibody further comprises a polypeptide wherein the Fab light chain variable region of the second Fab molecule shares a carboxy-terminal peptide bond with the Fab heavy chain constant region of the second Fab molecule (VL₍₂₎-CH1₍₂₎) and the Fab light chain polypeptide of the first Fab molecule (VL₍₁₎-CL₍₁₎). In some embodiments the antibody further comprises the Fab light chain polypeptide of a third Fab molecule (VL₍₃₎-CL₍₃₎).

In certain embodiments the antibody comprises a polypeptide wherein the Fab light chain variable region of the second Fab molecule shares a carboxy-terminal peptide bond with the Fab heavy chain constant region of the second Fab molecule (i.e. the second Fab molecule comprises a crossover Fab heavy chain, wherein the heavy chain variable region is replaced by a light chain variable region), which in turn shares a carboxy-terminal peptide bond with the Fab heavy chain of the first Fab molecule, which in turn shares a carboxy-terminal peptide bond with the Fab heavy chain of a third Fab molecule (VL₍₂₎-CH1₍₂₎-VH₍₁₎-CH1₍₁₎-VH₍₃₎-CH1₍₃₎). In some embodiments the antibody further comprises a polypeptide wherein the Fab heavy chain variable region of the second Fab molecule shares a carboxy-terminal peptide bond with the Fab light chain constant region of the second Fab molecule (VH₍₂₎-CL₍₂₎) and the Fab light chain polypeptide of the first Fab molecule (VL₍₁₎-CL₍₁₎). In some embodiments the antibody further comprises the Fab light chain polypeptide of a third Fab molecule (VL₍₃₎-CL₍₃₎).

In certain embodiments the antibody comprises a polypeptide wherein the Fab heavy chain variable region of the second Fab molecule shares a carboxy-terminal peptide bond with the Fab light chain constant region of the second Fab molecule (i.e. the second Fab molecule comprises a crossover Fab heavy chain, wherein the heavy chain constant region is replaced by a light chain constant region), which in turn shares a carboxy-terminal peptide bond with the Fab heavy chain of the first Fab molecule, which in turn shares a carboxy-terminal peptide bond with the Fab heavy chain of a third Fab molecule (VH₍₂₎-CL₍₂₎-VH₍₁₎-CH1₍₁₎-VH₍₃₎-CH1₍₃₎). In some embodiments the antibody further comprises a polypeptide wherein the Fab light chain variable region of the second Fab molecule shares a carboxy-terminal peptide bond with the Fab heavy chain constant region of the second Fab molecule (VL₍₂₎-CH1₍₂₎) and the Fab light chain polypeptide of the first Fab molecule (VL₍₁₎-CL₍₁₎). In some embodiments the antibody further comprises the Fab light chain polypeptide of a third Fab molecule (VL₍₃₎-CL₍₃₎).

In certain embodiments the antibody comprises a polypeptide wherein the Fab heavy chain of the first Fab molecule shares a carboxy-terminal peptide bond with the Fab light chain variable region of the second Fab molecule, which in turn shares a carboxy-terminal peptide bond with the Fab heavy chain constant region of the second Fab molecule (i.e. the second Fab molecule comprises a crossover Fab heavy chain, wherein the heavy chain variable region is replaced by a light chain variable region), which in turn shares a carboxy-terminal peptide bond with the Fab light chain variable region of a third Fab molecule, which in turn shares a carboxy-terminal peptide bond with the Fab heavy chain constant region of a third Fab molecule (i.e. the third Fab molecule comprises a crossover Fab heavy chain, wherein the heavy chain variable region is replaced by a light chain variable region) (VH₍₁₎-CH1₍₁₎-VL₍₂₎-CH1₍₂₎-VL₍₃₎-CH1₍₃₎). In some embodiments the antibody further comprises a polypeptide wherein the Fab heavy chain variable region of the second Fab molecule shares a carboxy-terminal peptide bond with the Fab light chain constant region of the second Fab molecule (VH₍₂₎-CL₍₂₎) and the Fab light chain polypeptide of the first Fab molecule (VL₍₁₎-CL₍₁₎). In some embodiments the antibody further comprises a polypeptide wherein the Fab heavy chain variable region of a third Fab molecule shares a carboxy-terminal peptide bond with the Fab light chain constant region of a third Fab molecule (VH₍₃₎-CL₍₃₎).

In certain embodiments the antibody comprises a polypeptide wherein the Fab heavy chain of the first Fab molecule shares a carboxy-terminal peptide bond with the Fab heavy chain variable region of the second Fab molecule, which in turn shares a carboxy-terminal peptide bond with the Fab light chain constant region of the second Fab molecule (i.e. the second Fab molecule comprises a crossover Fab heavy chain, wherein the heavy chain constant region is replaced by a light chain constant region), which in turn shares a carboxy-terminal peptide bond with the Fab heavy chain variable region of a third Fab molecule, which in turn shares a carboxy-terminal peptide bond with the Fab light chain constant region of a third Fab molecule (i.e. the third Fab molecule comprises a crossover Fab heavy chain, wherein the heavy chain constant region is replaced by a light chain constant region) (VH₍₁₎-CH1₍₁₎-VH₍₂₎-CL₍₂₎-VH₍₃₎-CL₍₃₎). In some embodiments the antibody further comprises a polypeptide wherein the Fab light chain variable region of the second Fab molecule shares a carboxy-terminal peptide bond with the Fab heavy chain constant region of the second Fab molecule (VL₍₂₎-CH1₍₂₎) and the Fab light chain polypeptide of the first Fab molecule (VL₍₁₎-CL₍₁₎). In some embodiments the antibody further comprises a polypeptide wherein the Fab light chain variable region of a third Fab molecule shares a carboxy-terminal peptide bond with the Fab heavy chain constant region of a third Fab molecule (VL₍₃₎-CH1₍₃₎).

In certain embodiments the antibody comprises a polypeptide wherein the Fab light chain variable region of a third Fab molecule shares a carboxy-terminal peptide bond with the Fab heavy chain constant region of a third Fab molecule (i.e. the third Fab molecule comprises a crossover Fab heavy chain, wherein the heavy chain variable region is replaced by a light chain variable region), which in turn shares a carboxy-terminal peptide bond with the Fab light chain variable region of the second Fab molecule, which in turn shares a carboxy-terminal peptide bond with the Fab heavy chain constant region of the second Fab molecule (i.e. the second Fab molecule comprises a crossover Fab heavy chain, wherein the heavy chain variable region is replaced by a light chain variable region), which in turn shares a carboxy-terminal peptide bond with the Fab heavy chain of the first Fab molecule (VL₍₃₎-CH1₍₃₎-VL₍₂₎-CH1₍₂₎-VH₍₁₎-CH1₍₁₎). In some embodiments the antibody further comprises a polypeptide wherein the Fab heavy chain variable region of the second Fab molecule shares a carboxy-terminal peptide bond with the Fab light chain constant region of the second Fab molecule (VH₍₂₎-CL₍₂₎) and the Fab light chain polypeptide of the first Fab molecule (VL₍₁₎-CL₍₁₎). In some embodiments the antibody further comprises a polypeptide wherein the Fab heavy chain variable region of a third Fab molecule shares a carboxy-terminal peptide bond with the Fab light chain constant region of a third Fab molecule (VH₍₃₎-CL₍₃₎).

In certain embodiments the antibody comprises a polypeptide wherein the Fab heavy chain variable region of a third Fab molecule shares a carboxy-terminal peptide bond with the Fab light chain constant region of a third Fab molecule (i.e. the third Fab molecule comprises a crossover Fab heavy chain, wherein the heavy chain constant region is replaced by a light chain constant region), which in turn shares a carboxy-terminal peptide bond with the Fab heavy chain variable region of the second Fab molecule, which in turn shares a carboxy-terminal peptide bond with the Fab light chain constant region of the second Fab molecule (i.e. the second Fab molecule comprises a crossover Fab heavy chain, wherein the heavy chain constant region is replaced by a light chain constant region), which in turn shares a carboxy-terminal peptide bond with the Fab heavy chain of the first Fab molecule (VH₍₃₎-CL₍₃₎-VH₍₂₎-CL₍₂₎-VH₍₁₎-CH1₍₁₎). In some embodiments the antibody further comprises a polypeptide wherein the Fab light chain variable region of the second Fab molecule shares a carboxy-terminal peptide bond with the Fab heavy chain constant region of the second Fab molecule (VL₍₂₎-CH1₍₂₎) and the Fab light chain polypeptide of the first Fab molecule (VL₍₁₎-CL₍₁₎). In some embodiments the antibody further comprises a polypeptide wherein the Fab light chain variable region of a third Fab molecule shares a carboxy-terminal peptide bond with the Fab heavy chain constant region of a third Fab molecule (VL₍₃₎-CH1₍₃₎).

According to any of the above embodiments, components of the antibody (e.g. Fab molecules, Fc domain) may be fused directly or through various linkers, particularly peptide linkers comprising one or more amino acids, typically about 2-20 amino acids, that are described herein or are known in the art. Suitable, non-immunogenic peptide linkers include, for example, (G₄S)ₙ, (SG₄)ₙ, (G₄S)ₙ or G₄(SG₄)ₙ peptide linkers, wherein n is generally an integer from 1 to 10, typically from 2 to 4.

### Fc domain

An antibody, e.g. a bispecific antibody, comprised in the therapeutic agent may comprise an Fc domain which consists of a pair of polypeptide chains comprising heavy chain domains of an antibody molecule. For example, the Fc domain of an immunoglobulin G (IgG) molecule is a dimer, each subunit of which comprises the CH2 and CH3 IgG heavy chain constant domains. The two subunits of the Fc domain are capable of stable association with each other.

In one embodiment, the Fc domain is an IgG Fc domain. In a particular embodiment the Fc domain is an IgG₁ Fc domain. In another embodiment the Fc domain is an IgG₄ Fc domain. In a more specific embodiment, the Fc domain is an IgG₄ Fc domain comprising an amino acid substitution at position S228 (Kabat numbering), particularly the amino acid substitution S228P. This amino acid substitution reduces in vivo Fab arm exchange of IgG₄ antibodies (see Stubenrauch et al., Drug Metabolism and Disposition 38, 84-91 (2010)). In a further particular embodiment the Fc domain is human. An exemplary sequence of a human IgG₁ Fc region is given in SEQ ID NO: 94.

### (i) Fc domain modifications promoting heterodimerization

Antibodies, particularly bispecific antibodies, comprised in the therapeutic agent may comprise different components (e.g. antigen binding domains) fused to one or the other of the two subunits of the Fc domain, thus the two subunits of the Fc domain are typically comprised in two non-identical polypeptide chains. Recombinant co-expression of these polypeptides and subsequent dimerization leads to several possible combinations of the two polypeptides. To improve the yield and purity of such antibodies in recombinant production, it will thus be advantageous to introduce in the Fc domain of the antibody a modification promoting the association of the desired polypeptides.

Accordingly, in particular embodiments the Fc domain comprises a modification promoting the association of the first and the second subunit of the Fc domain. The site of most extensive protein-protein interaction between the two subunits of a human IgG Fc domain is in the CH3 domain of the Fc domain. Thus, in one embodiment said modification is in the CH3 domain of the Fc domain.

There exist several approaches for modifications in the CH3 domain of the Fc domain in order to enforce heterodimerization, which are well described e.g. in WO 96/27011, WO 98/050431, EP 1870459, WO 2007/110205, WO 2007/147901, WO 2009/089004, WO 2010/129304, WO 2011/90754, WO 2011/143545, WO 2012058768, WO 2013157954, WO 2013096291. Typically, in all such approaches the CH3 domain of the first subunit of the Fc domain and the CH3 domain of the second subunit of the Fc domain are both engineered in a complementary manner so that each CH3 domain (or the heavy chain comprising it) can no longer homodimerize with itself but is forced to heterodimerize with the complementarily engineered other CH3 domain (so that the first and second CH3 domain heterodimerize and no homodimers between the two first or the two second CH3 domains are formed). These different approaches for improved heavy chain heterodimerization are contemplated as different alternatives in combination with heavy-light chain modifications (e.g. variable or constant region exchange/replacement in Fab arms, or introduction of substitutions of charged amino acids with opposite charges in the CH1/CL interface) which reduce light chain mispairing and Bence Jones-type side products.

In a specific embodiment said modification promoting the association of the first and the second subunit of the Fc domain is a so-called "knob-into-hole" modification, comprising a "knob" modification in one of the two subunits of the Fc domain and a "hole" modification in the other one of the two subunits of the Fc domain.

The knob-into-hole technology is described e.g. in US 5,731,168; US 7,695,936; Ridgway et al., Prot Eng 9, 617-621 (1996) and Carter, J Immunol Meth 248, 7-15 (2001). Generally, the method involves introducing a protuberance ("knob") at the interface of a first polypeptide and a corresponding cavity ("hole") in the interface of a second polypeptide, such that the protuberance can be positioned in the cavity so as to promote heterodimer formation and hinder homodimer formation. Protuberances are constructed by replacing small amino acid side chains from the interface of the first polypeptide with larger side chains (e.g. tyrosine or tryptophan). Compensatory cavities of identical or similar size to the protuberances are created in the interface of the second polypeptide by replacing large amino acid side chains with smaller ones (e.g. alanine or threonine).

Accordingly, in a particular embodiment, in the CH3 domain of the first subunit of the Fc domain an amino acid residue is replaced with an amino acid residue having a larger side chain volume, thereby generating a protuberance within the CH3 domain of the first subunit which is positionable in a cavity within the CH3 domain of the second subunit, and in the CH3 domain of the second subunit of the Fc domain an amino acid residue is replaced with an amino acid residue having a smaller side chain volume, thereby generating a cavity within the CH3 domain of the second subunit within which the protuberance within the CH3 domain of the first subunit is positionable.

Preferably said amino acid residue having a larger side chain volume is selected from the group consisting of arginine (R), phenylalanine (F), tyrosine (Y), and tryptophan (W).

Preferably said amino acid residue having a smaller side chain volume is selected from the group consisting of alanine (A), serine (S), threonine (T), and valine (V).

The protuberance and cavity can be made by altering the nucleic acid encoding the polypeptides, e.g. by site-specific mutagenesis, or by peptide synthesis.

In a specific embodiment, in the CH3 domain of the first subunit of the Fc domain (the "knobs" subunit) the threonine residue at position 366 is replaced with a tryptophan residue (T366W), and in the CH3 domain of the second subunit of the Fc domain (the "hole" subunit) the tyrosine residue at position 407 is replaced with a valine residue (Y407V). In one embodiment, in the second subunit of the Fc domain additionally the threonine residue at position 366 is replaced with a serine residue (T366S) and the leucine residue at position 368 is replaced with an alanine residue (L368A) (numberings according to Kabat EU index).

In yet a further embodiment, in the first subunit of the Fc domain additionally the serine residue at position 354 is replaced with a cysteine residue (S354C) or the glutamic acid residue at position 356 is replaced with a cysteine residue (E356C), and in the second subunit of the Fc domain additionally the tyrosine residue at position 349 is replaced by a cysteine residue (Y349C) (numberings according to Kabat EU index). Introduction of these two cysteine residues results in formation of a disulfide bridge between the two subunits of the Fc domain, further stabilizing the dimer (Carter, J Immunol Methods 248, 7-15 (2001)).

In a particular embodiment, the first subunit of the Fc domain comprises amino acid substitutions S354C and T366W, and the second subunit of the Fc domain comprises amino acid substitutions Y349C, T366S, L368A and Y407V (numbering according to Kabat EU index).

In a particular embodiment the CD3 antigen binding moiety described herein is fused to the first subunit of the Fc domain (comprising the "knob" modification). Without wishing to be bound by theory, fusion of the CD3 antigen binding moiety to the knob-containing subunit of the Fc domain will (further) minimize the generation of bispecific antibodies comprising two CD3 antigen binding moieties (steric clash of two knob-containing polypeptides).

Other techniques of CH3-modification for enforcing the heterodimerization are contemplated as alternatives according to the invention and are described e.g. in WO 96/27011, WO 98/050431, EP 1870459, WO 2007/110205, WO 2007/147901, WO 2009/089004, WO 2010/129304, WO 2011/90754, WO 2011/143545, WO 2012/058768, WO 2013/157954, WO 2013/096291.

In one embodiment the heterodimerization approach described in EP 1870459 A1, is used alternatively. This approach is based on the introduction of charged amino acids with opposite charges at specific amino acid positions in the CH3/CH3 domain interface between the two subunits of the Fc domain. One preferred embodiment are amino acid mutations R409D; K370E in one of the two CH3 domains (of the Fc domain) and amino acid mutations D399K; E357K in the other one of the CH3 domains of the Fc domain (numbering according to Kabat EU index).

In another embodiment the antibody comprises amino acid mutation T366W in the CH3 domain of the first subunit of the Fc domain and amino acid mutations T366S, L368A, Y407V in the CH3 domain of the second subunit of the Fc domain, and additionally amino acid mutations R409D; K370E in the CH3 domain of the first subunit of the Fc domain and amino acid mutations D399K; E357K in the CH3 domain of the second subunit of the Fc domain (numberings according to Kabat EU index).

In another embodiment the antibody comprises amino acid mutations S354C, T366W in the CH3 domain of the first subunit of the Fc domain and amino acid mutations Y349C, T366S, L368A, Y407V in the CH3 domain of the second subunit of the Fc domain, or the antibody comprises amino acid mutations Y349C, T366W in the CH3 domain of the first subunit of the Fc domain and amino acid mutations S354C, T366S, L368A, Y407V in the CH3 domains of the second subunit of the Fc domain and additionally amino acid mutations R409D; K370E in the CH3 domain of the first subunit of the Fc domain and amino acid mutations D399K; E357K in the CH3 domain of the second subunit of the Fc domain (all numberings according to Kabat EU index).

In one embodiment the heterodimerization approach described in WO 2013/157953 is used alternatively. In one embodiment a first CH3 domain comprises amino acid mutation T366K and a second CH3 domain comprises amino acid mutation L351D (numberings according to Kabat EU index). In a further embodiment the first CH3 domain comprises further amino acid mutation L351K. In a further embodiment the second CH3 domain comprises further an amino acid mutation selected from Y349E, Y349D and L368E (preferably L368E) (numberings according to Kabat EU index).

In one embodiment the heterodimerization approach described in WO 2012/058768 is used alternatively. In one embodiment a first CH3 domain comprises amino acid mutations L351Y, Y407A and a second CH3 domain comprises amino acid mutations T366A, K409F. In a further embodiment the second CH3 domain comprises a further amino acid mutation at position T411, D399, S400, F405, N390, or K392, e.g. selected from a) T411N, T411R, T411Q, T411K, T411D, T411E or T411W, b) D399R, D399W, D399Y or D399K, c) S400E, S400D, S400R, or S400K, d) F405I, F405M, F405T, F405S, F405V or F405W, e) N390R, N390K or N390D, f) K392V, K392M, K392R, K392L, K392F or K392E (numberings according to Kabat EU index). In a further embodiment a first CH3 domain comprises amino acid mutations L351Y, Y407A and a second CH3 domain comprises amino acid mutations T366V, K409F. In a further embodiment a first CH3 domain comprises amino acid mutation Y407A and a second CH3 domain comprises amino acid mutations T366A, K409F. In a further embodiment the second CH3 domain further comprises amino acid mutations K392E, T411E, D399R and S400R (numberings according to Kabat EU index).

In one embodiment the heterodimerization approach described in WO 2011/143545 is used alternatively, e.g. with the amino acid modification at a position selected from the group consisting of 368 and 409 (numbering according to Kabat EU index).

In one embodiment the heterodimerization approach described in WO 2011/090762, which also uses the knobs-into-holes technology described above, is used alternatively. In one embodiment a first CH3 domain comprises amino acid mutation T366W and a second CH3 domain comprises amino acid mutation Y407A. In one embodiment a first CH3 domain comprises amino acid mutation T366Y and a second CH3 domain comprises amino acid mutation Y407T (numberings according to Kabat EU index).

In one embodiment the antibody or its Fc domain is of IgG₂ subclass and the heterodimerization approach described in WO 2010/129304 is used alternatively.

In an alternative embodiment a modification promoting association of the first and the second subunit of the Fc domain comprises a modification mediating electrostatic steering effects, e.g. as described in PCT publication WO 2009/089004. Generally, this method involves replacement of one or more amino acid residues at the interface of the two Fc domain subunits by charged amino acid residues so that homodimer formation becomes electrostatically unfavorable but heterodimerization electrostatically favorable. In one such embodiment a first CH3 domain comprises amino acid substitution of K392 or N392 with a negatively charged amino acid (e.g. glutamic acid (E), or aspartic acid (D), preferably K392D or N392D) and a second CH3 domain comprises amino acid substitution of D399, E356, D356, or E357 with a positively charged amino acid (e.g. lysine (K) or arginine (R), preferably D399K, E356K, D356K, or E357K, and more preferably D399K and E356K). In a further embodiment the first CH3 domain further comprises amino acid substitution of K409 or R409 with a negatively charged amino acid (e.g. glutamic acid (E), or aspartic acid (D), preferably K409D or R409D). In a further embodiment the first CH3 domain further or alternatively comprises amino acid substitution of K439 and/or K370 with a negatively charged amino acid (e.g. glutamic acid (E), or aspartic acid (D)) (all numberings according to Kabat EU index).

In yet a further embodiment the heterodimerization approach described in WO 2007/147901 is used alternatively. In one embodiment a first CH3 domain comprises amino acid mutations K253E, D282K, and K322D and a second CH3 domain comprises amino acid mutations D239K, E240K, and K292D (numberings according to Kabat EU index).

In still another embodiment the heterodimerization approach described in WO 2007/110205 can be used alternatively.

In one embodiment, the first subunit of the Fc domain comprises amino acid substitutions K392D and K409D, and the second subunit of the Fc domain comprises amino acid substitutions D356K and D399K (numbering according to Kabat EU index).

### (ii) Fc domain modifications reducing Fc receptor binding and/or effector function

The Fc domain confers to an antibody, such as a bispecific antibody, favorable pharmacokinetic properties, including a long serum half-life which contributes to good accumulation in the target tissue and a favorable tissue-blood distribution ratio. At the same time it may, however, lead to undesirable targeting of the antibody to cells expressing Fc receptors rather than to the preferred antigen-bearing cells. Moreover, the co-activation of Fc receptor signaling pathways may lead to cytokine release which, in combination with other immunostimulatory properties the antibody may have and the long half-life of the antibody, results in excessive activation of cytokine receptors and severe side effects upon systemic administration.

Accordingly, in particular embodiments, the Fc domain of the antibody, particularly bispecific antibody, comprised in the therapeutic agent exhibits reduced binding affinity to an Fc receptor and/or reduced effector function, as compared to a native IgG₁ Fc domain. In one such embodiment the Fc domain (or the molecule, e.g. antibody, comprising said Fc domain) exhibits less than 50%, preferably less than 20%, more preferably less than 10% and most preferably less than 5% of the binding affinity to an Fc receptor, as compared to a native IgG₁ Fc domain (or a corresponding molecule comprising a native IgG₁ Fc domain), and/or less than 50%, preferably less than 20%, more preferably less than 10% and most preferably less than 5% of the effector function, as compared to a native IgG₁ Fc domain domain (or a corresponding molecule comprising a native IgG₁ Fc domain). In one embodiment, the Fc domain (or the molecule, e.g. antibody, comprising said Fc domain) does not substantially bind to an Fc receptor and/or induce effector function. In a particular embodiment the Fc receptor is an Fcγ receptor. In one embodiment the Fc receptor is a human Fc receptor. In one embodiment the Fc receptor is an activating Fc receptor. In a specific embodiment the Fc receptor is an activating human Fcγ receptor, more specifically human FcγRIIIa, FcγRI or FcγRIIa, most specifically human FcγRIIIa. In one embodiment the effector function is one or more selected from the group of CDC, ADCC, ADCP, and cytokine secretion. In a particular embodiment the effector function is ADCC. In one embodiment the Fc domain exhibits substantially similar binding affinity to neonatal Fc receptor (FcRn), as compared to a native IgG₁ Fc domain domain. Substantially similar binding to FcRn is achieved when the Fc domain (or the molecule, e.g. antibody, comprising said Fc domain) exhibits greater than about 70%, particularly greater than about 80%, more particularly greater than about 90% of the binding affinity of a native IgG₁ Fc domain (or the corresponding molecule comprising a native IgG₁ Fc domain) to FcRn.

In certain embodiments the Fc domain is engineered to have reduced binding affinity to an Fc receptor and/or reduced effector function, as compared to a non-engineered Fc domain. In particular embodiments, the Fc domain comprises one or more amino acid mutation that reduces the binding affinity of the Fc domain to an Fc receptor and/or effector function. Typically, the same one or more amino acid mutation is present in each of the two subunits of the Fc domain. In one embodiment the amino acid mutation reduces the binding affinity of the Fc domain to an Fc receptor. In one embodiment the amino acid mutation reduces the binding affinity of the Fc domain to an Fc receptor by at least 2-fold, at least 5-fold, or at least 10-fold. In embodiments where there is more than one amino acid mutation that reduces the binding affinity of the Fc domain to the Fc receptor, the combination of these amino acid mutations may reduce the binding affinity of the Fc domain to an Fc receptor by at least 10-fold, at least 20-fold, or even at least 50-fold. In one embodiment the molecule, e.g. antibody, comprising an engineered Fc domain exhibits less than 20%, particularly less than 10%, more particularly less than 5% of the binding affinity to an Fc receptor as compared to a corresponding molecule comprising a non-engineered Fc domain. In a particular embodiment the Fc receptor is an Fcγ receptor. In some embodiments the Fc receptor is a human Fc receptor. In some embodiments the Fc receptor is an activating Fc receptor. In a specific embodiment the Fc receptor is an activating human Fcγ receptor, more specifically human FcγRIIIa, FcγRI or FcγRIIa, most specifically human FcγRIIIa. Preferably, binding to each of these receptors is reduced. In some embodiments binding affinity to a complement component, specifically binding affinity to C1q, is also reduced. In one embodiment binding affinity to neonatal Fc receptor (FcRn) is not reduced. Substantially similar binding to FcRn, i.e. preservation of the binding affinity of the Fc domain to said receptor, is achieved when the Fc domain (or the molecule, e.g. antibody, comprising said Fc domain) exhibits greater than about 70% of the binding affinity of a non-engineered form of the Fc domain (or a corresponding molecule comprising said non-engineered form of the Fc domain) to FcRn. The Fc domain, or molecule (e.g. antibody) comprising said Fc domain, may exhibit greater than about 80% and even greater than about 90% of such affinity. In certain embodiments the Fc domain is engineered to have reduced effector function, as compared to a non-engineered Fc domain. The reduced effector function can include, but is not limited to, one or more of the following: reduced complement dependent cytotoxicity (CDC), reduced antibody-dependent cell-mediated cytotoxicity (ADCC), reduced antibody-dependent cellular phagocytosis (ADCP), reduced cytokine secretion, reduced immune complex-mediated antigen uptake by antigen-presenting cells, reduced binding to NK cells, reduced binding to macrophages, reduced binding to monocytes, reduced binding to polymorphonuclear cells, reduced direct signaling inducing apoptosis, reduced crosslinking of target-bound antibodies, reduced dendritic cell maturation, or reduced T cell priming. In one embodiment the reduced effector function is one or more selected from the group of reduced CDC, reduced ADCC, reduced ADCP, and reduced cytokine secretion. In a particular embodiment the reduced effector function is reduced ADCC. In one embodiment the reduced ADCC is less than 20% of the ADCC induced by a non-engineered Fc domain (or a corresponding molecule comprising a non-engineered Fc domain).

In one embodiment the amino acid mutation that reduces the binding affinity of the Fc domain to an Fc receptor and/or effector function is an amino acid substitution. In one embodiment the Fc domain comprises an amino acid substitution at a position selected from the group of E233, L234, L235, N297, P331 and P329 (numberings according to Kabat EU index). In a more specific embodiment the Fc domain comprises an amino acid substitution at a position selected from the group of L234, L235 and P329 (numberings according to Kabat EU index). In some embodiments the Fc domain comprises the amino acid substitutions L234A and L235A (numberings according to Kabat EU index). In one such embodiment, the Fc domain is an IgG₁ Fc domain, particularly a human IgG₁ Fc domain. In one embodiment the Fc domain comprises an amino acid substitution at position P329. In a more specific embodiment the amino acid substitution is P329A or P329G, particularly P329G (numberings according to Kabat EU index). In one embodiment the Fc domain comprises an amino acid substitution at position P329 and a further amino acid substitution at a position selected from E233, L234, L235, N297 and P331 (numberings according to Kabat EU index). In a more specific embodiment the further amino acid substitution is E233P, L234A, L235A, L235E, N297A, N297D or P331S. In particular embodiments the Fc domain comprises amino acid substitutions at positions P329, L234 and L235 (numberings according to Kabat EU index). In more particular embodiments the Fc domain comprises the amino acid mutations L234A, L235A and P329G ("P329G LALA"). In one such embodiment, the Fc domain is an IgG₁ Fc domain, particularly a human IgG₁ Fc domain. The "P329G LALA" combination of amino acid substitutions almost completely abolishes Fcγ receptor (as well as complement) binding of a human IgG₁ Fc domain, as described in PCT publication no. WO 2012/130831, incorporated herein by reference in its entirety. WO 2012/130831 also describes methods of preparing such mutant Fc domains and methods for determining its properties such as Fc receptor binding or effector functions.

IgG₄ antibodies exhibit reduced binding affinity to Fc receptors and reduced effector functions as compared to IgG₁ antibodies. Hence, in some embodiments the Fc domain is an IgG₄ Fc domain, particularly a human IgG₄ Fc domain. In one embodiment the IgG₄ Fc domain comprises amino acid substitutions at position S228, specifically the amino acid substitution S228P (numberings according to Kabat EU index). To further reduce its binding affinity to an Fc receptor and/or its effector function, in one embodiment the IgG₄ Fc domain comprises an amino acid substitution at position L235, specifically the amino acid substitution L235E (numberings according to Kabat EU index). In another embodiment, the IgG₄ Fc domain comprises an amino acid substitution at position P329, specifically the amino acid substitution P329G (numberings according to Kabat EU index). In a particular embodiment, the IgG₄ Fc domain comprises amino acid substitutions at positions S228, L235 and P329, specifically amino acid substitutions S228P, L235E and P329G (numberings according to Kabat EU index). Such IgG₄ Fc domain mutants and their Fcγ receptor binding properties are described in PCT publication no. WO 2012/130831, incorporated herein by reference in its entirety.

In a particular embodiment the Fc domain exhibiting reduced binding affinity to an Fc receptor and/or reduced effector function, as compared to a native IgG₁ Fc domain, is a human IgG₁ Fc domain comprising the amino acid substitutions L234A, L235A and optionally P329G, or a human IgG₄ Fc domain comprising the amino acid substitutions S228P, L235E and optionally P329G (numberings according to Kabat EU index).

In certain embodiments N-glycosylation of the Fc domain has been eliminated. In one such embodiment the Fc domain comprises an amino acid mutation at position N297, particularly an amino acid substitution replacing asparagine by alanine (N297A) or aspartic acid (N297D) or glycine (N297G) (numberings according to Kabat EU index).

In addition to the Fc domains described hereinabove and in PCT publication no. WO 2012/130831, Fc domains with reduced Fc receptor binding and/or effector function also include those with substitution of one or more of Fc domain residues 238, 265, 269, 270, 297, 327 and 329 (U.S. Patent No. 6,737,056) (numberings according to Kabat EU index). Such Fc mutants include Fc mutants with substitutions at two or more of amino acid positions 265, 269, 270, 297 and 327, including the so-called "DANA" Fc mutant with substitution of residues 265 and 297 to alanine (US Patent No. 7,332,581).

Mutant Fc domains can be prepared by amino acid deletion, substitution, insertion or modification using genetic or chemical methods well known in the art. Genetic methods may include site-specific mutagenesis of the encoding DNA sequence, PCR, gene synthesis, and the like. The correct nucleotide changes can be verified for example by sequencing.

Binding to Fc receptors can be easily determined e.g. by ELISA, or by Surface Plasmon Resonance (SPR) using standard instrumentation such as a BIAcore instrument (GE Healthcare), and Fc receptors such as may be obtained by recombinant expression. Alternatively, binding affinity of Fc domains or molecules comprising an Fc domain for Fc receptors may be evaluated using cell lines known to express particular Fc receptors, such as human NK cells expressing FcγIIIa receptor.

Effector function of an Fc domain, or a molecule (e.g. an antibody) comprising an Fc domain, can be measured by methods known in the art. A suitable assay for measuring ADCC is described herein. Other examples of *in vitro* assays to assess ADCC activity of a molecule of interest are described in U.S. Patent No. 5,500,362; Hellstrom et al. Proc Natl Acad Sci USA 83, 7059-7063 (1986) and Hellstrom et al., Proc Natl Acad Sci USA 82, 1499-1502 (1985); U.S. Patent No. 5,821,337; Bruggemann et al., J Exp Med 166, 1351-1361 (1987). Alternatively, non-radioactive assays methods may be employed (see, for example, ACTI™ non-radioactive cytotoxicity assay for flow cytometry (CellTechnology, Inc. Mountain View, CA); and CytoTox 96^{®} non-radioactive cytotoxicity assay (Promega, Madison, WI)). Useful effector cells for such assays include peripheral blood mononuclear cells (PBMC) and Natural Killer (NK) cells. Alternatively, or additionally, ADCC activity of the molecule of interest may be assessed *in vivo,* e.g. in a animal model such as that disclosed in Clynes et al., Proc Natl Acad Sci USA 95, 652-656 (1998).

In some embodiments, binding of the Fc domain to a complement component, specifically to C1q, is reduced. Accordingly, in some embodiments wherein the Fc domain is engineered to have reduced effector function, said reduced effector function includes reduced CDC. C1q binding assays may be carried out to determine whether the Fc domain, or molecule (e.g. antibody) comprising the Fc domain, is able to bind C1q and hence has CDC activity. See e.g., C1q and C3c binding ELISA in WO 2006/029879 and WO 2005/100402. To assess complement activation, a CDC assay may be performed (see, for example, Gazzano-Santoro et al., J Immunol Methods 202, 163 (1996); Cragg et al., Blood 101, 1045-1052 (2003); and Cragg and Glennie, Blood 103, 2738-2743 (2004)).

### Antigen Binding Moieties

The antibody comprised in the therapeutic agent may be bispecific, i.e. it comprises at least two antigen binding moieties capable of specific binding to two distinct antigenic determinants. According to particular embodiments, the antigen binding moieties are Fab molecules (i.e. antigen binding domains composed of a heavy and a light chain, each comprising a variable and a constant domain). In one embodiment said Fab molecules are human. In another embodiment said Fab molecules are humanized. In yet another embodiment said Fab molecules comprise human heavy and light chain constant domains.

In some embodiments, at least one of the antigen binding moieties is a crossover Fab molecule. Such modification reduces mispairing of heavy and light chains from different Fab molecules, thereby improving the yield and purity of the antibody in recombinant production. In a particular crossover Fab molecule useful for the antibody, the variable domains of the Fab light chain and the Fab heavy chain (VL and VH, respectively) are exchanged. Even with this domain exchange, however, the preparation of the antibody may comprise certain side products due to a so-called Bence Jones-type interaction between mispaired heavy and light chains (see Schaefer et al, PNAS, 108 (2011) 11187-11191). To further reduce mispairing of heavy and light chains from different Fab molecules and thus increase the purity and yield of the desired antibody, charged amino acids with opposite charges may be introduced at specific amino acid positions in the CH1 and CL domains of either the Fab molecule(s) specifically binding to a target cell antigen, or the Fab molecule specifically binding to an activating T cell antigen. Charge modifications are made either in the conventional Fab molecule(s) comprised in the antibody (such as shown e.g. in Figures 1 A-C, G-J), or in the VH/VL crossover Fab molecule(s) comprised in the antibody (such as shown e.g. in Figure 1 D-F, K-N) (but not in both). In particular embodiments, the charge modifications are made in the conventional Fab molecule(s) comprised in the antibody (which in particular embodiments specifically bind(s) to the target cell antigen).

In a particular embodiment according to the invention, the antibody is capable of simultaneous binding to a target cell antigen, particularly a tumor cell antigen, and an activating T cell antigen, particularly CD3. In one embodiment, the antibody is capable of crosslinking a T cell and a target cell by simultaneous binding to a target cell antigen and an activating T cell antigen. In an even more particular embodiment, such simultaneous binding results in lysis of the target cell, particularly a tumor cell. In one embodiment, such simultaneous binding results in activation of the T cell. In other embodiments, such simultaneous binding results in a cellular response of a T lymphocyte, particularly a cytotoxic T lymphocyte, selected from the group of: proliferation, differentiation, cytokine secretion, cytotoxic effector molecule release, cytotoxic activity, and expression of activation markers. In one embodiment, binding of the antibody to the activating T cell antigen, particularly CD3, without simultaneous binding to the target cell antigen does not result in T cell activation.

In one embodiment, the antibody is capable of re-directing cytotoxic activity of a T cell to a target cell. In a particular embodiment, said re-direction is independent of MHC-mediated peptide antigen presentation by the target cell and and/or specificity of the T cell.

Particularly, a T cell according to any of the embodiments of the invention is a cytotoxic T cell. In some embodiments the T cell is a CD4⁺ or a CD8⁺ T cell, particularly a CD8⁺ T cell.

### (i) Activating T cell antigen binding moiety

In some embodiments, an antibody comprised in the therapeutic agent, particularly a bispecific antibody, comprises at least one antigen binding moiety, particularly a Fab molecule, which specifically binds to an activating T cell antigen (also referred to herein as an "activating T cell antigen binding moiety, or activating T cell antigen binding Fab molecule"). In a particular embodiment, the antibody comprises not more than one antigen binding moiety capable of specific binding to an activating T cell antigen. In one embodiment, the antibody provides monovalent binding to the activating T cell antigen.

In particular embodiments, the antigen binding moiety which specifically binds an activating T cell antigen is a crossover Fab molecule as described herein, i.e. a Fab molecule wherein the variable domains VH and VL or the constant domains CH1 and CL of the Fab heavy and light chains are exchanged / replaced by each other. In such embodiments, the antigen binding moiety(ies) which specifically binds a target cell antigen is preferably a conventional Fab molecule. In embodiments where there is more than one antigen binding moiety, particularly Fab molecule, which specifically binds to a target cell antigen comprised in the antibody, the antigen binding moiety which specifically binds to an activating T cell antigen preferably is a crossover Fab molecule and the antigen binding moieties which specifically bind to a target cell antigen are conventional Fab molecules.

In alternative embodiments, the antigen binding moiety which specifically binds an activating T cell antigen is a conventional Fab molecule. In such embodiments, the antigen binding moiety(ies) which specifically binds a target cell antigen is a crossover Fab molecule as described herein, i.e. a Fab molecule wherein the variable domains VH and VL or the constant domains CH1 and CL of the Fab heavy and light chains are exchanged / replaced by each other.

In one embodiment, the activating T cell antigen is selected from the group consisting of CD3, CD28, CD 137 (also known as 4-1BB), CD40, CD226, OX40, GITR, CD27, HVEM, and CD127.

In a particular embodiment, the activating T cell antigen is CD3, particularly human CD3 (SEQ ID NO: 91) or cynomolgus CD3 (SEQ ID NO: 92), most particularly human CD3. In a particular embodiment the activating T cell antigen binding moiety is cross-reactive for (i.e. specifically binds to) human and cynomolgus CD3. In some embodiments, the activating T cell antigen is the epsilon subunit of CD3 (CD3 epsilon).

In some embodiments, the activating T cell antigen binding moiety specifically binds to CD3, particularly CD3 epsilon, and comprises at least one heavy chain complementarity determining region (CDR) selected from the group consisting of SEQ ID NO: 12, SEQ ID NO: 13 and SEQ ID NO: 14 and at least one light chain CDR selected from the group of SEQ ID NO: 15, SEQ ID NO: 16, SEQ ID NO: 17.

In one embodiment the CD3 binding antigen binding moiety, particularly Fab molecule, comprises a heavy chain variable region comprising the heavy chain CDR1 of SEQ ID NO: 12, the heavy chain CDR2 of SEQ ID NO: 13, the heavy chain CDR3 of SEQ ID NO: 14, and a light chain variable region comprising the light chain CDR1 of SEQ ID NO: 15, the light chain CDR2 of SEQ ID NO: 16, and the light chain CDR3 of SEQ ID NO: 17.

In one embodiment the CD3 binding antigen binding moiety, particularly Fab molecule, comprises a heavy chain variable region sequence that is at least about 95%, 96%, 97%, 98%, 99% or 100% identical to SEQ ID NO: 18 and a light chain variable region sequence that is at least about 95%, 96%, 97%, 98%, 99% or 100% identical to SEQ ID NO: 19.

In one embodiment the CD3 binding antigen binding moiety, particularly Fab molecule, comprises a heavy chain variable region comprising the amino acid sequence of SEQ ID NO: 18 and a light chain variable region comprising the amino acid sequence of SEQ ID NO: 19.

In one embodiment the CD3 binding antigen binding moiety, particularly Fab molecule, comprises the heavy chain variable region sequence of SEQ ID NO: 18 and the light chain variable region sequence of SEQ ID NO: 19.

### (ii) Target cell antigen binding moiety

In some embodiments, an antibody comprised in the therapeutic agent, particularly a bispecific antibody, comprises at least one antigen binding moiety, particularly a Fab molecule, which specifically binds to a target cell antigen. In certain embodiments, the antibody comprises two antigen binding moieties, particularly Fab molecules, which specifically bind to a target cell antigen. In a particular such embodiment, each of these antigen binding moieties specifically binds to the same antigenic determinant. In an even more particular embodiment, all of these antigen binding moieties are identical, i.e. they comprise the same amino acid sequences including the same amino acid substitutions in the CH1 and CL domain as described herein (if any). In one embodiment, the antibody comprises an immunoglobulin molecule which specifically binds to a target cell antigen. In one embodiment the antibody comprises not more than two antigen binding moieties, particularly Fab molecules, which specifically bind to a target cell antigen.

In particular embodiments, the antigen binding moiety(ies) which specficially bind to a target cell antigen is/are a conventional Fab molecule. In such embodiments, the antigen binding moiety(ies) which specifically binds an activating T cell antigen is a crossover Fab molecule as described herein, i.e. a Fab molecule wherein the variable domains VH and VL or the constant domains CH1 and CL of the Fab heavy and light chains are exchanged / replaced by each other.

In alternative embodiments, the antigen binding moiety(ies)which specficially bind to a target cell antigen is/are a crossover Fab molecule as described herein, i.e. a Fab molecule wherein the variable domains VH and VL or the constant domains CH1 and CL of the Fab heavy and light chains are exchanged / replaced by each other. In such embodiments, the antigen binding moiety(ies)which specifically binds an activating T cell antigen is a conventional Fab molecule.

The target cell antigen binding moiety is able to direct the antibody to a target site, for example to a specific type of tumor cell that expresses the target cell antigen.

In one embodiment, the target cell antigen is a B-cell antigen, particularly a malignant B-cell antigen. In one embodiment, the target cell antigen is a cell surface antigen. In one embodiment the target cell antigen is selected from the group consisting of CD20, CD19, CD22, ROR-1, CD37 and CD5.

In one embodiment, the target cell antigen is CD20, particularly human CD20.

In one embodiment, the antigen binding moiety, particularly Fab molecule, which specifically binds to CD20 comprises a heavy chain variable region comprising the heavy chain complementarity determining region (CDR) 1 of SEQ ID NO: 4, the heavy chain CDR 2 of SEQ ID NO: 5, and the heavy chain CDR 3 of SEQ ID NO: 6, and a light chain variable region comprising the light chain CDR 1 of SEQ ID NO: 7, the light chain CDR 2 of SEQ ID NO: 8 and the light chain CDR 3 of SEQ ID NO: 9. In a further embodiment, the antigen binding moiety, particularly Fab molecule, which specifically binds to CD20 comprises a heavy chain variable region that is at least 95%, 96%, 97%, 98%, or 99% identical to the sequence of SEQ ID NO: 10, and a light chain variable region that is at least 95%, 96%, 97%, 98%, or 99% identical to the sequence of SEQ ID NO: 11. In still a further embodiment, the antigen binding moiety, particularly Fab molecule, which specifically binds to CD20 comprises the heavy chain variable region sequence of SEQ ID NO: 10, and the light chain variable region sequence of SEQ ID NO: 11.

In one embodiment, the target cell antigen is CD 19, particularly human CD 19.

In one embodiment, the antigen binding moiety, particularly Fab molecule, which specifically binds to CD 19 comprises a heavy chain variable region comprising the heavy chain complementarity determining region (CDR) 1 of SEQ ID NO: 24, the heavy chain CDR 2 of SEQ ID NO: 25, and the heavy chain CDR 3 of SEQ ID NO: 26, and a light chain variable region comprising the light chain CDR 1 of SEQ ID NO: 27, the light chain CDR 2 of SEQ ID NO: 28 and the light chain CDR 3 of SEQ ID NO: 29. In a further embodiment, the antigen binding moiety, particularly Fab molecule, which specifically binds to CD 19 comprises a heavy chain variable region that is at least 95%, 96%, 97%, 98%, or 99% identical to the sequence of SEQ ID NO: 30, and a light chain variable region that is at least 95%, 96%, 97%, 98%, or 99% identical to the sequence of SEQ ID NO: 31. In still a further embodiment, the antigen binding moiety, particularly Fab molecule, which specifically binds to CD19 comprises the heavy chain variable region sequence of SEQ ID NO: 30, and the light chain variable region sequence of SEQ ID NO: 31.

In another embodiment, the antigen binding moiety, particularly Fab molecule, which specifically binds to CD19 comprises a heavy chain variable region comprising the heavy chain complementarity determining region (CDR) 1 of SEQ ID NO: 35, the heavy chain CDR 2 of SEQ ID NO: 36, and the heavy chain CDR 3 of SEQ ID NO: 37, and a light chain variable region comprising the light chain CDR 1 of SEQ ID NO: 38, the light chain CDR 2 of SEQ ID NO: 39 and the light chain CDR 3 of SEQ ID NO: 40. In a further embodiment, the antigen binding moiety, particularly Fab molecule, which specifically binds to CD 19 comprises a heavy chain variable region that is at least 95%, 96%, 97%, 98%, or 99% identical to the sequence of SEQ ID NO: 41, and a light chain variable region that is at least 95%, 96%, 97%, 98%, or 99% identical to the sequence of SEQ ID NO: 42. In still a further embodiment, the antigen binding moiety, particularly Fab molecule, which specifically binds to CD19 comprises the heavy chain variable region sequence of SEQ ID NO: 41, and the light chain variable region sequence of SEQ ID NO: 42.

In another embodiment, the antigen binding moiety, particularly Fab molecule, which specifically binds to CD19 comprises
(i) a heavy chain variable region comprising the heavy chain complementarity determining region (CDR) 1 of SEQ ID NO: 43, the heavy chain CDR 2 of SEQ ID NO: 44, and the heavy chain CDR 3 of SEQ ID NO: 45, and a light chain variable region comprising the light chain CDR 1 of SEQ ID NO: 46, the light chain CDR 2 of SEQ ID NO: 47 and the light chain CDR 3 of SEQ ID NO: 48;
(ii) a heavy chain variable region comprising the heavy chain complementarity determining region (CDR) 1 of SEQ ID NO: 51, the heavy chain CDR 2 of SEQ ID NO: 52, and the heavy chain CDR 3 of SEQ ID NO: 53, and a light chain variable region comprising the light chain CDR 1 of SEQ ID NO: 54, the light chain CDR 2 of SEQ ID NO: 55 and the light chain CDR 3 of SEQ ID NO: 56;
(iii) a heavy chain variable region comprising the heavy chain complementarity determining region (CDR) 1 of SEQ ID NO: 59, the heavy chain CDR 2 of SEQ ID NO: 60, and the heavy chain CDR 3 of SEQ ID NO: 61, and a light chain variable region comprising the light chain CDR 1 of SEQ ID NO: 62, the light chain CDR 2 of SEQ ID NO: 63 and the light chain CDR 3 of SEQ ID NO: 64;
(iv) a heavy chain variable region comprising the heavy chain complementarity determining region (CDR) 1 of SEQ ID NO: 67, the heavy chain CDR 2 of SEQ ID NO: 68, and the heavy chain CDR 3 of SEQ ID NO: 69, and a light chain variable region comprising the light chain CDR 1 of SEQ ID NO: 70, the light chain CDR 2 of SEQ ID NO: 71 and the light chain CDR 3 of SEQ ID NO: 72;
(v) a heavy chain variable region comprising the heavy chain complementarity determining region (CDR) 1 of SEQ ID NO: 75, the heavy chain CDR 2 of SEQ ID NO: 76, and the heavy chain CDR 3 of SEQ ID NO: 77, and a light chain variable region comprising the light chain CDR 1 of SEQ ID NO: 78, the light chain CDR 2 of SEQ ID NO: 79 and the light chain CDR 3 of SEQ ID NO: 80; or
(vi) a heavy chain variable region comprising the heavy chain complementarity determining region (CDR) 1 of SEQ ID NO: 83, the heavy chain CDR 2 of SEQ ID NO: 84, and the heavy chain CDR 3 of SEQ ID NO: 85, and a light chain variable region comprising the light chain CDR 1 of SEQ ID NO: 86, the light chain CDR 2 of SEQ ID NO: 87 and the light chain CDR 3 of SEQ ID NO: 88.

In a further embodiment, the antigen binding moiety, particularly Fab molecule, which specifically binds to CD19 comprises
(i) a heavy chain variable region that is at least 95%, 96%, 97%, 98%, or 99% identical to the sequence of SEQ ID NO: 49, and a light chain variable region that is at least 95%, 96%, 97%, 98%, or 99% identical to the sequence of SEQ ID NO: 50;
(ii) a heavy chain variable region that is at least 95%, 96%, 97%, 98%, or 99% identical to the sequence of SEQ ID NO: 57, and a light chain variable region that is at least 95%, 96%, 97%, 98%, or 99% identical to the sequence of SEQ ID NO: 58;
(iii) a heavy chain variable region that is at least 95%, 96%, 97%, 98%, or 99% identical to the sequence of SEQ ID NO: 65, and a light chain variable region that is at least 95%, 96%, 97%, 98%, or 99% identical to the sequence of SEQ ID NO: 66;
(iv) a heavy chain variable region that is at least 95%, 96%, 97%, 98%, or 99% identical to the sequence of SEQ ID NO: 73, and a light chain variable region that is at least 95%, 96%, 97%, 98%, or 99% identical to the sequence of SEQ ID NO: 74;
(v) a heavy chain variable region that is at least 95%, 96%, 97%, 98%, or 99% identical to the sequence of SEQ ID NO: 81, and a light chain variable region that is at least 95%, 96%, 97%, 98%, or 99% identical to the sequence of SEQ ID NO: 82; or
(vi) a heavy chain variable region that is at least 95%, 96%, 97%, 98%, or 99% identical to the sequence of SEQ ID NO: 89, and a light chain variable region that is at least 95%, 96%, 97%, 98%, or 99% identical to the sequence of SEQ ID NO: 90.

In still a further embodiment, the antigen binding moiety, particularly Fab molecule, which specifically binds to CD19 comprises
(i) the heavy chain variable region sequence of SEQ ID NO: 49, and the light chain variable region sequence of SEQ ID NO: 50;
(ii) the heavy chain variable region sequence of SEQ ID NO: 57, and the light chain variable region sequence of SEQ ID NO: 58;
(iii) the heavy chain variable region sequence of SEQ ID NO: 65, and the light chain variable region sequence of SEQ ID NO: 66;
(iv) the heavy chain variable region sequence of SEQ ID NO: 73, and the light chain variable region sequence of SEQ ID NO: 74;
(v) the heavy chain variable region sequence of SEQ ID NO: 81, and the light chain variable region sequence of SEQ ID NO: 82; or
(vi) the heavy chain variable region sequence of SEQ ID NO: 89, and the light chain variable region sequence of SEQ ID NO: 90.

### Charge modification

An antibody, particularly a multispecific antibody, comprised in the therapeutic agent may comprise amino acid substitutions in Fab molecules comprised therein which are particularly efficient in reducing mispairing of light chains with non-matching heavy chains (Bence-Jones-type side products), which can occur in the production of Fab-based bi-/multispecific antigen binding molecules with a VH/VL exchange in one (or more, in case of molecules comprising more than two antigen-binding Fab molecules) of their binding arms (see also PCT publication no. WO 2015/150447, particularly the examples therein, incorporated herein by reference in its entirety).

Accordingly, in particular embodiments, an antibody comprised in the therapeutic agent comprises
(a) a first Fab molecule which specifically binds to a first antigen
(b) a second Fab molecule which specifically binds to a second antigen, and wherein the variable domains VL and VH of the Fab light chain and the Fab heavy chain are replaced by each other,
wherein the first antigen is an activating T cell antigen and the second antigen is a target cell antigen, or the first antigen is a target cell antigen and the second antigen is an activating T cell antigen; and
wherein
i) in the constant domain CL of the first Fab molecule under a) the amino acid at position 124 is substituted by a positively charged amino acid (numbering according to Kabat), and wherein in the constant domain CH1 of the first Fab molecule under a) the amino acid at position 147 or the amino acid at position 213 is substituted by a negatively charged amino acid (numbering according to Kabat EU index); or
ii) in the constant domain CL of the second Fab molecule under b) the amino acid at position 124 is substituted by a positively charged amino acid (numbering according to Kabat), and wherein in the constant domain CH1 of the second Fab molecule under b) the amino acid at position 147 or the amino acid at position 213 is substituted by a negatively charged amino acid (numbering according to Kabat EU index).

The antibody does not comprise both modifications mentioned under i) and ii). The constant domains CL and CH1 of the second Fab molecule are not replaced by each other (i.e. remain unexchanged).

In one embodiment of the antibody, in the constant domain CL of the first Fab molecule under a) the amino acid at position 124 is substituted independently by lysine (K), arginine (R) or histidine (H) (numbering according to Kabat) (in one preferred embodiment independently by lysine (K) or arginine (R)), and in the constant domain CH1 of the first Fab molecule under a) the amino acid at position 147 or the amino acid at position 213 is substituted independently by glutamic acid (E), or aspartic acid (D) (numbering according to Kabat EU index).

In a further embodiment, in the constant domain CL of the first Fab molecule under a) the amino acid at position 124 is substituted independently by lysine (K), arginine (R) or histidine (H) (numbering according to Kabat), and in the constant domain CH1 of the first Fab molecule under a) the amino acid at position 147 is substituted independently by glutamic acid (E), or aspartic acid (D) (numbering according to Kabat EU index).

In a particular embodiment, in the constant domain CL of the first Fab molecule under a) the amino acid at position 124 is substituted independently by lysine (K), arginine (R) or histidine (H) (numbering according to Kabat) (in one preferred embodiment independently by lysine (K) or arginine (R)) and the amino acid at position 123 is substituted independently by lysine (K), arginine (R) or histidine (H) (numbering according to Kabat) (in one preferred embodiment independently by lysine (K) or arginine (R)), and in the constant domain CH1 of the first Fab molecule under a) the amino acid at position 147 is substituted independently by glutamic acid (E), or aspartic acid (D) (numbering according to Kabat EU index) and the amino acid at position 213 is substituted independently by glutamic acid (E), or aspartic acid (D) (numbering according to Kabat EU index).

In a more particular embodiment, in the constant domain CL of the first Fab molecule under a) the amino acid at position 124 is substituted by lysine (K) (numbering according to Kabat) and the amino acid at position 123 is substituted by lysine (K) or arginine (R) (numbering according to Kabat), and in the constant domain CH1 of the first Fab molecule under a) the amino acid at position 147 is substituted by glutamic acid (E) (numbering according to Kabat EU index) and the amino acid at position 213 is substituted by glutamic acid (E) (numbering according to Kabat EU index).

In an even more particular embodiment, in the constant domain CL of the first Fab molecule under a) the amino acid at position 124 is substituted by lysine (K) (numbering according to Kabat) and the amino acid at position 123 is substituted by arginine (R) (numbering according to Kabat), and in the constant domain CH1 of the first Fab molecule under a) the amino acid at position 147 is substituted by glutamic acid (E) (numbering according to Kabat EU index) and the amino acid at position 213 is substituted by glutamic acid (E) (numbering according to Kabat EU index).

In particular embodiments, the constant domain CL of the first Fab molecule under a) is of kappa isotype.

Alternatively, the amino acid substitutions according to the above embodiments may be made in the constant domain CL and the constant domain CH1 of the second Fab molecule under b) instead of in the constant domain CL and the constant domain CH1 of the first Fab molecule under a). In particular such embodiments, the constant domain CL of the second Fab molecule under b) is of kappa isotype.

The antibody may further comprise a third Fab molecule which specifically binds to the first antigen. In particular embodiments, said third Fab molecule is identical to the first Fab molecule under a). In these embodiments, the amino acid substitutions according to the above embodiments will be made in the constant domain CL and the constant domain CH1 of each of the first Fab molecule and the third Fab molecule. Alternatively, the amino acid substitutions according to the above embodiments may be made in the constant domain CL and the constant domain CH1 of the second Fab molecule under b), but not in the constant domain CL and the constant domain CH1 of the first Fab molecule and the third Fab molecule.

In particular embodiments, the antibody further comprises an Fc domain composed of a first and a second subunit capable of stable association.

### Treatment regimen

According to the invention, the Type II anti-CD20 antibody and the therapeutic agent may be administered in various ways (e.g. with regard to the route of administration, dose and/or timing), as long as the Type II anti-CD20 antibody is administered prior to the therapeutic agent and that the administration of the Type II anti-CD20 antibody has effectively induced a reduction of the number of B cells in the treated subject by the time the therapeutic agent is administered.

Without wishing to be bound by theory, the reduction of the number of B cells in the subject prior to administration of the therapeutic agent will reduce or prevent cytokine release associated with administration of the therapeutic agent, and will thus reduce or prevent adverse events (such as IRRs) in the subject associated with the administration of the therapeutic agent.

In one embodiment, the treatment regimen effectively reduces cytokine release in the subject associated with the administration of the therapeutic agent as compared to a corresponding treatment regimen without the administration of the Type II anti-CD20 antibody. In one embodiment, cytokine release is reduced at least 2-fold, at least 3-fold, at least 4-fold, at least 5-fold, at least 10-fold, at least 20-fold, at least 50-fold, or at least 100-fold as compared to a corresponding treatment regimen without the administration of the Type II anti-CD20 antibody. In one embodiment, cytokine release is essentially prevented. In one embodiment, the reduction or prevention of cytokine release is 1, 2, 3, 4, 5, 6, 7, 8, 9, 10, 11, 12, 13, 14, 15, 16, 17, 18, 19, 20, 21, 22, 23 or 24 hours after administration of the therapeutic agent. In one embodiment, the reduction or prevention of cytokine release is within the first 24 hours after administration of the therapeutic agent.

In one embodiment, the cytokine concentration in the subject (as measured e.g. in a blood sample taken from the subject) after administration of the therapeutic agent does not exceed the cytokine concentration in the subject prior to administration of the therapeutic agent. In one embodiment, the cytokine concentration in the subject after administration of the therapeutic agent does not exceed the cytokine concentration in the subject prior to administration of the therapeutic agent by more than 1.1-fold, more than 1.2-fold, more than 1.5-fold, more than 2-fold, more than 3-fold, more than 4-fold, more than 5-fold, more than 10-fold, more than 20-fold, more than 50-fold or more than 100-fold. In one embodiment, the cytokine concentration in the subject after administration of the therapeutic agent is increased less than 1.1-fold, less than 1.2-fold, less than 1.5-fold, less than 2-fold, less than 3-fold, less than 4-fold, less than 5-fold, less than 10-fold, less than 20-fold, less than 50-fold or less than 100-fold, as compared to the cytokine concentration in the subject prior to administration of the therapeutic agent. In one embodiment, the cytokine concentration in the subject after administration of the therapeutic agent is the cytokine concentration at 1, 2, 3, 4, 5, 6, 7, 8, 9, 10, 11, 12, 13, 14, 15, 16, 17, 18, 19, 20, 21, 22, 23 or 24 hours after administration of the therapeutic agent. In one embodiment, the cytokine concentration in the subject after administration of the therapeutic agent is the cytokine concentration within the first 24 hours after administration of the therapeutic agent.

In one embodiment, essentially no increase in the concentration of cytokines is detectable in the subject after administration of the therapeutic agent, particularly 1, 2, 3, 4, 5, 6, 7, 8, 9, 10, 11, 12, 13, 14, 15, 16, 17, 18, 19, 20, 21, 22, 23 or 24 hours after administration of the therapeutic agent.

Cytokines can be detected by methods known in the art, such as e.g. ELISA, FACS or Luminex® assay.

Cytokines can be detected e.g. in a blood sample taken from the subject. In one embodiment, the cytokine concentration is the blood of the subject.

In some embodiments, the cytokine is one or more cytokine(s) selected from the group consisting of tumor necrosis factor alpha (TNF-α), interferon gamma (IFN-γ), interleukin-6 (IL-6), interleukin-10 (IL-10), interleukin-2 (IL-2) and interleukin-8 (IL-8), particularly the group consisting of TNF-α, IFN-γ and IL-6. In some embodiments, the cytokine is TNF-α. In some embodiments, the cytokine is IFN-γ. In some embodiments, the cytokine is IL-6. In some embodiments, the cytokine is IL-10. In some embodiments, the cytokine is IL-2. In some embodiments, the cytokine is IL-8.

In some embodiments, the treatment regimen increases the safety of the therapeutic agent, as compared to a corresponding treatment regimen without the administration of the Type II anti-CD20 antibody. In some embodiments, the treatment regimen reduces adverse events in the subject, as compared to a corresponding treatment regimen without the administration of the Type II anti-CD20 antibody. In some embodiments, the treatment regimen increases the efficacy of the therapeutic agent, as compared to a corresponding treatment regimen without the administration of the Type II anti-CD20 antibody. In some embodiments, the treatment regimen increases the serum half-life of the therapeutic agent, as compared to a corresponding treatment regimen without the administration of the Type II anti-CD20 antibody. In some embodiments, the treatment regimen reduces toxicity of the therapeutic agent, as compared to a corresponding treatment regimen without the administration of the Type II anti-CD20 antibody.

According to the invention, the period of time between the administration of the Type II anti-CD20 antibody and the administration of the therapeutic agent is sufficient for reduction of the number of B-cells in the subject in response to the administration of the Type II CD20 antibody.

In one embodiment, the period of time is 3 days to 21 days, 5 days to 20 days, 7 days to 21 days, 7 days to 14 days, 5 days to 15 days, 7 days to 15 days, 8 days to 15 days, 10 days to 20 days, 10 days to 15 days, 11 days to 14 days, or 12 days to 13 days. In one embodiment, the period of time is 7 days to 14 days. In one embodiment, the period of time is 5 days to 10 days. In a particular embodiment, the period of time is 7 days.

In one embodiment, the period of time is about about 3 days, about 4 days, about 5 days, about 6 days, about 7 days, about 8 days, about 9 days, about 10 days, about 11 days, about 12 days, about 13 days, about 14 days, about 15 days, about 16 days, about 17 days, about 18 days, about 19 days, about 20 days, about 21 days, about 22 days, about 23 days, about 24 days, about 25 days, about 26 days, about 27 days, about 28 days, about 29 days, or about 30 days.

In one embodiment, the period of time is at least 3 days, at least 4 days, at least 5 days, at least 6 days, at least 7 days, at least 8 days, at least 9 days, at least 10 days, at least 11 days, at least 12 days, at least 13 days, at least 14 days, or at least 15 days. In a particular embodiment, the period of time is at least 5 days. In a further particular embodiment, the period of time is at least 7 days.

In one embodiment, the period of time is between the last administration of the Type II anti-CD20 antibody and the (first, if several) administration of the therapeutic agent. In one embodiment, no administration of the therapeutic agent is made during the period of time.

In a particular embodiment, the reduction of the number of B cells is in the blood of the subject. In one embodiment, the B cells are peripheral blood B cells. In one embodiment, the B cells are malignant and normal B cells. In one embodiment, the B cells are malignant B cells.

In some embodiments, the reduction of B cells is in a tissue of the subject. In one embodiment, the tissue is a tumor. In one embodiment, the tissue is a lymph node. In one embodiment, the tissue is spleen. In one embodiment, the tissue is the marginal zone of spleen. In one embodiment, the B cells are lymph node B cells. In one embodiment, the B cells are splenic B cells. In one embodiment, the B cells are splenic marginal zone B cells. In one embodiment, the B cells are CD20-positive B cells, i.e. B cells expressing CD20 on their surface.

In one embodiment, the reduction of the number of B cells is a reduction of at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, or at least about 95%. In one embodiment, the reduction of the number of B cells is a complete elimination of B cells. In a particular embodiment, the reduction of the number of B cells is a reduction of at least 90%, particularly at least 95%, of the number of B cells in the (peripheral) blood of the subject. In one embodiment, the reduction of the number of B cells is a reduction as compared to the number of B cells in the subject prior to the (first, if several) administration of the Type II anti-CD20 antibody to the subject.

The number of B cells in the subject may be determined by any method known in the art suitable for quantifying B cells in patient blood or tissue, such as flow cytometric, immunohistochemical or immunofluorescent methods, using antibodies against B cell markers such as CD20, CD 19, and/or PAX5.

The number of B cells may also be determined indirectly, by quantification of protein or mRNA levels of B-cell markers in patient blood or tissues. Suitable methods known in the art for the determination of specific protein levels include immunoassay methods such as enzyme-linked immunosorbent assay (ELISA), or Western Blot, methods for determination of mRNA levels include for example quantitative RT-PCR or microarray technologies. All the above mentioned methods and technologies are well known in the art and can be deduced from standard textbooks such as Lottspeich (Bioanalytik, Spektrum Akademisher Verlag, 1998) or Sambrook and Russell (Molecular Cloning: A Laboratory Manual, CSH Press, Cold Spring Harbor, NY, U.S.A., 2001).

In certain embodiments, the reduction of the number of B cells is determined by quantification of B cells in the blood of the subject (e.g. in a blood sample taken from the subject). In one such embodiment, B cells are quantified by flow cytometric analysis. Flow cytometric methods (FACS) are well known in the art for the quantification of cells in blood or tissue samples. In particular, they allow determining the number of cells expressing a specific antigen (e.g. CD20 and/or CD19) among a defined total number of cells in a blood or tissue sample (e.g. a blood sample, or (part of) a tissue biopsy). In one embodiment, B cells are quantified by flow cytometric analysis using an anti-CD 19 antibody and/or an anti-CD20 antibody.

In other embodiments, the reduction of the number of B cells is determined by quantification of B cells in a tissue, e.g. a tumor, of said individual (e.g. in a tissue biopsy taken from the subject). In one such embodiment, B cells are quantified by immunohistochemical or immunofluorescent analysis. In one embodiment, B cells are quantified by immunohistochemical analysis using an anti-CD 19 antibody, an anti-CD20 antibody and/or an anti-PAX5 antibody.

Methods of the present invention can be applied in the treatment of a variety of diseases, depending on the therapeutic agent(s) used. The methods are particularly useful, however, in the treatment of B-cell proliferative disorders, particularly CD20-positive B-cell disorders, where (CD20-positive) B-cells are present in large quantities (i.e. an increased number of B-cells is present in the subject suffering from the disorder, as compared to a healthy subject). Thus, in one embodiment, the disease is a B cell proliferative disorder, particularly a CD20-positive B-cell disorder.

In one embodiment, the disease is selected from the group consisting of Non-Hodgkin lymphoma (NHL), acute lymphocytic leukemia (ALL), chronic lymphocytic leukemia (CLL), diffuse large B-cell lymphoma (DLBCL), follicular lymphoma (FL), mantle-cell lymphoma (MCL), marginal zone lymphoma (MZL), Multiple myeloma (MM) or Hodgkin lymphoma (HL). In one embodiment, the disease is selected from the group consisting of Non-Hodgkin lymphoma (NHL), acute lymphocytic leukemia (ALL), chronic lymphocytic leukemia (CLL), diffuse large B-cell lymphoma (DLBCL), follicular lymphoma (FL), mantle-cell lymphoma (MCL) and marginal zone lymphoma (MZL).

In a particular embodiment, the disease is NHL, particularly relapsed/refractory (r/r) NHL. In one embodiment, the disease is DLBCL. In one embodiment, the disease is FL. In one embodiment, the disease is MCL. In one embodiment, the disease is MZL.

A skilled artisan readily recognizes that in many cases the therapeutic agent may not provide a cure but may only provide partial benefit. In some embodiments, a physiological change having some benefit is also considered therapeutically beneficial. Thus, in some embodiments, an amount of therapeutic agent that provides a physiological change is considered an "effective amount" or a "therapeutically effective amount".

The subject, patient, or individual in need of treatment is typically a mammal, more specifically a human.

In certain embodiments, the subject is a human. In one embodiment, the subject suffers from a B-cell proliferative disorder, particularly from Non-Hodgkin lymphoma (NHL), acute lymphocytic leukemia (ALL), chronic lymphocytic leukemia (CLL), diffuse large B-cell lymphoma (DLBCL), follicular lymphoma (FL), mantle-cell lymphoma (MCL), marginal zone lymphoma (MZL), Multiple myeloma (MM) or Hodgkin lymphoma (HL).

In one embodiment, the subject suffers from relapsed/refractory (r/r) NHL.

### Administration of the Type II anti-CD20 antibody

According to the invention, the period of time between the administration of the Type II anti-CD20 antibody and the administration of the therapeutic agent and the dose of the Type II anti-CD20 antibody are chosen such as to effectively reduce the number of B cells in the subject prior to administration of the therapeutic agent.

The Type II anti-CD20 antibody can be administered by any suitable means, including parenteral, intrapulmonary, and intranasal, and, if desired for local treatment, intralesional administration. Parenteral infusions include intramuscular, intravenous, intraarterial, intraperitoneal, or subcutaneous administration. Dosing can be by any suitable route, e.g. by injections, such as intravenous or subcutaneous injections, depending in part on whether the administration is brief or chronic. Various dosing schedules including but not limited to single or multiple administrations over various time-points, bolus administration, and pulse infusion are contemplated herein. In one embodiment, the Type II anti-CD20 antibody is administered parenterally, particularly intravenously, e.g. by intravenous infusion.

The Type II anti-CD20 antibody would be formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners.

In one embodiment, the administration of the Type II anti-CD20 antibody is a single administration. In another embodiment, the administration of the Type II anti-CD20 antibody is two or more separate administrations. In one embodiment, the two or more separate administrations are on two or more consecutive days. In one embodiment, no further administration of the Type II anti-CD20 antibody is made to the subject before or after the administration of the therapeutic agent. In one embodiment, the administration of the Type II anti-CD20 antibody is a single administration, or two administrations on two consecutive days, and no further administration of the Type II anti-CD20 antibody is made. In one embodiment, the period of time is between the last administration of the Type II anti-CD20 antibody and the (first, if several) administration of the therapeutic agent.

In one embodiment, the administration of the Type II anti-CD20 antibody is a dose of Type II anti-CD20 antibody effective for the reduction of B cells in the subject. In one embodiment, the dose of Type II anti-CD20 antibody is effective in reducing the number of B cells in the subject within the period of time between the administration of the Type II anti-CD20 antibody and the administration of the therapeutic agent. In one embodiment, the period of time between the administration of the Type II anti-CD20 antibody and the administration of the therapeutic agent and the administered dose of Type II anti-CD20 antibody is sufficient for reduction of the number of B-cells in the subject in response to the administration of the Type II CD20 antibody.

In one embodiment, the administration of the Type II anti-CD20 antibody is a dose of about 2 g Type II anti-CD20 antibody. The dose of about 2 g Type II anti-CD20 antibody may be administered to the subject as a single administration of about 2 g, or as several administrations, e.g. two administrations of about 1 g each or three administrations of e. g. 100 mg, 900 mg and 1000 mg. In one embodiment, one administration of about 2 g Type II anti-CD20 antibody is made to the subject. In another embodiment, two administrations of about 1 g Type II anti-CD20 antibody each are made to the subject on two consecutive days. In still another embodiment, three administrations ((i) to (iii)) of (i) about 100 mg Type II anti-CD20 antibody, (ii) about 900 mg Type II anti-CD20 antibody, and (iii) about 1000 mg Type II anti-CD20 antibody are made to the subject on three consecutive days. In one embodiment, two administration of about 1 g Type II anti-CD20 antibody are made to the subject on two consecutive days, 10 days to 15 days before the administration of the therapeutic agent. In one embodiment, one administration of about 2 g Type II anti-CD20 antibody is made to the subject 10 days to 15 days before the administration of the therapeutic agent. In one embodiment, no further administration of the Type II anti-CD20 antibody is made to the subject. In one embodiment, no administration of the therapeutic agent is made to the subject prior to the administration of the Type II anti-CD20 antibody (at least not within the same course of treatment).

In one embodiment, the administration of the Type II anti-CD20 antibody is a dose of about 1000 mg Type II anti-CD20 antibody. The dose of about 1000 mg Type II anti-CD20 antibody may be administered to the subject as a single administration of about 1000 mg, or as several administrations, e.g. two administrations of about 500 mg each. In a particular embodiment, one administration of about 1000 mg Type II anti-CD20 antibody is made to the subject. In another embodiment, two administrations of about 500 mg Type II anti-CD20 antibody each are made to the subject on two consecutive days. In one embodiment, one administration of about 1000 mg Type II anti-CD20 antibody is made to the subject, 7 days before the administration of the therapeutic agent. In one embodiment, no further administration of the Type II anti-CD20 antibody is made to the subject. In one embodiment, no administration of the therapeutic agent is made to the subject prior to the administration of the Type II anti-CD20 antibody (at least not within the same course of treatment).

In one embodiment, the treatment regimen further comprises administration of premedication prior to the administration of the Type II anti-CD20 antibody. In embodiment the premedication comprises a corticosteroid (such as e.g. prednisolone, dexamethasone, or methylprednisolone), paracetamol/acetaminophen, and/or an anti-histamine (such as e.g. diphenhydramine). In one embodiment, the premedication is administered at least 60 minutes prior to the administration of the Type II anti-CD20 antibody.

In one embodiment, the treatment regimen does not comprise administration of an immunosuppressive agent other than the Type II anti-CD20 antibody (and optionally the above-described premedication) prior to the administration of the therapeutic agent. In one embodiment, the treatment regimen does not comprise administration of an agent selected from the group of methotrexate, azathioprine, 6-mercaptopurine, leflunomide, cyclosporine, tacrolimus/FK506, mycophenolate mofetil and mycophenolate sodium prior to the administration of the therapeutic agent. In one embodiment, the treatment regimen does not comprise administration of a further antibody in addition to the Type II anti-CD20 antibody prior to the administration of the therapeutic agent.

### Administration of the therapeutic agent

The therapeutic agent can be administered by any suitable means, including parenteral, intrapulmonary, and intranasal, and, if desired for local treatment, intralesional administration. The methods of the present invention are particularly useful, however, in relation to therapeutic agents administered by parenteral, particularly intravenous, infusion. Parenteral infusions include intramuscular, intravenous, intraarterial, intraperitoneal, or subcutaneous administration. Dosing can be by any suitable route, e.g. by injections, such as intravenous or subcutaneous injections, depending in part on whether the administration is brief or chronic. Various dosing schedules including but not limited to single or multiple administrations over various time-points, bolus administration, and pulse infusion are contemplated herein. In one embodiment, the therapeutic agent is administered parenterally, particularly intravenously. In a particular embodiment, the therapeutic agent is administerd by intravenous infusion.

The therapeutic agent would be formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The therapeutic agent need not be, but is optionally formulated with one or more agents currently used to prevent or treat the disorder in question. The effective amount of such other agents depends on the amount of therapeutic agent present in the formulation, the type of disorder or treatment, and other factors discussed above. These are generally used in the same dosages and with administration routes as described herein, or about from 1 to 99% of the dosages described herein, or in any dosage and by any route that is empirically/clinically determined to be appropriate.

For the prevention or treatment of disease, the appropriate dosage of the therapeutic agent (when used alone or in combination with one or more other additional therapeutic agents) will depend on the type of disease to be treated, the type of therapeutic agent, the severity and course of the disease, whether the therapeutic agent is administered for preventive or therapeutic purposes, previous therapy, the patient's clinical history and response to the therapeutic agent, and the discretion of the attending physician. The therapeutic agent is suitably administered to the patient at one time or over a series of treatments. Depending on the type and severity of the disease, about 1 µg/kg to 15 mg/kg (e.g. 0.1 mg/kg - 10 mg/kg) of therapeutic agent can be an initial candidate dosage for administration to the subject, whether, for example, by one or more separate administrations, or by continuous infusion. One typical daily dosage might range from about 1 µg/kg to 100 mg/kg or more, depending on the factors mentioned above. For repeated administrations over several days or longer, depending on the condition, the treatment would generally be sustained until a desired suppression of disease symptoms occurs. One exemplary dosage of the therapeutic agent would be in the range from about 0.05 mg/kg to about 10 mg/kg. Thus, one or more doses of about 0.5 mg/kg, 2.0 mg/kg, 4.0 mg/kg or 10 mg/kg (or any combination thereof) may be administered to the subject. Such doses may be administered intermittently, e.g. every week, every two weeks, or every three weeks (*e.g*. such that the subject receives from about two to about twenty, or *e.g.* about six doses of the therapeutic agent). An initial higher loading dose, followed by one or more lower doses, or an initial lower dose, followed by one or more higher doses may be administered. An exemplary dosing regimen comprises administering an initial dose of about 10 mg, followed by a bi-weekly dose of about 20 mg of the therapeutic agent. However, other dosage regimens may be useful. The progress of this therapy is easily monitored by conventional techniques and assays.

In one embodiment, the administration of the therapeutic agent is a single administration. In certain embodiments, the administration of the therapeutic agent is two or more administrations. In one such embodiment, the therapeutic agent is administered every week, every two weeks, or every three weeks, particularly every two weeks. In one embodiment, the therapeutic agent is administered in a therapeutically effective amount. In one embodiment the therapeutic agent is administered at a dose of about 50 µg/kg, about 100 µg/kg, about 200 µg/kg, about 300 µg/kg, about 400 µg/kg, about 500 µg/kg, about 600 µg/kg, about 700 µg/kg, about 800 µg/kg, about 900 µg/kg or about 1000 µg/kg. In one embodiment, the therapeutic agent is administered at a dose which is higher than the dose of the therapeutic agent in a corresponding treatment regimen without the administration of the Type II anti-CD20 antibody. In one embodiment the administration of the therapeutic agent comprises an initial administration of a first dose of the therapeutic agent, and one or more subsequent administrations of a second dose the therapeutic agent, wherein the second dose is higher than the first dose. In one embodiment, the administration of the therapeutic agent comprises an initial administration of a first dose of the therapeutic agent, and one or more subsequent administrations of a second dose the therapeutic agent, wherein the first dose is not lower than the second dose.

In one embodiment, the administration of the therapeutic agent in the treatment regimen according to the invention is the first administration of that therapeutic agent to the subject (at least within the same course of treatment). In one embodiment, no administration of the therapeutic agent is made to the subject prior to the administration of the Type II anti-CD20 antibody.

In the present invention, the therapeutic agent can be used either alone or in combination with other agents in a therapy. For instance, the therapeutic agent may be co-administered with at least one additional therapeutic agent. In certain embodiments, an additional therapeutic agent is an immunotherapeutic agent.

Such combination therapies noted above encompass combined administration (where two or more therapeutic agents are included in the same or separate formulations), and separate administration, in which case, administration of the therapeutic agent can occur prior to, simultaneously, and/or following, administration of an additional therapeutic agent or agents. In one embodiment, administration of the therapeutic agent and administration of an additional therapeutic agent occur within about one month, or within about one, two or three weeks, or within about one, two, three, four, five, or six days, of each other.

### Articles of manufacture

In another aspect of the invention, an article of manufacture, e.g. a kit, is provided, containing materials useful for the treatment, prevention and/or diagnosis of a disease, or for the reduction of cytokine release as described herein. The article of manufacture comprises a container and a label or package insert on or associated with the container. Suitable containers include, for example, bottles, vials, syringes, IV solution bags, etc. The containers may be formed from a variety of materials such as glass or plastic. The container holds a composition which is by itself or combined with another composition effective for treating, preventing and/or diagnosing the condition, or holds a composition which is effective for reducing cytokine release, and may have a sterile access port (for example the container may be an intravenous solution bag or a vial having a stopper pierceable by a hypodermic injection needle). At least one active agent in the composition is a Type II anti-CD20 antibody or a therapeutic agent as described herein. The label or package insert indicates that the composition is used for treating the condition of choice and/or to reduce cytokine release. Moreover, the article of manufacture may comprise (a) a first container with a composition contained therein, wherein the composition comprises a Type II anti-CD20 antibody as described herein; and (b) a second container with a composition contained therein, wherein the composition comprises a therapeutic agent as described herein. The article of manufacture in this embodiment of the invention may further comprise a package insert indicating that the compositions can be used to treat a particular condition and/or to reduce cytokine release Alternatively, or additionally, the article of manufacture may further comprise a second (or third) container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

The term "PD-L1 binding antagonist" refers to a molecule that decreases, blocks, inhibits, abrogates or interferes with signal transduction resulting from the interaction of PD-L1 with either one or more of its binding partners, such as PD-1, B7-1. In some embodiments, a PD-L1 binding antagonist is a molecule that inhibits the binding of PD-L1 to its binding partners. In a specific aspect, the PD-L1 binding antagonist inhibits binding of PD-L1 to PD-1 and/or B7-1. In some embodiments, the PD-L1 binding antagonists include anti-PD-L1 antibodies, antigen binding fragments thereof, immunoadhesins, fusion proteins, oligopeptides and other molecules that decrease, block, inhibit, abrogate or interfere with signal transduction resulting from the interaction of PD-L1 with one or more of its binding partners, such as PD-1, B7-1. In one embodiment, a PD-L1 binding antagonist reduces the negative co-stimulatory signal mediated by or through cell surface proteins expressed on T lymphocytes mediated signaling through PD-L1 so as to render a dysfunctional T-cell less dysfunctional (e.g., enhancing effector responses to antigen recognition). In some embodiments, a PD-L1 binding antagonist is an anti-PD-L1 antibody. In some embodiments, the antibody is a humanized antibody, a chimeric antibody or a human antibody. In some embodiments, the antibody is an antigen binding fragment. In some embodiments, the antigen-binding fragment is selected from the group consisting of Fab, Fab', F(ab')2, and Fv.In a specific aspect, an anti-PD-L1 antibody is YW243.55.S70 described herein. In another specific aspect, an anti-PD-L1 antibody is MDX-1105 described herein. In still another specific aspect, an anti-PD-L1 antibody is MPDL3280A (atezolizumab) described herein. In still another specific aspect, an anti-PD-L1 antibody is MDX-1105 described herein. In still another specific aspect, an anti-PD-L1 antibody is YW243.55.S70 described herein. In still another specific aspect, an anti-PD-L1 antibody is MEDI4736 (durvalumab) described herein. In still another specific aspect, an anti-PD-L1 antibody is MSB0010718C (avelumab) described herein.

In some embodiments, the PD-1 axis binding antagonist is a PD-1 binding antagonist. In some embodiments, the PD-1 binding antagonist inhibits the binding of PD-1 to its ligand binding partners. In some embodiments, the PD-1 binding antagonist inhibits the binding of PD-1 to PD-L1. In some embodiments, the PD-1 binding antagonist inhibits the binding of PD-1 to PD-L2. In some embodiments, the PD-1 binding antagonist inhibits the binding of PD-1 to both PD-L1 and PD-L2. In some embodiments, the PD-1 binding antagonist is an antibody. In some embodiments, the PD-1 binding antagonist is selected from the group consisting of MDX 1106 (nivolumab), MK-3475 (pembrolizumab), CT-011 (pidilizumab), MEDI-0680 (AMP-514), PDR001, REGN2810, and BGB-108.

In some embodiments, the PD-1 axis binding antagonist is a PD-L1 binding antagonist. In some embodiments, the PD-L1 binding antagonist inhibits the binding of PD-L1 to PD-1. In some embodiments, the PD-L1 binding antagonist inhibits the binding of PD-L1 to B7-1. In some embodiments, the PD-L1 binding antagonist inhibits the binding of PD-L1 to both PD-1 and B7-1. In some embodiments, the PD-L1 binding antagonist is an anti-PD-L1 antibody. In some embodiments, the PD-L1 binding antagonist is selected from the group consisting of: MPDL3280A (atezolizumab), YW243.55.S70, MDX-1105, MEDI4736 (durvalumab), and MSB0010718C (avelumab). In particular embodiments, the anti-PD-L1 antibody is MPDL3280A (atezolizumab). In some embodiments, MPDL3280A is administered at a dose of about 800 mg to about 1500 mg every three weeks (e.g., about 1000 mg to about 1300 mg every three weeks, e.g., about 1100 mg to about 1200 mg every three weeks). In some embodiments, MPDL3280A is administered at a dose of about 1200 mg every three weeks. In some embodiments, the anti-PD-L1 antibody comprises a heavy chain comprising HVR-H1 sequence of SEQ ID NO: 107, HVR-H2 sequence of SEQ ID NO: 108, and HVR-H3 sequence of SEQ ID NO: 109; and/or a light chain comprising HVR-L1 sequence of SEQ ID NO: 110, HVR-L2 sequence of SEQ ID NO: 111, and HVR-L3 sequence of SEQ ID NO: 112. In some embodiments, the anti-PD-L1 antibody comprises a heavy chain variable region comprising the amino acid sequence of SEQ ID NO: 113 or 114 and/or a light chain variable region comprising the amino acid sequence of SEQ ID NO: 115. In some embodiments, the anti-PD-L1 antibody comprises a heavy chain variable region comprising the amino acid sequence of SEQ ID NO: 113 and a light chain variable region comprising the amino acid sequence of SEQ ID NO:115. In some embodiments, the anti-PD-L1 antibody comprises the three heavy chain HVR sequences of antibody YW243.55.S70 and/or the three light chain HVR sequences of antibody YW24355.S70 described in WO 2010/077634 and U.S. Patent No. 8,217,149, which are incorporated herein by reference. In some embodiments, the anti-PD-L1 antibody comprises the heavy chain variable region sequence of antibody YW243.55.S70 and/or the light chain variable region sequence of antibody YW24355.S70.

In some embodiments, the PD-1 axis binding antagonist is a PD-L2 binding antagonist. In some embodiments, the PD-L2 binding antagonist is an antibody. In some embodiments, the PD-L2 binding antagonist is an immunoadhesin.

In some embodiments, the PD-1 axis binding antagonist is an antibody (e.g., an anti-PD-1 antibody, an anti-PD-L1 antibody, or an anti-PD-L2 antibody) and comprises an aglycosylation site mutation. In some embodiments, the aglycosylation site mutation is a substitution mutation. In some embodiments, the substitution mutation is at amino acid residue N297, L234, L235, and/or D265 (EU numbering). In some embodiments, the substitution mutation is selected from the group consisting of N297G, N297A, L234A, L235A, and D265A. In some embodiments, the substitution mutation is a D265A mutation and an N297G mutation. In some embodiments, the aglycosylation site mutation reduces effector function of the antibody. In some embodiments, the PD-1 axis binding antagonist (e.g., an anti-PD-1 antibody, an anti-PD-L1 antibody, or an anti-PD-L2 antibody) is a human IgG1 having Asn to Ala substitution at position 297 according to EU numbering.

The term "PD-L2 binding antagonist" refers to a molecule that decreases, blocks, inhibits, abrogates or interferes with signal transduction resulting from the interaction of PD-L2 with either one or more of its binding partners, such as PD-1. In some embodiments, a PD-L2 binding antagonist is a molecule that inhibits the binding of PD-L2 to one or more of its binding partners. In a specific aspect, the PD-L2 binding antagonist inhibits binding of PD-L2 to PD-1. In some embodiments, the PD-L2 antagonists include anti-PD-L2 antibodies, antigen binding fragments thereof, immunoadhesins, fusion proteins, oligopeptides and other molecules that decrease, block, inhibit, abrogate or interfere with signal transduction resulting from the interaction of PD-L2 with either one or more of its binding partners, such as PD-1. In one embodiment, a PD-L2 binding antagonist reduces the negative co-stimulatory signal mediated by or through cell surface proteins expressed on T lymphocytes mediated signaling through PD-L2 so as render a dysfunctional T-cell less dysfunctional (e.g., enhancing effector responses to antigen recognition). In some embodiments, a PD-L2 binding antagonist is an immunoadhesin.

### FURTHER ASPECTS OF THE INVENTION

In a further embodiment of the invention the combination treatment includes an at least first administration of anti-CD20 antibody and at least a second administration of an anti-CD20/anti-CD3 bispecific antibody, wherein the period of time between the at least first administration and the at least second administration is insufficient for the reduction of the number of B-Cells in the individual in response to the administration of the Type II anti-CD20 antibody.

In a further embodiment, the combination treatment may comprise administering of an immunoadhesin, preferably an immunoadhesin comprising an extracellular or PD-1 binding portion of PD-L1 or PD-L2 fused to a constant region (e.g., an Fc region of an immunoglobulin sequence), more preferably an anti-PD-L1 antibody. In one embodiment the anti-PD-L1 antibody is selected from the group consisting of YW243.55.S70, MPDL3280A, MDX-1105, and MEDI4736. Antibody YW243.55.S70 is an anti-PD-L1 antibody described in WO 2010/077634. MDX-1105, also known as BMS-936559, is an anti-PD-L1 antibody described in WO2007/005874. MEDI4736 is an anti-PDL1 monoclonal antibody described in WO2011/066389 and US2013/034559. In one embodiment, the anti-PD-L1 antibody is atezolizumab.

### Embodiments

In the following, some of the embodiments of the invention are listed.
1. A method of treating a disease in a subject, the method comprising a treatment regimen comprising
   (i) administration to the subject of a Type II anti-CD20 antibody,
      and consecutively after a period of time
   (ii) administration to the subject of a T-cell activating therapeutic agent,
      wherein the period of time between the administration of the Type II anti-CD20 antibody and the administration of the therapeutic agent is sufficient for reduction of the number of B-cells in the subject in response to the administration of the CD20 antibody.
2. The method of embodiment 1, wherein the treatment regimen effectively reduces cytokine release in the subject associated with the administration of the therapeutic agent as compared to a corresponding treatment regimen without the administration of the Type II anti-CD20 antibody.
3. A method for reducing cytokine release associated with the administration of a therapeutic agent in a subject, comprising administration of a Type II anti-CD20 antibody to the subject prior to administration of the therapeutic agent.
4. The method of embodiment 3, wherein the period of time between the administration of the Type II anti-CD20 antibody and administration of the therapeutic agent is sufficient for reduction of the number of B-cells in the subject in response to the administration of the Type II anti-CD20 antibody.
5. The method of any one of the preceding embodiments, wherein the Type II anti-CD20 antibody comprises a heavy chain variable region comprising the heavy chain CDR (HCDR) 1 of SEQ ID NO: 4, the HCDR2 of SEQ ID NO: 5, and the HCDR3 of SEQ ID NO: 6; and a light chain variable region comprising the light chain CDR (LCDR) 1 of SEQ ID NO: 7, the LCDR2 of SEQ ID NO: 8 and the LCDR3 of SEQ ID NO: 9.
6. The method of any one of the preceding embodiments, wherein the Type II anti-CD20 antibody comprises the heavy chain variable region sequence of SEQ ID NO: 10 and the light chain variable region sequence of SEQ ID NO: 11.
7. The method of any one of the preceding embodiments, wherein the Type II anti-CD20 antibody is an IgG antibody, particularly an IgG₁ antibody.
8. The method of any one of the preceding embodiments, wherein the Type II anti-CD20 antibody is engineered to have an increased proportion of non-fucosylated oligosaccharides in the Fc region as compared to a non-engineered antibody.
9. The method of any one of the preceding embodiments, wherein at least about 40% of the N-linked oligosaccharides in the Fc region of the Type II anti-CD20 antibody are non-fucosylated.
10. The method of any one of the preceding embodiments, wherein the Type II anti-CD20 antibody is obinutuzumab.
11. The method of any one of the preceding embodiments, wherein the therapeutic agent comprises an antibody, particularly a multispecific antibody.
12. The method of embodiment 11, wherein the antibody specifically binds to an activating T cell antigen, particularly an antigen selected from the group consisting of CD3, CD28, CD137 (also known as 4-1BB), CD40, CD226, OX40, GITR, CD27, HVEM, and CD127, more particularly CD3, most particularly CD3ε.
13. The method of embodiment 11 or 12, wherein the antibody comprises a heavy chain variable region comprising the heavy chain CDR (HCDR) 1 of SEQ ID NO: 12, the HCDR2 of SEQ ID NO: 13, and the HCDR3 of SEQ ID NO: 14; and a light chain variable region comprising the light chain CDR (LCDR) 1 of SEQ ID NO: 15, the LCDR2 of SEQ ID NO: 16 and the LCDR3 of SEQ ID NO: 17.
14. The method of any one of embodiments 11 to 13, wherein the antibody comprises the heavy chain variable region sequence of SEQ ID NO: 18 and the light chain variable region sequence of SEQ ID NO: 19.
15. The method of any one of embodiments 11 to 14, wherein the antibody specifically binds to a B-cell antigen, particularly an antigen selected from the group consisting of CD20, CD19, CD22, ROR-1, CD37 and CD5, more particularly CD20 or CD19, most particularly CD20.
16. The method of embodiment 15, wherein the antibody comprises a heavy chain variable region comprising the heavy chain CDR (HCDR) 1 of SEQ ID NO: 4, the HCDR2 of SEQ ID NO: 5, and the HCDR3 of SEQ ID NO: 6; and a light chain variable region comprising the light chain CDR (LCDR) 1 of SEQ ID NO: 7, the LCDR2 of SEQ ID NO: 8 and the LCDR3 of SEQ ID NO: 9.
17. The method of embodiment 15 or 16, wherein the antibody comprises the heavy chain variable region sequence of SEQ ID NO: 10 and the light chain variable region sequence of SEQ ID NO: 11.
18. The method of any one of the preceding embodiments, wherein the antibody is a bispecific antibody comprising (i) an antibody as defined in any one of embodiments 12 to 14 and (ii) an antibody as defined .in any one of embodiments 15 to 17.
19. The method of any one of the preceding embodiments, wherein the therapeutic agent comprises CD20XCD3 bsAB.
20. The method of any one of embodiments 1 to 10, wherein the therapeutic agent comprises a T cell expressing a chimeric antigen receptor (CAR), particularly a CAR that specifically binds to a B-cell antigen, more particularly a CAR that specifically binds to an antigen selected from the group of CD20, CD 19, CD22, ROR-1, CD37 and CD5.
21. The method of any one of the preceding embodiments, wherein the disease is a B cell proliferative disorder, particularly a CD20-positive B-cell disorder, and/or is a disease selected from the group consisting of Non-Hodgkin lymphoma (NHL), acute lymphocytic leukemia (ALL), chronic lymphocytic leukemia (CLL), diffuse large B-cell lymphoma (DLBCL), follicular lymphoma (FL), mantle-cell lymphoma (MCL), marginal zone lymphoma (MZL), Multiple myeloma (MM) and Hodgkin lymphoma (HL).
22. A Type II anti-CD20 antibody for use in a method of treating a disease in a subject, the method comprising a treatment regimen comprising
   (i) administration to the subject of the Type II anti-CD20 antibody,
      and consecutively after a period of time
   (ii) administration to the subject of a T-cell activating therapeutic agent,
      wherein the period of time between the administration of the Type II anti-CD20 antibody and the administration of the therapeutic agent is sufficient for reduction of the number of B-cells in the subject in response to the administration of the Type II anti-CD20 antibody.
23. The Type II anti-CD20 antibody of embodiment 22, wherein the treatment regimen effectively reduces cytokine release in the subject associated with the administration of the therapeutic agent as compared to a corresponding treatment regimen without the administration of the Type II anti-CD20 antibody.
24. A Type II anti-CD20 antibody for use in a method for reducing cytokine release associated with the administration of a therapeutic agent in a subject, comprising administration of the Type II anti-CD20 antibody to the subject prior to administration of the therapeutic agent.
25. The Type II anti-CD20 antibody of embodiment 24, wherein the period of time between the administration of the Type II anti-CD20 antibody and administration of the therapeutic agent is sufficient for reduction of the number of B-cells in the subject in response to the administration of the Type II anti-CD20 antibody.
26. The Type II anti-CD20 antibody of any one of embodiments 22 to 25, wherein the Type II anti-CD20 antibody comprises a heavy chain variable region comprising the heavy chain CDR (HCDR) 1 of SEQ ID NO: 4, the HCDR2 of SEQ ID NO: 5, and the HCDR3 of SEQ ID NO: 6; and a light chain variable region comprising the light chain CDR (LCDR) 1 of SEQ ID NO: 7, the LCDR2 of SEQ ID NO: 8 and the LCDR3 of SEQ ID NO: 9.
27. The Type II anti-CD20 antibody of any one of embodiments 22 to 26, wherein the Type II anti-CD20 antibody comprises the heavy chain variable region sequence of SEQ ID NO: 10 and the light chain variable region sequence of SEQ ID NO: 11.
28. The Type II anti-CD20 antibody of any one of embodiments 22 to 27, wherein the Type II anti-CD20 antibody is an IgG antibody, particularly an IgG₁ antibody.
29. The Type II anti-CD20 antibody of any one of embodiments 22 to 28, wherein the Type II anti-CD20 antibody is engineered to have an increased proportion of non-fucosylated oligosaccharides in the Fc region as compared to a non-engineered antibody.
30. The Type II anti-CD20 antibody of any one of embodiments 22 to 29, wherein at least about 40% of the N-linked oligosaccharides in the Fc region of the Type II anti-CD20 antibody are non-fucosylated.
31. The Type II anti-CD20 antibody of any one of embodiments 22 to 30, wherein the Type II anti-CD20 antibody is obinutuzumab.
32. The Type II anti-CD20 antibody of any one of embodiments 22 to 31, wherein the therapeutic agent comprises an antibody, particularly a multispecific antibody.
33. The Type II anti-CD20 antibody of embodiment 32, wherein the antibody comprised in the therapeutic agent specifically binds to an activating T cell antigen, particularly an antigen selected from the group consisting of CD3, CD28, CD137 (also known as 4-1BB), CD40, CD226, OX40, GITR, CD27, HVEM, and CD 127, more particularly CD3, most particularly CD3ε.
34. The Type II anti-CD20 antibody of embodiment 32 or 33, wherein the antibody comprised in the therapeutic agent comprises a heavy chain variable region comprising the heavy chain CDR (HCDR) 1 of SEQ ID NO: 12, the HCDR2 of SEQ ID NO: 13, and the HCDR3 of SEQ ID NO: 14; and a light chain variable region comprising the light chain CDR (LCDR) 1 of SEQ ID NO: 15, the LCDR2 of SEQ ID NO: 16 and the LCDR3 of SEQ ID NO: 17.
35. The Type II anti-CD20 antibody of any one of embodiments 32 to 34, wherein the antibody comprised in the therapeutic agent comprises the heavy chain variable region sequence of SEQ ID NO: 18 and the light chain variable region sequence of SEQ ID NO: 19.
36. The Type II anti-CD20 antibody of any one of embodiments 32 to 35, wherein the antibody comprised in the therapeutic agent specifically binds to a B-cell antigen, particularly an antigen selected from the group consisting of CD20, CD 19, CD22, ROR-1, CD37 and CD5, more particularly CD20 or CD19, most particularly CD20.
37. The Type II anti-CD20 antibody of embodiment 36, wherein the antibody comprised in the therapeutic agent comprises a heavy chain variable region comprising the heavy chain CDR (HCDR) 1 of SEQ ID NO: 4, the HCDR2 of SEQ ID NO: 5, and the HCDR3 of SEQ ID NO: 6; and a light chain variable region comprising the light chain CDR (LCDR) 1 of SEQ ID NO: 7, the LCDR2 of SEQ ID NO: 8 and the LCDR3 of SEQ ID NO: 9.
38. The Type II anti-CD20 antibody of embodiment 36 or 37, wherein the antibody comprised in the therapeutic agent comprises the heavy chain variable region sequence of SEQ ID NO: 10 and the light chain variable region sequence of SEQ ID NO: 11.
39. The Type II anti-CD20 antibody of any one of embodiments 22 to 38, wherein the antibody comprised in the therapeutic agent is a bispecific antibody comprising (i) an antibody as defined in any one of embodiments 33 to 35 and (ii) an antibody as defined in any one of embodiments 36 to 38.
40. The Type II anti-CD20 antibody of any one of embodiments 22 to 39, wherein the therapeutic agent comprises CD20XCD3 bsAB.
41. The Type II anti-CD20 antibody of any one of embodiments 22 to 31, wherein the therapeutic agent comprises a T cell expressing a chimeric antigen receptor (CAR), particularly a CAR that specifically binds to a B-cell antigen, more particularly a CAR that specifically binds to an antigen selected from the group of CD20, CD19, CD22, ROR-1, CD37 and CD5.
42. The Type II anti-CD20 antibody of any one of embodiments 22 to 41, wherein the disease is a B cell proliferative disorder, particularly a CD20-positive B-cell disorder, and/or is a disease selected from the group consisting of Non-Hodgkin lymphoma (NHL), acute lymphocytic leukemia (ALL), chronic lymphocytic leukemia (CLL), diffuse large B-cell lymphoma (DLBCL), follicular lymphoma (FL), mantle-cell lymphoma (MCL), marginal zone lymphoma (MZL), Multiple myeloma (MM) and Hodgkin lymphoma (HL).
43. Use of a Type II anti-CD20 antibody in the manufacture of a medicament for reduction of cytokine release associated with the administration of a T-cell activating therapeutic agent in a subject, wherein the medicament is to be used in a treatment regimen comprising
   (i) administration to the subject of the Type II anti-CD20 antibody,
      and consecutively after a period of time
   (ii) administration to the subject of a T-cell activating therapeutic agent,
      wherein the period of time between the administration of the Type II anti-CD20 antibody and the administration of the therapeutic agent is sufficient for reduction of the number of B-cells in the subject in response to the administration of the CD20 antibody.
44. Use of a T-cell activating therapeutic agent in the manufacture of a medicament for treatment of a disease in a subject, wherein the treatment comprises a treatment regimen comprising
   (i) administration to the subject of a Type II anti-CD20 antibody,
      and consecutively after a period of time
   (ii) administration to the subject of the T-cell activating therapeutic agent,
      wherein the period of time between the administration of the Type II anti-CD20 antibody and the administration of the therapeutic agent is sufficient for reduction of the number of B-cells in the subject in response to the administration of the CD20 antibody.
45. The use of embodiment 43 or 44, wherein the treatment regimen effectively reduces cytokine release associated with the administration of the therapeutic agent in the subject as compared to a corresponding treatment regimen without the administration of the Type II anti-CD20 antibody.
46. The use of any one of embodiments 43 to 45, wherein the Type II anti-CD20 antibody comprises a heavy chain variable region comprising the heavy chain CDR (HCDR) 1 of SEQ ID NO: 4, the HCDR2 of SEQ ID NO: 5, and the HCDR3 of SEQ ID NO: 6; and a light chain variable region comprising the light chain CDR (LCDR) 1 of SEQ ID NO: 7, the LCDR2 of SEQ ID NO: 8 and the LCDR3 of SEQ ID NO: 9.
47. The use of any one of embodiments 43 to 46, wherein the Type II anti-CD20 antibody comprises the heavy chain variable region sequence of SEQ ID NO: 10 and the light chain variable region sequence of SEQ ID NO: 11.
48. The use of any one of embodiments 43 to 47, wherein the Type II anti-CD20 antibody is an IgG antibody, particularly an IgG₁ antibody.
49. The use of any one of embodiments 43 to 48, wherein the Type II anti-CD20 antibody is engineered to have an increased proportion of non-fucosylated oligosaccharides in the Fc region as compared to a non-engineered antibody.
50. The use of any one of embodiments 43 to 49, wherein at least about 40% of the N-linked oligosaccharides in the Fc region of the Type II anti-CD20 antibody are non-fucosylated.
51. The use of any one of embodiments 43 to 50, wherein the Type II anti-CD20 antibody is obinutuzumab.
52. The use of any one of embodiments 43 to 51, wherein the therapeutic agent comprises an antibody, particularly a multispecific antibody.
53. The use of embodiment 51, wherein the antibody specifically binds to an activating T cell antigen, particularly an antigen selected from the group consisting of CD3, CD28, CD137 (also known as 4-1BB), CD40, CD226, OX40, GITR, CD27, HVEM, and CD127, more particularly CD3, most particularly CD3ε.
54. The use of embodiment 52 or 53, wherein the antibody comprises a heavy chain variable region comprising the heavy chain CDR (HCDR) 1 of SEQ ID NO: 12, the HCDR2 of SEQ ID NO: 13, and the HCDR3 of SEQ ID NO: 14; and a light chain variable region comprising the light chain CDR (LCDR) 1 of SEQ ID NO: 15, the LCDR2 of SEQ ID NO: 16 and the LCDR3 of SEQ ID NO: 17.
55. The use of any one of embodiments 52 to 54, wherein the antibody comprises the heavy chain variable region sequence of SEQ ID NO: 18 and the light chain variable region sequence of SEQ ID NO: 19.
56. The use of any one of embodiments 52 to 55, wherein the antibody specifically binds to a B-cell antigen, particularly an antigen selected from the group consisting of CD20, CD19, CD22, ROR-1, CD37 and CD5, more particularly CD20 or CD19, most particularly CD20.
57. The use of embodiment 56, wherein the antibody comprises a heavy chain variable region comprising the heavy chain CDR (HCDR) 1 of SEQ ID NO: 4, the HCDR2 of SEQ ID NO: 5, and the HCDR3 of SEQ ID NO: 6; and a light chain variable region comprising the light chain CDR (LCDR) 1 of SEQ ID NO: 7, the LCDR2 of SEQ ID NO: 8 and the LCDR3 of SEQ ID NO: 9.
58. The use of embodiment 56 or 57, wherein the antibody comprises the heavy chain variable region sequence of SEQ ID NO: 10 and the light chain variable region sequence of SEQ ID NO: 11.
59. The use of any one of embodiments 43 to 58, wherein the antibody is a bispecific antibody comprising (i) an antibody as defined in any one of embodiments 53 to 55 and (ii) an antibody as defined .in any one of embodiments 56 to 58.
60. The use of any one of embodiments 43 to 59, wherein the therapeutic agent comprises CD20XCD3 bsAB.
61. The use of any one of embodiments 43 to 51, wherein the therapeutic agent comprises a T cell expressing a chimeric antigen receptor (CAR), particularly a CAR that specifically binds to a B-cell antigen, more particularly a CAR that specifically binds to an antigen selected from the group of CD20, CD19, CD22, ROR-1, CD37 and CD5.
62. The use of any one of embodiments 43 to 61, wherein the disease is a B cell proliferative disorder, particularly a CD20-positive B-cell disorder, and/or is a disease selected from the group consisting of Non-Hodgkin lymphoma (NHL), acute lymphocytic leukemia (ALL), chronic lymphocytic leukemia (CLL), diffuse large B-cell lymphoma (DLBCL), follicular lymphoma (FL), mantle-cell lymphoma (MCL), marginal zone lymphoma (MZL), Multiple myeloma (MM) and Hodgkin lymphoma (HL).
63. A kit for the reduction of cytokine release associated with the administration of a T-cell activating therapeutic agent in a subject, comprising a package comprising a Type II anti-CD20 antibody composition and instructions for using the Type II anti-CD20 antibody composition in a treatment regimen comprising
   (i) administration to the subject of the Type II anti-CD20 antibody composition,
      and consecutively after a period of time
   (ii) administration to the subject of a T-cell activating therapeutic agent,
      wherein the period of time between the administration of the Type II anti-CD20 antibody composition and the administration of the therapeutic agent is sufficient for reduction of the number of B-cells in the subject in response to the administration of the CD20 antibody.
64. The kit of embodiment 63, further comprising a T-cell activating therapeutic agent composition.
65. A kit for the treatment of a disease in a subject, comprising a package comprising a T-cell activating therapeutic agent composition and instructions for using the therapeutic agent composition in a treatment regimen comprising
   (iii) administration to the subject of a Type II anti-CD20 antibody,
      and consecutively after a period of time
   (iv) administration to the subject of the T-cell activating therapeutic agent composition,
      wherein the period of time between the administration of the Type II anti-CD20 antibody and the administration of the therapeutic agent composition is sufficient for reduction of the number of B-cells in the subject in response to the administration of the CD20 antibody.
66. The kit of embodiment 65, further comprising a Type II anti-CD20 antibody composition.
67. The kit of any one of embodiments 63 to 66, wherein the treatment regimen effectively reduces cytokine release associated with the administration of the therapeutic agent in the subject as compared to a corresponding treatment regimen without the administration of the Type II anti-CD20 antibody composition.
68. The kit of any one of embodiments 63 to 67, wherein the Type II anti-CD20 antibody comprises a heavy chain variable region comprising the heavy chain CDR (HCDR) 1 of SEQ ID NO: 4, the HCDR2 of SEQ ID NO: 5, and the HCDR3 of SEQ ID NO: 6; and a light chain variable region comprising the light chain CDR (LCDR) 1 of SEQ ID NO: 7, the LCDR2 of SEQ ID NO: 8 and the LCDR3 of SEQ ID NO: 9.
69. The kit of any one of embodiments 63 to 68, wherein the Type II anti-CD20 antibody comprises the heavy chain variable region sequence of SEQ ID NO: 10 and the light chain variable region sequence of SEQ ID NO: 11.
70. The kit of any one of embodiments 63 to 69, wherein the Type II anti-CD20 antibody is an IgG antibody, particularly an IgG₁ antibody.
71. The kit of any one of embodiments 63 to 70, wherein the Type II anti-CD20 antibody is engineered to have an increased proportion of non-fucosylated oligosaccharides in the Fc region as compared to a non-engineered antibody.
72. The kit of any one of embodiments 63 to 71, wherein at least about 40% of the N-linked oligosaccharides in the Fc region of the Type II anti-CD20 antibody are non-fucosylated.
73. The kit of any one of embodiments 63 to 72, wherein the Type II anti-CD20 antibody is obinutuzumab.
74. The kit of any one of embodiments 63 to 73, wherein the therapeutic agent comprises an antibody, particularly a multispecific antibody.
75. The kit of embodiment 74, wherein the antibody specifically binds to an activating T cell antigen, particularly an antigen selected from the group consisting of CD3, CD28, CD137 (also known as 4-1BB), CD40, CD226, OX40, GITR, CD27, HVEM, and CD127, more particularly CD3, most particularly CD3ε.
76. The kit of embodiment 74 or 75, wherein the antibody comprises a heavy chain variable region comprising the heavy chain CDR (HCDR) 1 of SEQ ID NO: 12, the HCDR2 of SEQ ID NO: 13, and the HCDR3 of SEQ ID NO: 14; and a light chain variable region comprising the light chain CDR (LCDR) 1 of SEQ ID NO: 15, the LCDR2 of SEQ ID NO: 16 and the LCDR3 of SEQ ID NO: 17.
77. The kit of any one of embodiments 74 to 76, wherein the antibody comprises the heavy chain variable region sequence of SEQ ID NO: 18 and the light chain variable region sequence of SEQ ID NO: 19.
78. The kit of any one of embodiments 74 to 77, wherein the antibody specifically binds to a B-cell antigen, particularly an antigen selected from the group consisting of CD20, CD19, CD22, ROR-1, CD37 and CD5, more particularly CD20 or CD19, most particularly CD20.
79. The kit of embodiment 78, wherein the antibody comprises a heavy chain variable region comprising the heavy chain CDR (HCDR) 1 of SEQ ID NO: 4, the HCDR2 of SEQ ID NO: 5, and the HCDR3 of SEQ ID NO: 6; and a light chain variable region comprising the light chain CDR (LCDR) 1 of SEQ ID NO: 7, the LCDR2 of SEQ ID NO: 8 and the LCDR3 of SEQ ID NO: 9.
80. The kit of embodiment 78 or 79, wherein the antibody comprises the heavy chain variable region sequence of SEQ ID NO: 10 and the light chain variable region sequence of SEQ ID NO: 11.
81. The kit of any one of embodiments 78 to 80, wherein the antibody is a bispecific antibody comprising (i) an antibody as defined in any one of embodiments 75 to 77 and (ii) an antibody as defined in any one of embodiments 78 to 80.
82. The kit of any one of embodiments 63 to 81, wherein the therapeutic agent comprises CD20XCD3 bsAB.
83. The kit of any one of embodiments 63 to 73, wherein the therapeutic agent comprises a T cell expressing a chimeric antigen receptor (CAR), particularly a CAR that specifically binds to a B-cell antigen, more particularly a CAR that specifically binds to an antigen selected from the group of CD20, CD19, CD22, ROR-1, CD37 and CD5.
84. The kit of any one of embodiments 63 to 83, wherein the disease is a B cell proliferative disorder, particularly a CD20-positive B-cell disorder, and/or is a disease selected from the group consisting of Non-Hodgkin lymphoma (NHL), acute lymphocytic leukemia (ALL), chronic lymphocytic leukemia (CLL), diffuse large B-cell lymphoma (DLBCL), follicular lymphoma (FL), mantle-cell lymphoma (MCL), marginal zone lymphoma (MZL), Multiple myeloma (MM) and Hodgkin lymphoma (HL).
85. A T-cell activating therapeutic agent for use in a method of treating a disease in a subject, the method comprising a treatment regimen comprising
   (i) administration to the subject of a Type II anti-CD20 antibody,
      and consecutively after a period of time
   (ii) administration to the subject of the T-cell activating therapeutic agent,
      wherein the period of time between the administration of the Type II anti-CD20 antibody and the administration of the therapeutic agent is sufficient for reduction of the number of B-cells in the subject in response to the administration of the CD20 antibody.
86. The T-cell activating therapeutic agent of embodiment 85, wherein the treatment regimen effectively reduces cytokine release in the subject associated with the administration of the therapeutic agent as compared to a corresponding treatment regimen without the administration of the Type II anti-CD20 antibody.
87. The T-cell activating therapeutic agent of embodiment 85 or 86, wherein the Type II anti-CD20 antibody comprises a heavy chain variable region comprising the heavy chain CDR (HCDR) 1 of SEQ ID NO: 4, the HCDR2 of SEQ ID NO: 5, and the HCDR3 of SEQ ID NO: 6; and a light chain variable region comprising the light chain CDR (LCDR) 1 of SEQ ID NO: 7, the LCDR2 of SEQ ID NO: 8 and the LCDR3 of SEQ ID NO: 9.
88. The T-cell activating therapeutic agent of any one of embodiments 85 to 87, wherein the Type II anti-CD20 antibody comprises the heavy chain variable region sequence of SEQ ID NO: 10 and the light chain variable region sequence of SEQ ID NO: 11.
89. The T-cell activating therapeutic agent of any one of embodiments 85 to 88, wherein the Type II anti-CD20 antibody is an IgG antibody, particularly an IgG₁ antibody.
90. The T-cell activating therapeutic agent of any one of embodiments 85 to 89, wherein the Type II anti-CD20 antibody is engineered to have an increased proportion of non-fucosylated oligosaccharides in the Fc region as compared to a non-engineered antibody.
91. The T-cell activating therapeutic agent of any one of embodiments 85 to 90, wherein at least about 40% of the N-linked oligosaccharides in the Fc region of the Type II anti-CD20 antibody are non-fucosylated.
92. The T-cell activating therapeutic agent of any one of embodiments 85 to 91, wherein the Type II anti-CD20 antibody is obinutuzumab.
93. The T-cell activating therapeutic agent of any one of embodiments 85 to 92, wherein the therapeutic agent comprises an antibody, particularly a multispecific antibody.
94. The T-cell activating therapeutic agent of embodiment 93, wherein the antibody specifically binds to an activating T cell antigen, particularly an antigen selected from the group consisting of CD3, CD28, CD137 (also known as 4-1BB), CD40, CD226, OX40, GITR, CD27, HVEM, and CD 127, more particularly CD3, most particularly CD3ε.
95. The T-cell activating therapeutic agent of embodiment 93 or 94, wherein the antibody comprises a heavy chain variable region comprising the heavy chain CDR (HCDR) 1 of SEQ ID NO: 12, the HCDR2 of SEQ ID NO: 13, and the HCDR3 of SEQ ID NO: 14; and a light chain variable region comprising the light chain CDR (LCDR) 1 of SEQ ID NO: 15, the LCDR2 of SEQ ID NO: 16 and the LCDR3 of SEQ ID NO: 17.
96. The T-cell activating therapeutic agent of any one of embodiments 93 to 95, wherein the antibody comprises the heavy chain variable region sequence of SEQ ID NO: 18 and the light chain variable region sequence of SEQ ID NO: 19.
97. The T-cell activating therapeutic agent of any one of embodiments 93 to 96, wherein the antibody specifically binds to a B-cell antigen, particularly an antigen selected from the group consisting of CD20, CD19, CD22, ROR-1, CD37 and CD5, more particularly CD20 or CD19, most particularly CD20.
98. The T-cell activating therapeutic agent of embodiment 97, wherein the antibody comprises a heavy chain variable region comprising the heavy chain CDR (HCDR) 1 of SEQ ID NO: 4, the HCDR2 of SEQ ID NO: 5, and the HCDR3 of SEQ ID NO: 6; and a light chain variable region comprising the light chain CDR (LCDR) 1 of SEQ ID NO: 7, the LCDR2 of SEQ ID NO: 8 and the LCDR3 of SEQ ID NO: 9.
99. The T-cell activating therapeutic agent of embodiment 97 or 98, wherein the antibody comprises the heavy chain variable region sequence of SEQ ID NO: 10 and the light chain variable region sequence of SEQ ID NO: 11.
100. The T-cell activating therapeutic agent of any one of embodiments 85 to 99, wherein the antibody is a bispecific antibody comprising (i) an antibody as defined in any one of embodiments 94 to 96 and (ii) an antibody as defined .in any one of embodiments 97 to 99.
101. The T-cell activating therapeutic agent of any one of embodiments 85 to 100, wherein the therapeutic agent comprises CD20XCD3 bsAB.
102. The T-cell activating therapeutic agent of any one of embodiments 85 to 92, wherein the therapeutic agent comprises a T cell expressing a chimeric antigen receptor (CAR), particularly a CAR that specifically binds to a B-cell antigen, more particularly a CAR that specifically binds to an antigen selected from the group of CD20, CD 19, CD22, ROR-1, CD37 and CD5.
103. The T-cell activating therapeutic agent of any one of embodiments 85 to 102, wherein the disease is a B cell proliferative disorder, particularly a CD20-positive B-cell disorder, and/or is a disease selected from the group consisting of Non-Hodgkin lymphoma (NHL), acute lymphocytic leukemia (ALL), chronic lymphocytic leukemia (CLL), diffuse large B-cell lymphoma (DLBCL), follicular lymphoma (FL), mantle-cell lymphoma (MCL), marginal zone lymphoma (MZL), Multiple myeloma (MM) and Hodgkin lymphoma (HL).

In the following, further aspects of the present invention are given.
I. A Type II anti-CD20 antibody for use in a method of treating a disease in a subject, the method comprising a treatment regimen comprising
   (i) administration to the subject of the Type II anti-CD20 antibody, and consecutively after a period of time
   (ii) administration to the subject of a T-cell activating therapeutic agent,
      wherein the period of time between the administration of the Type II anti-CD20 antibody and the administration of the therapeutic agent is sufficient for reduction of the number of B-cells in the subject in response to the administration of the CD20 antibody.
II. The Type II anti-CD20 antibody of aspect I, wherein the treatment regimen effectively reduces cytokine release in the subject associated with the administration of the therapeutic agent as compared to a corresponding treatment regimen without the administration of the Type II anti-CD20 antibody.
III. A Type II anti-CD20 antibody for use in a method for reducing cytokine release associated with the administration of a T-cell activating therapeutic agent in a subject, comprising administration of the Type II anti-CD20 antibody to the subject prior to administration of the therapeutic agent.
IV. The Type II anti-CD20 antibody of aspect III, wherein the period of time between the administration of the Type II anti-CD20 antibody and administration of the therapeutic agent is sufficient for reduction of the number of B-cells in the subject in response to the administration of the CD20 antibody.
V. The Type II anti-CD20 antibody of any one of the aspects I to IV, wherein the Type II anti-CD20 antibody comprises a heavy chain variable region comprising the heavy chain CDR (HCDR) 1 of SEQ ID NO: 4, the HCDR2 of SEQ ID NO: 5, and the HCDR3 of SEQ ID NO: 6; and a light chain variable region comprising the light chain CDR (LCDR) 1 of SEQ ID NO: 7, the LCDR2 of SEQ ID NO: 8 and the LCDR3 of SEQ ID NO: 9.
VI. The Type II anti-CD20 antibody of any one of the aspects I to V, wherein the Type II anti-CD20 antibody comprises the heavy chain variable region sequence of SEQ ID NO: 10 and the light chain variable region sequence of SEQ ID NO: 11.
VII. The Type II anti-CD20 antibody of any one of the aspects I to VI, wherein the Type II anti-CD20 antibody is an IgG antibody, particularly an IgG1 antibody, and wherein at least about 40% of the N-linked oligosaccharides in the Fc region of the Type II anti-CD20 antibody are non-fucosylated.
VIII. The Type II anti-CD20 antibody of any one of the aspects I to VII, wherein the Type II anti-CD20 antibody is obinutuzumab.
IX. The Type II anti-CD20 antibody of any one of the aspects I to XIII, wherein the therapeutic agent comprises an antibody, particularly a multispecific antibody.
X. The Type II anti-CD20 antibody of aspect IX, wherein the antibody comprised in the therapeutic agent specifically binds to an activating T cell antigen, particularly an antigen selected from the group consisting of CD3, CD28, CD137 (also known as 4-1BB), CD40, CD226, OX40, GITR, CD27, HVEM, and CD127, more particularly CD3, most particularly CD3ε.
XI. The Type II anti-CD20 antibody of aspect IX or X, wherein the antibody comprised in the therapeutic agent comprises a heavy chain variable region comprising the heavy chain CDR (HCDR) 1 of SEQ ID NO: 12, the HCDR2 of SEQ ID NO: 13, and the HCDR3 of SEQ ID NO: 14; and a light chain variable region comprising the light chain CDR (LCDR) 1 of SEQ ID NO: 15, the LCDR2 of SEQ ID NO: 16 and the LCDR3 of SEQ ID NO: 17.
XII. The Type II anti-CD20 antibody of any one of aspects IX to XI, wherein the antibody comprised in the therapeutic agent comprises the heavy chain variable region sequence of SEQ ID NO: 18 and the light chain variable region sequence of SEQ ID NO: 19.
XIII. The Type II anti-CD20 antibody of any one of aspect IX to XII, wherein the antibody comprised in the therapeutic agent specifically binds to a B-cell antigen, particularly an antigen selected from the group consisting of CD20, CD19, CD22, ROR-1, CD37 and CD5, more particularly CD20 or CD19, most particularly CD20.
XIV. The Type II anti-CD20 antibody of aspect XIII, wherein the antibody comprised in the therapeutic agent comprises a heavy chain variable region comprising the heavy chain CDR (HCDR) 1 of SEQ ID NO: 4, the HCDR2 of SEQ ID NO: 5, and the HCDR3 of SEQ ID NO: 6; and a light chain variable region comprising the light chain CDR (LCDR) 1 of SEQ ID NO: 7, the LCDR2 of SEQ ID NO: 8 and the LCDR3 of SEQ ID NO: 9.
XV. The Type II anti-CD20 antibody of aspect XIII or IV, wherein the antibody comprised in the therapeutic agent comprises the heavy chain variable region sequence of SEQ ID NO: 10 and the light chain variable region sequence of SEQ ID NO: 11.
XVI. The Type II anti-CD20 antibody of any one of the aspects I to XV, wherein the antibody comprised in the therapeutic agent is a bispecific antibody comprising (i) an antibody as defined in any one of claims 10 to 12 and (ii) an antibody as defined in any one of aspects XIII to XV.
XVII. The Type II anti-CD20 antibody of any one of aspects I to VIII, wherein the therapeutic agent comprises a T cell expressing a chimeric antigen receptor (CAR), particularly a CAR that specifically binds to a B-cell antigen, more particularly a CAR that specifically binds to an antigen selected from the group of CD20, CD 19, CD22, ROR-1, CD37 and CD5.
XVIII. The Type II anti-CD20 antibody of any one of the aspects I to XVII, wherein the disease is a B cell proliferative disorder, particularly a CD20-positive B-cell disorder, and/or is a disease selected from the group consisting of Non-Hodgkin lymphoma (NHL), acute lymphocytic leukemia (ALL), chronic lymphocytic leukemia (CLL), diffuse large B-cell lymphoma (DLBCL), follicular lymphoma (FL), mantle-cell lymphoma (MCL), marginal zone lymphoma (MZL), (Multiple myeloma (MM) and Hodgkin lymphoma (HL).

### Examples

The following are examples of methods and compositions of the invention. It is understood that various other embodiments may be practiced, given the general description provided above.

### Example 1

### Assessment of the Anti-Tumour Activity and Cytokine Release Mediated by CD20XCD3 bsAB ± Obinutuzumab Pre-Treatment (Gpt) in Fully Humanized Mice

We investigated whether Gpt could prevent the cytokine release associated with the first administration of CD20XCD3 bsAB in fully humanized NOG mice.

All treatment options (obinutuzumab, CD20XCD3 bsAB and Gpt + CD20XCD3 bsAB) led to efficient peripheral blood B-cell depletion detected already 24 hours after the first therapy administration (Figure 1A). T cell counts revealed a transient decrease in the peripheral blood 24 hours after the first administration of CD20XCD3 bsAB but not following obinutuzumab or Gpt + CD20XCD3 bsAB (Figure 1B). Therefore, when administered prior to CD20XCD3 bsAB, a single administration of obinutuzumab abrogates CD20XCD3 bsAB-mediated T cell decrease in the peripheral blood.

The analysis of cytokines released in blood of treated mice in the different experimental groups revealed that CD20XCD3 bsAB treatment induces a transient elevation of several cytokines in the blood, with a peak at 24 hours after the first administration and a return to near baseline levels by 72 hours (Figure 2). MIP-1b, IL-5, IL-10, MCP-1 show a similar trend to IFNγ, TNFα and IL-6 (not shown). Gpt strongly reduced the cytokine release in the peripheral blood associated with the first CD20XCD3 bsAB injection (Table 2).

**Table 2. Cytokines Released in the Peripheral Blood of Fully Humanized NOG Mice upon CD20XCD3 bsAB and Gpt + CD20XCD3 bsAB Treatments**

| | | **Treatment** | |
|---|---|---|---|
| **Cytokine** | **Vehicle (pg/ml)** | **CD20XCD3 bsAB (pg/ml)** | **Gpt + CD20XCD3 bsAB (pg/ml)** |
| **IFN-g** | 18.50 (18.07) | 756.95 (357.30) | 183.134 (171.91) |
| **TNF-a** | 12.47 (2.95) | 79.56 (28.98) | 14.89 (2.56) |
| **IL-6** | 15.39 (7.15) | 613.27 (140.60) | 178.34 (117.85) |
| **IL-8** | 11.44 (2.64) | 292.68 (132.36) | 150.58 (96.76) |
| **MIP-1b** | 272.70 (97.05) | 2129.44 (132.36) | 338.95 (71.25) |
| **MCP-1** | 73.49 (13.89) | 2146.31 (672.69) | 393.29 (188.86) |
| **IL-10** | 223.48 (62.48) | 15,278.89 (6584.50) | 945.04 (604.89) |
| **IL-4** | 0.75 (0.14) | 1.99 (0.77) | 0.81 (0.02) |
| **G-CSF** | 14.60 (5.14) | 21.23 (16.36) | 3.82 (2.02) |
| **GM-CSF** | 945.97 (155.74) | 1207.48 (299.83) | 626.18 (282.46) |
| **IL-5** | 10.42 (3.35) | 162.33 (140.82) | 13.58 (8.44) |
| **IL-2** | 19.1 (8.42) | 369.70 (360.64) | 19.59 (17.64) |
| **IL-13** | 5.39 (3.66) | 15.42 (11.18) | 2.96 (1.11) |
| **IL-1b** | 1.48 (0.2) | 6.40 (1.94) | 3.47 (1.88) |
| **IL-7** | 6.98 (0) | 4.27 (2.55) | 6.17 (1.79) |
| **IL-12p40** | 43.59 (19.45) | 51.31 (23.12) | 17.05 (2.62) |
| **IL-17** | 194.40 (96.32) | 274.79 (112.20) | 73.33 (32.43) |

| | | | |
|---|---|---|---|
| Notes: Data are displayed as the arithmetic mean (SD). N = 5 in both treatments. | | | |

The anti-tumour activity of CD20XCD3 bsAB was not affected by pre-treatment with obinutuzumab (Figure 3). Obinutuzumab treatment, as monotherapy, showed a strong anti-tumour activity, although with slower kinetics when compared to CD20XCD3 bsAB in this tumour and mouse model.

The data therefore indicate that Gpt reduces the cytokine release associated with the first CD20XCD3 bsAB injection, however, despite targeting the same antigen on tumour cells, the anti-tumour activity of CD20XCD3 bsAB is not affected by Gpt.

### Example 2

### Obinutuzumab pre-treatment study in cynomolgus monkeys

A mechanistic study (non-GLP) in male cynomolgus monkeys was performed to investigate the effects of pretreatment with obinutuzumab on CD20XCD3 bsAB at doses of 0.1, 0.3 and 1 mg/kg) (Table 3). In this study, 6 naive cynomolgus male monkeys/group (4 for Group 1), received an IV dose of either control article 1 (Groups 1 and 2) or obinutuzumab (50 mg/kg, Groups 3, 4, 5), followed 4 days later by treatment with control article 2 (Group 1), CD20XCD3 bsAB, 0.1 mg/kg (Group 2, Group 3), CD20XCD3 bsAB, 0.3 mg/kg (Group 4) or CD20XCD3 bsAB, 1 mg/kg (Group 5). Four days between the obinutuzumab and CD20XCD3 bsAB dosing was considered sufficient to allow depletion of B cells in peripheral blood, lymph nodes and spleen by obinutuzumab. On Day 12, 2 animals from Group 1, and 4 from Groups 2 to 5 were necropsied (terminal necropsy). Two animals from each group were retained for an 8-week recovery period.

**Table 3. Study Design: Obinutuzumab Pre-Treatment in Cynomolgus Monkeys.**

| **Group No**. | **Test Article** | **Dosing Day** | **Dose Level (mg/kg)** | **Number of Males** | |
|---|---|---|---|---|---|
| | | | | **Main**^{a} | **Recovery**^{b} |
| | Control Article 1 | 1 | 0 | | |
| 1 | Control Article 2 | 5 | 0 | 2 | 2 |
| | Control Article 1 | 1 | 0 | | |
| 2 | CD20XCD3 bsAB | 5 | 0.1 | 4 | 2 |
| | obinutuzumab | 1 | 50 | | |
| 3 | CD20XCD3 bsAB | 5 | 0.1 | 4 | 2 |
| | obinutuzumab | 1 | 50 | | |
| 4 | CD20XCD3 bsAB | 5 | 0.3 | 4 | 2 |
| | obinutuzumab | 1 | 50 | | |
| 5 | CD20XCD3 bsAB | 5 | 1 | 4 | 2 |

| | | | | | |
|---|---|---|---|---|---|
| Note: Control Article 1 = Control for obinutuzumab: Control article 2 = Control for CD20XCD3 bsAB. ^{a}Main group animals, terminal necropsy Day 12. ^{b}Recovery animals, necropsy week 8. | | | | | |

The following preliminary data are available from this currently ongoing study:
- Following pretreatment with obinutuzumab (50 mg/kg, Gpt), IV administration of CD20XCD3 bsAB was tolerated up to 1 mg/kg, the highest tested dose. Clinical signs, observed with CD20XCD3 bsAB alone (emesis, hunched posture and hypoactivity) were markedly reduced by Gpt at all doses of CD20XCD3 bsAB.
- CD20XCD3 bsAB administration alone resulted in the reduction of B lymphocytes and the activation and expansion of T-lymphocyte (CD4+ and CD8+) subsets and NK cells. Furthermore, the administration of obinutuzumab prior to CD20XCD3 bsAB administration resulted in B-lymphocyte depletion, as well as the subsequent attenuation of T-lymphocyte activation as demonstrated by reductions in the transient reductions of lymphocyte and monocyte populations after CD20XCD3 bsAB administration, as well as reductions in T-cell activation marker up-regulation and expansion, relative to changes present for animals that were treated with CD20XCD3 bsAB alone.
- The release of IFNγ, IL-8, TNFα, IL-2 and IL-6, 4-hour post- 0.1 mg/kg CD20XCD3 bsAB treatment, was markedly reduced in the Gpt groups. Similarly, low levels of cytokine release were noted at higher doses of CD20XCD3 bsAB in Gpt groups. CD20XCD3 bsAB-related histopathologic findings were restricted to the lymphoid organs (e.g. decreased cellularity specifically affecting the CD20-positive cells was present in the lymphoid follicles of the spleen). The CD20-positive cell decreases were almost completely reversed after the 8 week treatment-free period. No other histopathological changes were present, including in brain, spinal cord and sciatic nerve in monkeys treated with CD20XCD3 bsAB at 0.1 mg/kg and in animals administered CD20XCD3 bsAB at 0.1, 0.3 or 1 mg/kg following Gpt.

### Example 3

### Clinical evaluation of safety, tolerability and pharmacokinetics of CD20XCD3 bsAB with obinituzumab pre-treatment in patients with r/r NHL

A phase I dose-escalation study will be performed, the primary objectives of which include evaluation of the safety, tolerability and pharmacokinetics CD20XCD3 bsAB with obinutuzumab pre-treatment in patients with relapsed/refractory (r/r) NHL.

The study will enroll patients with r/r NHL, whose tumours are expected to express CD20 in B cells. Patients with CLL will not be enrolled. Patients are expected to have relapsed after or failed to respond to at least one prior treatment regimen.

Obinutuzumab and CD20XCD3 bsAB will be administered intravenously (IV).

Prior to administration of obinutuzumab and CD20XCD3 bsAB, premedication with corticosteroids (e.g., 100 mg IV prednisolone or equivalent) will be administered, along with anti-histamines and acetaminophen. Prophylactic measures for other events, such as tumor lysis syndrome will also be either recommended as needed or mandated.

CD20XCD3 bsAB will be initiated on Cycle 1/Day 1 (C1/D1) as a single agent by intravenous (IV) infusion, following pre-treatment with a single dose of obinutuzumab (1000 mg; IV) seven days in advance (Cycle 1/Day -7) of the first CD20XCD3 bsAB dose (Cycle 1/Day 1). The anticipated starting dose of CD20XCD3 bsAB will be 5 micrograms (flat dosing). All dosing cycles are 14 days (Q2W) long. The dosing scheme is for administration of CD20XCD3 bsAB on Days 1 and 8 in Cycle 1 (C1/D1; C1/D8), followed by dosing in all subsequent Cycles on Day 1 only (Q2W) for a total of 12 cycles (24 weeks) of treatment or until unacceptable toxicity or progression occurs.

Blood samples will be collected at appropriate timepoints to determine the relevant PK properties of CD20XCD3 bsAB, as well as a range of PD markers in blood, to assess e.g. magnitude and kinetics of B-cell depletion following Gpt and CD20XCD3 bsAB dose initiation, T-cell phenotypes, and to assess soluble mediator release (cytokines and chemokines), following administration of Gpt and CD20XCD3 bsAB at selected timepoints.

### Example 4

### GAZYVA pre-treatment to avoid cytokine release after adoptive T cell therapy with CAR-T cells

Cytokine release syndrome (CRS) is a very frequent phenomenon following treatment with CD19 CAR-T cells as well as CAR-T cells directed against CD20 or CD22 that can result in lethal side effects. Strategies to avoid or reduce CRS focus on various aspects of CAR-T therapy (reviewed in Xu and Tang, Cancer Letters (2014) 343, 172-178).

We suggest a novel approach to avoid CRS following treatment with CAR-T cells in B cell proliferative disorders, by depletion of peripheral and malignant B cells using obinutuzumab pre-treatment.

For this purpose, patients with a B-cell proliferative disorder (e.g. NHL) are randomized into an obinutuzumab pretreatment arm and a control arm without obinutuzumab pretreatment. The patients in the obinutuzumab pretreatment arm receive 1 g of obinutuzumab, administered on Day -7 (+/- 2 days) before administration of CD19, CD20 or CD22 CAR-T cells.

Patients are infused with autologous T cells transduced with a CAR lentiviral vector at an appropriate dose for the specific CAR-T cell used, the patient and the disease to be treated (e.g. 0.76×10⁶ to 20.6×10⁶ CAR-T cells per kilogram of body weight as described in Maude et al., N Engl J Med (2014) 371,1507-1517; 1.4×10⁶ to 1.2×10⁷ CAR-T cells per kilogram of body weight as described in Grupp et al., New Engl J Med (2013) 368, 1509-1518; or 0.14 × 10⁸ to 11 × 10⁸ CAR-T cells as described in Porter et al., Sci Transl Med (2015) 7, 303ra139). Patients are monitored for a response, toxic effects, and the expansion and persistence of circulating CAR-T cells.

Pre-medication is given prior to each obinutuzumab dosing. Blood samples are collected before and during the treatment period for the monitoring of B lymphocyte counts. B cell counts are obtained using flow cytometry and staining for CD 19. In addition, incidence of CRS is screened by measuring cytokines including IL-6.

### Example 5

### Combination treatment of an anti-CD20/anti-CD3 bispecific antibody with Obinutuzmab or anti-PD-L1 antibody

Figure 6 shows the efficacy of a combination treatment of an anti-CD20/anti-CD3 bispecific antibody with Obinutuzumab (Figure 6D) and of a combination treatment of an anti-CD20/anti-CD3 bispecific antibody with an anti-PD-L1 antibody (Figure 6E).

The anti-CD20/anti-CD3 bispecific antibody is a "2:1" T-cell bispecific humanized monoclonal antibody that binds to human CD20 on tumor cells through two fragment antigen-binding (Fab) domains, and to the human CD3 epsilon subunit (CD3e) of the T-cell receptor (TCR) complex on T cells through a single Fab domain. The molecule is based on the human IgG1 isotype, but contains an Fc-part devoid of Fc gamma receptor (FcyR) and complement (C1q) binding. The molecular weight is approximately 194 kDa. In example 5, the anti-CD20/anti-CD3 bispecific comprises the heavy chain according to SEQ ID NO: 116, the heavy chain according to SEQ ID NO: 117, twice the light chain according to SEQ ID NO: 118 and the light chain according to SEQ ID NO: 119.

The anti-PD-L1 antibody is based on the YW243.55.S70 PD-L1 antibody described in WO 2010/077634 (sequence shown in Figure 1I of WO 2010/077634). This antibody contained a DAPG mutation to abolish FcγR interaction. The variable region of YW243.55.S70 was attached to a murine IgG1 constant domain with DAPG Fc mutations. The anti-PD-L1 antibody used in example 5 comprises heavy chains according to SEQ ID NO: 120 and light chains according to SEQ ID NO: 121.

Anti-tumor activity of the anti-CD20/anti-CD3 bispecific antibody upon combination with Obinutuzumab (GAZYVA; Figure 6D) and anti-PD-L1 antibody (Figure 6E) was analyzed in human hematopoietic stem-cell humanized mice (HSC-NSG mice) bearing aggressive lymphoma model (WSU-DLCL2 tumor), which was injected subcutaneously on day 0. The therapy started when the tumor average volume was 600 mm3 as indicated by the arrow in Figures 6A-F. The tumor average volume of 600 mm3 was reached at study day 15.

For the combination treatment of the anti-CD20/anti-CD3 bispecific antibody with Obinutuzumab the anti-CD20/anti-CD3 bispecific antibody was administered intravenously at a sub-optimally efficacious dose of 0.15 mg/kg once per week. Obinutuzumab was administered intravenously at 10 mg/kg, once per week (Figure 6D). The two partners were injected simultaneously.

For the combination treatment of the anti-CD20/anti-CD3 bispecific antibody with the anti-PD-L1 antibody the anti-CD20/anti-CD3 bispecific antibody was administered intravenously at a sub-optimally efficacious dose of 0.15 mg/kg once per week and the anti-PD-L1 antibody was administered intravenously at 10 mg/kg, once per week (Figure 6E). The two partners were also injected simultaneously.

The animals in the vehicle group received weekly intravenous injections of Phosphate Buffer Saline (Figure 6A). In the monotherapy groups, the anti-CD20/anti-CD3 bispecific antibody (Figure 6B) was administered intravenously at 10 mg/kg, once per week, Obinutuzumab (Figure 6C) was administered intravenously at 10 mg/kg, once per week, and the anti-PD-L1 (Figure 6F) antibody was administered intravenously at 10 mg/kg, once per week. Each group contained 10 animals. The monotherapy groups were not statistically different between each other according to one-way ANOVA of the standardized area-under-the-curve (sAUC) with the Dunnet's method (Table 4).

**Table 4. Statistical analysis of in vivo-data of monotherapy groups.**

| **Study group** | **Vehicle** | **anti-CD20/anti-CD3 bispecific antibody** | **Obinutuzumab** | **anti-PD-L1 antibody** |
|---|---|---|---|---|
| **Vehicle** | 1.0000 | 0.2250 | 0.2038 | 0.3319 |
| **anti-CD20/anti-CD3 bispecific antibody** | 0.2250 | 1.0000 | 1.0000 | 0.9989 |
| **Obinutuzumab** | 0.2038 | 1.0000 | 1.0000 | 0.9974 |
| **anti-PD-L1 antibody** | 0.3319 | 0.9989 | 0.9974 | 1.0000 |

The combination treatments of either anti-CD20/anti-CD3 bispecific antibody with Obinutuzumab or anti-CD20/anti-CD3 bispecific antibody with the anti-PD-L1 antibody, as indicated above, show a significant decrease in tumor average size during the course of the study. This indicates a superior potential of the combination treatments to reduce tumor average size compared to the individual treatment of either the anti-CD20/anti-CD3 bispecific antibody, Obinutzumab or the anti-PD-L1 antibody.

The above in-vivo data relating to the combination treatment of example 5 were statistically analyzed according to one-way ANOVA of the sAUC with the Dunnet's method (Table 5).

**Table 5. Statistical analysis of in-vivo data of combination treatments.**

| Antibody combination | vs antibody or antibody combination | p-value |
|---|---|---|
| anti-CD20/anti-CD3 bispecific antibody and Obinutuzumab | anti-CD20/anti-CD3 bispecific antibody | <0.0001 |
| anti-CD20/anti-CD3 bispecific antibody and anti-PD-L1 antibody | anti-CD20/anti-CD3 bispecific antibody | 0.0084 |
| anti-CD20/anti-CD3 bispecific antibody and anti-PD-L1 antibody | anti-CD20/anti-CD3 bispecific antibody and Obinutuzumab | 0.0007 |

In the tested conditions the combination treatment of anti-CD20/anti-CD3 bispecific antibody with Obinutuzumab showed a stronger effect on the reduction of average tumor size compared to the combination treatment of anti-CD20/anti-CD3 bispecific antibody with the anti-PD-L1 antibody.

Although the foregoing invention has been described in some detail by way of illustration and example for purposes of clarity of understanding, the descriptions and examples should not be construed as limiting the scope of the invention. The disclosures of all patent and scientific literature cited herein are expressly incorporated in their entirety by reference.

## Claims

**1.** A Type II anti-CD20 antibody for the use in a method for treating or delaying progression of cancer of an individual, wherein the Type II anti-CD20 antibody is used in combination with an anti-CD20/anti-CD3 bispecific antibody.

**2.** The Type II anti-CD20 antibody for the use in a method according to claim 1, wherein the anti-CD20/anti-CD3 bispecific antibody and the Type II anti-CD20 antibody are administered together in a single composition or administered separately in two or more different compositions.

**3.** The Type II anti-CD20 antibody for the use in a method according to claim 1 or 2, wherein the anti-CD20/anti-CD3 bispecific antibody and the Type II anti-CD20 antibody are administered in two or more different composition, wherein the two or more different compositions are administered at different points in time.

**4.** The Type II anti-CD20 antibody for the use in a method according to any of claims 1 to 3, wherein the Type II anti-CD20 antibody comprises a heavy chain variable region comprising the heavy chain CDR(HCDR) 1 of SEQ ID NO: 4, the HCDR2 of SEQ ID NO: 5, and the HCDR3 of SEQ ID NO: 6; and a light chain variable region comprising the light chain CDR (LCDR) 1 of SEQ IDNO: 7, the LCDR2 of SEQ ID NO: 8 and the LCDR3 of SEQ ID NO: 9.

**5.** The Type II anti-CD20 antibody for the use in a method according to any of claims 1 to 4, wherein the Type II anti-CD20 antibody comprises the heavy chain variable region sequence of SEQ ID NO: 10 and the light chain variable region sequence of SEQ ID NO: 11.

**6.** The Type II anti-CD20 antibody the use in a method according to any of claims 1 to 5, wherein the Type II anti-CD20 antibody is an IgG antibody, particularly an IgG1 antibody, and wherein at least about 40% of the N-linked oligosaccharides in the Fc region of the anti-CD20 antibody are nonfucosylated.

**7.** The Type II anti-CD20 antibody for the use in a method according to any of claims 1 to 6, wherein the Type II anti-CD20 antibody is Obinutuzumab.

**8.** The Type II anti-CD20 antibody for the use in a method according to any of claims 1 to 7, wherein the Type II anti-CD20 antibody is administered concurrently with, prior to, or subsequently to the anti-CD20/anti-CD3 bispecific antibody.

**9.** The Type II anti-CD20 antibody for the use in a method according to any of claims 1 to 8, wherein furthermore an anti-PD-L1 antibody, preferably Atezolizumab, is administered.

**10.** The Type II anti-CD20 antibody for the use in a method according to claim 9, wherein the anti-PD-L1 antibody is administered separately or in combination with at least one of the anti-CD20/anti-CD3 bispecific antibody and the Type II anti-CD20 antibody.

**11.** The Type II anti-CD20 antibody for the use in a method of any one of the preceding claims, wherein the anti-CD20/anti-CD3 bispecific antibody comprises a first antigen binding domain that binds to CD3, and a second antigen binding domain that binds to CD20.

**12.** The Type II anti-CD20 antibody for use in a method according to claim 11, wherein the anti-CD20/anti-CD3 bispecific antibody comprises a first antigen binding domain comprising a heavy chain variable region (VHCD3) and a light chain variable region (VLCD3), and a second antigen binding domain comprising a heavy chain variable region (VHCD20) and a light chain variable region (VLCD20).

**13.** The Type II anti-CD20 antibody for use in a method of any one of claims 11 to 12, wherein the first antigen binding domain of the anti-CD20/anti-CD3 bispecific antibody comprises a heavy chain variable region (VHCD3) comprising CDR-H1 sequence of SEQ ID NO: 97, CDR-H2 sequence of SEQ ID NO: 98, and CDR-H3 sequence of SEQ ID NO: 99; and/or a light chain variable region (VLCD3) comprising CDR-L1 sequence of SEQ ID NO: 100, CDR-L2 sequence of SEQ ID NO: 101, and CDR-L3 sequence of SEQ ID NO: 102.

**14.** The Type II anti-CD20 antibody for use in a method of any one of claims 11 to 12, wherein the first antigen binding domain of the anti-CD20/anti-CD3 bispecific antibody comprises a heavy chain variable region (VHCD3) comprising the amino acid sequence of SEQ ID NO: 103 and/or a light chain variable region (VLCD3) comprising the amino acid sequence of SEQ ID NO: 104.

**15.** The Type II anti-CD20 antibody for use in a method of any one of claims 11 to 14, wherein the second antigen binding domain of the anti-CD20/anti-CD3 bispecific antibody comprises a heavy chain variable region (VHCD20) comprising CDR-H1 sequence of SEQ ID NO: 4, CDR-H2 sequence of SEQ ID NO: 5, and CDR-H3 sequence of SEQ ID NO: 6, and/or a light chain variable region (VLCD20) comprising CDR-L1 sequence of SEQ ID NO: 7, CDR-L2 sequence of SEQ ID NO: 8, and CDR-L3 sequence of SEQ ID NO: 9.

**16.** The Type II anti-CD20 antibody for use in a method of any of claims 11 to 14, wherein the second antigen binding domain of the anti-CD20/anti-CD3 bispecific antibody comprises a heavy chain variable region (VHCD20) comprising the amino acid sequence of SEQ ID NO: 10 and/or a light chain variable region (VLCD20) comprising the amino acid sequence of SEQ ID NO: 11.

**17.** The Type II anti-CD20 antibody for use in a method of any of claims 11 to 16, wherein the anti-CD20/anti-CD3 bispecific antibody comprises a third antigen binding domain that binds to CD20.

**18.** The Type II anti-CD20 antibody for use in a method of claim 17, wherein the third antigen binding domain of the anti-CD20/anti-CD3 bispecific antibody comprises a heavy chain variable region (VHCD20) comprising CDR-H1 sequence of SEQ ID NO: 4, CDR-H2 sequence of SEQ ID NO: 5, and CDR-H3 sequence of SEQ ID NO: 6; and/or a light chain variable region (VLCD20) comprising CDR-L1 sequence of SEQ ID NO: 7, CDR-L2 sequence of SEQ ID NO: 8, and CDR-L3 sequence of SEQ ID NO: 9.

**19.** The Type II anti-CD20 antibody for use in a method of claim 17 or 18, wherein the third antigen binding domain of the anti-CD20/anti-CD3 bispecific antibody comprises a heavy chain variable region (VHCD20) comprising the amino acid sequence of SEQ ID NO: 10 and/or a light chain variable region (VLCD20) comprising the amino acid sequence of SEQ ID NO: 11.

**20.** The Type II anti-CD20 antibody for use in a method of any of claims 11 to 19, wherein the first antigen binding domain of the anti-CD20/anti-CD3 bispecific antibody is a cross-Fab molecule wherein the variable domains or the constant domains of the Fab heavy and light chain are exchanged, and the second and third, if present, antigen binding domain is a conventional Fab molecule.

**21.** The Type II anti-CD20 antibody for use in a method of claim 20, wherein the anti-CD20/anti-CD3 bispecific antibody comprises an IgG1 Fc domain.

**22.** The Type II anti-CD20 antibody for use in a method of claim 21, wherein the IgG1 Fc domain of the anti-CD20/anti-CD3 bispecific antibody comprises one or more amino acid substitutions that reduce binding to an Fc receptor and/or effector function.

**23.** The Type II anti-CD20 antibody for use in a method of claims 21 or 22, wherein the IgG1 Fc domain of the anti-CD20/anti-CD3 bispecific antibody comprises the amino acid substitutions L234A, L235A and P329G (numbering according to Kabat EU index).

**24.** The Type II anti-CD20 antibody for use in a method of any of claims 21 to 23, wherein the anti-CD20/anti-CD3 bispecific antibody comprises a third antigen binding domain,
wherein (i) the second antigen binding domain of the anti-CD20/anti-CD3 bispecific antibody is fused at the C-terminus of the Fab heavy chain to the N-terminus of the Fab heavy chain of the first antigen binding domain, the first antigen binding domain of the anti-CD20/anti-CD3 bispecific antibody is fused at the C-terminus of the Fab heavy chain to the N-terminus of the first subunit of the Fc domain, and the third antigen binding domain of the anti-CD20/anti-CD3 bispecific antibody is fused at the C-terminus of the Fab heavy chain to the N-terminus of the second subunit of the Fc domain, or (ii) the first antigen binding domain of the anti-CD20/anti-CD3 bispecific antibody is fused at the C-terminus of the Fab heavy chain to the N-terminus of the Fab heavy chain of the second antigen binding domain, the second antigen binding domain of the anti-CD20/anti-CD3 bispecific antibody is fused at the C-terminus of the Fab heavy chain to the N-terminus of the first subunit of the Fc domain, and the third antigen binding domain of the anti-CD20/anti-CD3 bispecific antibody is fused at the C-terminus of the Fab heavy chain to the N-terminus of the second subunit of the Fc domain.

**25.** The Type II anti-CD20 antibody for use in a method of any one of the preceding claims, wherein the combination is administered at intervals from about one week to three weeks.

**26.** The Type II anti-CD20 antibody for the use in a method according to any of claims 1 to 25, wherein a pretreatment with a Type II anti-CD20 antibody, preferably Obinutuzumab, is performed prior to the combination treatment, wherein the period of time between the pretreatment and the combination treatment is sufficient for the reduction of B-cells in the individual in response to the Type II anti-CD20 antibody, preferably Obinutuzumab.

**27.** A method for treating or delaying progression of a proliferative disease, particularly cancer, in an individual comprising administering an Type II anti-CD20 antibody and an anti-CD20/anti-CD3 bispecific antibody, wherein the Type II anti-CD20 antibody and the anti-CD20/anti-CD3 bispecific antibody are administered in a single composition or in two or more compositions.

**28.** A pharmaceutical composition comprising a Type II anti-CD20 antibody for the use in a combination treatment and an optional pharmaceutically acceptable carrier, and a second medicament comprising an anti-CD20/anti-CD3 bispecific antibody and an optional pharmaceutically acceptable carrier, and optionally a third medicament comprising an anti-PD-L1 antibody and an optional pharmaceutically acceptable carrier, for the use in the combined treatment of a disease, in particular cancer.

**29.** A kit comprising a first medicament comprising a Type II anti-CD20 antibody and an optional pharmaceutically acceptable carrier, and a second medicament comprising an anti-CD20/anti-CD3 bispecific antibody and an optional pharmaceutically acceptable carrier, and optionally a third medicament comprising an anti-PD-L1 antibody and an optional pharmaceutically acceptable carrier, for the use in the combined treatment of a disease, in particular cancer.

**30.** The kit according to claim 29, wherein the kit comprises instructions for use of the first medicament and the second medicament and optionally the third medicament for treating or delaying the progression of cancer in an individual.

**31.** Use of a combination of a Type II anti-CD20 antibody and an anti-CD20/anti-CD3 bispecific antibody in the manufacture of a medicament for therapeutic application, preferably for treating or delaying the progression of a proliferative disease, particularly cancer, in an individual.

**32.** Use of a Type II anti-CD20 antibody in the manufacture of a medicament for treating or delaying progression of cancer in an individual, wherein the medicament comprises the Type II anti-CD20 antibody and an optional pharmaceutically acceptable carrier, and wherein the treatment comprises administration of the medicament in combination with a composition comprising an anti-CD20/anti-CD3 bispecific antibody and an optional pharmaceutically acceptable carrier.

**33.** Use of an anti-CD20/anti-CD3 bispecific antibody in the manufacture of a medicament for treating or delaying progression of cancer in an individual, wherein the medicament comprises the anti-CD20/anti-CD3 bispecific antibody and an optional pharmaceutically acceptable carrier, and wherein the treatment comprises administration of the medicament in combination with a composition comprising an anti-CD20 antibody and an optional pharmaceutically acceptable carrier.

**36.** A method for treating or delaying progression of cancer in an individual comprising administering a Type II anti-CD20 antibody and of an anti-CD20/anti-CD3 antibody to the individual.

**37.** The method according to clam 36, wherein furthermore an anti-PD-L1 antibody is administered to the individual.

**39.** An anti-CD20/anti-CD3 bispecific antibody for the use in a method for treating or delaying progression of cancer of an individual, wherein the anti-CD20/anti-CD3 bispecific antibody is used in combination with a Type II anti-CD20 antibody.

**40.** The anti-CD20/anti-CD3 bispecific antibody for the use in a method according to claim 39, wherein the anti-CD20/anti-CD3 bispecific antibody and the Type II anti-CD20 antibody are administered together in a single composition or administered separately in two or more different compositions.

**41.** The anti-CD20/anti-CD3 bispecific antibody for the use in a method according to claim 39 or 40, wherein the anti-CD20/anti-CD3 bispecific antibody and the Type II anti-CD20 antibody are administered in two or more different composition, wherein the two or more different compositions are administered at different points in time.

**42.** The anti-CD20/anti-CD3 bispecific antibody for the use in a method according to any of claims 39 to 41, wherein the Type II anti-CD20 antibody comprises a heavy chain variable region comprising the heavy chain CDR(HCDR)1 of SEQ ID NO: 4, the HCDR2 of SEQ ID NO: 5, and the HCDR3 of SEQ ID NO: 6; and a light chain variable region comprising the light chain CDR (LCDR) 1 of SEQ IDNO: 7, the LCDR2 of SEQ ID NO: 8 and the LCDR3 of SEQ ID NO: 9.

**43.** The anti-CD20/anti-CD3 bispecific antibody for the use in a method according to any of claims 39 to 42, wherein the Type II anti-CD20 antibody comprises the heavy chain variable region sequence of SEQ ID NO: 10 and the light chain variable region sequence of SEQ ID NO: 11.

**44.** The anti-CD20/anti-CD3 bispecific antibody the use in a method according to any of claims 39 to 42, wherein the Type II anti-CD20 antibody is an IgG antibody, particularly an IgG1 antibody, and wherein at least about 40% of the N-linked oligosaccharides in the Fc region of the anti-CD20 antibody are nonfucosylated.

**45.** The anti-CD20/anti-CD3 bispecific antibody for the use in a method according to any of claims 39 to 44, wherein the Type II anti-CD20 antibody is Obinutuzumab.

**46.** The anti-CD20/anti-CD3 bispecific antibody for the use in a method according to any of claims 39 to 45, wherein the Type II anti-CD20 antibody is administered concurrently with, prior to, or subsequently to the anti-CD20/anti-CD3 bispecific antibody.

**47.** The anti-CD20/anti-CD3 bispecific antibody for the use in a method according to any of claims 39 to 46, wherein furthermore an anti-PD-L1 antibody, preferably Atezolizumab, is administered.

**48.** The anti-CD20/anti-CD3 bispecific antibody for the use in a method according to claim 47, wherein the anti-PD-L1 antibody is administered separately or in combination with at least one of the anti-CD20/anti-CD3 bispecific antibody and the Type II anti-CD20 antibody.

**49.** The anti-CD20/anti-CD3 bispecific antibody for use in a method of any one of claims 39 to 48, wherein the anti-CD20/anti-CD3 bispecific antibody comprises a first antigen binding domain that binds to CD3, and a second antigen binding domain that binds to CD20.

**50.** The anti-CD20/anti-CD3 bispecific antibody for use in a method according to claim 49, wherein the anti-CD20/anti-CD3 bispecific antibody comprises a first antigen binding domain comprising a heavy chain variable region (VHCD3) and a light chain variable region (VLCD3), and a second antigen binding domain comprising a heavy chain variable region (VHCD20) and a light chain variable region (VLCD20).

**51.** The anti-CD20/anti-CD3 bispecific antibody for use in a method of any one of claims 48 to 50, wherein the first antigen binding domain of the anti-CD20/anti-CD3 bispecific antibody comprises a heavy chain variable region (VHCD3) comprising CDR-H1 sequence of SEQ ID NO: 97, CDR-H2 sequence of SEQ ID NO: 98, and CDR-H3 sequence of SEQ ID NO: 99; and/or a light chain variable region (VLCD3) comprising CDR-L1 sequence of SEQ ID NO: 100, CDR-L2 sequence of SEQ ID NO: 101, and CDR-L3 sequence of SEQ ID NO: 102.

**52.** The anti-CD20/anti-CD3 bispecific antibody for use in a method of any one of claims 48 to 51, wherein the first antigen binding domain of the anti-CD20/anti-CD3 bispecific antibody comprises a heavy chain variable region (VHCD3) comprising the amino acid sequence of SEQ ID NO: 103 and/or a light chain variable region (VLCD3) comprising the amino acid sequence of SEQ ID NO: 104.

**53.** The anti-CD20/anti-CD3 bispecific antibody for use in a method of any one of claims 48 to 52, wherein the second antigen binding domain of the anti-CD20/anti-CD3 bispecific antibody comprises a heavy chain variable region (VHCD20) comprising CDR-H1 sequence of SEQ ID NO: 4, CDR-H2 sequence of SEQ ID NO: 5, and CDR-H3 sequence of SEQ ID NO: 6, and/or a light chain variable region (VLCD20) comprising CDR-L1 sequence of SEQ ID NO: 7, CDR-L2 sequence of SEQ ID NO: 8, and CDR-L3 sequence of SEQ ID NO: 9.

**54.** The anti-CD20/anti-CD3 bispecific antibody for use in a method of any of claims 48 to 52, wherein the second antigen binding domain of the anti-CD20/anti-CD3 bispecific antibody comprises a heavy chain variable region (VHCD20) comprising the amino acid sequence of SEQ ID NO: 10 and/or a light chain variable region (VLCD20) comprising the amino acid sequence of SEQ ID NO: 11.

**55.** The anti-CD20/anti-CD3 bispecific antibody for use in a method of any of claims 48 to 54, wherein the anti-CD20/anti-CD3 bispecific antibody comprises a third antigen binding domain that binds to CD20.

**56.** The anti-CD20/anti-CD3 bispecific antibody for use in a method of claim 55, wherein the third antigen binding domain of the anti-CD20/anti-CD3 bispecific antibody comprises a heavy chain variable region (VHCD20) comprising CDR-H1 sequence of SEQ ID NO: 4, CDR-H2 sequence of SEQ ID NO: 5, and CDR-H3 sequence of SEQ ID NO: 6; and/or a light chain variable region (VLCD20) comprising CDR-L1 sequence of SEQ ID NO: 7, CDR-L2 sequence of SEQ ID NO: 8, and CDR-L3 sequence of SEQ ID NO: 9.

**57.** The anti-CD20/anti-CD3 bispecific antibody for use in a method of claim 55 or 56, wherein the third antigen binding domain of the anti-CD20/anti-CD3 bispecific antibody comprises a heavy chain variable region (VHCD20) comprising the amino acid sequence of SEQ ID NO: 10 and/or a light chain variable region (VLCD20) comprising the amino acid sequence of SEQ ID NO: 11.

**58.** The anti-CD20/anti-CD3 bispecific antibody for use in a method of any of claims 48 to 57, wherein the first antigen binding domain of the anti-CD20/anti-CD3 bispecific antibody is a cross-Fab molecule wherein the variable domains or the constant domains of the Fab heavy and light chain are exchanged, and the second and third, if present, antigen binding domain is a conventional Fab molecule.

**59.** The anti-CD20/anti-CD3 bispecific antibody for use in a method of claim 58, wherein the anti-CD20/anti-CD3 bispecific antibody comprises an IgG1 Fc domain.

**60.** The anti-CD20/anti-CD3 bispecific antibody for use in a method of claim 59, wherein the IgG1 Fc domain of the anti-CD20/anti-CD3 bispecific antibody comprises one or more amino acid substitutions that reduce binding to an Fc receptor and/or effector function.

**61.** The anti-CD20/anti-CD3 bispecific antibody for use in a method of claims 59 or 60, wherein the IgG1 Fc domain of the anti-CD20/anti-CD3 bispecific antibody comprises the amino acid substitutions L234A, L235A and P329G (numbering according to Kabat EU index).

**62.** The anti-CD20/anti-CD3 bispecific antibody for use in a method of any of claims 59 to 61, wherein the anti-CD20/anti-CD3 bispecific antibody comprises a third antigen binding domain,
wherein (i) the second antigen binding domain of the anti-CD20/anti-CD3 bispecific antibody is fused at the C-terminus of the Fab heavy chain to the N-terminus of the Fab heavy chain of the first antigen binding domain, the first antigen binding domain of the anti-CD20/anti-CD3 bispecific antibody is fused at the C-terminus of the Fab heavy chain to the N-terminus of the first subunit of the Fc domain, and the third antigen binding domain of the anti-CD20/anti-CD3 bispecific antibody is fused at the C-terminus of the Fab heavy chain to the N-terminus of the second subunit of the Fc domain, or (ii) the first antigen binding domain of the anti-CD20/anti-CD3 bispecific antibody is fused at the C-terminus of the Fab heavy chain to the N-terminus of the Fab heavy chain of the second antigen binding domain, the second antigen binding domain of the anti-CD20/anti-CD3 bispecific antibody is fused at the C-terminus of the Fab heavy chain to the N-terminus of the first subunit of the Fc domain, and the third antigen binding domain of the anti-CD20/anti-CD3 bispecific antibody is fused at the C-terminus of the Fab heavy chain to the N-terminus of the second subunit of the Fc domain.

**63.** The anti-CD20/anti-CD3 bispecific antibody for use in a method of claims 39 to 62, wherein the combination is administered at intervals from about one week to three weeks.

**64.** The anti-CD20/anti-CD3 bispecific antibody for the use in a method according to any of claims 39 to 63, wherein a pretreatment with an Type II anti-CD20 antibody, preferably Obinutuzumab, is performed prior to the combination treatment, wherein the period of time between the pretreatment and the combination treatment is sufficient for the reduction of B-cells in the individual in response to the Type II anti-CD20 antibody, preferably Obinutuzumab.

**65.** A pharmaceutical composition comprising an anti-CD20/anti-CD3 bispecific antibody for the use in a combination treatment and an optional pharmaceutically acceptable carrier, and a second medicament comprising an Type II anti-CD20 antibody and an optional pharmaceutically acceptable carrier, and optionally a third medicament comprising an anti-PD-L1 antibody and an optional pharmaceutically acceptable carrier, for the use in the combined treatment of a disease, in particular cancer.

**66.** The invention as described hereinbefore.


SEQUENCE LISTING

<110>  F. Hoffmann-La Roche AG

<120>  Treatment method

<130>  PX

<160>  121

<170>  PatentIn version 3.5

<210>  1
<211>  297
<212>  PRT
<213>  Homo sapiens

<400>  1

Met Thr Thr Pro Arg Asn Ser Val Asn Gly Thr Phe Pro Ala Glu Pro
1               5                   10                  15

Met Lys Gly Pro Ile Ala Met Gln Ser Gly Pro Lys Pro Leu Phe Arg
            20                  25                  30

Arg Met Ser Ser Leu Val Gly Pro Thr Gln Ser Phe Phe Met Arg Glu
        35                  40                  45

Ser Lys Thr Leu Gly Ala Val Gln Ile Met Asn Gly Leu Phe His Ile
    50                  55                  60

Ala Leu Gly Gly Leu Leu Met Ile Pro Ala Gly Ile Tyr Ala Pro Ile
65                  70                  75                  80

Cys Val Thr Val Trp Tyr Pro Leu Trp Gly Gly Ile Met Tyr Ile Ile
                85                  90                  95

Ser Gly Ser Leu Leu Ala Ala Thr Glu Lys Asn Ser Arg Lys Cys Leu
            100                 105                 110

Val Lys Gly Lys Met Ile Met Asn Ser Leu Ser Leu Phe Ala Ala Ile
        115                 120                 125

Ser Gly Met Ile Leu Ser Ile Met Asp Ile Leu Asn Ile Lys Ile Ser
    130                 135                 140

His Phe Leu Lys Met Glu Ser Leu Asn Phe Ile Arg Ala His Thr Pro
145                 150                 155                 160

Tyr Ile Asn Ile Tyr Asn Cys Glu Pro Ala Asn Pro Ser Glu Lys Asn
                165                 170                 175

Ser Pro Ser Thr Gln Tyr Cys Tyr Ser Ile Gln Ser Leu Phe Leu Gly
            180                 185                 190

Ile Leu Ser Val Met Leu Ile Phe Ala Phe Phe Gln Glu Leu Val Ile
        195                 200                 205

Ala Gly Ile Val Glu Asn Glu Trp Lys Arg Thr Cys Ser Arg Pro Lys
    210                 215                 220

Ser Asn Ile Val Leu Leu Ser Ala Glu Glu Lys Lys Glu Gln Thr Ile
225                 230                 235                 240

Glu Ile Lys Glu Glu Val Val Gly Leu Thr Glu Thr Ser Ser Gln Pro
                245                 250                 255

Lys Asn Glu Glu Asp Ile Glu Ile Ile Pro Ile Gln Glu Glu Glu Glu
            260                 265                 270

Glu Glu Thr Glu Thr Asn Phe Pro Glu Pro Pro Gln Asp Gln Glu Ser
        275                 280                 285

Ser Pro Ile Glu Asn Asp Ser Ser Pro
    290                 295

<210>  2
<211>  112
<212>  PRT
<213>  Mus musculus

<400>  2

Gly Pro Glu Leu Val Lys Pro Gly Ala Ser Val Lys Ile Ser Cys Lys
1               5                   10                  15

Ala Ser Gly Tyr Ala Phe Ser Tyr Ser Trp Met Asn Trp Val Lys Leu
            20                  25                  30

Arg Pro Gly Gln Gly Leu Glu Trp Ile Gly Arg Ile Phe Pro Gly Asp
        35                  40                  45

Gly Asp Thr Asp Tyr Asn Gly Lys Phe Lys Gly Lys Ala Thr Leu Thr
    50                  55                  60

Ala Asp Lys Ser Ser Asn Thr Ala Tyr Met Gln Leu Thr Ser Leu Thr
65                  70                  75                  80

Ser Val Asp Ser Ala Val Tyr Leu Cys Ala Arg Asn Val Phe Asp Gly
                85                  90                  95

Tyr Trp Leu Val Tyr Trp Gly Gln Gly Thr Leu Val Thr Val Ser Ala
            100                 105                 110

<210>  3
<211>  103
<212>  PRT
<213>  Mus musculus

<400>  3

Asn Pro Val Thr Leu Gly Thr Ser Ala Ser Ile Ser Cys Arg Ser Ser
1               5                   10                  15

Lys Ser Leu Leu His Ser Asn Gly Ile Thr Tyr Leu Tyr Trp Tyr Leu
            20                  25                  30

Gln Lys Pro Gly Gln Ser Pro Gln Leu Leu Ile Tyr Gln Met Ser Asn
        35                  40                  45

Leu Val Ser Gly Val Pro Asp Arg Phe Ser Ser Ser Gly Ser Gly Thr
    50                  55                  60

Asp Phe Thr Leu Arg Ile Ser Arg Val Glu Ala Glu Asp Val Gly Val
65                  70                  75                  80

Tyr Tyr Cys Ala Gln Asn Leu Glu Leu Pro Tyr Thr Phe Gly Gly Gly
                85                  90                  95

Thr Lys Leu Glu Ile Lys Arg
            100

<210>  4
<211>  5
<212>  PRT
<213>  Artificial Sequence

<220>
<223>  CD20 HCDR1

<400>  4

Tyr Ser Trp Ile Asn
1               5

<210>  5
<211>  17
<212>  PRT
<213>  Artificial Sequence

<220>
<223>  CD20 HCDR2

<400>  5

Arg Ile Phe Pro Gly Asp Gly Asp Thr Asp Tyr Asn Gly Lys Phe Lys
1               5                   10                  15

Gly

<210>  6
<211>  10
<212>  PRT
<213>  Artificial Sequence

<220>
<223>  CD20 HCDR3

<400>  6

Asn Val Phe Asp Gly Tyr Trp Leu Val Tyr
1               5                   10

<210>  7
<211>  16
<212>  PRT
<213>  Artificial Sequence

<220>
<223>  CD20 LCDR1

<400>  7

Arg Ser Ser Lys Ser Leu Leu His Ser Asn Gly Ile Thr Tyr Leu Tyr
1               5                   10                  15

<210>  8
<211>  7
<212>  PRT
<213>  Artificial Sequence

<220>
<223>  CD20 LCDR2

<400>  8

Gln Met Ser Asn Leu Val Ser
1               5

<210>  9
<211>  9
<212>  PRT
<213>  Artificial Sequence

<220>
<223>  CD20 LCDR3

<400>  9

Ala Gln Asn Leu Glu Leu Pro Tyr Thr
1               5

<210>  10
<211>  119
<212>  PRT
<213>  Artificial Sequence

<220>
<223>  CD20 VH

<400>  10

Gln Val Gln Leu Val Gln Ser Gly Ala Glu Val Lys Lys Pro Gly Ser
1               5                   10                  15

Ser Val Lys Val Ser Cys Lys Ala Ser Gly Tyr Ala Phe Ser Tyr Ser
            20                  25                  30

Trp Ile Asn Trp Val Arg Gln Ala Pro Gly Gln Gly Leu Glu Trp Met
        35                  40                  45

Gly Arg Ile Phe Pro Gly Asp Gly Asp Thr Asp Tyr Asn Gly Lys Phe
    50                  55                  60

Lys Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Asn Val Phe Asp Gly Tyr Trp Leu Val Tyr Trp Gly Gln Gly
            100                 105                 110

Thr Leu Val Thr Val Ser Ser
        115


<210>  11
<211>  115
<212>  PRT
<213>  Artificial Sequence

<220>
<223>  CD20 VL

<400>  11

Asp Ile Val Met Thr Gln Thr Pro Leu Ser Leu Pro Val Thr Pro Gly
1               5                   10                  15

Glu Pro Ala Ser Ile Ser Cys Arg Ser Ser Lys Ser Leu Leu His Ser
            20                  25                  30

Asn Gly Ile Thr Tyr Leu Tyr Trp Tyr Leu Gln Lys Pro Gly Gln Ser
        35                  40                  45

Pro Gln Leu Leu Ile Tyr Gln Met Ser Asn Leu Val Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Ala Gln Asn
                85                  90                  95

Leu Glu Leu Pro Tyr Thr Phe Gly Gly Gly Thr Lys Val Glu Ile Lys
            100                 105                 110

Arg Thr Val
        115

<210>  12
<211>  5
<212>  PRT
<213>  Artificial Sequence

<220>
<223>  CD3 HCDR1

<400>  12

Thr Tyr Ala Met Asn
1               5

<210>  13
<211>  19
<212>  PRT
<213>  Artificial Sequence

<220>
<223>  CD3 HCDR2

<400>  13

Arg Ile Arg Ser Lys Tyr Asn Asn Tyr Ala Thr Tyr Tyr Ala Asp Ser
1               5                   10                  15

Val Lys Gly

<210>  14
<211>  14
<212>  PRT
<213>  Artificial Sequence

<220>
<223>  CD3 HCDR3

<400>  14

His Gly Asn Phe Gly Asn Ser Tyr Val Ser Trp Phe Ala Tyr
1               5                   10

<210>  15
<211>  14
<212>  PRT
<213>  Artificial Sequence

<220>
<223>  CD3 LCDR1

<400>  15

Gly Ser Ser Thr Gly Ala Val Thr Thr Ser Asn Tyr Ala Asn
1               5                   10

<210>  16
<211>  7
<212>  PRT
<213>  Artificial Sequence

<220>
<223>  CD3 LCDR2

<400>  16

Gly Thr Asn Lys Arg Ala Pro
1               5

<210>  17
<211>  9
<212>  PRT
<213>  Artificial Sequence

<220>
<223>  CD3 LCDR3

<400>  17

Ala Leu Trp Tyr Ser Asn Leu Trp Val
1               5

<210>  18
<211>  125
<212>  PRT
<213>  Artificial Sequence

<220>
<223>  CD3 VH

<400>  18

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Thr Tyr
            20                  25                  30

Ala Met Asn Trp Val Arg Gln Ala Pro Gly Lys Gly Leu Glu Trp Val
        35                  40                  45

Ser Arg Ile Arg Ser Lys Tyr Asn Asn Tyr Ala Thr Tyr Tyr Ala Asp
    50                  55                  60

Ser Val Lys Gly Arg Phe Thr Ile Ser Arg Asp Asp Ser Lys Asn Thr
65                  70                  75                  80

Leu Tyr Leu Gln Met Asn Ser Leu Arg Ala Glu Asp Thr Ala Val Tyr
                85                  90                  95

Tyr Cys Val Arg His Gly Asn Phe Gly Asn Ser Tyr Val Ser Trp Phe
            100                 105                 110

Ala Tyr Trp Gly Gln Gly Thr Leu Val Thr Val Ser Ser
        115                 120                 125

<210>  19
<211>  109
<212>  PRT
<213>  Artificial Sequence

<220>
<223>  CD3 VL

<400>  19

Gln Ala Val Val Thr Gln Glu Pro Ser Leu Thr Val Ser Pro Gly Gly
1               5                   10                  15

Thr Val Thr Leu Thr Cys Gly Ser Ser Thr Gly Ala Val Thr Thr Ser
            20                  25                  30

Asn Tyr Ala Asn Trp Val Gln Glu Lys Pro Gly Gln Ala Phe Arg Gly
        35                  40                  45

Leu Ile Gly Gly Thr Asn Lys Arg Ala Pro Gly Thr Pro Ala Arg Phe
    50                  55                  60

Ser Gly Ser Leu Leu Gly Gly Lys Ala Ala Leu Thr Leu Ser Gly Ala
65                  70                  75                  80

Gln Pro Glu Asp Glu Ala Glu Tyr Tyr Cys Ala Leu Trp Tyr Ser Asn
                85                  90                  95

Leu Trp Val Phe Gly Gly Gly Thr Lys Leu Thr Val Leu
            100                 105

<210>  20
<211>  672
<212>  PRT
<213>  Artificial Sequence

<220>
<223>  CD20 VH-CH1(EE)-CD3 VL-CH1-Fc (knob, P329G LALA)

<400>  20

Gln Val Gln Leu Val Gln Ser Gly Ala Glu Val Lys Lys Pro Gly Ser
1               5                   10                  15

Ser Val Lys Val Ser Cys Lys Ala Ser Gly Tyr Ala Phe Ser Tyr Ser
            20                  25                  30

Trp Ile Asn Trp Val Arg Gln Ala Pro Gly Gln Gly Leu Glu Trp Met
        35                  40                  45

Gly Arg Ile Phe Pro Gly Asp Gly Asp Thr Asp Tyr Asn Gly Lys Phe
    50                  55                  60

Lys Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Asn Val Phe Asp Gly Tyr Trp Leu Val Tyr Trp Gly Gln Gly
            100                 105                 110

Thr Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Glu Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Glu Lys Val Glu Pro Lys Ser Cys Asp Gly
    210                 215                 220

Gly Gly Gly Ser Gly Gly Gly Gly Ser Gln Ala Val Val Thr Gln Glu
225                 230                 235                 240

Pro Ser Leu Thr Val Ser Pro Gly Gly Thr Val Thr Leu Thr Cys Gly
                245                 250                 255

Ser Ser Thr Gly Ala Val Thr Thr Ser Asn Tyr Ala Asn Trp Val Gln
            260                 265                 270

Glu Lys Pro Gly Gln Ala Phe Arg Gly Leu Ile Gly Gly Thr Asn Lys
        275                 280                 285

Arg Ala Pro Gly Thr Pro Ala Arg Phe Ser Gly Ser Leu Leu Gly Gly
    290                 295                 300

Lys Ala Ala Leu Thr Leu Ser Gly Ala Gln Pro Glu Asp Glu Ala Glu
305                 310                 315                 320

Tyr Tyr Cys Ala Leu Trp Tyr Ser Asn Leu Trp Val Phe Gly Gly Gly
                325                 330                 335

Thr Lys Leu Thr Val Leu Ser Ser Ala Ser Thr Lys Gly Pro Ser Val
            340                 345                 350

Phe Pro Leu Ala Pro Ser Ser Lys Ser Thr Ser Gly Gly Thr Ala Ala
        355                 360                 365

Leu Gly Cys Leu Val Lys Asp Tyr Phe Pro Glu Pro Val Thr Val Ser
    370                 375                 380

Trp Asn Ser Gly Ala Leu Thr Ser Gly Val His Thr Phe Pro Ala Val
385                 390                 395                 400

Leu Gln Ser Ser Gly Leu Tyr Ser Leu Ser Ser Val Val Thr Val Pro
                405                 410                 415

Ser Ser Ser Leu Gly Thr Gln Thr Tyr Ile Cys Asn Val Asn His Lys
            420                 425                 430

Pro Ser Asn Thr Lys Val Asp Lys Lys Val Glu Pro Lys Ser Cys Asp
        435                 440                 445

Lys Thr His Thr Cys Pro Pro Cys Pro Ala Pro Glu Ala Ala Gly Gly
    450                 455                 460

Pro Ser Val Phe Leu Phe Pro Pro Lys Pro Lys Asp Thr Leu Met Ile
465                 470                 475                 480

Ser Arg Thr Pro Glu Val Thr Cys Val Val Val Asp Val Ser His Glu
                485                 490                 495

Asp Pro Glu Val Lys Phe Asn Trp Tyr Val Asp Gly Val Glu Val His
            500                 505                 510

Asn Ala Lys Thr Lys Pro Arg Glu Glu Gln Tyr Asn Ser Thr Tyr Arg
        515                 520                 525

Val Val Ser Val Leu Thr Val Leu His Gln Asp Trp Leu Asn Gly Lys
    530                 535                 540

Glu Tyr Lys Cys Lys Val Ser Asn Lys Ala Leu Gly Ala Pro Ile Glu
545                 550                 555                 560

Lys Thr Ile Ser Lys Ala Lys Gly Gln Pro Arg Glu Pro Gln Val Tyr
                565                 570                 575

Thr Leu Pro Pro Cys Arg Asp Glu Leu Thr Lys Asn Gln Val Ser Leu
            580                 585                 590

Trp Cys Leu Val Lys Gly Phe Tyr Pro Ser Asp Ile Ala Val Glu Trp
        595                 600                 605

Glu Ser Asn Gly Gln Pro Glu Asn Asn Tyr Lys Thr Thr Pro Pro Val
    610                 615                 620

Leu Asp Ser Asp Gly Ser Phe Phe Leu Tyr Ser Lys Leu Thr Val Asp
625                 630                 635                 640

Lys Ser Arg Trp Gln Gln Gly Asn Val Phe Ser Cys Ser Val Met His
                645                 650                 655

Glu Ala Leu His Asn His Tyr Thr Gln Lys Ser Leu Ser Leu Ser Pro
            660                 665                 670

<210>  21
<211>  447
<212>  PRT
<213>  Artificial Sequence

<220>
<223>  CD20 VH-CH1(EE)-Fc (hole, P329G LALA)

<400>  21

Gln Val Gln Leu Val Gln Ser Gly Ala Glu Val Lys Lys Pro Gly Ser
1               5                   10                  15

Ser Val Lys Val Ser Cys Lys Ala Ser Gly Tyr Ala Phe Ser Tyr Ser
            20                  25                  30

Trp Ile Asn Trp Val Arg Gln Ala Pro Gly Gln Gly Leu Glu Trp Met
        35                  40                  45

Gly Arg Ile Phe Pro Gly Asp Gly Asp Thr Asp Tyr Asn Gly Lys Phe
    50                  55                  60

Lys Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Asn Val Phe Asp Gly Tyr Trp Leu Val Tyr Trp Gly Gln Gly
            100                 105                 110

Thr Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Glu Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Glu Lys Val Glu Pro Lys Ser Cys Asp Lys
    210                 215                 220

Thr His Thr Cys Pro Pro Cys Pro Ala Pro Glu Ala Ala Gly Gly Pro
225                 230                 235                 240

Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser His Glu Asp
            260                 265                 270

Pro Glu Val Lys Phe Asn Trp Tyr Val Asp Gly Val Glu Val His Asn
        275                 280                 285

Ala Lys Thr Lys Pro Arg Glu Glu Gln Tyr Asn Ser Thr Tyr Arg Val
    290                 295                 300

Val Ser Val Leu Thr Val Leu His Gln Asp Trp Leu Asn Gly Lys Glu
305                 310                 315                 320

Tyr Lys Cys Lys Val Ser Asn Lys Ala Leu Gly Ala Pro Ile Glu Lys
                325                 330                 335

Thr Ile Ser Lys Ala Lys Gly Gln Pro Arg Glu Pro Gln Val Cys Thr
            340                 345                 350

Leu Pro Pro Ser Arg Asp Glu Leu Thr Lys Asn Gln Val Ser Leu Ser
        355                 360                 365

Cys Ala Val Lys Gly Phe Tyr Pro Ser Asp Ile Ala Val Glu Trp Glu
    370                 375                 380

Ser Asn Gly Gln Pro Glu Asn Asn Tyr Lys Thr Thr Pro Pro Val Leu
385                 390                 395                 400

Asp Ser Asp Gly Ser Phe Phe Leu Val Ser Lys Leu Thr Val Asp Lys
                405                 410                 415

Ser Arg Trp Gln Gln Gly Asn Val Phe Ser Cys Ser Val Met His Glu
            420                 425                 430

Ala Leu His Asn His Tyr Thr Gln Lys Ser Leu Ser Leu Ser Pro
        435                 440                 445

<210> 22
<211> 219
<212> PRT
<213> Artificial Sequence

<220>
<223> CD20 VL-CL(RK)

<400> 22

Asp Ile Val Met Thr Gln Thr Pro Leu Ser Leu Pro Val Thr Pro Gly
1               5                   10                  15

Glu Pro Ala Ser Ile Ser Cys Arg Ser Ser Lys Ser Leu Leu His Ser
            20                  25                  30

Asn Gly Ile Thr Tyr Leu Tyr Trp Tyr Leu Gln Lys Pro Gly Gln Ser
        35                  40                  45

Pro Gln Leu Leu Ile Tyr Gln Met Ser Asn Leu Val Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Ala Gln Asn
                85                  90                  95

Leu Glu Leu Pro Tyr Thr Phe Gly Gly Gly Thr Lys Val Glu Ile Lys
            100                 105                 110

Arg Thr Val Ala Ala Pro Ser Val Phe Ile Phe Pro Pro Ser Asp Arg
        115                 120                 125

Lys Leu Lys Ser Gly Thr Ala Ser Val Val Cys Leu Leu Asn Asn Phe
    130                 135                 140

Tyr Pro Arg Glu Ala Lys Val Gln Trp Lys Val Asp Asn Ala Leu Gln
145                 150                 155                 160

Ser Gly Asn Ser Gln Glu Ser Val Thr Glu Gln Asp Ser Lys Asp Ser
                165                 170                 175

Thr Tyr Ser Leu Ser Ser Thr Leu Thr Leu Ser Lys Ala Asp Tyr Glu
            180                 185                 190

Lys His Lys Val Tyr Ala Cys Glu Val Thr His Gln Gly Leu Ser Ser
        195                 200                 205

Pro Val Thr Lys Ser Phe Asn Arg Gly Glu Cys
    210                 215

<210>  23
<211>  232
<212>  PRT
<213>  Artificial Sequence

<220>
<223>  CD3 VH-CL

<400>  23

Glu Val Gln Leu Leu Glu Ser Gly Gly Gly Leu Val Gln Pro Gly Gly

1               5                   10                  15

Ser Leu Arg Leu Ser Cys Ala Ala Ser Gly Phe Thr Phe Ser Thr Tyr
            20                  25                  30

Ala Met Asn Trp Val Arg Gln Ala Pro Gly Lys Gly Leu Glu Trp Val
        35                  40                  45

Ser Arg Ile Arg Ser Lys Tyr Asn Asn Tyr Ala Thr Tyr Tyr Ala Asp
    50                  55                  60

Ser Val Lys Gly Arg Phe Thr Ile Ser Arg Asp Asp Ser Lys Asn Thr
65                  70                  75                  80

Leu Tyr Leu Gln Met Asn Ser Leu Arg Ala Glu Asp Thr Ala Val Tyr
                85                  90                  95

Tyr Cys Val Arg His Gly Asn Phe Gly Asn Ser Tyr Val Ser Trp Phe
            100                 105                 110

Ala Tyr Trp Gly Gln Gly Thr Leu Val Thr Val Ser Ser Ala Ser Val
        115                 120                 125

Ala Ala Pro Ser Val Phe Ile Phe Pro Pro Ser Asp Glu Gln Leu Lys
    130                 135                 140

Ser Gly Thr Ala Ser Val Val Cys Leu Leu Asn Asn Phe Tyr Pro Arg
145                 150                 155                 160

Glu Ala Lys Val Gln Trp Lys Val Asp Asn Ala Leu Gln Ser Gly Asn
                165                 170                 175

Ser Gln Glu Ser Val Thr Glu Gln Asp Ser Lys Asp Ser Thr Tyr Ser
            180                 185                 190

Leu Ser Ser Thr Leu Thr Leu Ser Lys Ala Asp Tyr Glu Lys His Lys
        195                 200                 205

Val Tyr Ala Cys Glu Val Thr His Gln Gly Leu Ser Ser Pro Val Thr
    210                 215                 220

Lys Ser Phe Asn Arg Gly Glu Cys
225                 230

<210>  24
<211>  5
<212>  PRT
<213>  Artificial Sequence

<220>
<223>  CD19 HCDR1

<400>  24

Asp Tyr Ile Met His
1               5


<210>  25
<211>  17
<212>  PRT
<213>  Artificial Sequence

<220>
<223>  CD19 HCDR2

<400>  25

Tyr Ile Asn Pro Tyr Asn Asp Gly Ser Lys Tyr Thr Glu Lys Phe Gln
1               5                   10                  15


Gly


<210>  26
<211>  12
<212>  PRT
<213>  Artificial Sequence

<220>
<223>  CD19 HCDR3

<400>  26

Gly Thr Tyr Tyr Tyr Gly Ser Ala Leu Phe Asp Tyr
1               5                   10


<210>  27
<211>  16
<212>  PRT
<213>  Artificial Sequence

<220>
<223>  CD19 LCDR1

<400>  27

Lys Ser Ser Gln Ser Leu Glu Asn Pro Asn Gly Asn Thr Tyr Leu Asn
1               5                   10                  15


<210>  28
<211>  7
<212>  PRT
<213>  Artificial Sequence

<220>
<223>  CD19 LCDR2

<400>  28

Arg Val Ser Lys Arg Phe Ser
1               5


<210>  29
<211>  9
<212>  PRT
<213>  Artificial Sequence

<220>
<223>  CD19 LCDR3

<400>  29

Leu Gln Leu Thr His Val Pro Tyr Thr
1               5


<210>  30
<211>  121
<212>  PRT
<213>  Artificial Sequence

<220>
<223>  CD19 VH

<400>  30

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asp Tyr
            20                  25                  30

Ile Met His Trp Val Arg Gln Ala Pro Gly Gln Gly Leu Glu Trp Met
        35                  40                  45

Gly Tyr Ile Asn Pro Tyr Asn Asp Gly Ser Lys Tyr Thr Glu Lys Phe
    50                  55                  60

Gln Gly Arg Val Thr Met Thr Ser Asp Thr Ser Ile Ser Thr Ala Tyr
65                  70                  75                  80

Met Glu Leu Ser Arg Leu Arg Ser Asp Asp Thr Ala Val Tyr Tyr Cys
                85                  90                  95

Ala Arg Gly Thr Tyr Tyr Tyr Gly Ser Ala Leu Phe Asp Tyr Trp Gly
            100                 105                 110

Gln Gly Thr Thr Val Thr Val Ser Ser
        115                 120

<210>  31
<211>  112
<212>  PRT
<213>  Artificial Sequence

<220>
<223>  CD19 VL

<400>  31

Asp Ile Val Met Thr Gln Thr Pro Leu Ser Leu Ser Val Thr Pro Gly
1               5                   10                  15

Gln Pro Ala Ser Ile Ser Cys Lys Ser Ser Gln Ser Leu Glu Asn Pro
            20                  25                  30

Asn Gly Asn Thr Tyr Leu Asn Trp Tyr Leu Gln Lys Pro Gly Gln Ser
        35                  40                  45

Pro Gln Leu Leu Ile Tyr Arg Val Ser Lys Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Leu Gln Leu
                85                  90                  95

Thr His Val Pro Tyr Thr Phe Gly Gln Gly Thr Lys Leu Glu Ile Lys
            100                 105                 110


<210>  32
<211>  449
<212>  PRT
<213>  Artificial Sequence

<220>
<223>  CD19 VH-CH1(EE)-Fc(hole, P329G LALA)

<400>  32

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asp Tyr
            20                  25                  30

Ile Met His Trp Val Arg Gln Ala Pro Gly Gln Gly Leu Glu Trp Met
        35                  40                  45

Gly Tyr Ile Asn Pro Tyr Asn Asp Gly Ser Lys Tyr Thr Glu Lys Phe
    50                  55                  60

Gln Gly Arg Val Thr Met Thr Ser Asp Thr Ser Ile Ser Thr Ala Tyr
65                  70                  75                  80

Met Glu Leu Ser Arg Leu Arg Ser Asp Asp Thr Ala Val Tyr Tyr Cys
                85                  90                  95

Ala Arg Gly Thr Tyr Tyr Tyr Gly Ser Ala Leu Phe Asp Tyr Trp Gly
            100                 105                 110

Gln Gly Thr Thr Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Glu Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205

Lys Pro Ser Asn Thr Lys Val Asp Glu Lys Val Glu Pro Lys Ser Cys
    210                 215                 220

Asp Lys Thr His Thr Cys Pro Pro Cys Pro Ala Pro Glu Ala Ala Gly
225                 230                 235                 240

Gly Pro Ser Val Phe Leu Phe Pro Pro Lys Pro Lys Asp Thr Leu Met
                245                 250                 255

Ile Ser Arg Thr Pro Glu Val Thr Cys Val Val Val Asp Val Ser His
            260                 265                 270

Glu Asp Pro Glu Val Lys Phe Asn Trp Tyr Val Asp Gly Val Glu Val
        275                 280                 285

His Asn Ala Lys Thr Lys Pro Arg Glu Glu Gln Tyr Asn Ser Thr Tyr
    290                 295                 300

Arg Val Val Ser Val Leu Thr Val Leu His Gln Asp Trp Leu Asn Gly
305                 310                 315                 320

Lys Glu Tyr Lys Cys Lys Val Ser Asn Lys Ala Leu Gly Ala Pro Ile
                325                 330                 335

Glu Lys Thr Ile Ser Lys Ala Lys Gly Gln Pro Arg Glu Pro Gln Val
            340                 345                 350

Cys Thr Leu Pro Pro Ser Arg Asp Glu Leu Thr Lys Asn Gln Val Ser
        355                 360                 365

Leu Ser Cys Ala Val Lys Gly Phe Tyr Pro Ser Asp Ile Ala Val Glu
    370                 375                 380

Trp Glu Ser Asn Gly Gln Pro Glu Asn Asn Tyr Lys Thr Thr Pro Pro
385                 390                 395                 400

Val Leu Asp Ser Asp Gly Ser Phe Phe Leu Val Ser Lys Leu Thr Val
                405                 410                 415

Asp Lys Ser Arg Trp Gln Gln Gly Asn Val Phe Ser Cys Ser Val Met
            420                 425                 430

His Glu Ala Leu His Asn His Tyr Thr Gln Lys Ser Leu Ser Leu Ser
        435                 440                 445

Pro

<210>  33
<211>  674
<212>  PRT
<213>  Artificial Sequence

<220>
<223>  CD19 VH-CH1(EE)-CD3 VL-CH1-Fc(knob, P329G LALA)

<400>  33

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asp Tyr
            20                  25                  30

Ile Met His Trp Val Arg Gln Ala Pro Gly Gln Gly Leu Glu Trp Met
        35                  40                  45

Gly Tyr Ile Asn Pro Tyr Asn Asp Gly Ser Lys Tyr Thr Glu Lys Phe
    50                  55                  60

Gln Gly Arg Val Thr Met Thr Ser Asp Thr Ser Ile Ser Thr Ala Tyr
65                  70                  75                  80

Met Glu Leu Ser Arg Leu Arg Ser Asp Asp Thr Ala Val Tyr Tyr Cys
                85                  90                  95

Ala Arg Gly Thr Tyr Tyr Tyr Gly Ser Ala Leu Phe Asp Tyr Trp Gly
            100                 105                 110

Gln Gly Thr Thr Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Glu Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205

Lys Pro Ser Asn Thr Lys Val Asp Glu Lys Val Glu Pro Lys Ser Cys
    210                 215                 220

Asp Gly Gly Gly Gly Ser Gly Gly Gly Gly Ser Gln Ala Val Val Thr
225                 230                 235                 240

Gln Glu Pro Ser Leu Thr Val Ser Pro Gly Gly Thr Val Thr Leu Thr
                245                 250                 255

Cys Gly Ser Ser Thr Gly Ala Val Thr Thr Ser Asn Tyr Ala Asn Trp
            260                 265                 270

Val Gln Glu Lys Pro Gly Gln Ala Phe Arg Gly Leu Ile Gly Gly Thr
        275                 280                 285

Asn Lys Arg Ala Pro Gly Thr Pro Ala Arg Phe Ser Gly Ser Leu Leu
    290                 295                 300

Gly Gly Lys Ala Ala Leu Thr Leu Ser Gly Ala Gln Pro Glu Asp Glu
305                 310                 315                 320

Ala Glu Tyr Tyr Cys Ala Leu Trp Tyr Ser Asn Leu Trp Val Phe Gly
                325                 330                 335

Gly Gly Thr Lys Leu Thr Val Leu Ser Ser Ala Ser Thr Lys Gly Pro
            340                 345                 350

Ser Val Phe Pro Leu Ala Pro Ser Ser Lys Ser Thr Ser Gly Gly Thr
        355                 360                 365

Ala Ala Leu Gly Cys Leu Val Lys Asp Tyr Phe Pro Glu Pro Val Thr
    370                 375                 380

Val Ser Trp Asn Ser Gly Ala Leu Thr Ser Gly Val His Thr Phe Pro
385                 390                 395                 400

Ala Val Leu Gln Ser Ser Gly Leu Tyr Ser Leu Ser Ser Val Val Thr
                405                 410                 415

Val Pro Ser Ser Ser Leu Gly Thr Gln Thr Tyr Ile Cys Asn Val Asn
            420                 425                 430

His Lys Pro Ser Asn Thr Lys Val Asp Lys Lys Val Glu Pro Lys Ser
        435                 440                 445

Cys Asp Lys Thr His Thr Cys Pro Pro Cys Pro Ala Pro Glu Ala Ala
    450                 455                 460

Gly Gly Pro Ser Val Phe Leu Phe Pro Pro Lys Pro Lys Asp Thr Leu
465                 470                 475                 480

Met Ile Ser Arg Thr Pro Glu Val Thr Cys Val Val Val Asp Val Ser
                485                 490                 495

His Glu Asp Pro Glu Val Lys Phe Asn Trp Tyr Val Asp Gly Val Glu
            500                 505                 510

Val His Asn Ala Lys Thr Lys Pro Arg Glu Glu Gln Tyr Asn Ser Thr
        515                 520                 525

Tyr Arg Val Val Ser Val Leu Thr Val Leu His Gln Asp Trp Leu Asn
    530                 535                 540

Gly Lys Glu Tyr Lys Cys Lys Val Ser Asn Lys Ala Leu Gly Ala Pro
545                 550                 555                 560

Ile Glu Lys Thr Ile Ser Lys Ala Lys Gly Gln Pro Arg Glu Pro Gln

565                 570                 575

Val Tyr Thr Leu Pro Pro Cys Arg Asp Glu Leu Thr Lys Asn Gln Val
            580                 585                 590

Ser Leu Trp Cys Leu Val Lys Gly Phe Tyr Pro Ser Asp Ile Ala Val
        595                 600                 605

Glu Trp Glu Ser Asn Gly Gln Pro Glu Asn Asn Tyr Lys Thr Thr Pro
    610                 615                 620

Pro Val Leu Asp Ser Asp Gly Ser Phe Phe Leu Tyr Ser Lys Leu Thr
625                 630                 635                 640

Val Asp Lys Ser Arg Trp Gln Gln Gly Asn Val Phe Ser Cys Ser Val
                645                 650                 655

Met His Glu Ala Leu His Asn His Tyr Thr Gln Lys Ser Leu Ser Leu
            660                 665                 670

Ser Pro

<210>  34
<211>  219
<212>  PRT
<213>  Artificial Sequence

<220>
<223>  CD19 VL-CL(RK)

<400>  34

Asp Ile Val Met Thr Gln Thr Pro Leu Ser Leu Ser Val Thr Pro Gly
1               5                   10                  15

Gln Pro Ala Ser Ile Ser Cys Lys Ser Ser Gln Ser Leu Glu Asn Pro
            20                  25                  30

Asn Gly Asn Thr Tyr Leu Asn Trp Tyr Leu Gln Lys Pro Gly Gln Ser
        35                  40                  45

Pro Gln Leu Leu Ile Tyr Arg Val Ser Lys Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Leu Gln Leu
                85                  90                  95

Thr His Val Pro Tyr Thr Phe Gly Gln Gly Thr Lys Leu Glu Ile Lys
            100                 105                 110

Arg Thr Val Ala Ala Pro Ser Val Phe Ile Phe Pro Pro Ser Asp Arg
        115                 120                 125

Lys Leu Lys Ser Gly Thr Ala Ser Val Val Cys Leu Leu Asn Asn Phe
    130                 135                 140

Tyr Pro Arg Glu Ala Lys Val Gln Trp Lys Val Asp Asn Ala Leu Gln
145                 150                 155                 160

Ser Gly Asn Ser Gln Glu Ser Val Thr Glu Gln Asp Ser Lys Asp Ser
                165                 170                 175

Thr Tyr Ser Leu Ser Ser Thr Leu Thr Leu Ser Lys Ala Asp Tyr Glu
            180                 185                 190

Lys His Lys Val Tyr Ala Cys Glu Val Thr His Gln Gly Leu Ser Ser
        195                 200                 205

Pro Val Thr Lys Ser Phe Asn Arg Gly Glu Cys
    210                 215

<210> 35
<211> 5
<212> PRT
<213> Artificial Sequence

<220>
<223> CD19 HCDR1

<400> 35

Asp Tyr Ile Met His
1               5

<210> 36
<211> 17
<212> PRT
<213> Artificial Sequence

<220>
<223> CD19 HCDR2

<400> 36

Tyr Ile Asn Pro Tyr Asn Asp Gly Ser Lys Tyr Thr Glu Lys Phe Gln
1               5                   10                  15

Gly

<210>  37
<211>  12
<212>  PRT
<213>  Artificial Sequence

<220>
<223>  CD19 HCDR3

<400>  37

Gly Thr Tyr Tyr Tyr Gly Pro Gln Leu Phe Asp Tyr
1               5                   10


<210>  38
<211>  16
<212>  PRT
<213>  Artificial Sequence

<220>
<223>  CD19 LCDR1

<400>  38

Lys Ser Ser Gln Ser Leu Glu Thr Ser Thr Gly Thr Thr Tyr Leu Asn
1               5                   10                  15


<210>  39
<211>  7
<212>  PRT
<213>  Artificial Sequence

<220>
<223>  CD19 LCDR2

<400>  39

Arg Val Ser Lys Arg Phe Ser
1               5


<210>  40
<211>  9
<212>  PRT
<213>  Artificial Sequence

<220>
<223>  CD19 LCDR3

<400>  40

Leu Gln Leu Leu Glu Asp Pro Tyr Thr
1               5


<210>  41
<211>  121
<212>  PRT
<213>  Artificial Sequence

<220>
<223>  CD19 VH

<400>  41

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asp Tyr
            20                  25                  30

Ile Met His Trp Val Arg Gln Ala Pro Gly Gln Gly Leu Glu Trp Met
        35                  40                  45

Gly Tyr Ile Asn Pro Tyr Asn Asp Gly Ser Lys Tyr Thr Glu Lys Phe
    50                  55                  60

Gln Gly Arg Val Thr Met Thr Ser Asp Thr Ser Ile Ser Thr Ala Tyr
65                  70                  75                  80

Met Glu Leu Ser Arg Leu Arg Ser Asp Asp Thr Ala Val Tyr Tyr Cys
                85                  90                  95

Ala Arg Gly Thr Tyr Tyr Tyr Gly Pro Gln Leu Phe Asp Tyr Trp Gly
            100                 105                 110

Gln Gly Thr Thr Val Thr Val Ser Ser
        115                 120


<210>  42
<211>  112
<212>  PRT
<213>  Artificial Sequence

<220>
<223>  CD19 VL

<400>  42

Asp Ile Val Met Thr Gln Thr Pro Leu Ser Leu Ser Val Thr Pro Gly
1               5                   10                  15

Gln Pro Ala Ser Ile Ser Cys Lys Ser Ser Gln Ser Leu Glu Thr Ser
            20                  25                  30

Thr Gly Thr Thr Tyr Leu Asn Trp Tyr Leu Gln Lys Pro Gly Gln Ser
        35                  40                  45

Pro Gln Leu Leu Ile Tyr Arg Val Ser Lys Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Leu Gln Leu
                85                  90                  95

Leu Glu Asp Pro Tyr Thr Phe Gly Gln Gly Thr Lys Leu Glu Ile Lys
            100                 105                 110

<210>  43
<211>  5
<212>  PRT
<213>  Artificial Sequence

<220>
<223>  CD19 HCDR1

<400>  43

Asp Tyr Ile Met His
1               5

<210>  44
<211>  17
<212>  PRT
<213>  Artificial Sequence

<220>
<223>  CD19 HCDR2

<400>  44

Tyr Ile Asn Pro Tyr Asn Asp Gly Ser Lys Tyr Thr Glu Lys Phe Gln
1               5                   10                  15

Gly

<210>  45
<211>  12
<212>  PRT
<213>  Artificial Sequence

<220>
<223>  CD19 HCDR3

<400>  45

Gly Thr Tyr Tyr Tyr Gly Ser Ala Leu Phe Asp Tyr
1               5                   10

<210>  46
<211>  16
<212>  PRT
<213>  Artificial Sequence

<220>
<223>  CD19 LCDR1

<400>  46

Lys Ser Ser Gln Ser Leu Glu Ser Ser Thr Gly Asn Thr Tyr Leu Asn
1               5                   10                  15


<210>  47
<211>  7
<212>  PRT
<213>  Artificial Sequence

<220>
<223>  CD19 LCDR2

<400>  47

Arg Val Ser Lys Arg Phe Ser
1               5


<210>  48
<211>  9
<212>  PRT
<213>  Artificial Sequence

<220>
<223>  CD19 LCDR3

<400>  48

Leu Gln Leu Ile Asp Tyr Pro Val Thr
1               5


<210>  49
<211>  121
<212>  PRT
<213>  Artificial Sequence

<220>
<223>  CD19 VH

<400>  49

Gln Val Gln Leu Val Gln Ser Gly Ala Glu Val Lys Lys Pro Gly Ala
1               5                   10                  15


Ser Val Lys Val Ser Cys Lys Ala Ser Gly Tyr Thr Phe Thr Asp Tyr
            20                  25                  30


Ile Met His Trp Val Arg Gln Ala Pro Gly Gln Gly Leu Glu Trp Met
        35                  40                  45


Gly Tyr Ile Asn Pro Tyr Asn Asp Gly Ser Lys Tyr Thr Glu Lys Phe
    50                  55                  60

Gln Gly Arg Val Thr Met Thr Ser Asp Thr Ser Ile Ser Thr Ala Tyr
65                  70                  75                  80

Met Glu Leu Ser Arg Leu Arg Ser Asp Asp Thr Ala Val Tyr Tyr Cys
                85                  90                  95

Ala Arg Gly Thr Tyr Tyr Tyr Gly Ser Ala Leu Phe Asp Tyr Trp Gly
            100                 105                 110

Gln Gly Thr Thr Val Thr Val Ser Ser
        115                 120

<210>  50
<211>  112
<212>  PRT
<213>  Artificial Sequence

<220>
<223>  CD19 VL

<400>  50

Asp Ile Val Met Thr Gln Thr Pro Leu Ser Leu Ser Val Thr Pro Gly
1               5                   10                  15

Gln Pro Ala Ser Ile Ser Cys Lys Ser Ser Gln Ser Leu Glu Ser Ser
            20                  25                  30

Thr Gly Asn Thr Tyr Leu Asn Trp Tyr Leu Gln Lys Pro Gly Gln Ser
        35                  40                  45

Pro Gln Leu Leu Ile Tyr Arg Val Ser Lys Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Leu Gln Leu
                85                  90                  95

Ile Asp Tyr Pro Val Thr Phe Gly Gln Gly Thr Lys Leu Glu Ile Lys
            100                 105                 110

<210>  51
<211>  5
<212>  PRT
<213>  Artificial Sequence

<220>
<223>  CD19 HCDR1

<400>  51

Asp Tyr Ile Met His
1               5



<210>  52
<211>  17
<212>  PRT
<213>  Artificial Sequence

<220>
<223>  CD19 HCDR2

<400>  52

Tyr Ile Asn Pro Tyr Asn Asp Gly Ser Lys Tyr Thr Glu Lys Phe Gln
1               5                   10                  15


Gly




<210>  53
<211>  12
<212>  PRT
<213>  Artificial Sequence

<220>
<223>  CD19 HCDR3

<400>  53

Gly Thr Tyr Tyr Tyr Gly Ser Glu Leu Phe Asp Tyr
1               5                   10



<210>  54
<211>  16
<212>  PRT
<213>  Artificial Sequence

<220>
<223>  CD19 LCDR1

<400>  54

Lys Ser Ser Gln Ser Leu Glu Thr Ser Thr Gly Asn Thr Tyr Leu Asn
1               5                   10                  15



<210>  55
<211>  7
<212>  PRT
<213>  Artificial Sequence

<220>
<223>  CD19 LCDR2

<400>  55

Arg Val Ser Lys Arg Phe Ser

1               5


<210>  56
<211>  9
<212>  PRT
<213>  Artificial Sequence

<220>
<223>  CD19 LCDR3

<400>  56

Leu Gln Ala Thr His Ile Pro Tyr Thr
1               5


<210>  57
<211>  121
<212>  PRT
<213>  Artificial Sequence

<220>
<223>  CD19 VH

<400>  57

Gln Val Gln Leu Val Gln Ser Gly Ala Glu Val Lys Lys Pro Gly Ala
1               5                   10                  15


Ser Val Lys Val Ser Cys Lys Ala Ser Gly Tyr Thr Phe Thr Asp Tyr
            20                  25                  30


Ile Met His Trp Val Arg Gln Ala Pro Gly Gln Gly Leu Glu Trp Met
        35                  40                  45


Gly Tyr Ile Asn Pro Tyr Asn Asp Gly Ser Lys Tyr Thr Glu Lys Phe
    50                  55                  60


Gln Gly Arg Val Thr Met Thr Ser Asp Thr Ser Ile Ser Thr Ala Tyr
65                  70                  75                  80


Met Glu Leu Ser Arg Leu Arg Ser Asp Asp Thr Ala Val Tyr Tyr Cys
                85                  90                  95


Ala Arg Gly Thr Tyr Tyr Tyr Gly Ser Glu Leu Phe Asp Tyr Trp Gly
            100                 105                 110


Gln Gly Thr Thr Val Thr Val Ser Ser
        115                 120


<210>  58
<211>  112
<212>  PRT
<213>  Artificial Sequence

<220>
<223>  CD19 VL

<400>  58

Asp Ile Val Met Thr Gln Thr Pro Leu Ser Leu Ser Val Thr Pro Gly
1               5                   10                  15

Gln Pro Ala Ser Ile Ser Cys Lys Ser Ser Gln Ser Leu Glu Thr Ser
            20                  25                  30

Thr Gly Asn Thr Tyr Leu Asn Trp Tyr Leu Gln Lys Pro Gly Gln Ser
        35                  40                  45

Pro Gln Leu Leu Ile Tyr Arg Val Ser Lys Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Leu Gln Ala
                85                  90                  95

Thr His Ile Pro Tyr Thr Phe Gly Gln Gly Thr Lys Leu Glu Ile Lys
            100                 105                 110


<210>  59
<211>  5
<212>  PRT
<213>  Artificial Sequence

<220>
<223>  CD19 HCDR1

<400>  59

Asp Tyr Ile Thr His
1               5


<210>  60
<211>  17
<212>  PRT
<213>  Artificial Sequence

<220>
<223>  CD19 HCDR2

<400>  60

Tyr Ile Asn Pro Tyr Asn Asp Gly Ser Lys Tyr Thr Glu Lys Phe Gln
1               5                   10                  15

Gly

<210>  61
<211>  12
<212>  PRT
<213>  Artificial Sequence

<220>
<223>  CD19 HCDR3

<400>  61

Gly Thr Tyr Tyr Tyr Gly Pro Asp Leu Phe Asp Tyr
1               5                   10


<210>  62
<211>  16
<212>  PRT
<213>  Artificial Sequence

<220>
<223>  CD19 LCDR1

<400>  62

Lys Ser Ser Gln Ser Leu Glu Thr Ser Thr Gly Asn Thr Tyr Leu Asn
1               5                   10                  15


<210>  63
<211>  7
<212>  PRT
<213>  Artificial Sequence

<220>
<223>  CD19 LCDR2

<400>  63

Arg Val Ser Lys Arg Phe Ser
1               5


<210>  64
<211>  9
<212>  PRT
<213>  Artificial Sequence

<220>
<223>  CD19 LCDR3

<400>  64

Leu Gln Leu Thr His Val Pro Tyr Thr
1               5


<210>  65
<211>  121
<212>  PRT
<213>  Artificial Sequence

<220>
<223>  CD19 VH

<400>  65

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asp Tyr
            20                  25                  30

Ile Thr His Trp Val Arg Gln Ala Pro Gly Gln Gly Leu Glu Trp Met
        35                  40                  45

Gly Tyr Ile Asn Pro Tyr Asn Asp Gly Ser Lys Tyr Thr Glu Lys Phe
    50                  55                  60

Gln Gly Arg Val Thr Met Thr Ser Asp Thr Ser Ile Ser Thr Ala Tyr
65                  70                  75                  80

Met Glu Leu Ser Arg Leu Arg Ser Asp Asp Thr Ala Val Tyr Tyr Cys
                85                  90                  95

Ala Arg Gly Thr Tyr Tyr Tyr Gly Pro Asp Leu Phe Asp Tyr Trp Gly
            100                 105                 110

Gln Gly Thr Thr Val Thr Val Ser Ser
        115                 120


<210>  66
<211>  112
<212>  PRT
<213>  Artificial Sequence

<220>
<223>  CD19 VL


<220>
<221>  misc_feature
<222>  (107)..(107)
<223>  Xaa can be any naturally occurring amino acid

<400>  66

Asp Ile Val Met Thr Gln Thr Pro Leu Ser Leu Ser Val Thr Pro Gly
1               5                   10                  15

Gln Pro Ala Ser Ile Ser Cys Lys Ser Ser Gln Ser Leu Glu Thr Ser
            20                  25                  30

Thr Gly Asn Thr Tyr Leu Asn Trp Tyr Leu Gln Lys Pro Gly Gln Ser

35                  40                  45

Pro Gln Leu Leu Ile Tyr Arg Val Ser Lys Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Leu Gln Leu
                85                  90                  95

Thr His Val Pro Tyr Thr Phe Gly Gln Gly Xaa Lys Leu Glu Ile Lys
            100                 105                 110

<210>  67
<211>  5
<212>  PRT
<213>  Artificial Sequence

<220>
<223>  CD19 HCDR1

<400>  67

Asp Tyr Ile Met His
1               5

<210>  68
<211>  17
<212>  PRT
<213>  Artificial Sequence

<220>
<223>  CD19 HCDR2

<400>  68

Tyr Ile Asn Pro Tyr Asn Asp Gly Ser Lys Tyr Thr Glu Lys Phe Gln
1               5                   10                  15

Gly

<210>  69
<211>  12
<212>  PRT
<213>  Artificial Sequence

<220>
<223>  CD19 HCDR3

<400>  69

Gly Thr Tyr Tyr Tyr Gly Ser Ala Leu Phe Asp Tyr
1               5                   10

<210>  70
<211>  16
<212>  PRT
<213>  Artificial Sequence

<220>
<223>  CD19 LCDR1

<400>  70

Lys Ser Ser Gln Ser Leu Glu Thr Ser Thr Gly Asn Thr Tyr Leu Asn
1               5                   10                  15


<210>  71
<211>  7
<212>  PRT
<213>  Artificial Sequence

<220>
<223>  CD19 LCDR2

<400>  71

Arg Val Ser Lys Arg Phe Ser
1               5


<210>  72
<211>  9
<212>  PRT
<213>  Artificial Sequence

<220>
<223>  CD19 LCDR3

<400>  72

Leu Gln Pro Gly His Tyr Pro Gly Thr
1               5


<210>  73
<211>  121
<212>  PRT
<213>  Artificial Sequence

<220>
<223>  CD19 VH

<400>  73

Gln Val Gln Leu Val Gln Ser Gly Ala Glu Val Lys Lys Pro Gly Ala
1               5                   10                  15


Ser Val Lys Val Ser Cys Lys Ala Ser Gly Tyr Thr Phe Thr Asp Tyr
            20                  25                  30


Ile Met His Trp Val Arg Gln Ala Pro Gly Gln Gly Leu Glu Trp Met

35                  40                  45

Gly Tyr Ile Asn Pro Tyr Asn Asp Gly Ser Lys Tyr Thr Glu Lys Phe
    50                  55                  60

Gln Gly Arg Val Thr Met Thr Ser Asp Thr Ser Ile Ser Thr Ala Tyr
65                  70                  75                  80

Met Glu Leu Ser Arg Leu Arg Ser Asp Asp Thr Ala Val Tyr Tyr Cys
                85                  90                  95

Ala Arg Gly Thr Tyr Tyr Tyr Gly Ser Ala Leu Phe Asp Tyr Trp Gly
            100                 105                 110

Gln Gly Thr Thr Val Thr Val Ser Ser
        115                 120

<210>  74
<211>  112
<212>  PRT
<213>  Artificial Sequence

<220>
<223>  CD19 VL

<400>  74

Asp Ile Val Met Thr Gln Thr Pro Leu Ser Leu Ser Val Thr Pro Gly
1               5                   10                  15

Gln Pro Ala Ser Ile Ser Cys Lys Ser Ser Gln Ser Leu Glu Thr Ser
            20                  25                  30

Thr Gly Asn Thr Tyr Leu Asn Trp Tyr Leu Gln Lys Pro Gly Gln Ser
        35                  40                  45

Pro Gln Leu Leu Ile Tyr Arg Val Ser Lys Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Leu Gln Pro
                85                  90                  95

Gly His Tyr Pro Gly Thr Phe Gly Gln Gly Thr Lys Leu Glu Ile Lys
            100                 105                 110

<210>  75
<211>  5

<212>  PRT
<213>  Artificial Sequence

<220>
<223>  CD19 HCDR1

<400>  75

Asp Tyr Ile Met His
1               5


<210>  76
<211>  17
<212>  PRT
<213>  Artificial Sequence

<220>
<223>  CD19 HCDR2

<400>  76

Tyr Ile Asn Pro Tyr Asn Asp Gly Ser Lys Tyr Thr Glu Lys Phe Gln
1               5                   10                  15


Gly




<210>  77
<211>  12
<212>  PRT
<213>  Artificial Sequence

<220>
<223>  CD19 HCDR3

<400>  77

Gly Thr Tyr Tyr Tyr Gly Pro Gln Leu Phe Asp Tyr
1               5                   10


<210>  78
<211>  16
<212>  PRT
<213>  Artificial Sequence

<220>
<223>  CD19 LCDR1

<400>  78

Lys Ser Ser Gln Ser Leu Glu Thr Ser Thr Gly Asn Thr Tyr Leu Asn
1               5                   10                  15


<210>  79
<211>  7
<212>  PRT
<213>  Artificial Sequence

<220>
<223>  CD19 LCDR2

<400>  79

Arg Val Ser Lys Arg Phe Ser
1               5


<210>  80
<211>  9
<212>  PRT
<213>  Artificial Sequence

<220>
<223>  CD19 LCDR3

<400>  80

Leu Gln Leu Asp Ser Tyr Pro Asn Thr
1               5


<210>  81
<211>  121
<212>  PRT
<213>  Artificial Sequence

<220>
<223>  CD19 VH

<400>  81

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asp Tyr
            20                  25                  30

Ile Met His Trp Val Arg Gln Ala Pro Gly Gln Gly Leu Glu Trp Met
        35                  40                  45

Gly Tyr Ile Asn Pro Tyr Asn Asp Gly Ser Lys Tyr Thr Glu Lys Phe
    50                  55                  60

Gln Gly Arg Val Thr Met Thr Ser Asp Thr Ser Ile Ser Thr Ala Tyr
65                  70                  75                  80

Met Glu Leu Ser Arg Leu Arg Ser Asp Asp Thr Ala Val Tyr Tyr Cys
                85                  90                  95

Ala Arg Gly Thr Tyr Tyr Tyr Gly Pro Gln Leu Phe Asp Tyr Trp Gly
            100                 105                 110

Gln Gly Thr Thr Val Thr Val Ser Ser
        115                 120

<210>  82
<211>  112
<212>  PRT
<213>  Artificial Sequence

<220>
<223>  CD19 VL

<400>  82

Asp Ile Val Met Thr Gln Thr Pro Leu Ser Leu Ser Val Thr Pro Gly
1               5                   10                  15

Gln Pro Ala Ser Ile Ser Cys Lys Ser Ser Gln Ser Leu Glu Thr Ser
            20                  25                  30

Thr Gly Asn Thr Tyr Leu Asn Trp Tyr Leu Gln Lys Pro Gly Gln Ser
        35                  40                  45

Pro Gln Leu Leu Ile Tyr Arg Val Ser Lys Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Leu Gln Leu
                85                  90                  95

Asp Ser Tyr Pro Asn Thr Phe Gly Gln Gly Thr Lys Leu Glu Ile Lys
            100                 105                 110


<210>  83
<211>  5
<212>  PRT
<213>  Artificial Sequence

<220>
<223>  CD19 HCDR1

<400>  83

Asp Tyr Ile Met His
1               5


<210>  84
<211>  17
<212>  PRT
<213>  Artificial Sequence

<220>
<223>  CD19 HCDR2

<400>  84

Tyr Ile Asn Pro Tyr Asn Asp Gly Ser Lys Tyr Thr Glu Lys Phe Gln
1               5                   10                  15

Gly

<210>  85
<211>  12
<212>  PRT
<213>  Artificial Sequence

<220>
<223>  CD19 HCDR3

<400>  85

Gly Thr Tyr Tyr Tyr Gly Ser Glu Leu Phe Asp Tyr
1               5                   10

<210>  86
<211>  16
<212>  PRT
<213>  Artificial Sequence

<220>
<223>  CD19 LCDR1

<400>  86

Lys Ser Ser Gln Ser Leu Glu Thr Ser Thr Gly Asn Thr Tyr Leu Asn
1               5                   10                  15

<210>  87
<211>  7
<212>  PRT
<213>  Artificial Sequence

<220>
<223>  CD19 LCDR2

<400>  87

Arg Val Ser Lys Arg Phe Ser
1               5

<210>  88
<211>  9
<212>  PRT
<213>  Artificial Sequence

<220>
<223>  CD19 LCDR3

<400>  88

Leu Gln Leu Thr His Glu Pro Tyr Thr
1               5

<210>  89
<211>  121
<212>  PRT
<213>  Artificial Sequence

<220>
<223>  CD19 VH

<400>  89

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asp Tyr
            20                  25                  30

Ile Met His Trp Val Arg Gln Ala Pro Gly Gln Gly Leu Glu Trp Met
        35                  40                  45

Gly Tyr Ile Asn Pro Tyr Asn Asp Gly Ser Lys Tyr Thr Glu Lys Phe
    50                  55                  60

Gln Gly Arg Val Thr Met Thr Ser Asp Thr Ser Ile Ser Thr Ala Tyr
65                  70                  75                  80

Met Glu Leu Ser Arg Leu Arg Ser Asp Asp Thr Ala Val Tyr Tyr Cys
                85                  90                  95

Ala Arg Gly Thr Tyr Tyr Tyr Gly Ser Glu Leu Phe Asp Tyr Trp Gly
            100                 105                 110

Gln Gly Thr Thr Val Thr Val Ser Ser
        115                 120


<210>  90
<211>  112
<212>  PRT
<213>  Artificial Sequence

<220>
<223>  CD19 VL

<400>  90

Asp Ile Val Met Thr Gln Thr Pro Leu Ser Leu Ser Val Thr Pro Gly
1               5                   10                  15

Gln Pro Ala Ser Ile Ser Cys Lys Ser Ser Gln Ser Leu Glu Thr Ser
            20                  25                  30

Thr Gly Asn Thr Tyr Leu Asn Trp Tyr Leu Gln Lys Pro Gly Gln Ser

        35                  40                  45

Pro Gln Leu Leu Ile Tyr Arg Val Ser Lys Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Leu Gln Leu
                85                  90                  95

Thr His Glu Pro Tyr Thr Phe Gly Gln Gly Thr Lys Leu Glu Ile Lys
            100                 105                 110

<210> 91
<211> 207
<212> PRT
<213> Homo sapiens

<400> 91

Met Gln Ser Gly Thr His Trp Arg Val Leu Gly Leu Cys Leu Leu Ser
1               5                   10                  15

Val Gly Val Trp Gly Gln Asp Gly Asn Glu Glu Met Gly Gly Ile Thr
            20                  25                  30

Gln Thr Pro Tyr Lys Val Ser Ile Ser Gly Thr Thr Val Ile Leu Thr
        35                  40                  45

Cys Pro Gln Tyr Pro Gly Ser Glu Ile Leu Trp Gln His Asn Asp Lys
    50                  55                  60

Asn Ile Gly Gly Asp Glu Asp Asp Lys Asn Ile Gly Ser Asp Glu Asp
65                  70                  75                  80

His Leu Ser Leu Lys Glu Phe Ser Glu Leu Glu Gln Ser Gly Tyr Tyr
                85                  90                  95

Val Cys Tyr Pro Arg Gly Ser Lys Pro Glu Asp Ala Asn Phe Tyr Leu
            100                 105                 110

Tyr Leu Arg Ala Arg Val Cys Glu Asn Cys Met Glu Met Asp Val Met
        115                 120                 125

Ser Val Ala Thr Ile Val Ile Val Asp Ile Cys Ile Thr Gly Gly Leu
    130                 135                 140

Leu Leu Leu Val Tyr Tyr Trp Ser Lys Asn Arg Lys Ala Lys Ala Lys

145                 150                 155                 160

Pro Val Thr Arg Gly Ala Gly Ala Gly Gly Arg Gln Arg Gly Gln Asn
                165                 170                 175

Lys Glu Arg Pro Pro Pro Val Pro Asn Pro Asp Tyr Glu Pro Ile Arg
            180                 185                 190

Lys Gly Gln Arg Asp Leu Tyr Ser Gly Leu Asn Gln Arg Arg Ile
        195                 200                 205

<210>  92
<211>  198
<212>  PRT
<213>  Macaca fascicularis

<400>  92

Met Gln Ser Gly Thr Arg Trp Arg Val Leu Gly Leu Cys Leu Leu Ser
1               5                   10                  15

Ile Gly Val Trp Gly Gln Asp Gly Asn Glu Glu Met Gly Ser Ile Thr
            20                  25                  30

Gln Thr Pro Tyr Gln Val Ser Ile Ser Gly Thr Thr Val Ile Leu Thr
        35                  40                  45

Cys Ser Gln His Leu Gly Ser Glu Ala Gln Trp Gln His Asn Gly Lys
    50                  55                  60

Asn Lys Glu Asp Ser Gly Asp Arg Leu Phe Leu Pro Glu Phe Ser Glu
65                  70                  75                  80

Met Glu Gln Ser Gly Tyr Tyr Val Cys Tyr Pro Arg Gly Ser Asn Pro
                85                  90                  95

Glu Asp Ala Ser His His Leu Tyr Leu Lys Ala Arg Val Cys Glu Asn
            100                 105                 110

Cys Met Glu Met Asp Val Met Ala Val Ala Thr Ile Val Ile Val Asp
        115                 120                 125

Ile Cys Ile Thr Leu Gly Leu Leu Leu Leu Val Tyr Tyr Trp Ser Lys
    130                 135                 140

Asn Arg Lys Ala Lys Ala Lys Pro Val Thr Arg Gly Ala Gly Ala Gly
145                 150                 155                 160

Gly Arg Gln Arg Gly Gln Asn Lys Glu Arg Pro Pro Pro Val Pro Asn

                165                 170                 175

Pro Asp Tyr Glu Pro Ile Arg Lys Gly Gln Gln Asp Leu Tyr Ser Gly
            180                 185                 190

Leu Asn Gln Arg Arg Ile
        195

<210> 93
<211> 556
<212> PRT
<213> Homo sapiens

<400> 93

<210>  94
<211>  225
<212>  PRT
<213>  Homo sapiens

<400>  94

Asp Lys Thr His Thr Cys Pro Pro Cys Pro Ala Pro Glu Leu Leu Gly
1               5                   10                  15

Gly Pro Ser Val Phe Leu Phe Pro Pro Lys Pro Lys Asp Thr Leu Met
            20                  25                  30

Ile Ser Arg Thr Pro Glu Val Thr Cys Val Val Val Asp Val Ser His
        35                  40                  45

Glu Asp Pro Glu Val Lys Phe Asn Trp Tyr Val Asp Gly Val Glu Val
    50                  55                  60

His Asn Ala Lys Thr Lys Pro Arg Glu Glu Gln Tyr Asn Ser Thr Tyr
65                  70                  75                  80

Arg Val Val Ser Val Leu Thr Val Leu His Gln Asp Trp Leu Asn Gly
                85                  90                  95

Lys Glu Tyr Lys Cys Lys Val Ser Asn Lys Ala Leu Pro Ala Pro Ile
            100                 105                 110

Glu Lys Thr Ile Ser Lys Ala Lys Gly Gln Pro Arg Glu Pro Gln Val
        115                 120                 125

Tyr Thr Leu Pro Pro Ser Arg Asp Glu Leu Thr Lys Asn Gln Val Ser
    130                 135                 140

Leu Thr Cys Leu Val Lys Gly Phe Tyr Pro Ser Asp Ile Ala Val Glu
145                 150                 155                 160

Trp Glu Ser Asn Gly Gln Pro Glu Asn Asn Tyr Lys Thr Thr Pro Pro
                165                 170                 175

Val Leu Asp Ser Asp Gly Ser Phe Phe Leu Tyr Ser Lys Leu Thr Val
            180                 185                 190

Asp Lys Ser Arg Trp Gln Gln Gly Asn Val Phe Ser Cys Ser Val Met
        195                 200                 205

His Glu Ala Leu His Asn His Tyr Thr Gln Lys Ser Leu Ser Leu Ser
    210                 215                 220

Pro
225

<210>  95
<211>  10
<212>  PRT
<213>  Artificial Sequence

<220>
<223>  linker

<400>  95

Gly Gly Gly Gly Ser Gly Gly Gly Gly Ser
1               5                   10

<210>  96
<211>  11
<212>  PRT
<213>  Artificial Sequence

<220>
<223>  linker

<400>  96

Asp Gly Gly Gly Gly Ser Gly Gly Gly Gly Ser
1               5                   10

<210>  97
<211>  5
<212>  PRT
<213>  Artificial Sequence

<220>
<223>  Synthetic Construct

<400>  97

Thr Tyr Ala Met Asn
1               5


<210>  98
<211>  19
<212>  PRT
<213>  Artificial Sequence

<220>
<223>  Synthetic Construct

<400>  98

Arg Ile Arg Ser Lys Tyr Asn Asn Tyr Ala Thr Tyr Tyr Ala Asp Ser
1               5                   10                  15


Val Lys Gly



<210>  99
<211>  14
<212>  PRT
<213>  Artificial Sequence

<220>
<223>  Synthetic Construct

<400>  99

His Gly Asn Phe Gly Asn Ser Tyr Val Ser Trp Phe Ala Tyr
1               5                   10


<210>  100
<211>  14
<212>  PRT
<213>  Artificial Sequence

<220>
<223>  Synthetic Construct

<400>  100

Gly Ser Ser Thr Gly Ala Val Thr Thr Ser Asn Tyr Ala Asn
1               5                   10


<210>  101
<211>  7

<212>  PRT
<213>  Artificial Sequence

<220>
<223>  Synthetic Construct

<400>  101

Gly Thr Asn Lys Arg Ala Pro
1               5


<210>  102
<211>  9
<212>  PRT
<213>  Artificial Sequence

<220>
<223>  Synthetic Construct

<400>  102

Ala Leu Trp Tyr Ser Asn Leu Trp Val
1               5


<210>  103
<211>  125
<212>  PRT
<213>  Artificial Sequence

<220>
<223>  Synthetic Construct

<400>  103

Glu Val Gln Leu Leu Glu Ser Gly Gly Gly Leu Val Gln Pro Gly Gly
1               5                   10                  15


Ser Leu Arg Leu Ser Cys Ala Ala Ser Gly Phe Thr Phe Ser Thr Tyr
            20                  25                  30


Ala Met Asn Trp Val Arg Gln Ala Pro Gly Lys Gly Leu Glu Trp Val
        35                  40                  45


Ser Arg Ile Arg Ser Lys Tyr Asn Asn Tyr Ala Thr Tyr Tyr Ala Asp
    50                  55                  60


Ser Val Lys Gly Arg Phe Thr Ile Ser Arg Asp Asp Ser Lys Asn Thr
65                  70                  75                  80


Leu Tyr Leu Gln Met Asn Ser Leu Arg Ala Glu Asp Thr Ala Val Tyr
                85                  90                  95


Tyr Cys Val Arg His Gly Asn Phe Gly Asn Ser Tyr Val Ser Trp Phe
            100                 105                 110

Ala Tyr Trp Gly Gln Gly Thr Leu Val Thr Val Ser Ser
        115                 120                 125

<210>  104
<211>  109
<212>  PRT
<213>  Artificial Sequence

<220>
<223>  Synthetic Construct

<400>  104

Gln Ala Val Val Thr Gln Glu Pro Ser Leu Thr Val Ser Pro Gly Gly
1               5                   10                  15

Thr Val Thr Leu Thr Cys Gly Ser Ser Thr Gly Ala Val Thr Thr Ser
            20                  25                  30

Asn Tyr Ala Asn Trp Val Gln Glu Lys Pro Gly Gln Ala Phe Arg Gly
        35                  40                  45

Leu Ile Gly Gly Thr Asn Lys Arg Ala Pro Gly Thr Pro Ala Arg Phe
    50                  55                  60

Ser Gly Ser Leu Leu Gly Gly Lys Ala Ala Leu Thr Leu Ser Gly Ala
65                  70                  75                  80

Gln Pro Glu Asp Glu Ala Glu Tyr Tyr Cys Ala Leu Trp Tyr Ser Asn
                85                  90                  95

Leu Trp Val Phe Gly Gly Gly Thr Lys Leu Thr Val Leu
            100                 105

<210>  105
<211>  207
<212>  PRT
<213>  Homo sapiens

<400>  105

Met Gln Ser Gly Thr His Trp Arg Val Leu Gly Leu Cys Leu Leu Ser
1               5                   10                  15

Val Gly Val Trp Gly Gln Asp Gly Asn Glu Glu Met Gly Gly Ile Thr
            20                  25                  30

Gln Thr Pro Tyr Lys Val Ser Ile Ser Gly Thr Thr Val Ile Leu Thr
        35                  40                  45

Cys Pro Gln Tyr Pro Gly Ser Glu Ile Leu Trp Gln His Asn Asp Lys

50                  55                  60

Asn Ile Gly Gly Asp Glu Asp Asp Lys Asn Ile Gly Ser Asp Glu Asp
65                  70                  75                  80

His Leu Ser Leu Lys Glu Phe Ser Glu Leu Glu Gln Ser Gly Tyr Tyr
                85                  90                  95

Val Cys Tyr Pro Arg Gly Ser Lys Pro Glu Asp Ala Asn Phe Tyr Leu
            100                 105                 110

Tyr Leu Arg Ala Arg Val Cys Glu Asn Cys Met Glu Met Asp Val Met
        115                 120                 125

Ser Val Ala Thr Ile Val Ile Val Asp Ile Cys Ile Thr Gly Gly Leu
    130                 135                 140

Leu Leu Leu Val Tyr Tyr Trp Ser Lys Asn Arg Lys Ala Lys Ala Lys
145                 150                 155                 160

Pro Val Thr Arg Gly Ala Gly Ala Gly Gly Arg Gln Arg Gly Gln Asn
                165                 170                 175

Lys Glu Arg Pro Pro Pro Val Pro Asn Pro Asp Tyr Glu Pro Ile Arg
            180                 185                 190

Lys Gly Gln Arg Asp Leu Tyr Ser Gly Leu Asn Gln Arg Arg Ile
        195                 200                 205

<210>  106
<211>  198
<212>  PRT
<213>  Homo sapiens

<400>  106

Met Gln Ser Gly Thr Arg Trp Arg Val Leu Gly Leu Cys Leu Leu Ser
1               5                   10                  15

Ile Gly Val Trp Gly Gln Asp Gly Asn Glu Glu Met Gly Ser Ile Thr
            20                  25                  30

Gln Thr Pro Tyr Gln Val Ser Ile Ser Gly Thr Thr Val Ile Leu Thr
        35                  40                  45

Cys Ser Gln His Leu Gly Ser Glu Ala Gln Trp Gln His Asn Gly Lys
    50                  55                  60

Asn Lys Glu Asp Ser Gly Asp Arg Leu Phe Leu Pro Glu Phe Ser Glu

65                  70                  75                  80

Met Glu Gln Ser Gly Tyr Tyr Val Cys Tyr Pro Arg Gly Ser Asn Pro
                85                  90                  95

Glu Asp Ala Ser His His Leu Tyr Leu Lys Ala Arg Val Cys Glu Asn
            100                 105                 110

Cys Met Glu Met Asp Val Met Ala Val Ala Thr Ile Val Ile Val Asp
        115                 120                 125

Ile Cys Ile Thr Leu Gly Leu Leu Leu Leu Val Tyr Tyr Trp Ser Lys
    130                 135                 140

Asn Arg Lys Ala Lys Ala Lys Pro Val Thr Arg Gly Ala Gly Ala Gly
145                 150                 155                 160

Gly Arg Gln Arg Gly Gln Asn Lys Glu Arg Pro Pro Pro Val Pro Asn
                165                 170                 175

Pro Asp Tyr Glu Pro Ile Arg Lys Gly Gln Gln Asp Leu Tyr Ser Gly
            180                 185                 190

Leu Asn Gln Arg Arg Ile
        195

<210>  107
<211>  10
<212>  PRT
<213>  Artificial Sequence

<220>
<223>  Synthetic Construct

<400>  107

Gly Phe Thr Phe Ser Asp Ser Trp Ile His
1               5                   10

<210>  108
<211>  18
<212>  PRT
<213>  Artificial Sequence

<220>
<223>  Synthetic Construct

<400>  108

Ala Trp Ile Ser Pro Tyr Gly Gly Ser Thr Tyr Tyr Ala Asp Ser Val
1               5                   10                  15

Lys Gly

<210> 109
<211> 9
<212> PRT
<213> Artificial Sequence

<220>
<223> Synthetic Construct

<400> 109

Arg His Trp Pro Gly Gly Phe Asp Tyr
1               5

<210> 110
<211> 11
<212> PRT
<213> Artificial Sequence

<220>
<223> Artificial Contruct

<400> 110

Arg Ala Ser Gln Asp Val Ser Thr Ala Val Ala
1               5                   10

<210> 111
<211> 7
<212> PRT
<213> Artificial Sequence

<220>
<223> Synthetic Construct

<400> 111

Ser Ala Ser Phe Leu Tyr Ser
1               5

<210> 112
<211> 9
<212> PRT
<213> Artificial Sequence

<220>
<223> Synthetic Construct

<400> 112

Gln Gln Tyr Leu Tyr His Pro Ala Thr
1               5

<210> 113
<211> 118
<212> PRT

<213>  Artificial Sequence

<220>
<223>  Synthetic Construct

<400>  113


<210>  114
<211>  122
<212>  PRT
<213>  Artificial Sequence

<220>
<223>  Synthetic Construct

<400>  114

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Asp Ser
            20                  25                  30

Trp Ile His Trp Val Arg Gln Ala Pro Gly Lys Gly Leu Glu Trp Val
        35                  40                  45

Ala Trp Ile Ser Pro Tyr Gly Gly Ser Thr Tyr Tyr Ala Asp Ser Val
    50                  55                  60

Lys Gly Arg Phe Thr Ile Ser Ala Asp Thr Ser Lys Asn Thr Ala Tyr
65                  70                  75                  80

Leu Gln Met Asn Ser Leu Arg Ala Glu Asp Thr Ala Val Tyr Tyr Cys
                85                  90                  95

Ala Arg Arg His Trp Pro Gly Gly Phe Asp Tyr Trp Gly Gln Gly Thr
            100                 105                 110

Leu Val Thr Val Ser Ser Ala Ser Thr Lys
        115                 120

<210>  115
<211>  108
<212>  PRT
<213>  Artificial Sequence

<220>
<223>  Synthetic Construct

<400>  115

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Val Ser Thr Ala
            20                  25                  30

Val Ala Trp Tyr Gln Gln Lys Pro Gly Lys Ala Pro Lys Leu Leu Ile
        35                  40                  45

Tyr Ser Ala Ser Phe Leu Tyr Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Leu Tyr His Pro Ala
                85                  90                  95

Thr Phe Gly Gln Gly Thr Lys Val Glu Ile Lys Arg
            100                 105

<210>  116
<211>  673
<212>  PRT
<213>  Artificial Sequence

<220>
<223>  Synthetic Construct

<400>  116

Gln Val Gln Leu Val Gln Ser Gly Ala Glu Val Lys Lys Pro Gly Ser
1               5                   10                  15

Ser Val Lys Val Ser Cys Lys Ala Ser Gly Tyr Ala Phe Ser Tyr Ser
            20                  25                  30

Trp Ile Asn Trp Val Arg Gln Ala Pro Gly Gln Gly Leu Glu Trp Met
        35                  40                  45

Gly Arg Ile Phe Pro Gly Asp Gly Asp Thr Asp Tyr Asn Gly Lys Phe
    50                  55                  60

Lys Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Asn Val Phe Asp Gly Tyr Trp Leu Val Tyr Trp Gly Gln Gly
            100                 105                 110

Thr Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Glu Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Glu Lys Val Glu Pro Lys Ser Cys Asp Gly
    210                 215                 220

Gly Gly Gly Ser Gly Gly Gly Gly Ser Gln Ala Val Val Thr Gln Glu
225                 230                 235                 240

Pro Ser Leu Thr Val Ser Pro Gly Gly Thr Val Thr Leu Thr Cys Gly
                245                 250                 255

Ser Ser Thr Gly Ala Val Thr Thr Ser Asn Tyr Ala Asn Trp Val Gln
            260                 265                 270

Glu Lys Pro Gly Gln Ala Phe Arg Gly Leu Ile Gly Gly Thr Asn Lys
        275                 280                 285

Arg Ala Pro Gly Thr Pro Ala Arg Phe Ser Gly Ser Leu Leu Gly Gly
    290                 295                 300

Lys Ala Ala Leu Thr Leu Ser Gly Ala Gln Pro Glu Asp Glu Ala Glu
305                 310                 315                 320

Tyr Tyr Cys Ala Leu Trp Tyr Ser Asn Leu Trp Val Phe Gly Gly Gly
                325                 330                 335

Thr Lys Leu Thr Val Leu Ser Ser Ala Ser Thr Lys Gly Pro Ser Val
            340                 345                 350

Phe Pro Leu Ala Pro Ser Ser Lys Ser Thr Ser Gly Gly Thr Ala Ala
        355                 360                 365

Leu Gly Cys Leu Val Lys Asp Tyr Phe Pro Glu Pro Val Thr Val Ser
    370                 375                 380

Trp Asn Ser Gly Ala Leu Thr Ser Gly Val His Thr Phe Pro Ala Val
385                 390                 395                 400

Leu Gln Ser Ser Gly Leu Tyr Ser Leu Ser Ser Val Val Thr Val Pro
                405                 410                 415

Ser Ser Ser Leu Gly Thr Gln Thr Tyr Ile Cys Asn Val Asn His Lys
            420                 425                 430

Pro Ser Asn Thr Lys Val Asp Lys Lys Val Glu Pro Lys Ser Cys Asp
        435                 440                 445

Lys Thr His Thr Cys Pro Pro Cys Pro Ala Pro Glu Ala Ala Gly Gly
    450                 455                 460

Pro Ser Val Phe Leu Phe Pro Pro Lys Pro Lys Asp Thr Leu Met Ile
465                 470                 475                 480

Ser Arg Thr Pro Glu Val Thr Cys Val Val Val Asp Val Ser His Glu
                485                 490                 495

Asp Pro Glu Val Lys Phe Asn Trp Tyr Val Asp Gly Val Glu Val His
            500                 505                 510

Asn Ala Lys Thr Lys Pro Arg Glu Glu Gln Tyr Asn Ser Thr Tyr Arg
        515                 520                 525

Val Val Ser Val Leu Thr Val Leu His Gln Asp Trp Leu Asn Gly Lys
    530                 535                 540

Glu Tyr Lys Cys Lys Val Ser Asn Lys Ala Leu Gly Ala Pro Ile Glu
545                 550                 555                 560

Lys Thr Ile Ser Lys Ala Lys Gly Gln Pro Arg Glu Pro Gln Val Tyr
                565                 570                 575

Thr Leu Pro Pro Cys Arg Asp Glu Leu Thr Lys Asn Gln Val Ser Leu
            580                 585                 590

Trp Cys Leu Val Lys Gly Phe Tyr Pro Ser Asp Ile Ala Val Glu Trp
        595                 600                 605

Glu Ser Asn Gly Gln Pro Glu Asn Asn Tyr Lys Thr Thr Pro Pro Val
    610                 615                 620

Leu Asp Ser Asp Gly Ser Phe Phe Leu Tyr Ser Lys Leu Thr Val Asp
625                 630                 635                 640

Lys Ser Arg Trp Gln Gln Gly Asn Val Phe Ser Cys Ser Val Met His
                645                 650                 655

Glu Ala Leu His Asn His Tyr Thr Gln Lys Ser Leu Ser Leu Ser Pro
            660                 665                 670

Gly

<210>  117
<211>  448
<212>  PRT
<213>  Artificial Sequence

<220>
<223>  Synthetic Construct

<400>  117

Gln Val Gln Leu Val Gln Ser Gly Ala Glu Val Lys Lys Pro Gly Ser
1               5                   10                  15

Ser Val Lys Val Ser Cys Lys Ala Ser Gly Tyr Ala Phe Ser Tyr Ser
        20                  25                  30

Trp Ile Asn Trp Val Arg Gln Ala Pro Gly Gln Gly Leu Glu Trp Met
    35                  40                  45

Gly Arg Ile Phe Pro Gly Asp Gly Asp Thr Asp Tyr Asn Gly Lys Phe
    50                  55                  60

Lys Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Asn Val Phe Asp Gly Tyr Trp Leu Val Tyr Trp Gly Gln Gly
            100                 105                 110

Thr Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Glu Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Glu Lys Val Glu Pro Lys Ser Cys Asp Lys
    210                 215                 220

Thr His Thr Cys Pro Pro Cys Pro Ala Pro Glu Ala Ala Gly Gly Pro
225                 230                 235                 240

Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser His Glu Asp
            260                 265                 270

Pro Glu Val Lys Phe Asn Trp Tyr Val Asp Gly Val Glu Val His Asn
        275                 280                 285

Ala Lys Thr Lys Pro Arg Glu Glu Gln Tyr Asn Ser Thr Tyr Arg Val
    290                 295                 300

Val Ser Val Leu Thr Val Leu His Gln Asp Trp Leu Asn Gly Lys Glu
305                 310                 315                 320

Tyr Lys Cys Lys Val Ser Asn Lys Ala Leu Gly Ala Pro Ile Glu Lys
                325                 330                 335

Thr Ile Ser Lys Ala Lys Gly Gln Pro Arg Glu Pro Gln Val Cys Thr
            340                 345                 350

Leu Pro Pro Ser Arg Asp Glu Leu Thr Lys Asn Gln Val Ser Leu Ser
        355                 360                 365

Cys Ala Val Lys Gly Phe Tyr Pro Ser Asp Ile Ala Val Glu Trp Glu
    370                 375                 380

Ser Asn Gly Gln Pro Glu Asn Asn Tyr Lys Thr Thr Pro Pro Val Leu
385                 390                 395                 400

Asp Ser Asp Gly Ser Phe Phe Leu Val Ser Lys Leu Thr Val Asp Lys
                405                 410                 415

Ser Arg Trp Gln Gln Gly Asn Val Phe Ser Cys Ser Val Met His Glu
            420                 425                 430

Ala Leu His Asn His Tyr Thr Gln Lys Ser Leu Ser Leu Ser Pro Gly
        435                 440                 445

<210>  118
<211>  219
<212>  PRT
<213>  Artificial Sequence

<220>
<223>  Synthetic Construct

<400>  118

Asp Ile Val Met Thr Gln Thr Pro Leu Ser Leu Pro Val Thr Pro Gly
1               5                   10                  15

Glu Pro Ala Ser Ile Ser Cys Arg Ser Ser Lys Ser Leu Leu His Ser
            20                  25                  30

Asn Gly Ile Thr Tyr Leu Tyr Trp Tyr Leu Gln Lys Pro Gly Gln Ser
        35                  40                  45

Pro Gln Leu Leu Ile Tyr Gln Met Ser Asn Leu Val Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Ala Gln Asn
                85                  90                  95

Leu Glu Leu Pro Tyr Thr Phe Gly Gly Gly Thr Lys Val Glu Ile Lys
            100                 105                 110

Arg Thr Val Ala Ala Pro Ser Val Phe Ile Phe Pro Pro Ser Asp Arg
        115                 120                 125

Lys Leu Lys Ser Gly Thr Ala Ser Val Val Cys Leu Leu Asn Asn Phe
    130                 135                 140

Tyr Pro Arg Glu Ala Lys Val Gln Trp Lys Val Asp Asn Ala Leu Gln
145                 150                 155                 160

Ser Gly Asn Ser Gln Glu Ser Val Thr Glu Gln Asp Ser Lys Asp Ser
                165                 170                 175

Thr Tyr Ser Leu Ser Ser Thr Leu Thr Leu Ser Lys Ala Asp Tyr Glu
            180                 185                 190

Lys His Lys Val Tyr Ala Cys Glu Val Thr His Gln Gly Leu Ser Ser
        195                 200                 205

Pro Val Thr Lys Ser Phe Asn Arg Gly Glu Cys
    210                 215

<210>  119
<211>  232
<212>  PRT
<213>  Artificial Sequence

<220>
<223>  Synthetic Construct

<400>  119

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Thr Tyr

20                  25                  30

Ala Met Asn Trp Val Arg Gln Ala Pro Gly Lys Gly Leu Glu Trp Val
        35                  40                  45

Ser Arg Ile Arg Ser Lys Tyr Asn Asn Tyr Ala Thr Tyr Tyr Ala Asp
    50                  55                  60

Ser Val Lys Gly Arg Phe Thr Ile Ser Arg Asp Asp Ser Lys Asn Thr
65                  70                  75                  80

Leu Tyr Leu Gln Met Asn Ser Leu Arg Ala Glu Asp Thr Ala Val Tyr
                85                  90                  95

Tyr Cys Val Arg His Gly Asn Phe Gly Asn Ser Tyr Val Ser Trp Phe
            100                 105                 110

Ala Tyr Trp Gly Gln Gly Thr Leu Val Thr Val Ser Ser Ala Ser Val
        115                 120                 125

Ala Ala Pro Ser Val Phe Ile Phe Pro Pro Ser Asp Glu Gln Leu Lys
    130                 135                 140

Ser Gly Thr Ala Ser Val Val Cys Leu Leu Asn Asn Phe Tyr Pro Arg
145                 150                 155                 160

Glu Ala Lys Val Gln Trp Lys Val Asp Asn Ala Leu Gln Ser Gly Asn
                165                 170                 175

Ser Gln Glu Ser Val Thr Glu Gln Asp Ser Lys Asp Ser Thr Tyr Ser
            180                 185                 190

Leu Ser Ser Thr Leu Thr Leu Ser Lys Ala Asp Tyr Glu Lys His Lys
        195                 200                 205

Val Tyr Ala Cys Glu Val Thr His Gln Gly Leu Ser Ser Pro Val Thr
    210                 215                 220

Lys Ser Phe Asn Arg Gly Glu Cys
225                 230

<210>  120
<211>  442
<212>  PRT
<213>  Artificial Sequence

<220>
<223>  Synthetic Construct

<400>  120

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Asp Ser
            20                  25                  30

Trp Ile His Trp Val Arg Gln Ala Pro Gly Lys Gly Leu Glu Trp Val
        35                  40                  45

Ala Trp Ile Ser Pro Tyr Gly Gly Ser Thr Tyr Tyr Ala Asp Ser Val
    50                  55                  60

Lys Gly Arg Phe Thr Ile Ser Ala Asp Thr Ser Lys Asn Thr Ala Tyr
65                  70                  75                  80

Leu Gln Met Asn Ser Leu Arg Ala Glu Asp Thr Ala Val Tyr Tyr Cys
                85                  90                  95

Ala Arg Arg His Trp Pro Gly Gly Phe Asp Tyr Trp Gly Gln Gly Thr
            100                 105                 110

Leu Val Thr Val Ser Ala Ala Lys Thr Thr Pro Pro Ser Val Tyr Pro
        115                 120                 125

Leu Ala Pro Gly Ser Ala Ala Gln Thr Asn Ser Met Val Thr Leu Gly
    130                 135                 140

Cys Leu Val Lys Gly Tyr Phe Pro Glu Pro Val Thr Val Thr Trp Asn
145                 150                 155                 160

Ser Gly Ser Leu Ser Ser Gly Val His Thr Phe Pro Ala Val Leu Gln
                165                 170                 175

Ser Asp Leu Tyr Thr Leu Ser Ser Ser Val Thr Val Pro Ser Ser Thr
            180                 185                 190

Trp Pro Ser Glu Thr Val Thr Cys Asn Val Ala His Pro Ala Ser Ser
        195                 200                 205

Thr Lys Val Asp Lys Lys Ile Val Pro Arg Asp Cys Gly Cys Lys Pro
    210                 215                 220

Cys Ile Cys Thr Val Pro Glu Val Ser Ser Val Phe Ile Phe Pro Pro
225                 230                 235                 240

Lys Pro Lys Asp Val Leu Thr Ile Thr Leu Thr Pro Lys Val Thr Cys

245                 250                 255

Val Val Val Asp Ile Ser Lys Asp Ala Pro Glu Val Gln Phe Ser Trp
            260                 265                 270

Phe Val Asp Asp Val Glu Val His Thr Ala Gln Thr Gln Pro Arg Glu
        275                 280                 285

Glu Gln Phe Asn Ser Thr Phe Arg Ser Val Ser Glu Leu Pro Ile Met
    290                 295                 300

His Gln Asp Trp Leu Asn Gly Lys Glu Phe Lys Cys Arg Val Asn Ser
305                 310                 315                 320

Ala Ala Phe Gly Ala Pro Ile Glu Lys Thr Ile Ser Lys Thr Lys Gly
                325                 330                 335

Arg Pro Lys Ala Pro Gln Val Tyr Thr Ile Pro Pro Pro Lys Glu Gln
            340                 345                 350

Met Ala Lys Asp Lys Val Ser Leu Thr Cys Met Ile Thr Asp Phe Phe
        355                 360                 365

Pro Glu Asp Ile Thr Val Glu Trp Gln Trp Asn Gly Gln Pro Ala Glu
    370                 375                 380

Asn Tyr Lys Asn Thr Gln Pro Ile Met Asp Thr Asp Gly Ser Tyr Phe
385                 390                 395                 400

Val Tyr Ser Lys Leu Asn Val Gln Lys Ser Asn Trp Glu Ala Gly Asn
                405                 410                 415

Thr Phe Thr Cys Ser Val Leu His Glu Gly Leu His Asn His His Thr
            420                 425                 430

Glu Lys Ser Leu Ser His Ser Pro Gly Lys
        435                 440

<210>  121
<211>  214
<212>  PRT
<213>  Artificial Sequence

<220>
<223>  Synthetic Construct

<400>  121

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Val Ser Thr Ala
            20                  25                  30

Val Ala Trp Tyr Gln Gln Lys Pro Gly Lys Ala Pro Lys Leu Leu Ile
        35                  40                  45

Tyr Ser Ala Ser Phe Leu Tyr Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Leu Tyr His Pro Ala
                85                  90                  95

Thr Phe Gly Gln Gly Thr Lys Val Glu Ile Lys Arg Ala Asp Ala Ala
            100                 105                 110

Pro Thr Val Ser Ile Phe Pro Pro Ser Ser Glu Gln Leu Thr Ser Gly
        115                 120                 125

Gly Ala Ser Val Val Cys Phe Leu Asn Asn Phe Tyr Pro Lys Asp Ile
    130                 135                 140

Asn Val Lys Trp Lys Ile Asp Gly Ser Glu Arg Gln Asn Gly Val Leu
145                 150                 155                 160

Asn Ser Trp Thr Asp Gln Asp Ser Lys Asp Ser Thr Tyr Ser Met Ser
                165                 170                 175

Ser Thr Leu Thr Leu Thr Lys Asp Glu Tyr Glu Arg His Asn Ser Tyr
            180                 185                 190

Thr Cys Glu Ala Thr His Lys Thr Ser Thr Ser Pro Ile Val Lys Ser
        195                 200                 205

Phe Asn Arg Asn Glu Cys
    210